(19) Europäisches Patentamt
European Patent Office
Office européen des brevets

(11) EP 3 974 433 B1

# (12) EUROPEAN PATENT SPECIFICATION

(45) Date of publication and mention of the grant of the patent:
**21.02.2024 Bulletin 2024/08**

(21) Application number: **20809291.6**

(22) Date of filing: **19.05.2020**

(51) International Patent Classification (IPC):
***C07D 491/048*** (2006.01) ***C07D 495/04*** (2006.01)
***C09K 11/06*** (2006.01) ***H10K 85/60*** (2023.01)
***H10K 101/40*** (2023.01) ***H10K 101/20*** (2023.01)

(52) Cooperative Patent Classification (CPC):
**C09K 11/06; C07D 491/048; C07D 495/04; H10K 85/657; H10K 85/6572;** C09K 2211/1007; C09K 2211/1029; C09K 2211/1033; C09K 2211/1037; C09K 2211/1044; C09K 2211/1048; C09K 2211/1051; C09K 2211/1059; H10K 50/11; H10K 2101/20;

(Cont.)

(86) International application number:
**PCT/JP2020/019779**

(87) International publication number:
**WO 2020/235558 (26.11.2020 Gazette 2020/48)**

## (54) ORGANIC ELECTROLUMINESCENT ELEMENT, COMPOUND, AND ELECTRONIC APPLIANCE

ORGANISCHES ELEKTROLUMINESZENTES ELEMENT, VERBINDUNG UND ELEKTRONISCHES GERÄT

ÉLÉMENT ÉLECTROLUMINESCENT ORGANIQUE, COMPOSÉ ET APPAREIL ÉLECTRONIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.05.2019 JP 2019094653**

(43) Date of publication of application:
**30.03.2022 Bulletin 2022/13**

(73) Proprietor: **Idemitsu Kosan Co.,Ltd.**
**Tokyo 100-8321 (JP)**

(72) Inventors:
- **SHIOMI Takushi**
  **Sodegaura-shi, Chiba 299-0293 (JP)**
- **NAKANO Hiromi**
  **Sodegaura-shi, Chiba 299-0293 (JP)**

(74) Representative: **Gille Hrabal Partnerschaftsgesellschaft mbB Patentanwälte**
**Brucknerstraße 20**
**40593 Düsseldorf (DE)**

(56) References cited:
**EP-A1- 3 015 457**
**WO-A1-2011/049063**
**WO-A1-2011/136755**
**WO-A1-2015/022987**
**WO-A1-2017/118155**
**WO-A1-2018/202840**
**CN-A- 107 325 035**
**CN-A- 108 484 576**
**CN-A- 108 586 453**
**JP-A- 2019 096 876**
**US-A1- 2012 235 133**
**US-A1- 2018 083 201**
**US-A1- 2018 130 955**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)
H10K 2101/40

## Description

TECHNICAL FIELD

The present invention relates to an organic electroluminescence device, a compound, and an electronic device.

BACKGROUND ART

When a voltage is applied to an organic electroluminescence device (hereinafter, occasionally referred to as "organic EL device"), holes are injected from an anode and electrons are injected from a cathode into an emitting layer. The injected electrons and holes are recombined in the emitting layer to form excitons. Specifically, according to the electron spin statistics theory, singlet excitons and triplet excitons are generated at a ratio of 25%:75%.

A fluorescent organic EL device using light emission from singlet excitons has been applied to a full-color display such as a mobile phone and a television set, but an internal quantum efficiency is said to be at a limit of 25%.Accordingly, studies has been made to improve a performance of the organic EL device.

For instance, it is expected to further efficiently emit the organic EL device using triplet excitons in addition to singlet excitons. In view of the above, a highly efficient fluorescent organic EL device using thermally activated delayed fluorescence (hereinafter, sometimes simply referred to as "delayed fluorescence") has been proposed and studied.

A TADF (Thermally Activated Delayed Fluorescence) mechanism uses such a phenomenon that inverse intersystem crossing from triplet excitons to singlet excitons thermally occurs when a material having a small energy difference ($\Delta ST$) between singlet energy level and triplet energy level is used. Thermally activated delayed fluorescence is explained in "Yuki Hando-tai no Debaisu Bussei (Device Physics of Organic Semiconductors)" (edited by ADACHI, Chihaya, published by Kodansha, issued on April 1, 2012, on pages 261-268).

As a compound exhibiting TADF properties (hereinafter also referred to as a TADF compound), for example, a compound in which a donor moiety and an acceptor moiety are bonded in a molecule is known.

Examples of literatures relating to an organic EL device include Patent Literatures 1, 2, 3, 4, 5, 6 and 7 and Non-Patent Literature 1.

CITATION LIST

PATENT LITERATURE(S)

Patent Literature 1: Chinese Patent Application Publication No. 107793398
Patent Literature 2: International Publication No. WO2014/166585
Patent Literature 3: Korean Patent Publication No. 2014-143618
Patent Literature 4: EP 3015457 A1
Patent Literature 5: WO2017/118155 A1
Patent Literature 6: CN 107 325 035 A
Patent Literature 7: WO2015/022987 A1

EP 3015457 A1 discloses an organic electroluminescence device comprising an anode, namely an indium tin oxide (ITO) anode, a cathode, namely an Al cathode, and an emitting layer provided between the anode and the cathode. In contrast to the present invention, however, the emitting layer as disclosed in EP 3015457 A1 does not comprise a delayed fluorescent compound.

WO2017/118155 A1 discloses an OLED with an ITO anode and an Al cathode comprising an organic compound designed to solve the problem that the existing electroluminescent luminescent materials have high cost, short life and low quantum yield of TADF organic luminescent materials. The compounds of the emitting layer of the device as disclosed in WO2017/118155 A1, however, do not display the delayed fluorescence properties as required by the specific compounds of the emitting layer according to the present invention.

CN 107 325 035 A relates to the technical field of display and in particular relates to a biphenyl compound, an organic electroluminescence device and a display device.

Therein, CN 107 325 035 A discloses compounds serving as an organic functional layer of the organic electroluminescence device, so that the luminous efficiency of the organic electroluminescence device is improved, and the drive voltage of the organic electroluminescence device is reduced. The biphenyl compounds, however, are structurally different from the specific compounds of the emitting layer of the present invention.

WO2015/022987 A1 discloses organic electroluminescence devices containing a fluorescent compound and a host

compound in the emitting layer. The host compounds of the emitting layer are structurally different from the specific host compounds of the emitting layer of the present invention.

NON-PATENT LITERATURE(S)

Non-Patent Literature 1: Nature Communications, 2014, 5, 4016

SUMMARY OF THE INVENTION

PROBLEMS TO BE SOLVED BY THE INVENTION

In order to improve performance of an electronic device such as a display, an organic EL device has been required to be highly improved in performance.

An object of the invention is to provide a compound and an organic electroluminescence device capable of achieving higher performance, particularly an improved luminous efficiency.

MEANS FOR SOLVING THE PROBLEM(S)

According to an aspect of the invention, an organic electroluminescence device includes: an anode; a cathode; an emitting layer provided between the anode and the cathode, in which the emitting layer contains a delayed fluorescent compound M2, and a compound M3 represented by a formula (3) below, and a singlet energy $S_1(M2)$ of the compound M2 and a singlet energy $S_1(M3)$ of the compound M3 satisfy a relationship of a numerical formula (Numerical Formula 1) below,

$$S_1(M3) > S_1(M2) \ldots \text{(Numerical Formula 1)}.$$

[Formula 1]

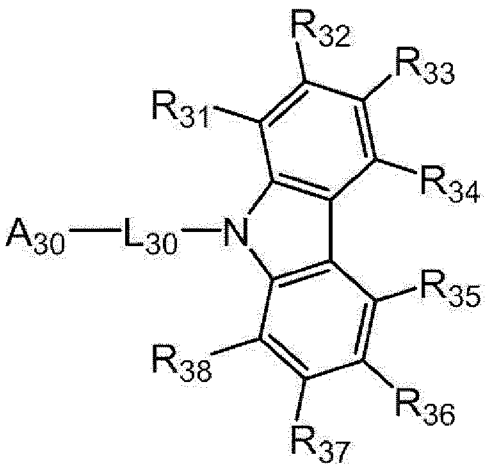

(3)

In the formula (3): $A_{30}$ is a group represented by a formula (3a), (3b), (3c), (3d), (3e) or (3f) below.

[Formula 2]

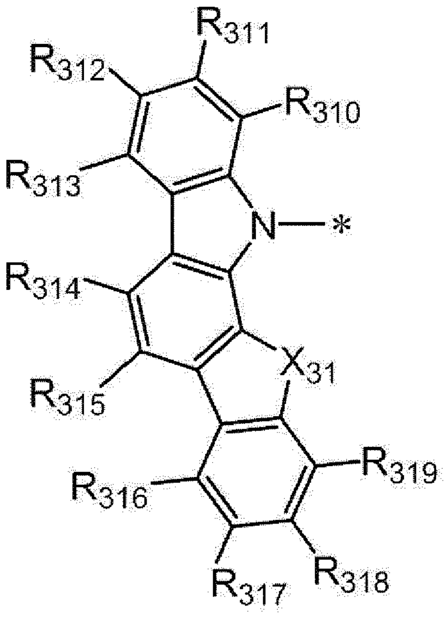

(3a)

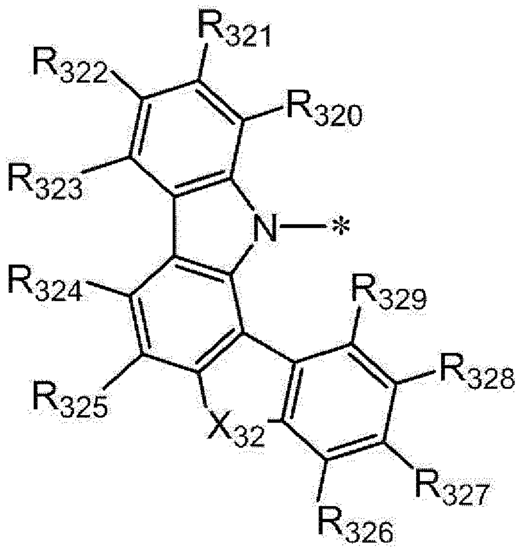

(3b)

[Formula 3]

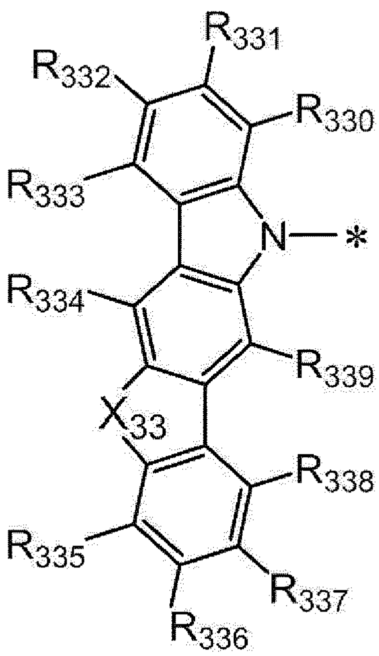

(3c)

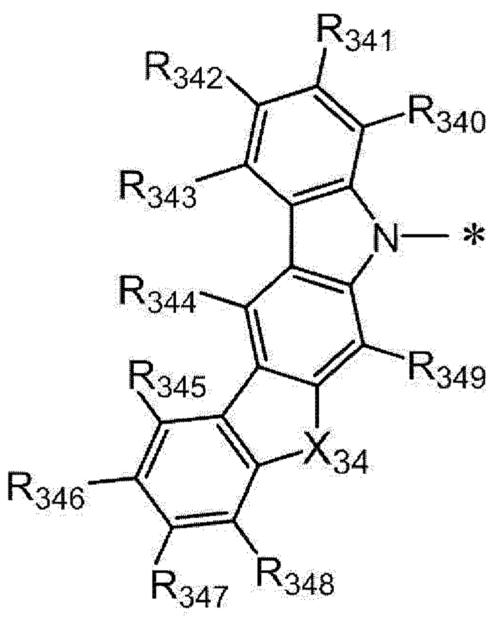

(3d)

[Formula 4]

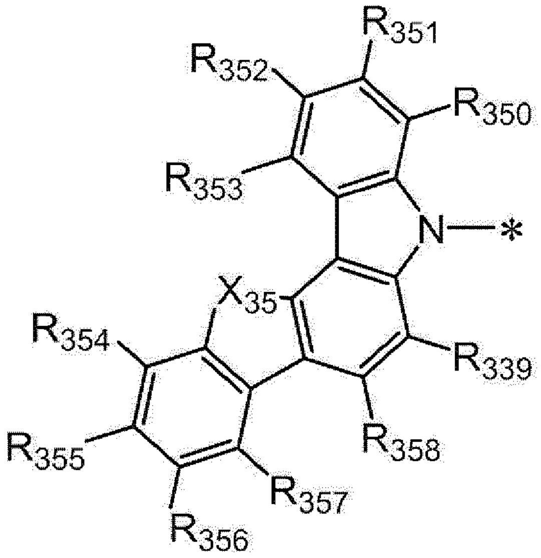

(3e)

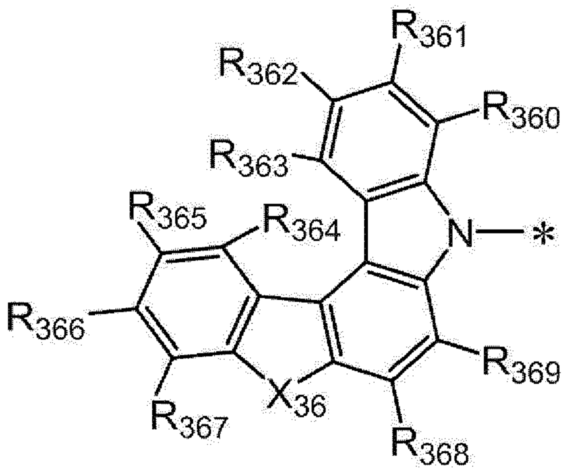

(3f)

In the formulae (3a), (3b), (3c), (3d), (3e) and (3f):

$X_{31}$, $X_{32}$, $X_{33}$, $X_{34}$, $X_{35}$ and $X_{36}$ are each independently an oxygen atom, a sulfur atom or $SiR_{372}R_{373}$;
$R_{372}$ and $R_{373}$ are each independently a hydrogen atom or a substituent;
$R_{310}$ to $R_{319}$ are each independently a hydrogen atom or a substituent, or at least one pair of a pair of $R_{310}$ and $R_{311}$, a pair of $R_{311}$ and $R_{312}$, a pair of $R_{312}$ and $R_{313}$, a pair of $R_{314}$ and $R_{315}$, a pair of $R_{316}$ and $R_{317}$, a pair of $R_{317}$ and $R_{318}$, or a pair of $R_{318}$ and $R_{319}$ are mutually bonded to form a ring;
$R_{320}$ to $R_{329}$ are each independently a hydrogen atom or a substituent, or at least one pair of a pair of $R_{320}$ and $R_{321}$, a pair of $R_{321}$ and $R_{322}$, a pair of $R_{322}$ and $R_{323}$, a pair of $R_{324}$ and $R_{325}$, a pair of $R_{326}$ and $R_{327}$, a pair of $R_{327}$ and $R_{328}$, or a pair of $R_{328}$ and $R_{329}$ are mutually bonded to form a ring;
$R_{330}$ to $R_{339}$ are each independently a hydrogen atom or a substituent, or at least one pair of a pair of $R_{330}$ and $R_{331}$, a pair of $R_{331}$ and $R_{332}$, a pair of $R_{332}$ and $R_{333}$, a pair of $R_{335}$ and $R_{336}$, a pair of $R_{336}$ and $R_{337}$, or a pair of $R_{337}$ and $R_{338}$ are mutually bonded to form a ring;
$R_{340}$ to $R_{349}$ are each independently a hydrogen atom or a substituent, or at least one pair of a pair of $R_{340}$ and $R_{341}$, a pair of $R_{341}$ and $R_{342}$, a pair of $R_{342}$ and $R_{343}$, a pair of $R_{345}$ and $R_{346}$, a pair of $R_{346}$ and $R_{347}$, or a pair of $R_{347}$ and $R_{348}$ are mutually bonded to form a ring;
$R_{350}$ to $R_{359}$ are each independently a hydrogen atom or a substituent, or at least one pair of a pair of $R_{350}$ and $R_{351}$, a pair of $R_{351}$ and $R_{352}$, a pair of $R_{352}$ and $R_{353}$, a pair of $R_{354}$ and $R_{355}$, a pair of $R_{355}$ and $R_{356}$, a pair of $R_{356}$ and $R_{357}$, or a pair of $R_{358}$ and $R_{359}$ are mutually bonded to form a ring;
$R_{360}$ to $R_{369}$ are each independently a hydrogen atom or a substituent, or at least one pair of a pair of $R_{360}$ and $R_{361}$, a pair of $R_{361}$ and $R_{362}$, a pair of $R_{362}$ and $R_{363}$, a pair of $R_{364}$ and $R_{365}$, a pair of $R_{365}$ and $R_{366}$, a pair of $R_{366}$ and $R_{367}$, or a pair of $R_{368}$ and $R_{369}$ are mutually bonded to form a ring;
$R_{372}$ and $R_{373}$ as a substituent and $R_{310}$ to $R_{319}$, $R_{320}$ to $R_{329}$, $R_{330}$ to $R_{339}$, $R_{340}$ to $R_{349}$, $R_{350}$ to $R_{359}$ and $R_{360}$ to $R_{369}$ as a substituent are each independently a halogen atom, a cyano group, a substituted or unsubstituted aryl group having 6 to 30 ring carbon atoms, a substituted or unsubstituted heterocyclic group having 5 to 30 ring atoms, a substituted or unsubstituted alkyl group having 1 to 30 carbon atoms, a substituted or unsubstituted alkyl halide group having 1 to 30 carbon atoms, a substituted or unsubstituted alkenyl group having 2 to 30 carbon atoms, a substituted or unsubstituted alkynyl group having 2 to 30 carbon atoms, a substituted or unsubstituted alkylsilyl group having 3 to 30 carbon atoms, a substituted or unsubstituted arylsilyl group having 6 to 60 ring carbon atoms, a substituted or unsubstituted arylphosphoryl group having 6 to 60 ring carbon atoms, a hydroxy group, a substituted or unsubstituted alkoxy group having 1 to 30 carbon atoms, a substituted or unsubstituted aryloxy group having 6 to 30 ring carbon atoms, an amino group, a substituted or unsubstituted alkylamino group having 2 to 30 carbon atoms, a substituted or unsubstituted arylamino group having 6 to 60 ring carbon atoms, a thiol group, a substituted or unsubstituted alkylthio group having 1 to 30 carbon atoms, or a substituted or unsubstituted arylthio group having 6 to 30 ring carbon atoms; and

* represents a bonding position to $L_{30}$.

In the formula (3):

$L_{30}$ is a substituted or unsubstituted arylene group having 6 to 22 ring carbon atoms, a group formed by bonding two substituted or unsubstituted arylene groups having 6 to 22 ring carbon atoms, or a group formed by bonding three substituted or unsubstituted arylene groups having 6 to 22 ring carbon atoms;

$R_{31}$ to $R_{38}$ are each independently a hydrogen atom or a substituent, or at least one pair of a pair of $R_{31}$ and $R_{32}$, a pair of $R_{32}$ and $R_{33}$, a pair of $R_{33}$ and $R_{34}$, a pair of $R_{35}$ and $R_{36}$, a pair of $R_{36}$ and $R_{37}$, or a pair of $R_{37}$ and $R_{38}$ are mutually bonded to form a ring; and

a substituent for substituting $L_{30}$, and $R_{31}$ to $R_{38}$ as a substituent are each independently a halogen atom, a cyano group, a substituted or unsubstituted aryl group having 6 to 30 ring carbon atoms, a substituted or unsubstituted heterocyclic group having 5 to 30 ring atoms, a substituted or unsubstituted alkyl group having 1 to 30 carbon atoms, a substituted or unsubstituted alkyl halide group having 1 to 30 carbon atoms, a substituted or unsubstituted alkenyl group having 2 to 30 carbon atoms, a substituted or unsubstituted alkynyl group having 2 to 30 carbon atoms, a substituted or unsubstituted alkylsilyl group having 3 to 30 carbon atoms, a substituted or unsubstituted arylsilyl group having 6 to 60 ring carbon atoms, a substituted or unsubstituted arylphosphoryl group having 6 to 60 ring carbon atoms, a hydroxy group, a substituted or unsubstituted alkoxy group having 1 to 30 carbon atoms, a substituted or unsubstituted aryloxy group having 6 to 30 ring carbon atoms, an amino group, a substituted or unsubstituted alkylamino group having 2 to 30 carbon atoms, a substituted or unsubstituted arylamino group having 6 to 60 ring carbon atoms, a thiol group, a substituted or unsubstituted alkylthio group having 1 to 30 carbon atoms, or a substituted or unsubstituted arylthio group having 6 to 30 ring carbon atoms.

According to another aspect of the invention, a compound represented by a formula (301) below is provided.

[Formula 5]

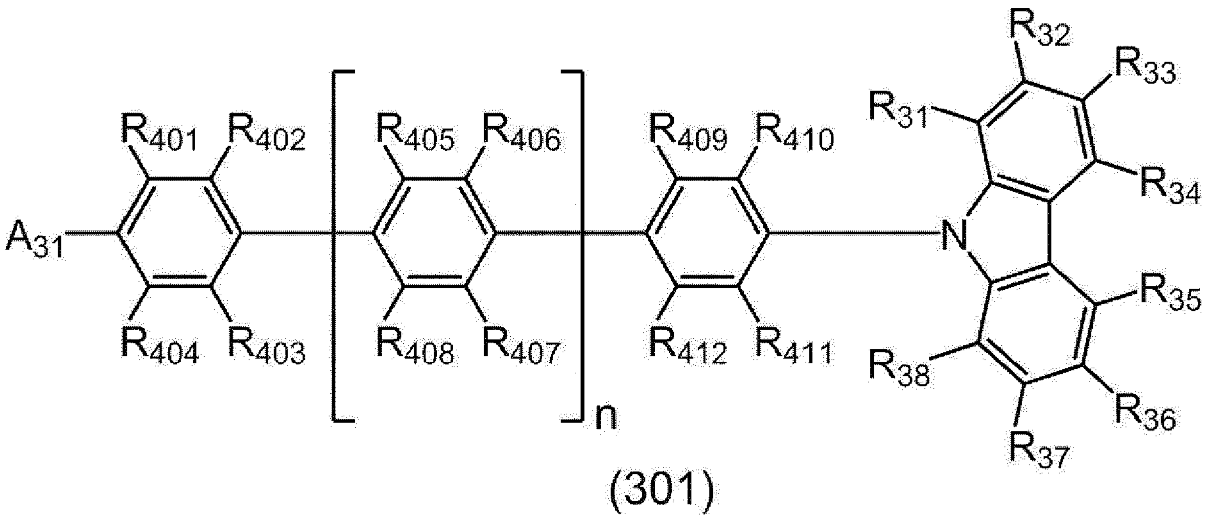

(301)

In the formula (301): $A_{31}$ is a group represented by a formula (31a), (31b), (31c), (31d), (31e) or (31f) below.

[Formula 6]

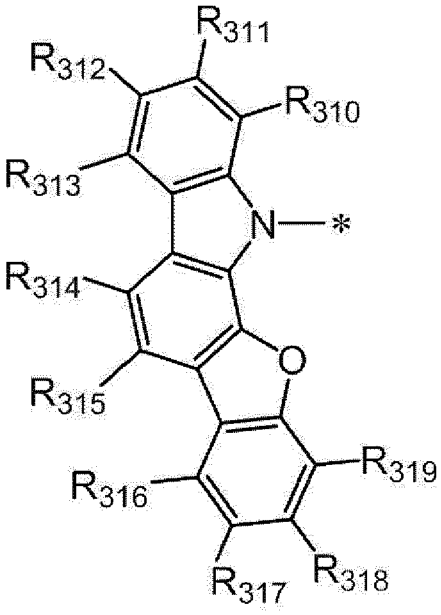

(31a)

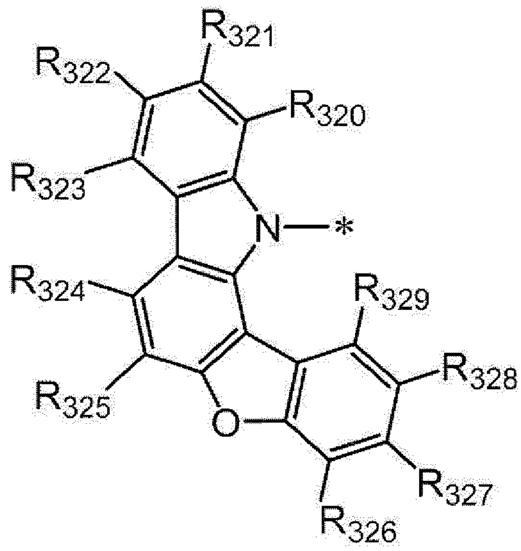

(31b)

[Formula 7]

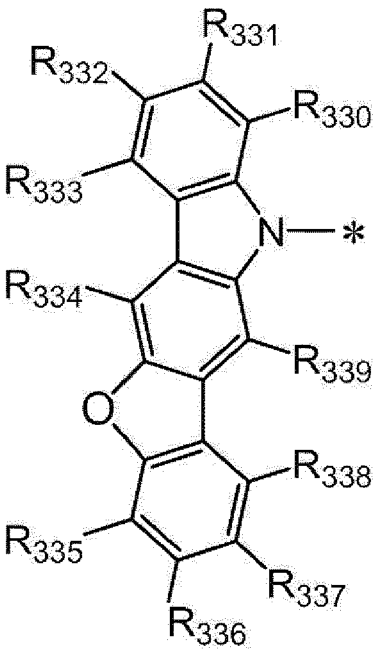

(31c)

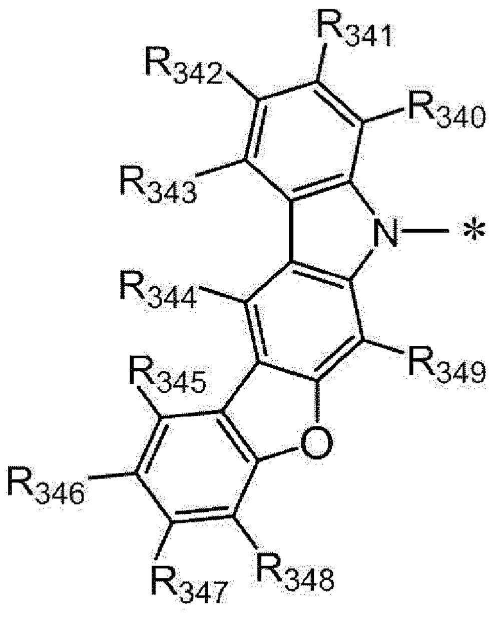

(31d)

[Formula 8]

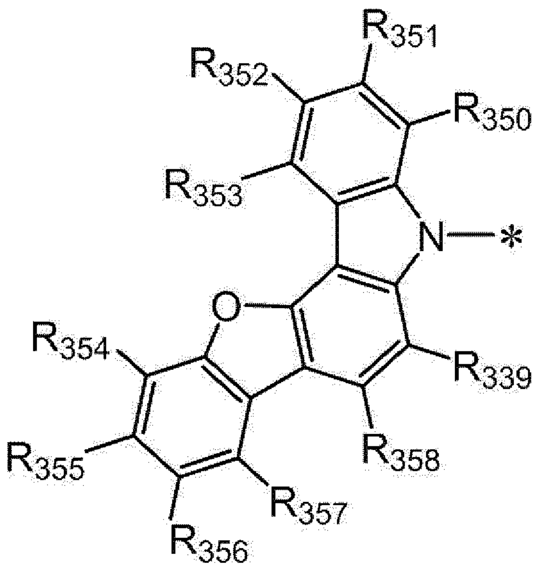

(31e)

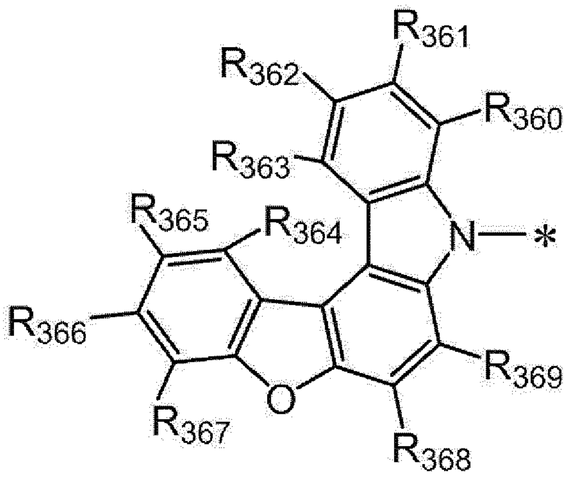

(31f)

In the formulae (31a), (31b), (31c), (31d), (31e) and (31f):

$R_{310}$ to $R_{319}$ are each independently a hydrogen atom or a substituent;
$R_{320}$ to $R_{329}$ are each independently a hydrogen atom or a substituent;
$R_{330}$ to $R_{339}$ are each independently a hydrogen atom or a substituent;
$R_{340}$ to $R_{349}$ are each independently a hydrogen atom or a substituent;
$R_{350}$ to $R_{359}$ are each independently a hydrogen atom or a substituent;
$R_{360}$ to $R_{369}$ are each independently a hydrogen atom or a substituent;
$R_{310}$ to $R_{319}$, $R_{320}$ to $R_{329}$, $R_{330}$ to $R_{339}$, $R_{340}$ to $R_{349}$, $R_{350}$ to $R_{359}$ and $R_{360}$ to $R_{369}$ as a substituent are each independently a halogen atom, a substituted or unsubstituted aryl group having 6 to 30 ring carbon atoms, a substituted or unsubstituted heterocyclic group having 5 to 30 ring atoms, a substituted or unsubstituted alkyl group having 1 to 30 carbon atoms, a substituted or unsubstituted alkyl halide group having 1 to 30 carbon atoms, a substituted or unsubstituted alkenyl group having 2 to 30 carbon atoms, a substituted or unsubstituted alkynyl group having 2 to 30 carbon atoms, a substituted or unsubstituted alkylsilyl group having 3 to 30 carbon atoms, a substituted or unsubstituted arylsilyl group having 6 to 60 ring carbon atoms, a substituted or unsubstituted arylphosphoryl group having 6 to 60 ring carbon atoms, a hydroxy group, a substituted or unsubstituted alkoxy group having 1 to 30 carbon atoms, a substituted or unsubstituted aryloxy group having 6 to 30 ring carbon atoms, an amino group, a substituted or unsubstituted alkylamino group having 2 to 30 carbon atoms, a substituted or unsubstituted arylamino group having 6 to 60 ring carbon atoms, a thiol group, a substituted or unsubstituted alkylthio group having 1 to 30 carbon atoms, or a substituted or unsubstituted arylthio group having 6 to 30 ring carbon atoms; and
* represents a bonding position to a benzene ring bonded to $R_{401}$ to $R_{404}$.

In the formula (301):

$R_{31}$ to $R_{38}$ are each independently a hydrogen atom or a substituent;
$R_{401}$ to $R_{412}$ are each independently a hydrogen atom or a substituent;
$R_{31}$ to $R_{38}$ as a substituent are each independently a halogen atom, a substituted or unsubstituted aryl group having 6 to 30 ring carbon atoms, a substituted or unsubstituted heterocyclic group having 5 to 30 ring atoms, a substituted or unsubstituted alkyl group having 1 to 30 carbon atoms, a substituted or unsubstituted alkyl halide group having 1 to 30 carbon atoms, a substituted or unsubstituted alkenyl group having 2 to 30 carbon atoms, a substituted or unsubstituted alkynyl group having 2 to 30 carbon atoms, a substituted or unsubstituted alkylsilyl group having 3 to 30 carbon atoms, a substituted or unsubstituted arylsilyl group having 6 to 60 ring carbon atoms, a substituted or unsubstituted arylphosphoryl group having 6 to 60 ring carbon atoms, a hydroxy group, a substituted or unsubstituted alkoxy group having 1 to 30 carbon atoms, a substituted or unsubstituted aryloxy group having 6 to 30 ring carbon atoms, an amino group, a substituted or unsubstituted alkylamino group having 2 to 30 carbon atoms, a substituted or unsubstituted arylamino group having 6 to 60 ring carbon atoms, a thiol group, a substituted or unsubstituted

alkylthio group having 1 to 30 carbon atoms, or a substituted or unsubstituted arylthio group having 6 to 30 ring carbon atoms; and

$R_{401}$ to $R_{412}$ as a substituent are each independently a halogen atom, a substituted or unsubstituted aryl group having 6 to 30 ring carbon atoms, a substituted or unsubstituted heterocyclic group having 5 to 30 ring atoms, a substituted or unsubstituted alkyl group having 1 to 30 carbon atoms, a substituted or unsubstituted alkyl halide group having 1 to 30 carbon atoms, a substituted or unsubstituted alkenyl group having 2 to 30 carbon atoms, a substituted or unsubstituted alkynyl group having 2 to 30 carbon atoms, a substituted or unsubstituted alkylsilyl group having 3 to 30 carbon atoms, a substituted or unsubstituted arylsilyl group having 6 to 60 ring carbon atoms, a substituted or unsubstituted arylphosphoryl group having 6 to 60 ring carbon atoms, a hydroxy group, a substituted or unsubstituted alkoxy group having 1 to 30 carbon atoms, a substituted or unsubstituted aryloxy group having 6 to 30 ring carbon atoms, a thiol group, a substituted or unsubstituted alkylthio group having 1 to 30 carbon atoms, or a substituted or unsubstituted arylthio group having 6 to 30 ring carbon atoms;

n is 0, 1, 2 or 3;

when a plurality of $R_{405}$ are present, the plurality of $R_{405}$ are mutually the same or different;

when a plurality of $R_{406}$ are present, the plurality of $R_{406}$ are mutually the same or different;

when a plurality of $R_{407}$ are present, the plurality of $R_{407}$ are mutually the same or different; and

when a plurality of $R_{408}$ are present, the plurality of $R_{408}$ are mutually the same or different.

According to still another aspect of the invention, a compound represented by a formula (310) below is provided.

[Formula 9]

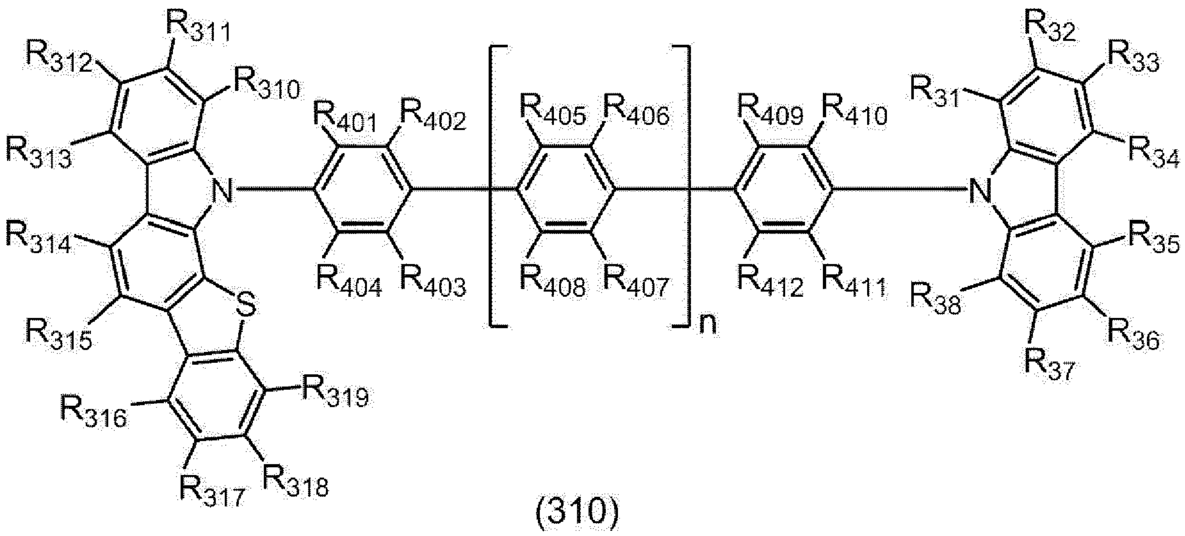

(310)

In the formula (310):

$R_{310}$ to $R_{319}$ are each independently a hydrogen atom or a substituent;

$R_{31}$ to $R_{38}$ are each independently a hydrogen atom or a substituent;

$R_{401}$ to $R_{412}$ are each independently a hydrogen atom or a substituent;

$R_{310}$ to $R_{319}$ as a substituent, $R_{31}$ to $R_{38}$ as a substituent and $R_{401}$ to $R_{412}$ as a substituent are each independently a halogen atom, a substituted or unsubstituted aryl group having 6 to 30 ring carbon atoms, a substituted or unsubstituted heterocyclic group having 5 to 30 ring atoms, a substituted or unsubstituted alkyl group having 1 to 30 carbon atoms, a substituted or unsubstituted alkyl halide group having 1 to 30 carbon atoms, a substituted or unsubstituted alkenyl group having 2 to 30 carbon atoms, a substituted or unsubstituted alkynyl group having 2 to 30 carbon atoms, a substituted or unsubstituted alkylsilyl group having 3 to 30 carbon atoms, a substituted or unsubstituted arylsilyl group having 6 to 60 ring carbon atoms, a substituted or unsubstituted aryl phosphoryl group having 6 to 60 ring carbon atoms, a hydroxy group, a substituted or unsubstituted alkoxy group having 1 to 30 carbon atoms, a substituted or unsubstituted aryloxy group having 6 to 30 ring carbon atoms, an amino group, a substituted or unsubstituted alkylamino group having 2 to 30 carbon atoms, a substituted or unsubstituted arylamino group having 6 to 60 ring carbon atoms, a thiol group, a substituted or unsubstituted alkylthio group having 1 to 30 carbon atoms, or a substituted or unsubstituted arylthio group having 6 to 30 ring carbon atoms;

n is 0, 1, 2 or 3;

when a plurality of $R_{405}$ are present, the plurality of $R_{405}$ are mutually the same or different;
when a plurality of $R_{406}$ are present, the plurality of $R_{406}$ are mutually the same or different;
when a plurality of $R_{407}$ are present, the plurality of $R_{407}$ are mutually the same or different; and
when a plurality of $R_{408}$ are present, the plurality of $R_{408}$ are mutually the same or different.

According to a further aspect of the invention, an organic electroluminescence device includes: an anode; a cathode; an emitting layer provided between the anode and the cathode, in which the emitting layer contains a delayed fluorescent compound M2 and the compound according to any one of the above aspects of the invention.
According to the above aspects of the invention, a compound and an organic electroluminescence device capable of achieving higher performance, particularly an improved luminous efficiency, can be provided.

BRIEF DESCRIPTION OF DRAWING(S)

Fig. 1 schematically shows an exemplary arrangement of an organic electroluminescence device according to a first exemplary embodiment of the invention.
Fig. 2 schematically shows a device of measuring transient PL.
Fig. 3 shows an example of decay curves of the transient PL.
Fig. 4 schematically shows a relationship in energy level and energy transfer between a compound M3 and a compound M2 in an emitting layer of an exemplary organic electroluminescence device according to the first exemplary embodiment of the invention.
Fig. 5 schematically shows a relationship in energy level and energy transfer between the compound M3, the compound M2, and a compound M1 in an emitting layer of an exemplary organic electroluminescence device according to a second exemplary embodiment of the invention.

DESCRIPTION OF EMBODIMENT(S)

First Exemplary Embodiment

An arrangement of an organic EL device according to a first exemplary embodiment of the invention will be described below.
The organic EL device includes an anode, a cathode, and an organic layer between the anode and the cathode. This organic layer includes at least one layer formed of an organic compound(s). Alternatively, the organic layer includes a plurality of layers formed of an organic compound(s). The organic layer may further include an inorganic compound. In the organic EL device according to the exemplary embodiment, at least one layer of the organic layer is an emitting layer. Accordingly, the organic layer may, for instance, be provided by a single emitting layer, or include a layer(s) usable for an organic EL device. Examples of the layer(s) usable for the organic EL device, which are not particularly limited, include at least one layer selected from the group consisting of a hole injecting layer, hole transporting layer, electron injecting layer, electron transporting layer, and blocking layer.
The organic EL device according to the exemplary embodiment includes an emitting layer between the anode and the cathode.
Fig. 1 schematically shows an exemplary arrangement of an organic EL device according to the first exemplary embodiment.
An organic EL device 1 includes a light-transmissive substrate 2, an anode 3, a cathode 4, and an organic layer 10 provided between the anode 3 and the cathode 4. The organic layer 10 includes a hole injecting layer 6, a hole transporting layer 7, an emitting layer 5, an electron transporting layer 8, and an electron injecting layer 9, which are sequentially laminated on the anode 3.
In an exemplary arrangement of the exemplary embodiment, the emitting layer may contain a metal complex.
In an exemplary arrangement of the exemplary embodiment, the emitting layer preferably does not contain a phosphorescent material (dopant material).
In an exemplary arrangement of the exemplary embodiment, the emitting layer preferably does not contain a heavy-metal complex and a phosphorescent rare earth metal complex. Examples of the heavy-metal complex herein include iridium complex, osmium complex, and platinum complex.
In an exemplary arrangement of the exemplary embodiment, the emitting layer also preferably does not contain a metal complex.
In the organic EL device according to the exemplary embodiment, the emitting layer contains a delayed fluorescent compound M2, and a compound M3 represented by a formula (3) below.

In this arrangement, the compound M2 is preferably a dopant material (also referred to as a guest material, emitter or luminescent material), and the compound M3 is preferably a host material (also referred to as a matrix material).

Emitting Layer

Compound M3

The emitting layer of the exemplary embodiment contains the compound M3 represented by the formula (3).

The compound M3 of the exemplary embodiment may be a thermally activated delayed fluorescent compound or a compound exhibiting no thermally activated delayed fluorescence. However, the compound M3 is preferably a compound exhibiting no thermally activated delayed fluorescence.

[Formula 10]

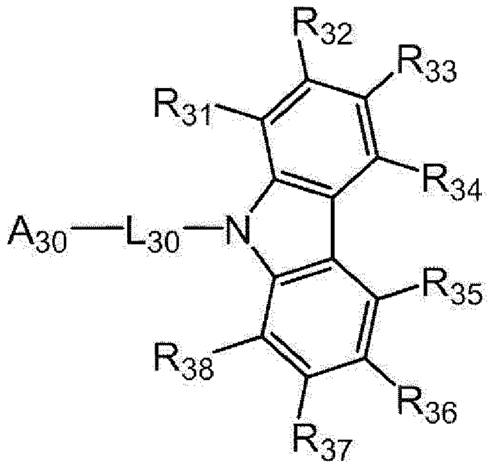

(3)

In the formula (3), $A_{30}$ is a group represented by a formula (3a), (3b), (3c), (3d), (3e) or (3f) below.

[Formula 11]

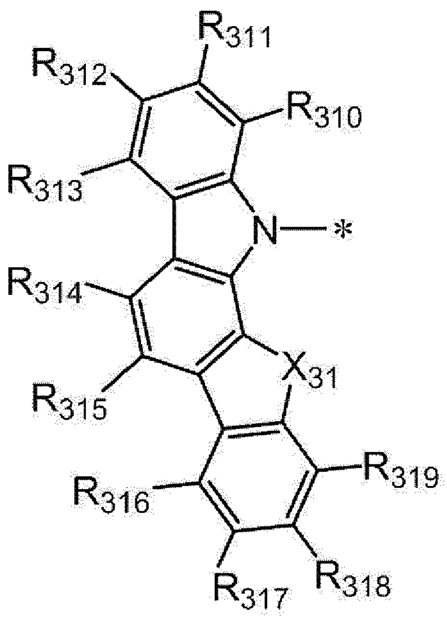

(3a)

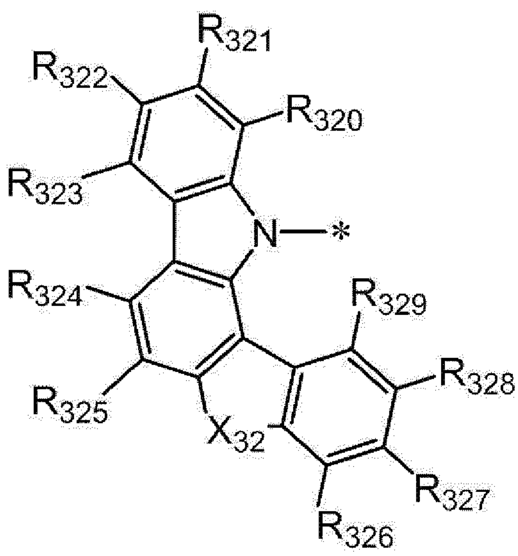

(3b)

[Formula 12]

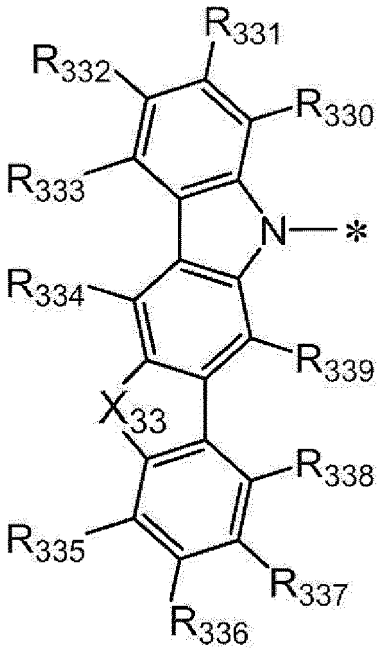

(3c)

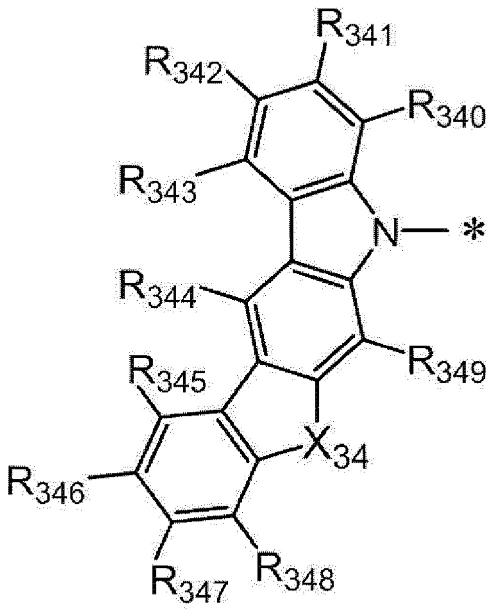

(3d)

[Formula 13]

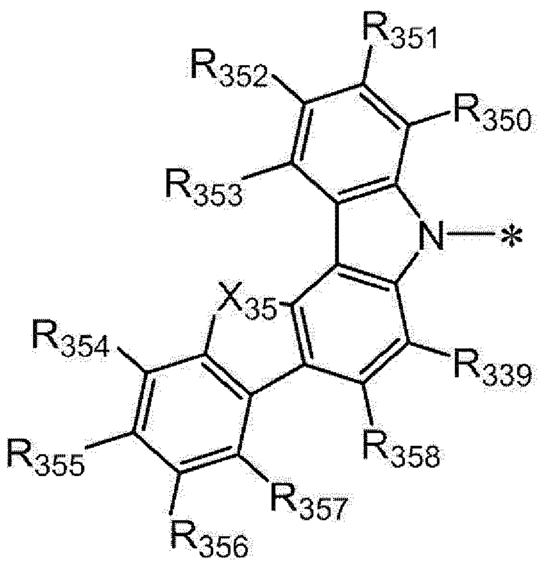

(3e)

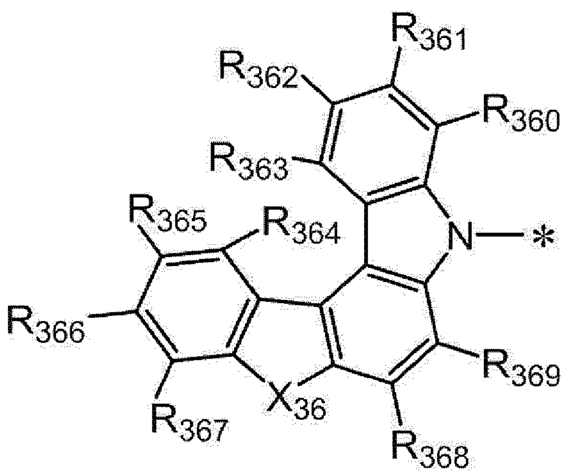

(3f)

In the formulae (3a), (3b), (3c), (3d), (3e) and (3f):

$X_{31}$, $X_{32}$, $X_{33}$, $X_{34}$, $X_{35}$ and $X_{36}$ are each independently an oxygen atom, a sulfur atom or $SiR_{372}R_{373}$;
$R_{372}$ and $R_{373}$ are each independently a hydrogen atom or a substituent;
$R_{310}$ to $R_{319}$ are each independently a hydrogen atom or a substituent, or at least one pair of a pair of $R_{310}$ and $R_{311}$, a pair of $R_{311}$ and $R_{312}$, a pair of $R_{312}$ and $R_{313}$, a pair of $R_{314}$ and $R_{315}$, a pair of $R_{316}$ and $R_{317}$, a pair of $R_{317}$ and $R_{318}$, or a pair of $R_{318}$ and $R_{319}$ are mutually bonded to form a ring;

$R_{320}$ to $R_{329}$ are each independently a hydrogen atom or a substituent, or at least one pair of a pair of $R_{320}$ and $R_{321}$, a pair of $R_{321}$ and $R_{322}$, a pair of $R_{322}$ and $R_{323}$, a pair of $R_{324}$ and $R_{325}$, a pair of $R_{326}$ and $R_{327}$, a pair of $R_{327}$ and $R_{328}$, or a pair of $R_{328}$ and $R_{329}$ are mutually bonded to form a ring;

$R_{330}$ to $R_{339}$ are each independently a hydrogen atom or a substituent, or at least one pair of a pair of $R_{330}$ and $R_{331}$, a pair of $R_{331}$ and $R_{332}$, a pair of $R_{332}$ and $R_{333}$, a pair of $R_{335}$ and $R_{336}$, a pair of $R_{336}$ and $R_{337}$, or a pair of $R_{337}$ and $R_{338}$ are mutually bonded to form a ring;

$R_{340}$ to $R_{349}$ are each independently a hydrogen atom or a substituent, or at least one pair of a pair of $R_{340}$ and $R_{341}$, a pair of $R_{341}$ and $R_{342}$, a pair of $R_{342}$ and $R_{343}$, a pair of $R_{345}$ and $R_{346}$, a pair of $R_{346}$ and $R_{347}$, or a pair of $R_{347}$ and $R_{348}$ are mutually bonded to form a ring;

$R_{350}$ to $R_{359}$ are each independently a hydrogen atom or a substituent, or at least one pair of a pair of $R_{350}$ and $R_{351}$, a pair of $R_{351}$ and $R_{352}$, a pair of $R_{352}$ and $R_{353}$, a pair of $R_{354}$ and $R_{355}$, a pair of $R_{355}$ and $R_{356}$, a pair of $R_{356}$ and $R_{357}$, or a pair of $R_{358}$ and $R_{359}$ are mutually bonded to form a ring;

$R_{360}$ to $R_{369}$ are each independently a hydrogen atom or a substituent, or at least one pair of a pair of $R_{360}$ and $R_{361}$, a pair of $R_{361}$ and $R_{362}$, a pair of $R_{362}$ and $R_{363}$, a pair of $R_{364}$ and $R_{365}$, a pair of $R_{365}$ and $R_{366}$, a pair of $R_{366}$ and $R_{367}$, or a pair of $R_{368}$ and $R_{369}$ are mutually bonded to form a ring;

$R_{372}$ and $R_{373}$ as a substituent and $R_{310}$ to $R_{319}$, $R_{320}$ to $R_{329}$, $R_{330}$ to $R_{339}$, $R_{340}$ to $R_{349}$, $R_{350}$ to $R_{359}$ and $R_{360}$ to $R_{369}$ as a substituent are each independently a halogen atom, a cyano group, a substituted or unsubstituted aryl group having 6 to 30 ring carbon atoms, a substituted or unsubstituted heterocyclic group having 5 to 30 ring atoms, a substituted or unsubstituted alkyl group having 1 to 30 carbon atoms, a substituted or unsubstituted alkyl halide group having 1 to 30 carbon atoms, a substituted or unsubstituted alkenyl group having 2 to 30 carbon atoms, a substituted or unsubstituted alkynyl group having 2 to 30 carbon atoms, a substituted or unsubstituted alkylsilyl group having 3 to 30 carbon atoms, a substituted or unsubstituted arylsilyl group having 6 to 60 ring carbon atoms, a substituted or unsubstituted arylphosphoryl group having 6 to 60 ring carbon atoms, a hydroxy group, a substituted or unsubstituted alkoxy group having 1 to 30 carbon atoms, a substituted or unsubstituted aryloxy group having 6 to 30 ring carbon atoms, an amino group, a substituted or unsubstituted alkylamino group having 2 to 30 carbon atoms, a substituted or unsubstituted arylamino group having 6 to 60 ring carbon atoms, a thiol group, a substituted or unsubstituted alkylthio group having 1 to 30 carbon atoms, or a substituted or unsubstituted arylthio group having 6 to 30 ring carbon atoms; and

* represents a bonding position to $L_{30}$.

In the formula (3):

$L_{30}$ is a substituted or unsubstituted arylene group having 6 to 22 ring carbon atoms, a group formed by bonding two substituted or unsubstituted arylene groups having 6 to 22 ring carbon atoms, or a group formed by bonding three substituted or unsubstituted arylene groups having 6 to 22 ring carbon atoms;

$R_{31}$ to $R_{38}$ are each independently a hydrogen atom or a substituent, or at least one pair of a pair of $R_{31}$ and $R_{32}$, a pair of $R_{32}$ and $R_{33}$, a pair of $R_{33}$ and $R_{34}$, a pair of $R_{35}$ and $R_{36}$, a pair of $R_{36}$ and $R_{37}$, or a pair of $R_{37}$ and $R_{38}$ are mutually bonded to form a ring; and

a substituent for substituting $L_{30}$, and $R_{31}$ to $R_{38}$ as a substituent are each independently a halogen atom, a cyano group, a substituted or unsubstituted aryl group having 6 to 30 ring carbon atoms, a substituted or unsubstituted heterocyclic group having 5 to 30 ring atoms, a substituted or unsubstituted alkyl group having 1 to 30 carbon atoms, a substituted or unsubstituted alkyl halide group having 1 to 30 carbon atoms, a substituted or unsubstituted alkenyl group having 2 to 30 carbon atoms, a substituted or unsubstituted alkynyl group having 2 to 30 carbon atoms, a substituted or unsubstituted alkylsilyl group having 3 to 30 carbon atoms, a substituted or unsubstituted arylsilyl group having 6 to 60 ring carbon atoms, a substituted or unsubstituted aryl phosphoryl group having 6 to 60 ring carbon atoms, a hydroxy group, a substituted or unsubstituted alkoxy group having 1 to 30 carbon atoms, a substituted or unsubstituted aryloxy group having 6 to 30 ring carbon atoms, an amino group, a substituted or unsubstituted alkylamino group having 2 to 30 carbon atoms, a substituted or unsubstituted arylamino group having 6 to 60 ring carbon atoms, a thiol group, a substituted or unsubstituted alkylthio group having 1 to 30 carbon atoms, or a substituted or unsubstituted arylthio group having 6 to 30 ring carbon atoms.

In the compound M3, $A_{30}$ is preferably the group represented by the formula (3a) or (3d).

In the compound M3, $A_{30}$ is more preferably the group represented by the formula (3a). When $A_{30}$ is represented by the formula (3a), the compound M3 is represented by a formula (3A) below.

[Formula 14]

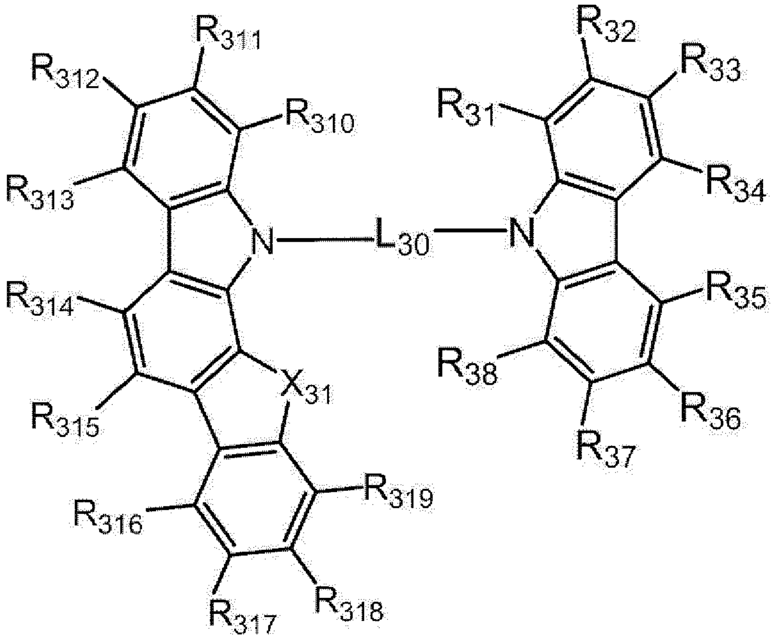

(3A)

$R_{31}$ to $R_{36}$, $L_{30}$, $X_{31}$ and $R_{310}$ to $R_{319}$ in the formula (3A) respectively represent the same as $R_{31}$ to $R_{36}$, $L_{30}$, $X_{31}$ and $R_{310}$ to $R_{319}$ in the formula (3) or (3a).

In the compound M3, it is also preferable that a pair of $R_{31}$ and $R_{32}$, a pair of $R_{32}$ and $R_{33}$, a pair of $R_{33}$ and $R_{34}$, a pair of $R_{35}$ and $R_{36}$, a pair of $R_{36}$ and $R_{37}$, and a pair of $R_{37}$ and $R_{38}$ are not mutually bonded. In other words, in the compound M3, $R_{31}$ to $R_{38}$ are also preferably each independently a hydrogen atom or a substituent.

In the compound M3, it is also preferable that at least one pair of a pair of $R_{31}$ and $R_{32}$, a pair of $R_{32}$ and $R_{33}$, a pair of $R_{33}$ and $R_{34}$, a pair of $R_{35}$ and $R_{36}$, a pair of $R_{36}$ and $R_{37}$, or a pair of $R_{37}$ and $R_{38}$ are mutually bonded to form a ring.

The compound M3 is also preferably represented by a formula (31), (32), (33), (34), (35) or (36) below.

[Formula 15]

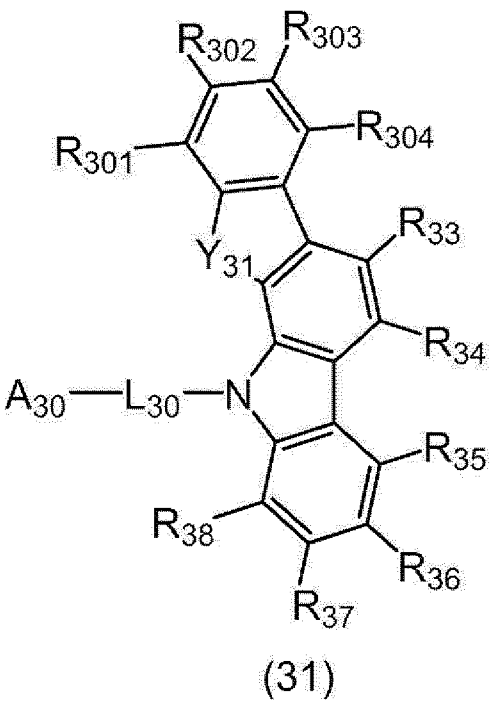

(31)

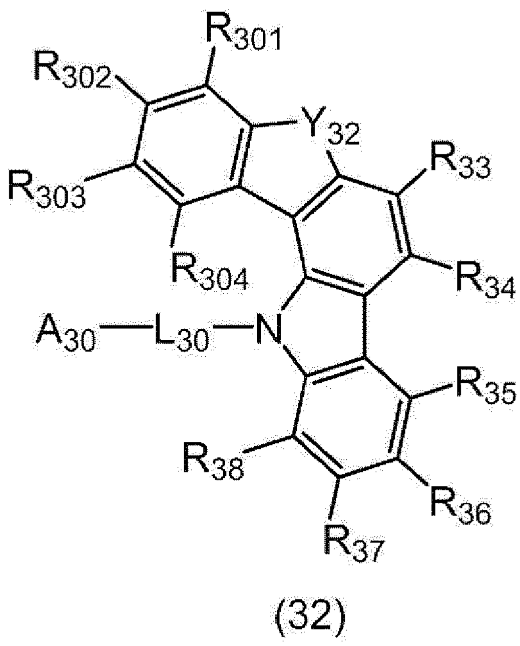

(32)

[Formula 16]

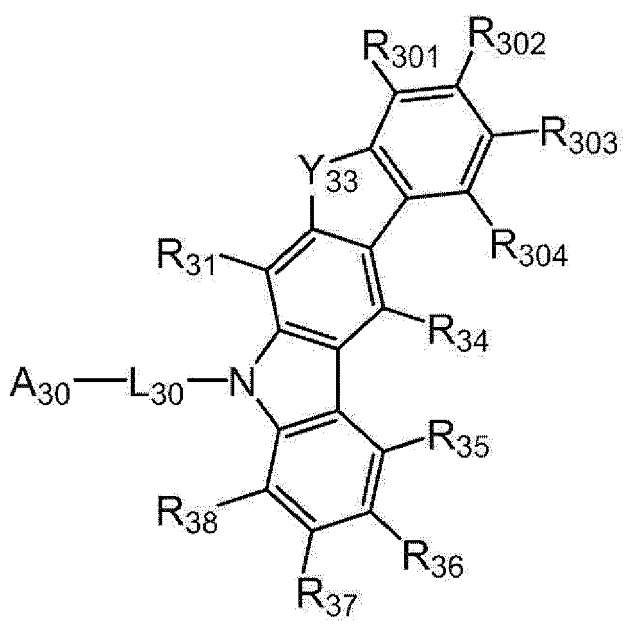

(33)

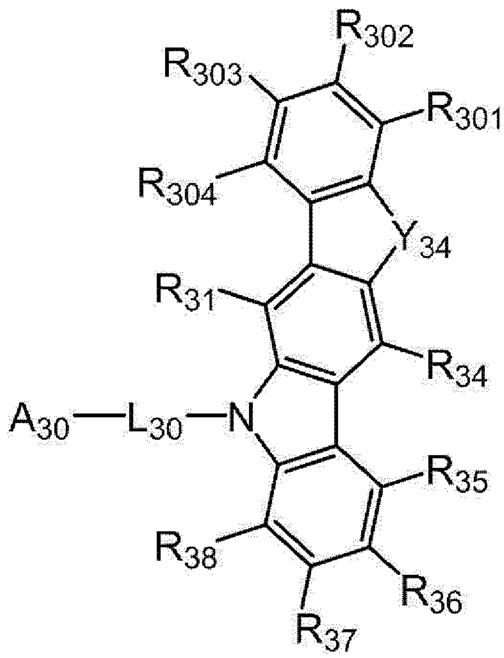

(34)

[Formula 17]

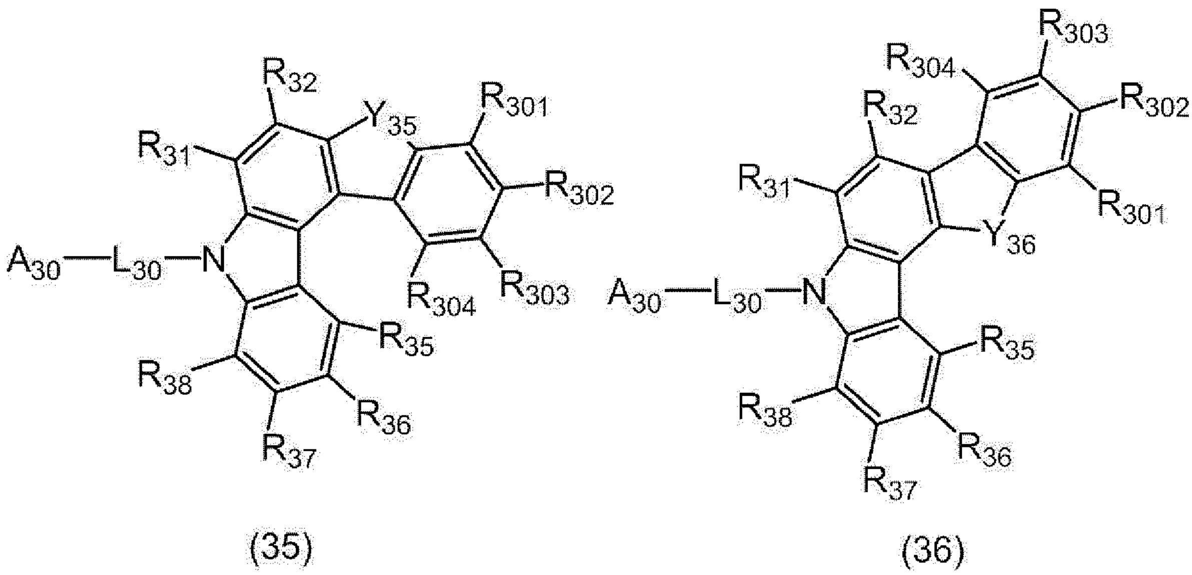

(35) (36)

In the formulae (31) to (36):

$Y_{31}$, $Y_{32}$, $Y_{33}$, $Y_{34}$, $Y_{35}$ and $Y_{36}$ are each independently an oxygen atom, a sulfur atom, $NR_{374}$ or $SiR_{375}R_{376}$;
$R_{374}$, $R_{375}$ and $R_{376}$ are each independently a hydrogen atom or a substituent;
$A_{30}$, $L_{30}$ and $R_{31}$ to $R_{38}$ respectively represent the same as $A_{30}$, $L_{30}$ and $R_{31}$ to $R_{38}$ in the formula (3);
$R_{301}$ to $R_{304}$ are each independently a hydrogen atom or a substituent, or at least one pair of a pair of $R_{301}$ and $R_{302}$, a pair of $R_{302}$ and $R_{303}$, or a pair of $R_{303}$ and $R_{304}$ are mutually bonded to form a ring; and
$R_{301}$ to $R_{304}$ as a substituent and $R_{374}$, $R_{375}$ and $R_{376}$ as a substituent are each independently a halogen atom, a cyano group, a substituted or unsubstituted aryl group having 6 to 30 ring carbon atoms, a substituted or unsubstituted

heterocyclic group having 5 to 30 ring atoms, a substituted or unsubstituted alkyl group having 1 to 30 carbon atoms, a substituted or unsubstituted alkyl halide group having 1 to 30 carbon atoms, a substituted or unsubstituted alkenyl group having 2 to 30 carbon atoms, a substituted or unsubstituted alkynyl group having 2 to 30 carbon atoms, a substituted or unsubstituted alkylsilyl group having 3 to 30 carbon atoms, a substituted or unsubstituted arylsilyl group having 6 to 60 ring carbon atoms, a substituted or unsubstituted arylphosphoryl group having 6 to 60 ring carbon atoms, a hydroxy group, a substituted or unsubstituted alkoxy group having 1 to 30 carbon atoms, a substituted or unsubstituted aryloxy group having 6 to 30 ring carbon atoms, an amino group, a substituted or unsubstituted alkylamino group having 2 to 30 carbon atoms, a substituted or unsubstituted arylamino group having 6 to 60 ring carbon atoms, a thiol group, a substituted or unsubstituted alkylthio group having 1 to 30 carbon atoms, or a substituted or unsubstituted arylthio group having 6 to 30 ring carbon atoms.

In the compound M3, it is preferable that none of a pair of $R_{301}$ and $R_{302}$, a pair of $R_{302}$ and $R_{303}$, and a pair of $R_{303}$ and $R_{304}$ are mutually bonded.

In the compound M3, $R_{301}$ to $R_{304}$ are preferably each independently a hydrogen atom, a substituted or unsubstituted aryl group having 6 to 30 ring carbon atoms, a substituted or unsubstituted heterocyclic group having 5 to 30 ring atoms, or a substituted or unsubstituted alkyl group having 1 to 30 carbon atoms.

In the compound M3, $R_{301}$ to $R_{304}$ are more preferably each independently a hydrogen atom, a substituted or unsubstituted aryl group having 6 to 30 ring carbon atoms, or a substituted or unsubstituted heterocyclic group having 5 to 30 ring atoms.

In the compound M3, $R_{301}$ to $R_{304}$ are further preferably each independently a hydrogen atom or a substituted or unsubstituted aryl group having 6 to 30 ring carbon atoms.

In the compound M3, $R_{301}$ to $R_{304}$ are further more preferably a hydrogen atom.

In the compound M3, $Y_{31}$, $Y_{32}$, $Y_{33}$, $Y_{34}$, $Y_{35}$ and $Y_{36}$ are preferably each independently an oxygen atom or a sulfur atom.

In the compound M3, $R_{31}$ to $R_{38}$ are preferably each independently a hydrogen atom, a substituted or unsubstituted aryl group having 6 to 30 ring carbon atoms, a substituted or unsubstituted heterocyclic group having 5 to 30 ring atoms, or a substituted or unsubstituted alkyl group having 1 to 30 carbon atoms.

In the compound M3, $R_{31}$ to $R_{38}$ are more preferably each independently a hydrogen atom, a substituted or unsubstituted aryl group having 6 to 30 ring carbon atoms, or a substituted or unsubstituted heterocyclic group having 5 to 30 ring atoms.

In the compound M3, $R_{31}$ to $R_{38}$ are further preferably each independently a hydrogen atom or a substituted or unsubstituted aryl group having 6 to 30 ring carbon atoms.

In the compound M3, $R_{31}$ to $R_{38}$ are further more preferably a hydrogen atom.

In the compound M3, $R_{31}$ to $R_{38}$ and $R_{301}$ to $R_{304}$ are preferably each independently a hydrogen atom, a substituted or unsubstituted aryl group having 6 to 30 ring carbon atoms, a substituted or unsubstituted heterocyclic group having 5 to 30 ring atoms, or a substituted or unsubstituted alkyl group having 1 to 30 carbon atoms.

In the compound M3, $R_{31}$ to $R_{38}$ and $R_{301}$ to $R_{304}$ are more preferably each independently a hydrogen atom, a substituted or unsubstituted aryl group having 6 to 30 ring carbon atoms, or a substituted or unsubstituted heterocyclic group having 5 to 30 ring atoms.

In the compound M3, $R_{31}$ to $R_{38}$ and $R_{301}$ to $R_{304}$ are further preferably each independently a hydrogen atom or a substituted or unsubstituted aryl group having 6 to 30 ring carbon atoms.

In the compound M3, $R_{31}$ to $R_{38}$ and $R_{301}$ to $R_{304}$ are further more preferably a hydrogen atom.

In the compound M3, $L_{30}$ is preferably a substituted or unsubstituted phenylene group, a substituted or unsubstituted biphenylene group, or a substituted or unsubstituted terphenylene group.

In the compound M3, $L_{30}$ is more preferably a substituted or unsubstituted biphenylene group.

In the compound M3, it is preferable that a substituent for substituting $L_{30}$ (this substituent is sometimes referred to as a substituent $F_L$) is a substituted or unsubstituted aryl group having 6 to 30 ring carbon atoms or a substituted or unsubstituted alkyl group having 1 to 30 carbon atoms.

In the compound M3, $L_{30}$ is also preferably at least one group selected from the group consisting of groups represented by formulae (L30-1) to (L30-4), (L30-4-2) and (L30-5) to (L30-11) below.

[Formula 18]

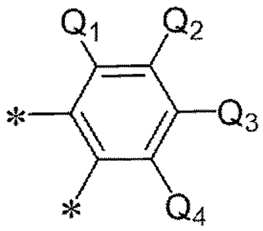

(L30-1)

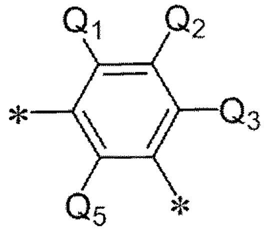

(L30-2)

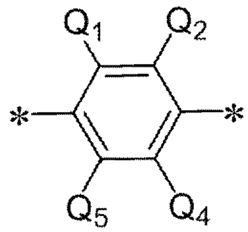

(L30-3)

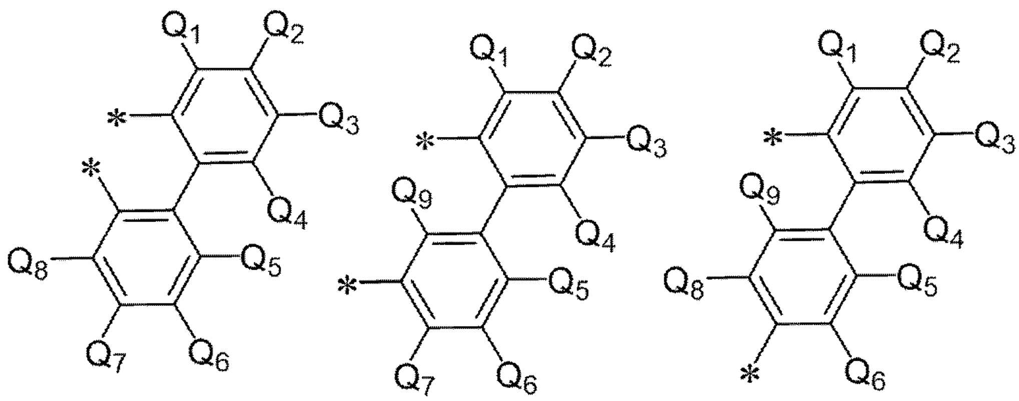

(L30-4) (L30-4-2) (L30-5)

[Formula 19]

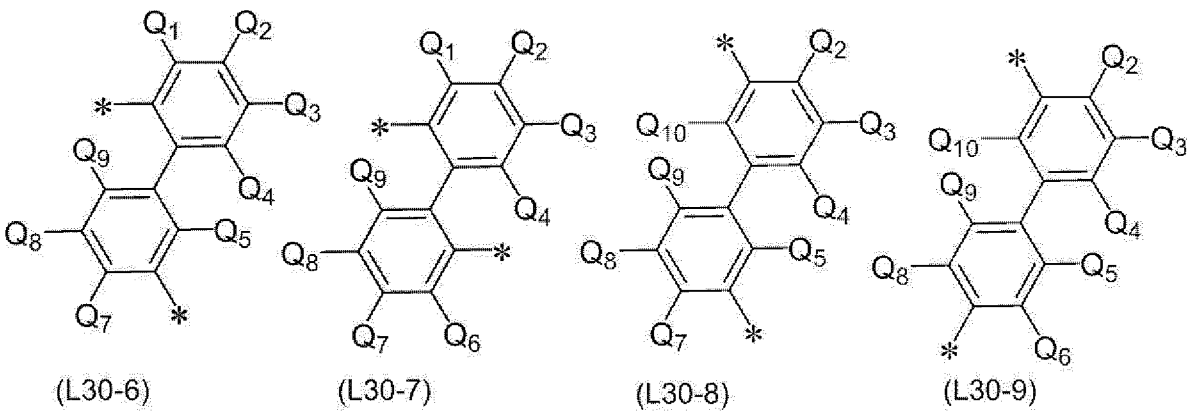

(L30-6) (L30-7) (L30-8) (L30-9)

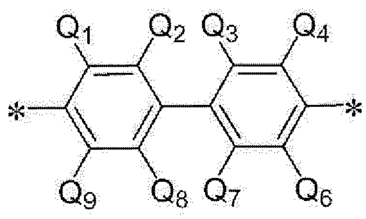

(L30-10)

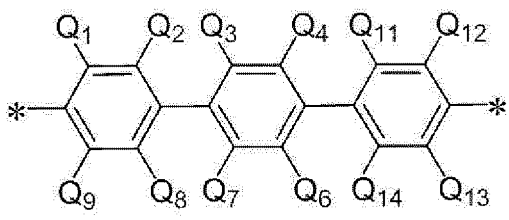

(L30-11)

In the formulae (L30-1) to (L30-4), (L30-4-2) and (L30-5) to (L30-11):

$Q_1$ to $Q_{14}$ are each independently a hydrogen atom or a substituent; and

$Q_1$ to $Q_{14}$ as a substituent are each independently a halogen atom, a cyano group, a substituted or unsubstituted aryl group having 6 to 30 ring carbon atoms, a substituted or unsubstituted heterocyclic group having 5 to 30 ring atoms, a substituted or unsubstituted alkyl group having 1 to 30 carbon atoms, a substituted or unsubstituted alkyl halide group having 1 to 30 carbon atoms, a substituted or unsubstituted alkenyl group having 2 to 30 carbon atoms, a substituted or unsubstituted alkynyl group having 2 to 30 carbon atoms, a substituted or unsubstituted alkylsilyl group having 3 to 30 carbon atoms, a substituted or unsubstituted arylsilyl group having 6 to 60 ring carbon atoms, a substituted or unsubstituted arylphosphoryl group having 6 to 60 ring carbon atoms, a hydroxy group, a substituted or unsubstituted alkoxy group having 1 to 30 carbon atoms, a substituted or unsubstituted aryloxy group having 6 to 30 ring carbon atoms, an amino group, a substituted or unsubstituted alkylamino group having 2 to 30 carbon atoms, a substituted or unsubstituted arylamino group having 6 to 60 ring carbon atoms, a thiol group, a substituted or unsubstituted alkylthio group having 1 to 30 carbon atoms, or a substituted or unsubstituted arylthio group having 6 to 30 ring carbon atoms.

$Q_1$ to $Q_{14}$ as a substituent are preferably each independently a hydrogen atom, a substituted or unsubstituted aryl group having 6 to 30 ring carbon atoms, or a substituted or unsubstituted alkyl group having 1 to 30 carbon atoms.

In the compound M3, $X_{31}$, $X_{32}$, $X_{33}$, $X_{34}$, $X_{35}$ and $X_{36}$ are also preferably each independently an oxygen atom or $SiR_{302}R_{303}$.

In the compound M3, $X_{31}$, $X_{32}$, $X_{33}$, $X_{34}$, $X_{35}$ and $X_{36}$ are also preferably each independently an oxygen atom or a sulfur atom.

In the compound M3, $X_{31}$, $X_{32}$, $X_{33}$, $X_{34}$, $X_{35}$ and $X_{36}$ are also preferably an oxygen atom.

In the compound M3: the formula (3a) is represented by, for instance, a formula (3a-1) below when $X_{31}$ is an oxygen atom; the formula (3a) is represented by, for instance, a formula (3a-2) below when $X_{31}$ is a sulfur atom; and the formula (3a) is represented by, for instance, a formula (3a-4) when $X_{31}$ is $SiR_{372}R_{373}$.

[Formula 20]

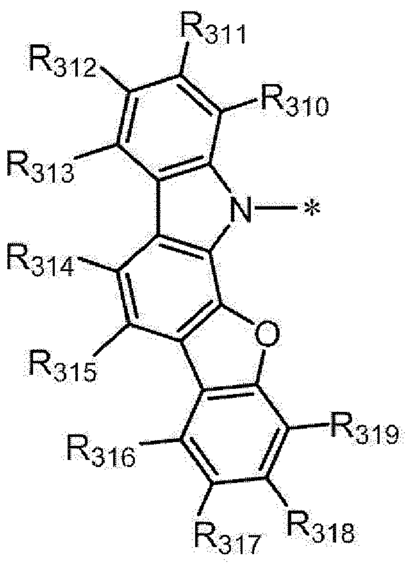

(3a-1)

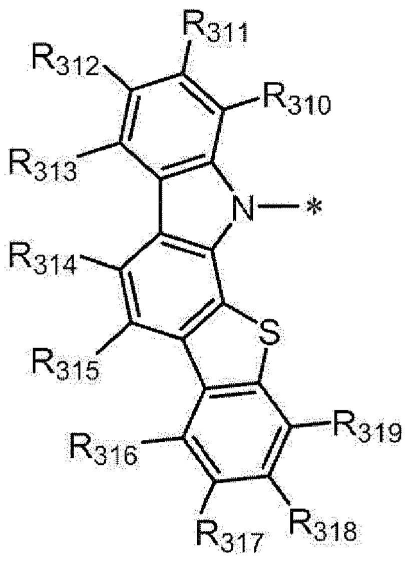

(3a-2)

[Formula 21]

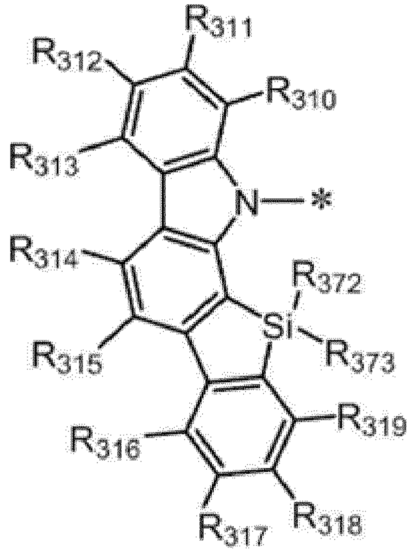

(3a-4)

In the compound M3, it is preferable that: a pair of $R_{310}$ and $R_{311}$, a pair of $R_{311}$ and $R_{312}$, a pair of $R_{312}$ and $R_{313}$, a pair of $R_{314}$ and $R_{315}$, a pair of $R_{316}$ and $R_{317}$, a pair of $R_{317}$ and $R_{318}$, and a pair of $R_{318}$ and $R_{319}$ are not mutually bonded; a pair of $R_{320}$ and $R_{321}$, a pair of $R_{321}$ and $R_{322}$, a pair of $R_{322}$ and $R_{323}$, a pair of $R_{324}$ and $R_{325}$, a pair of $R_{326}$ and $R_{327}$, a pair of $R_{327}$ and $R_{328}$, and a pair of $R_{328}$ and $R_{329}$ are not mutually bonded; a pair of $R_{330}$ and $R_{331}$, a pair of $R_{331}$ and $R_{332}$, a pair of $R_{332}$ and $R_{333}$, a pair of $R_{335}$ and $R_{336}$, a pair of $R_{336}$ and $R_{337}$, and a pair of $R_{337}$ and $R_{338}$ are not mutually bonded; a pair of $R_{340}$ and $R_{341}$, a pair of $R_{341}$ and $R_{342}$, a pair of $R_{342}$ and $R_{343}$, a pair of $R_{345}$ and $R_{346}$, a pair of $R_{346}$ and $R_{347}$, and a pair of $R_{347}$ and $R_{348}$ are not mutually bonded; a pair of $R_{350}$ and $R_{351}$, a pair of $R_{351}$ and $R_{352}$, a pair of $R_{352}$ and $R_{353}$, a pair of $R_{354}$ and $R_{355}$, a pair of $R_{355}$ and $R_{356}$, a pair of $R_{356}$ and $R_{357}$, and a pair of $R_{358}$ and $R_{359}$ are not mutually bonded; and a pair of $R_{360}$ and $R_{361}$, a pair of $R_{361}$ and $R_{362}$, a pair of $R_{362}$ and $R_{363}$, a pair of $R_{364}$ and $R_{365}$, a pair of $R_{365}$ and $R_{366}$, a pair of $R_{366}$ and $R_{367}$, and a pair of $R_{368}$ and $R_{369}$ are not mutually bonded.

**In** the compound M3, $R_{310}$ to $R_{319}$, $R_{320}$ to $R_{329}$, $R_{330}$ to $R_{339}$, $R_{340}$ to $R_{349}$, $R_{350}$ to $R_{359}$ and $R_{360}$ to $R_{369}$ are preferably each independently a hydrogen atom, a substituted or unsubstituted aryl group having 6 to 30 ring carbon atoms, a substituted or unsubstituted heterocyclic group having 5 to 30 ring atoms, or a substituted or unsubstituted alkyl group having 1 to 30 carbon atoms.

In the compound M3, $R_{310}$ to $R_{319}$, $R_{320}$ to $R_{329}$, $R_{330}$ to $R_{339}$, $R_{340}$ to $R_{349}$, $R_{350}$ to $R_{359}$ and $R_{360}$ to $R_{369}$ are more preferably each independently a hydrogen atom, a substituted or unsubstituted aryl group having 6 to 30 ring carbon atoms, or a substituted or unsubstituted heterocyclic group having 5 to 30 ring atoms.

In the compound M3, $R_{310}$ to $R_{319}$, $R_{320}$ to $R_{329}$, $R_{330}$ to $R_{339}$, $R_{340}$ to $R_{349}$, $R_{350}$ to $R_{359}$ and $R_{360}$ to $R_{369}$ are further preferably each independently a hydrogen atom or a substituted or unsubstituted aryl group having 6 to 30 ring carbon atoms.

In the compound M3, $R_{310}$ to $R_{319}$, $R_{320}$ to $R_{329}$, $R_{330}$ to $R_{339}$, $R_{340}$ to $R_{349}$, $R_{350}$ to $R_{359}$ and $R_{360}$ to $R_{369}$ are further more preferably a hydrogen atom.

Manufacturing Method of Compound M3 According to Exemplary Embodiment

The compound M3 of the exemplary embodiment can be manufactured, for instance, by a method described later in Examples. The compound M3 of the exemplary embodiment can be manufactured, for instance, by application of known substitution reactions and/or materials depending on a target compound according to reactions described later in Examples.

Specific Examples of Compound M3

Specific examples of the compound M3 of the exemplary embodiment include compounds below. It should

however be noted that the invention is not limited by the specific examples of the compound.

[Formula 22]

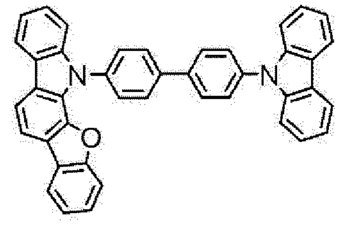
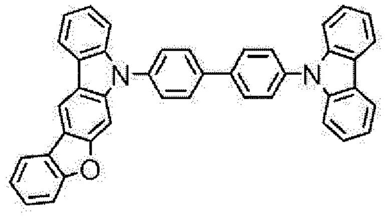
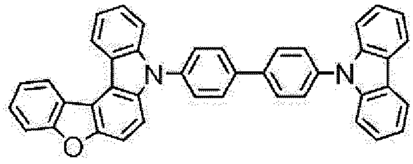
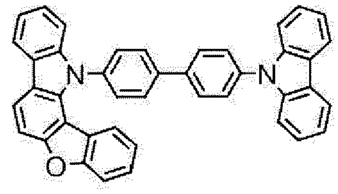
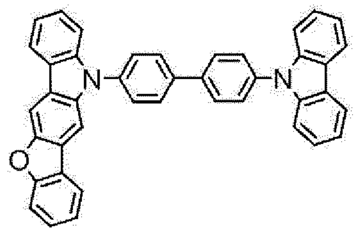

[Formula 23]

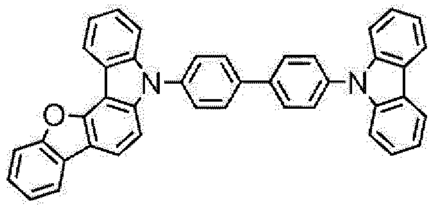
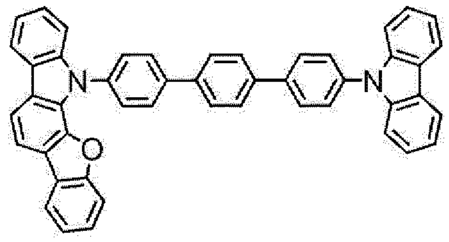
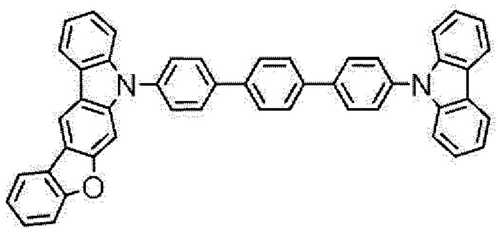
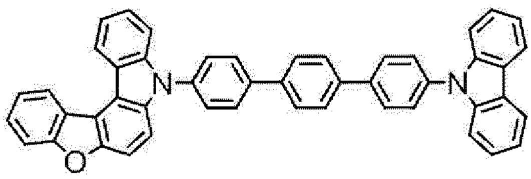

[Formula 24]

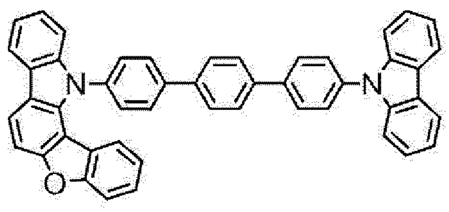
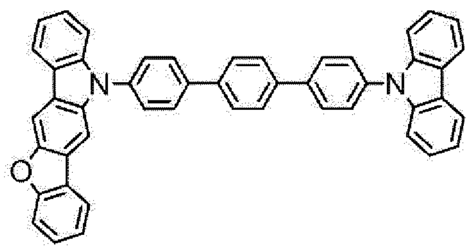
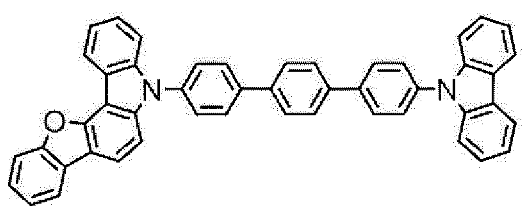

[Formula 25]

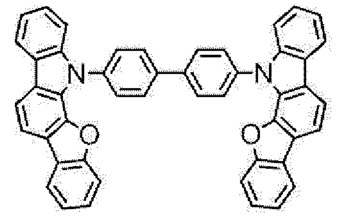

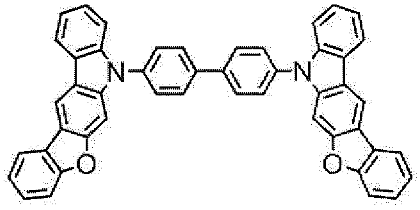

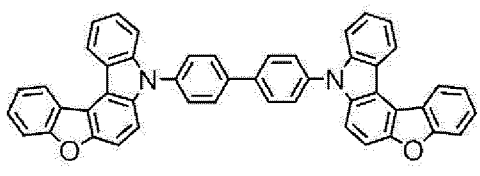

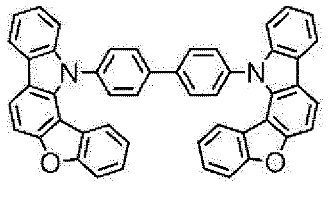

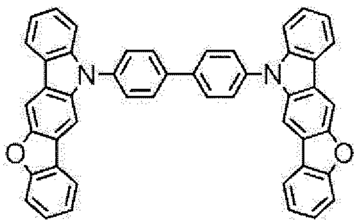

[Formula 26]

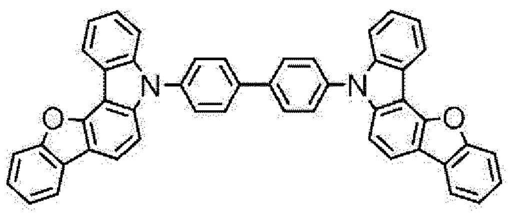

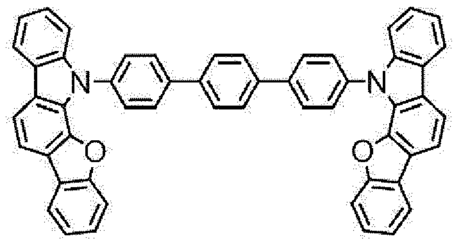

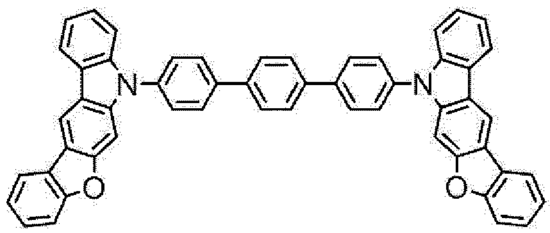

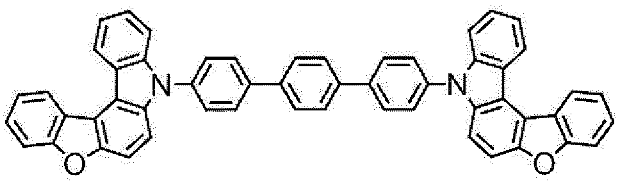

[Formula 27]

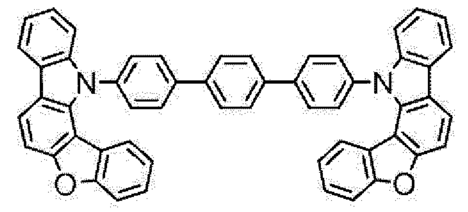

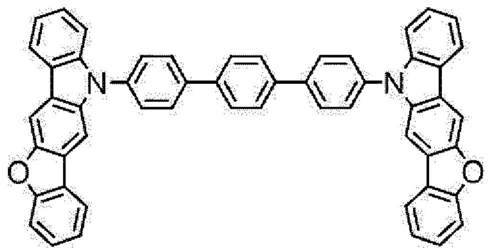

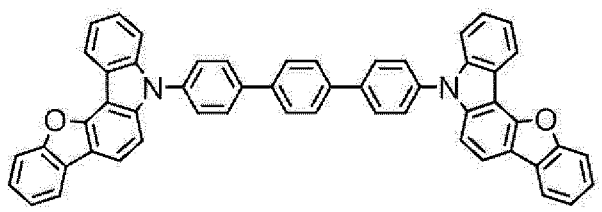

[Formula 28]

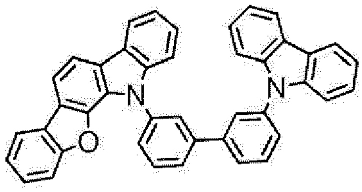
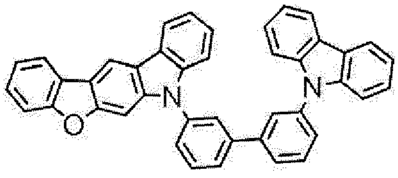
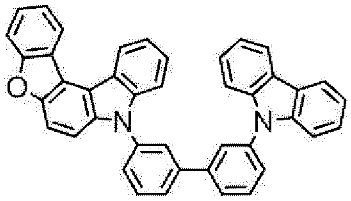
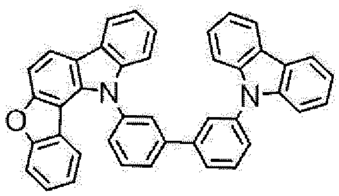
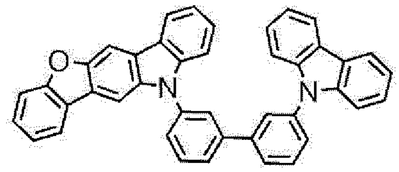

[Formula 29]

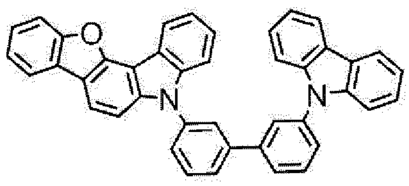
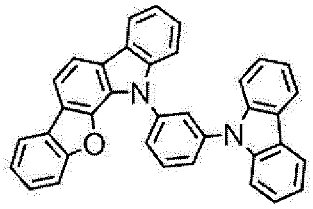
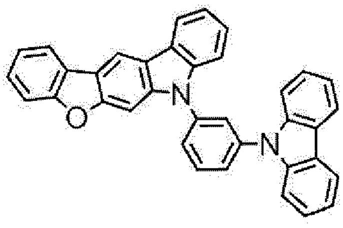
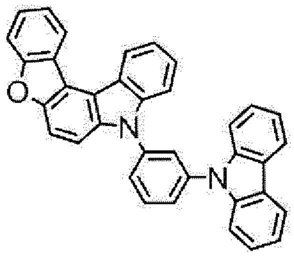
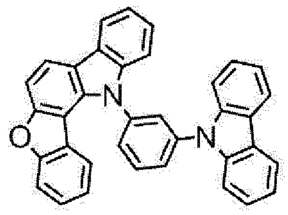

[Formula 30]

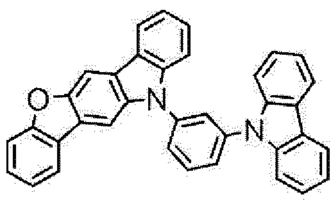
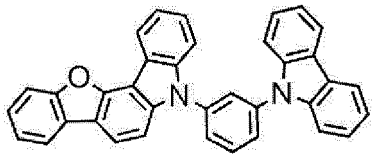
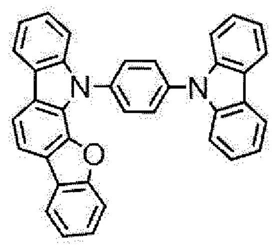
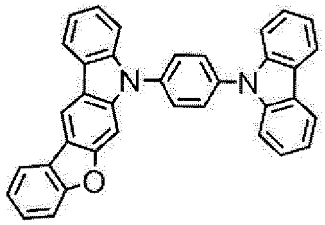
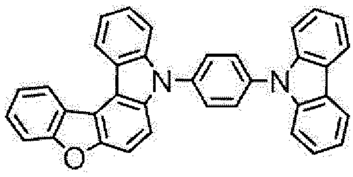

[Formula 31]

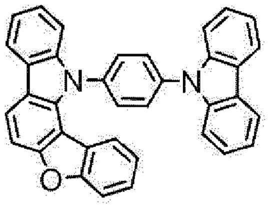
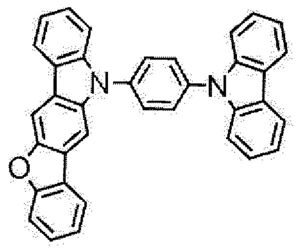
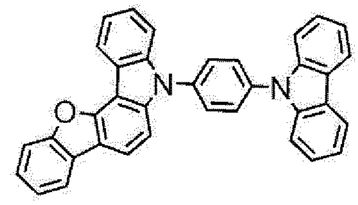

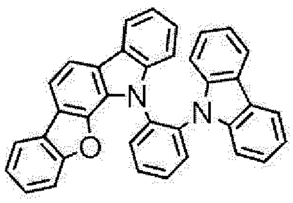
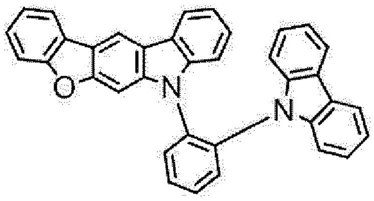
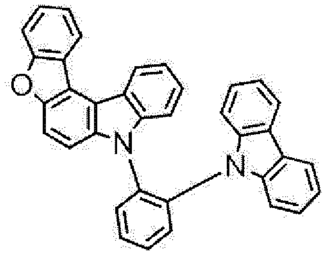

[Formula 32]

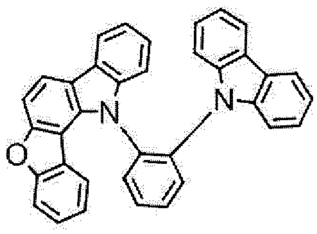
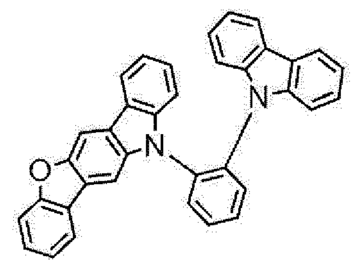
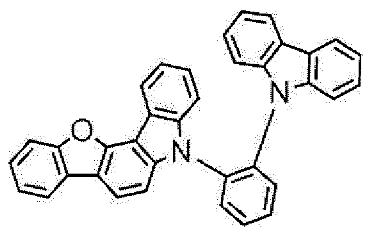

[Formula 33]

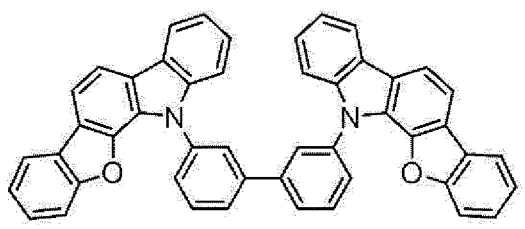
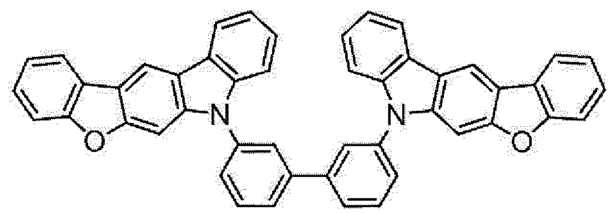

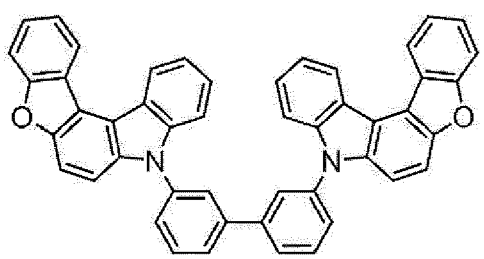
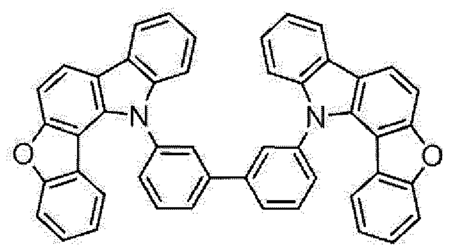

[Formula 34]

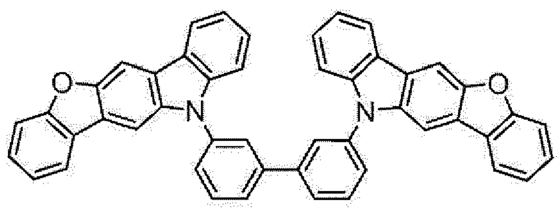

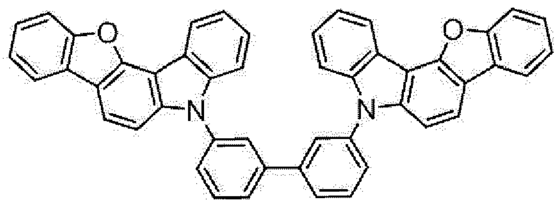

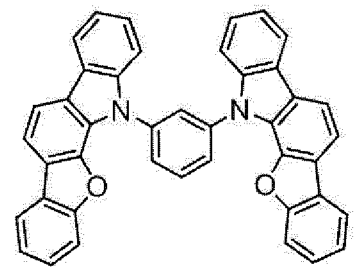

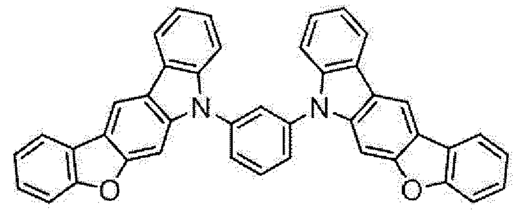

[Formula 35]

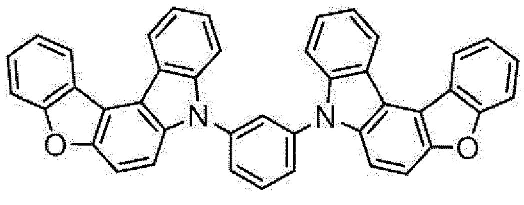

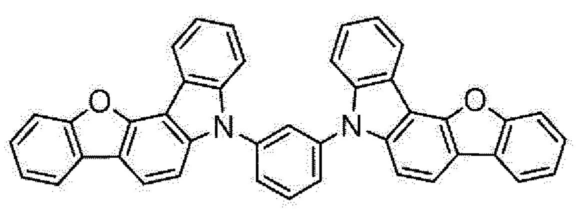

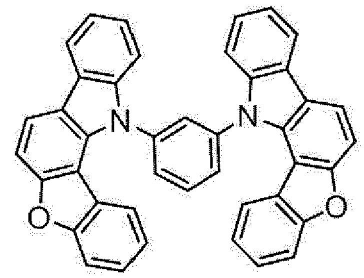

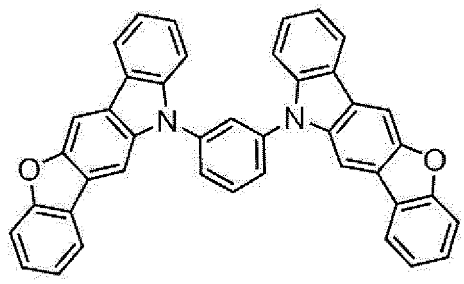

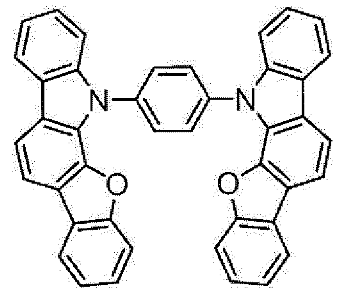

[Formula 36]

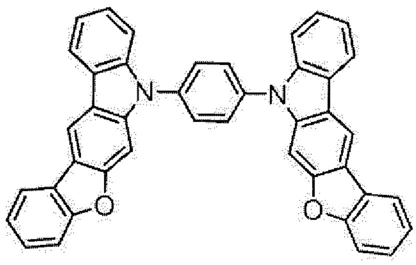

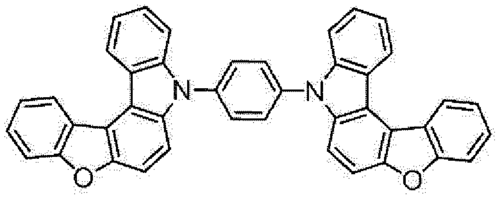

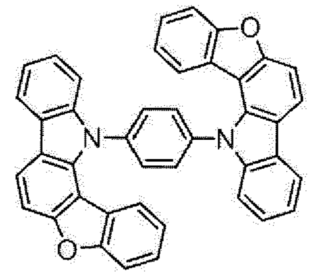

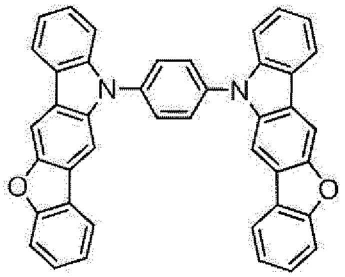

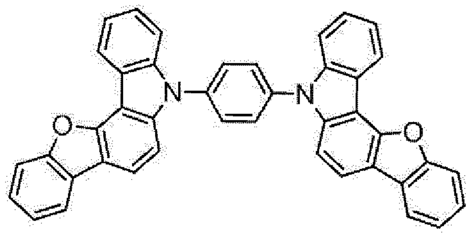

[Formula 37]

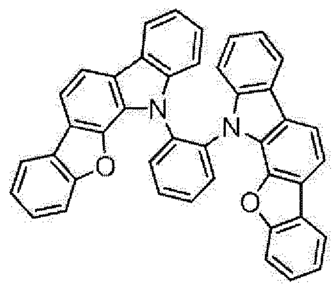

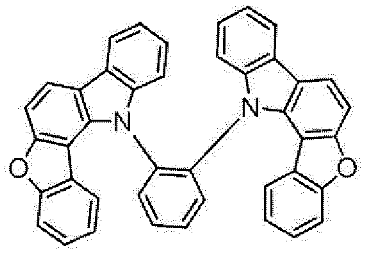

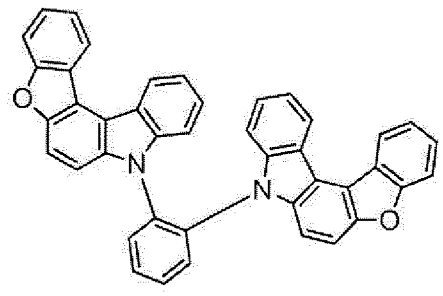

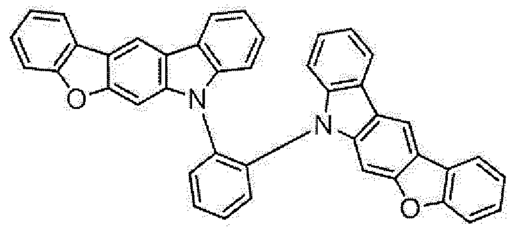

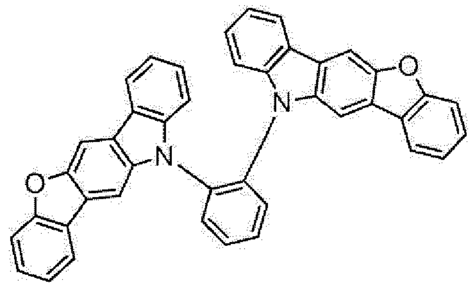

[Formula 38]

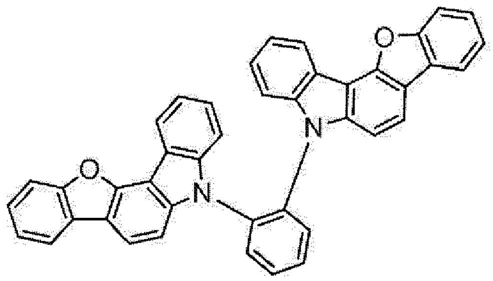

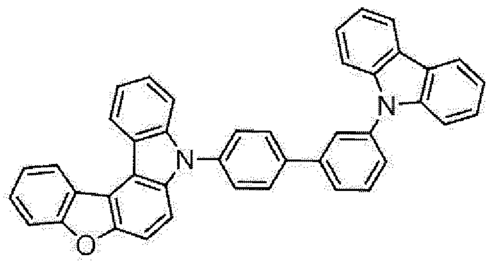

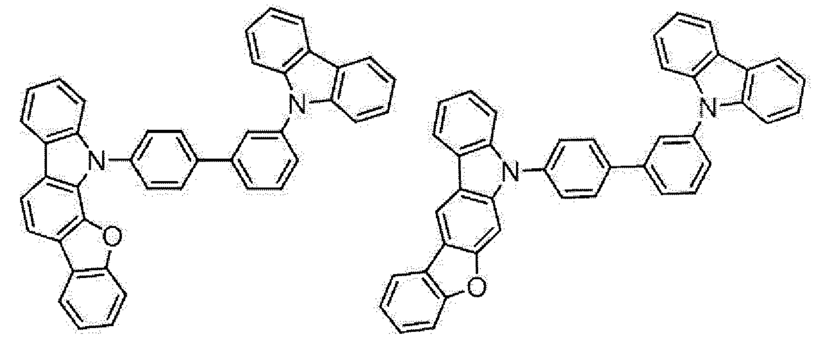

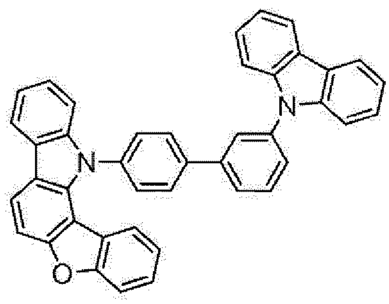

[Formula 39]

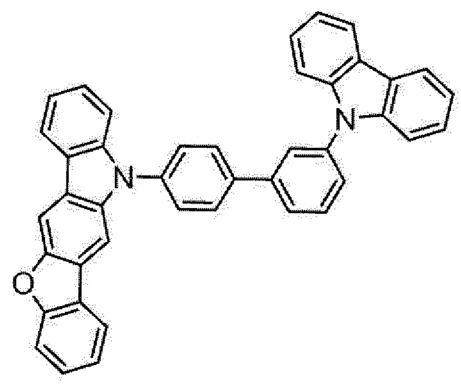

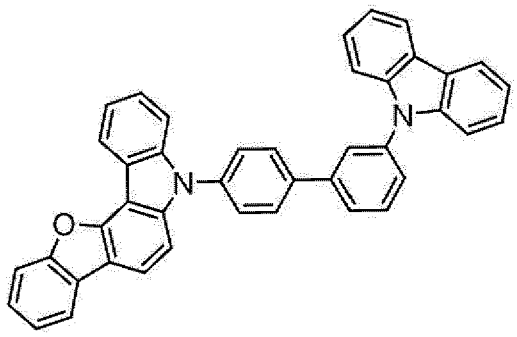

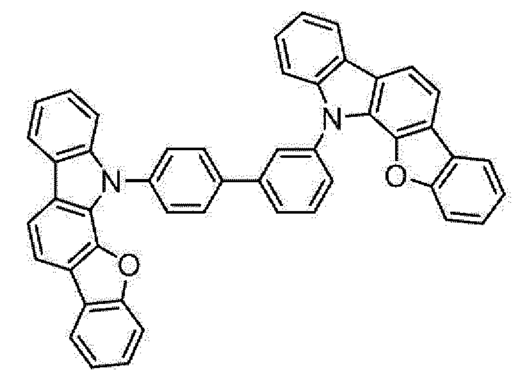

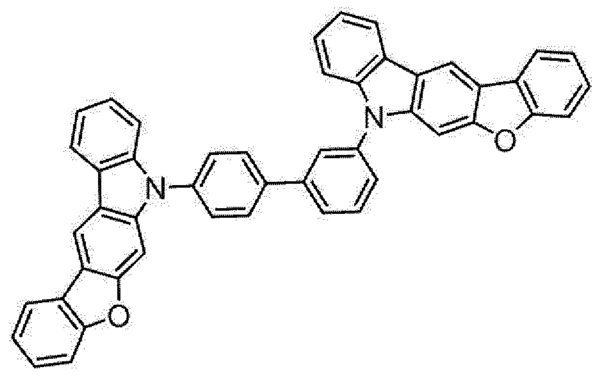

[Formula 40]

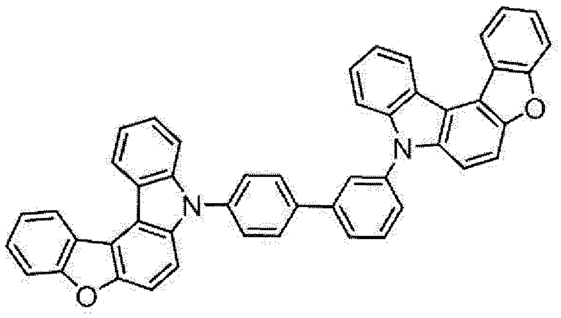
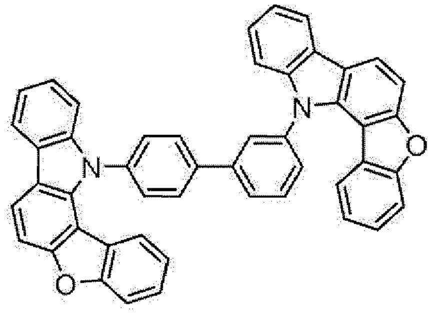
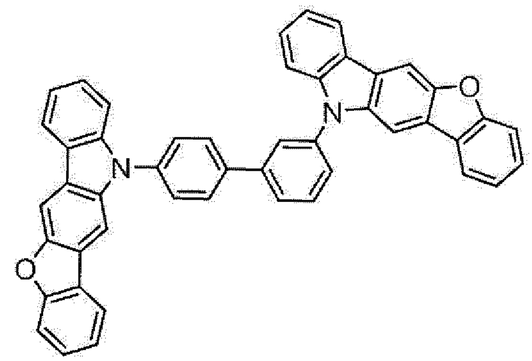
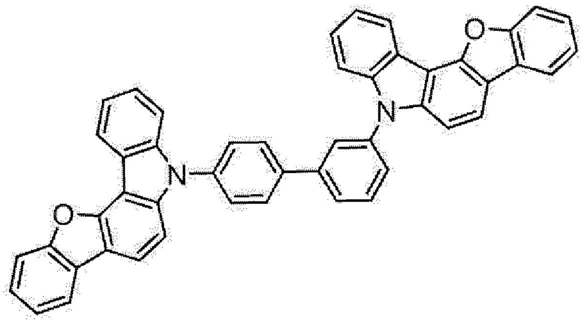

[Formula 41]

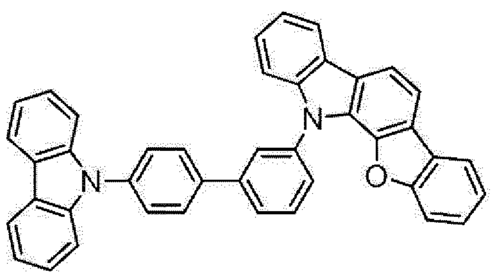
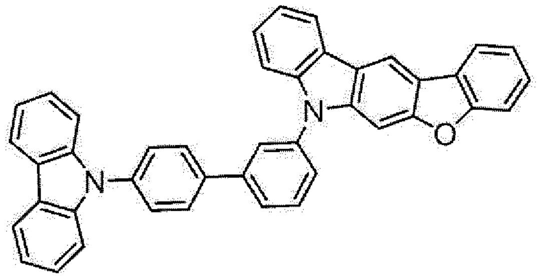
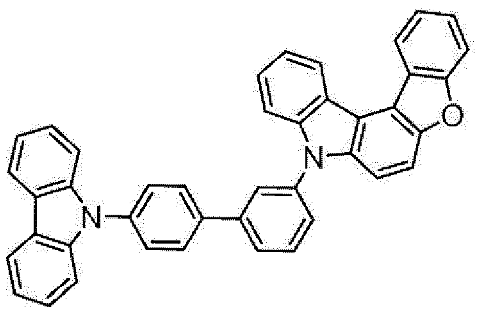
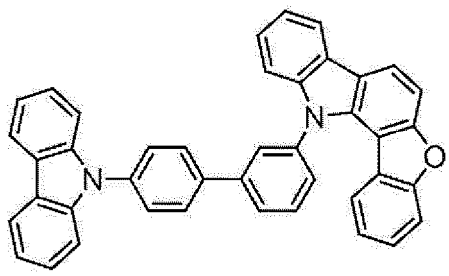
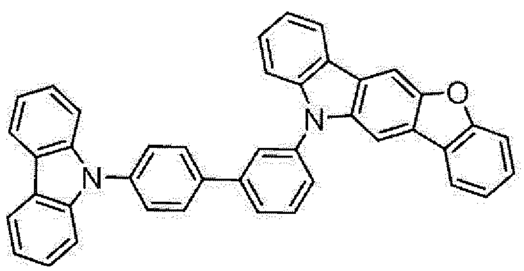
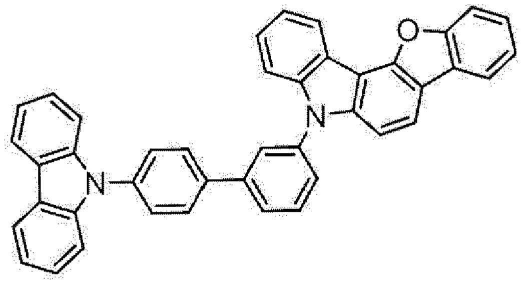

[Formula 42]

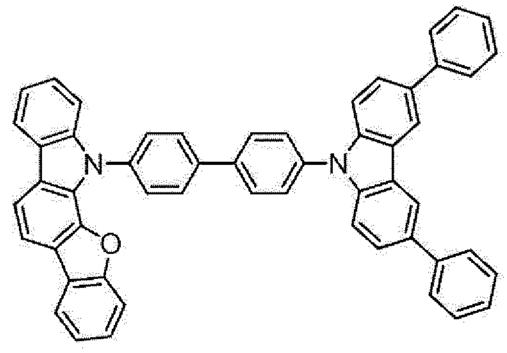

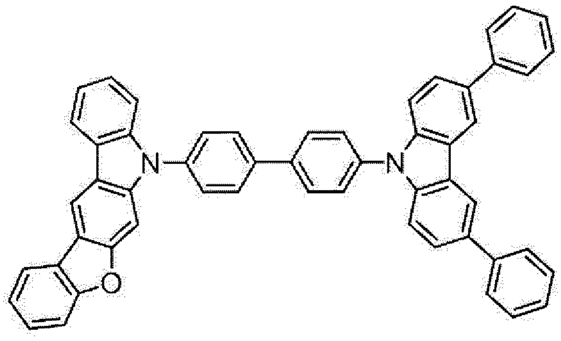

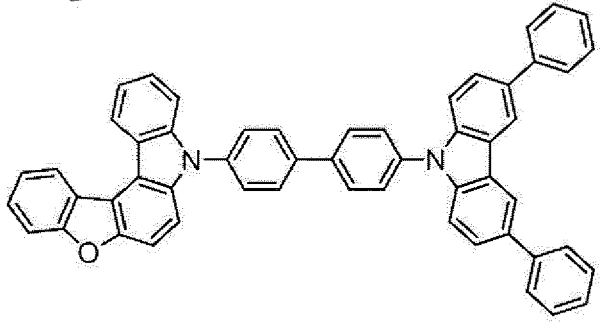

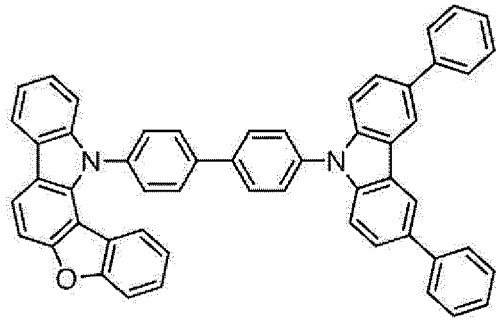

[Formula 43]

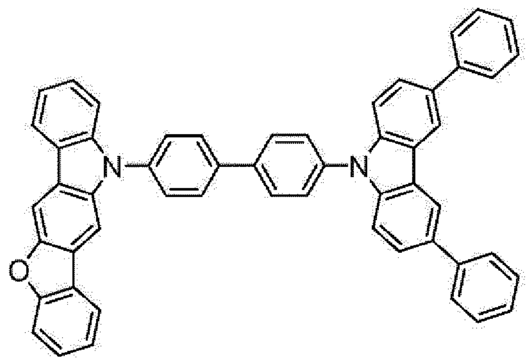

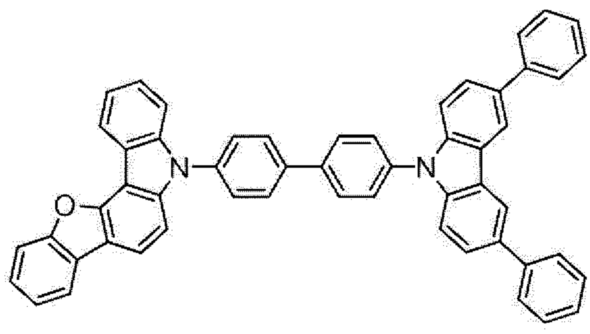

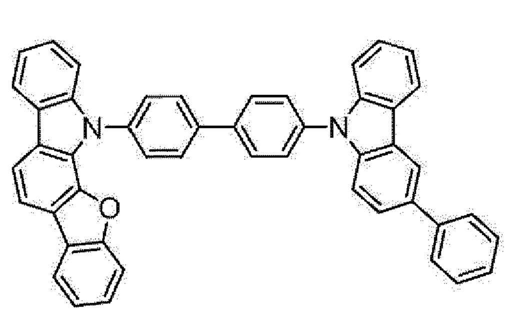

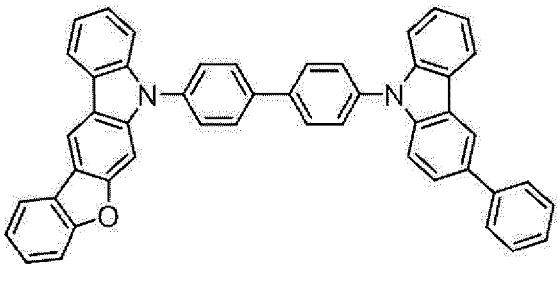

[Formula 44]

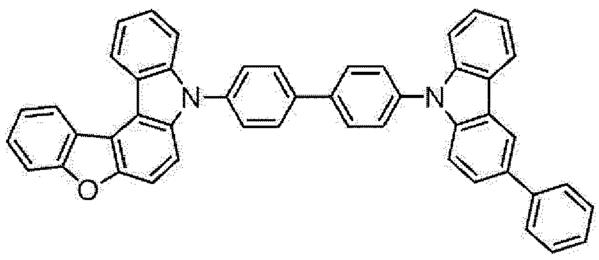

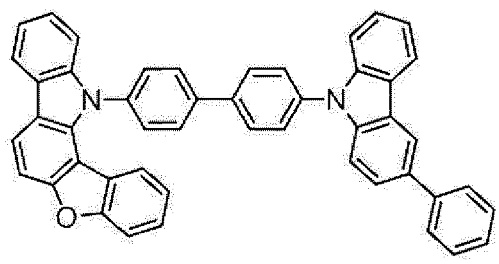

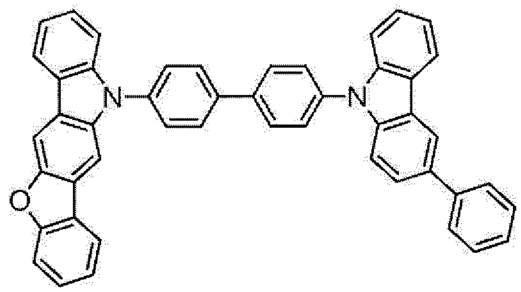

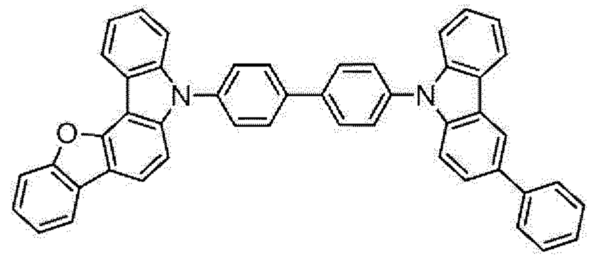

[Formula 45]

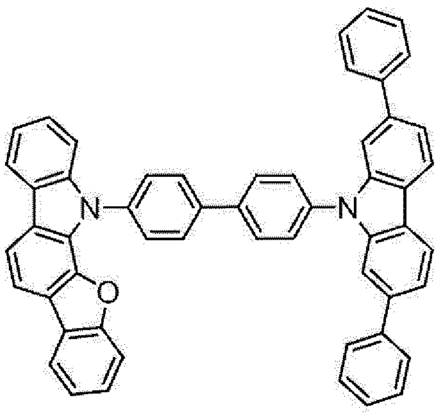

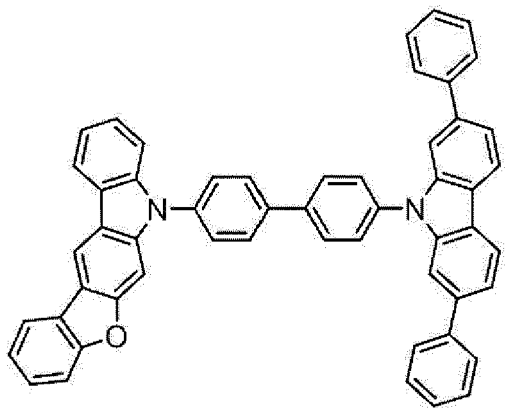

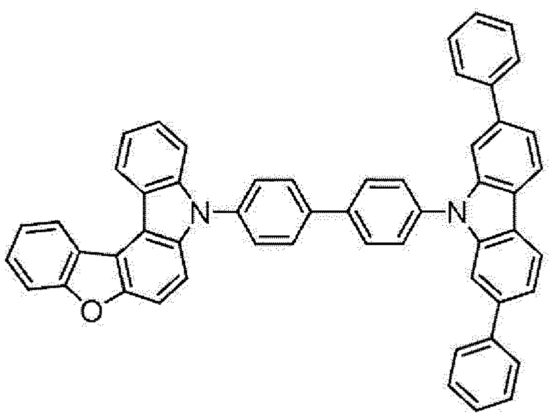

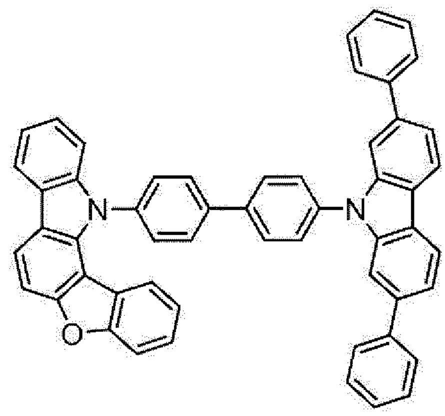

[Formula 46]

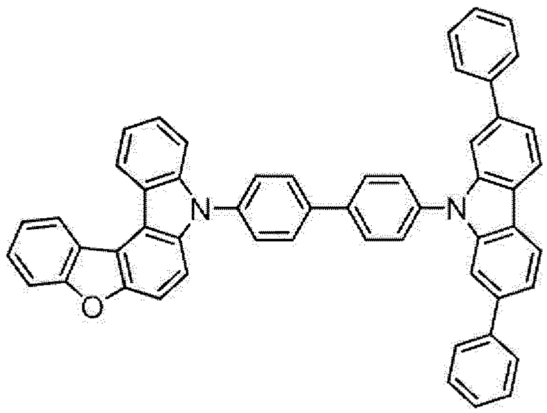

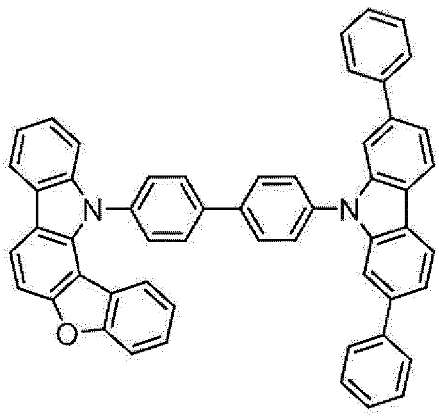

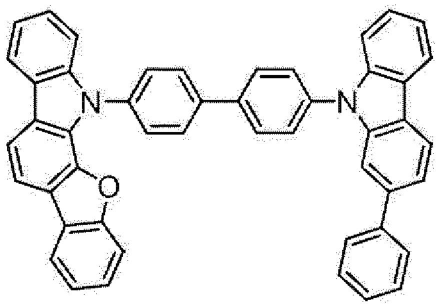

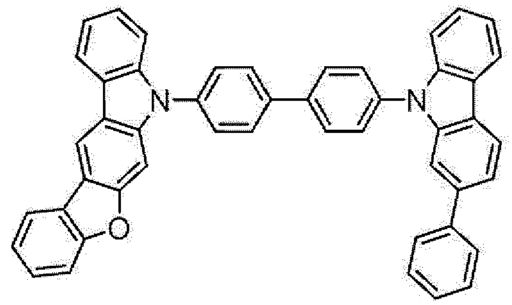

[Formula 47]

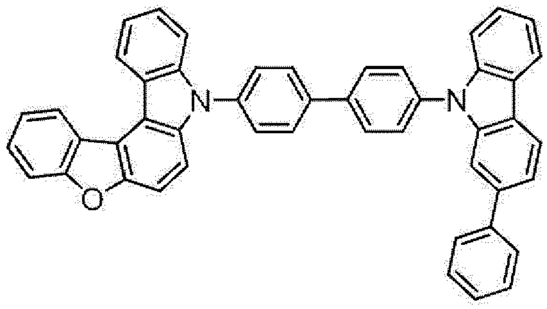

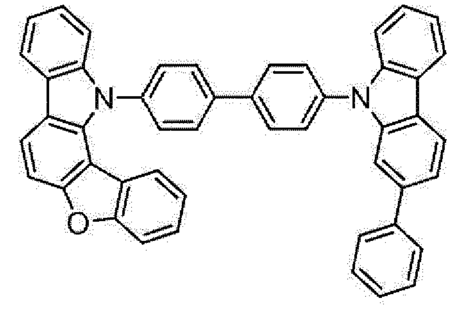

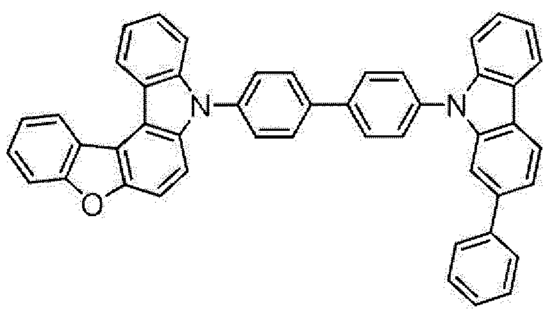

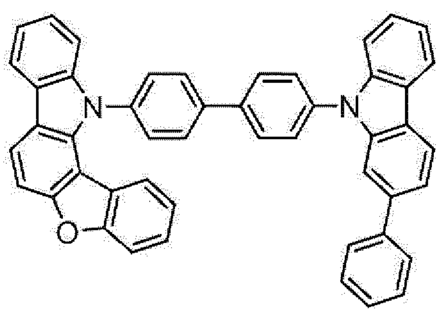

[Formula 48]

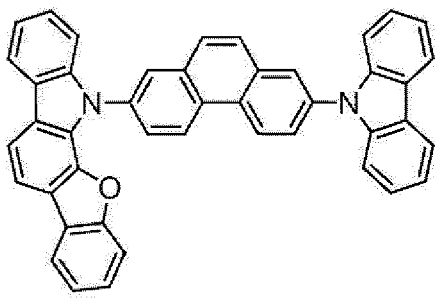

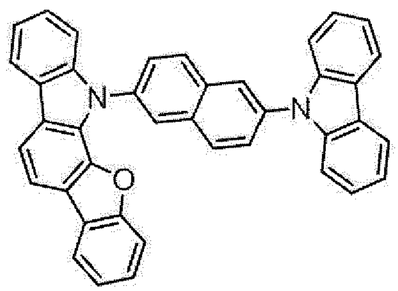

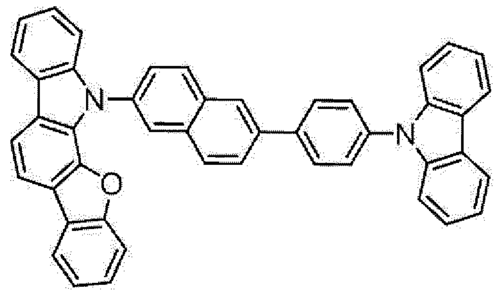

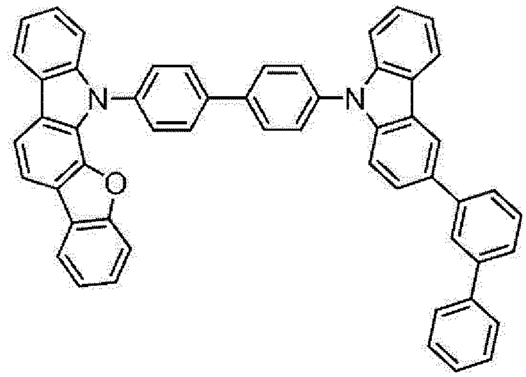

[Formula 49]

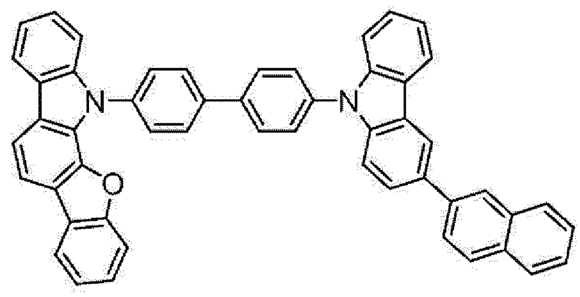

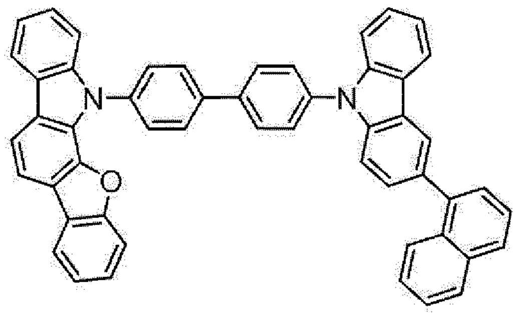

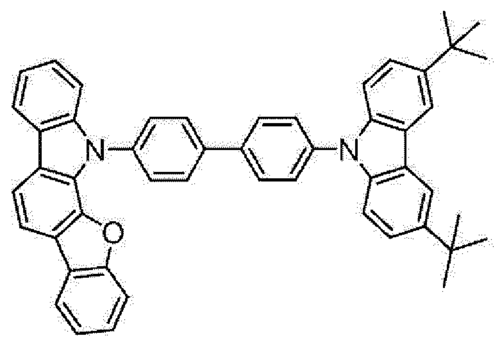

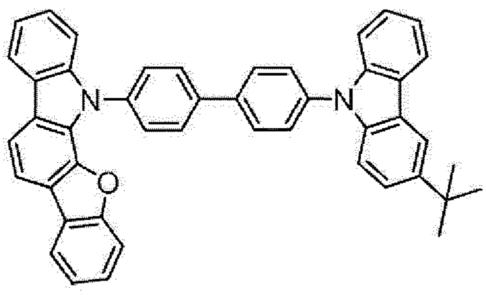

[Formula 50]

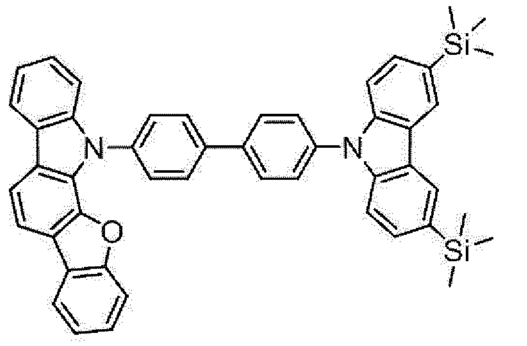

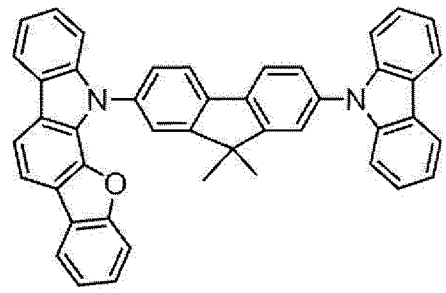

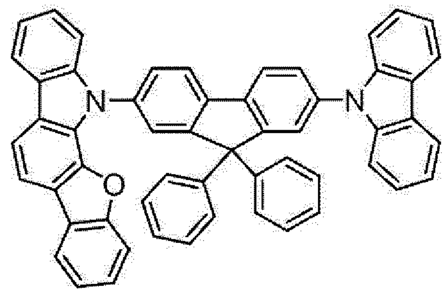

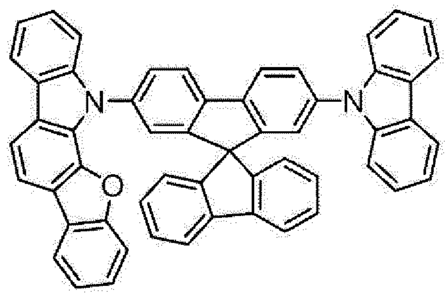

[Formula 51]

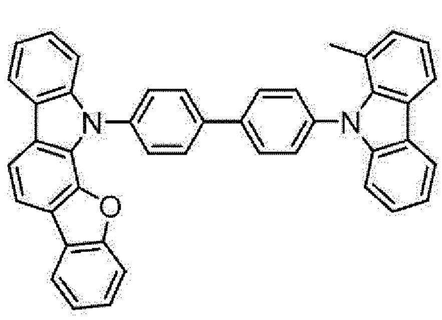

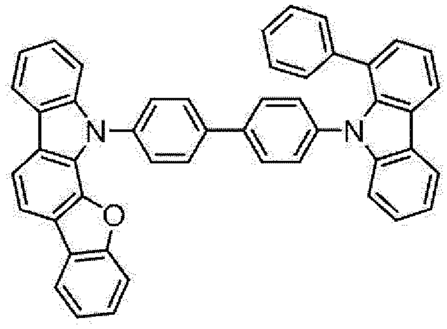

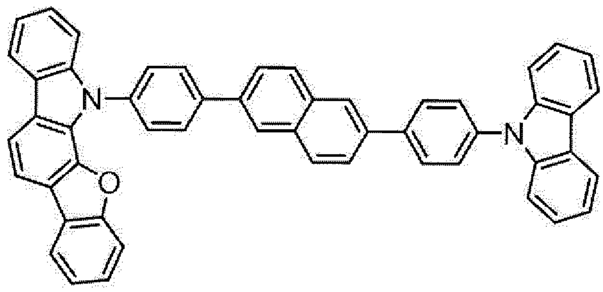

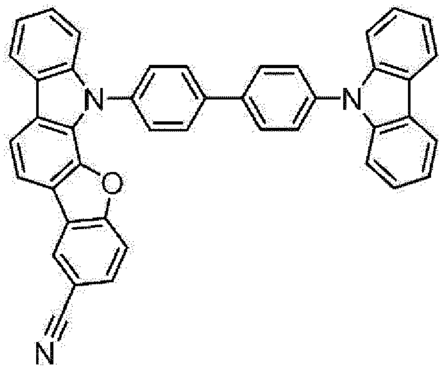

[Formula 52]

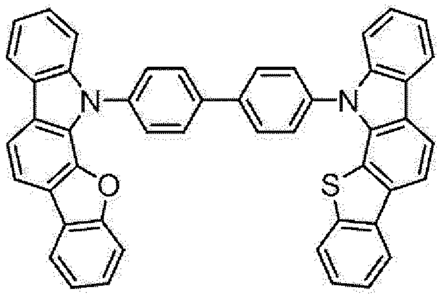

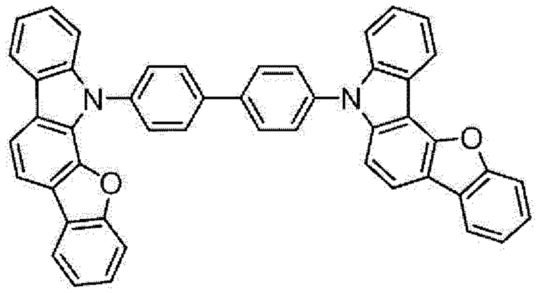

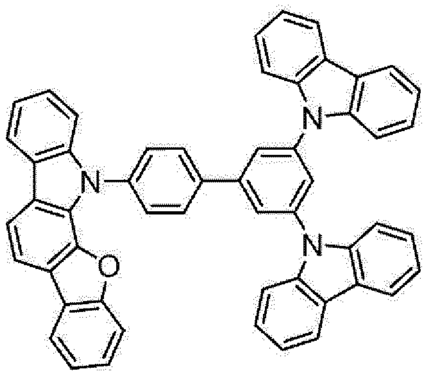

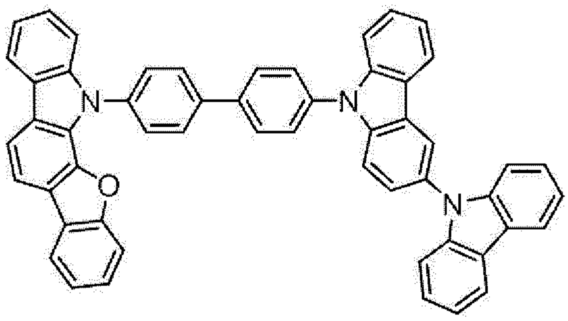

[Formula 53]

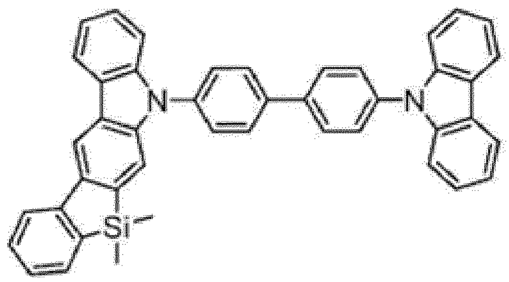

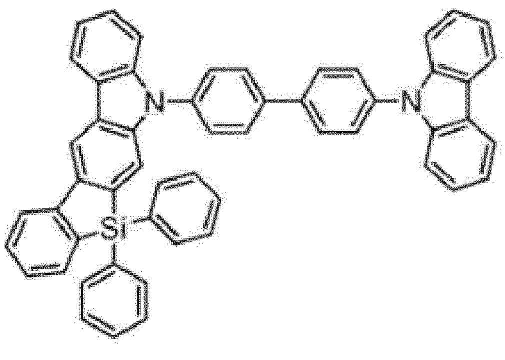

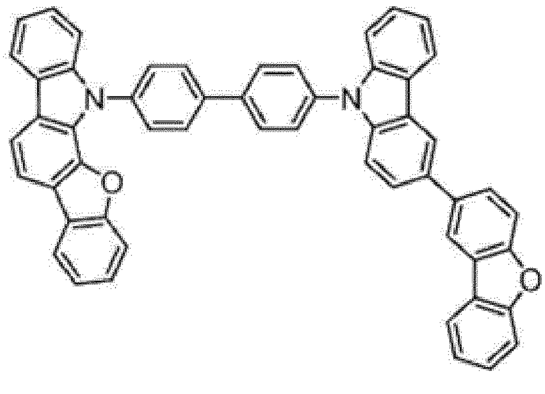

[Formula 54]

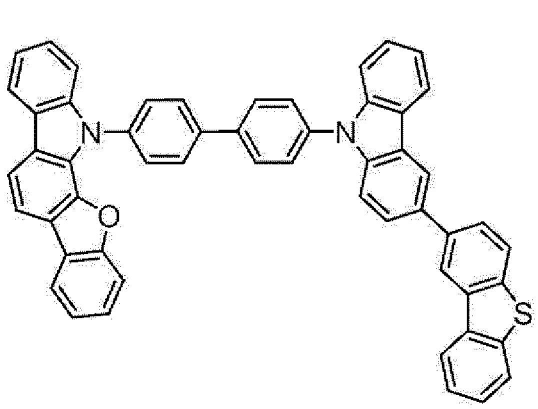

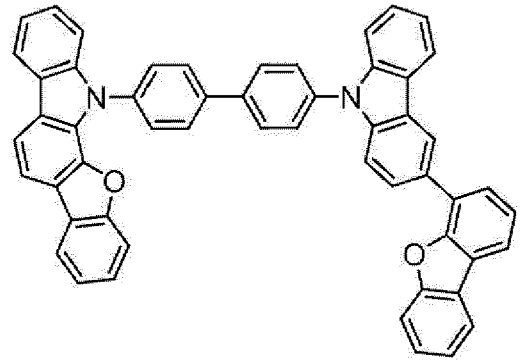

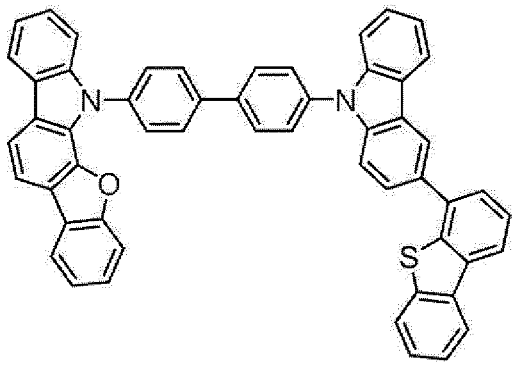

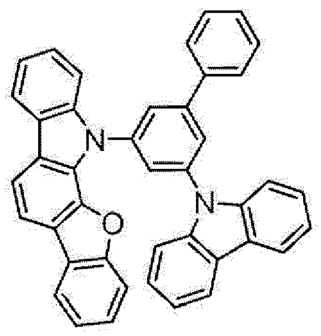

[Formula 55]

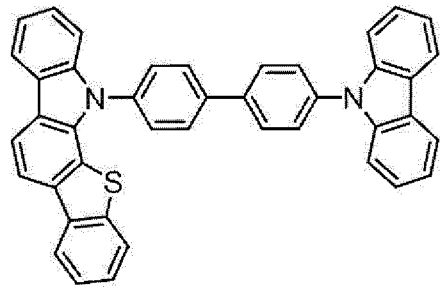

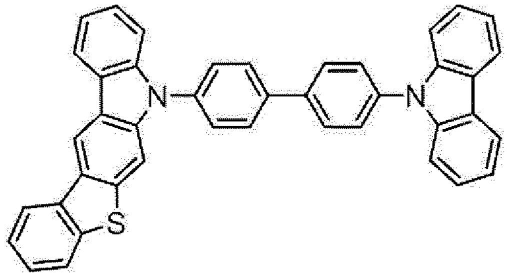

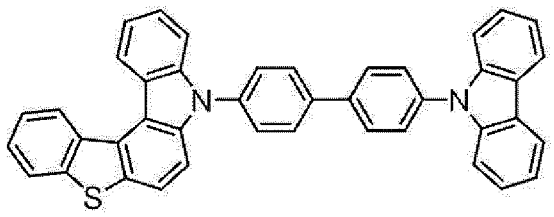

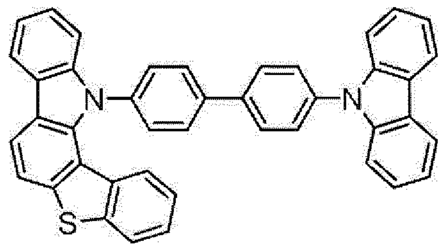

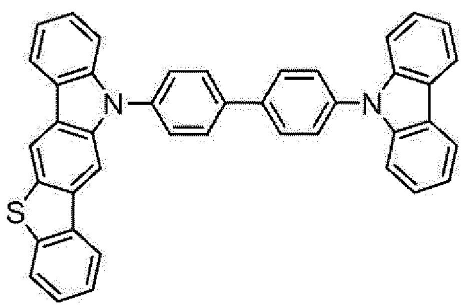

[Formula 56]

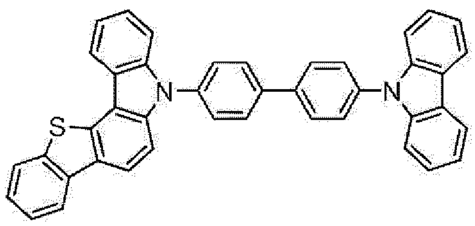

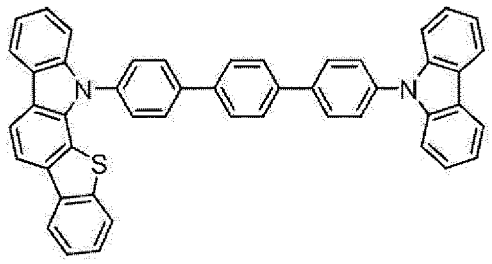

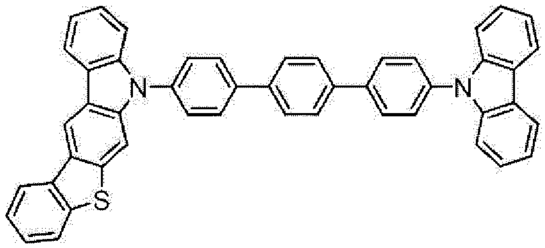

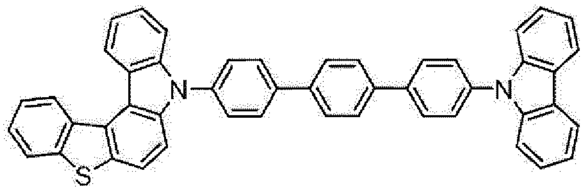

[Formula 57]

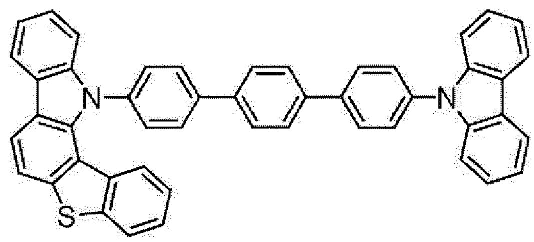

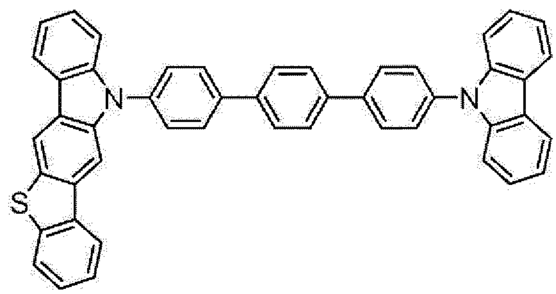

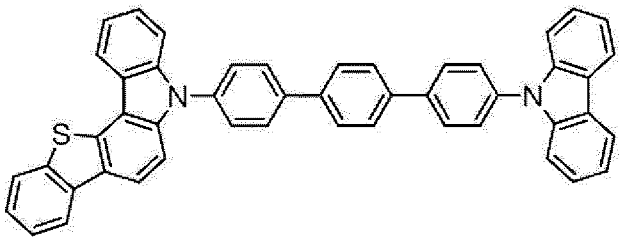

[Formula 58]

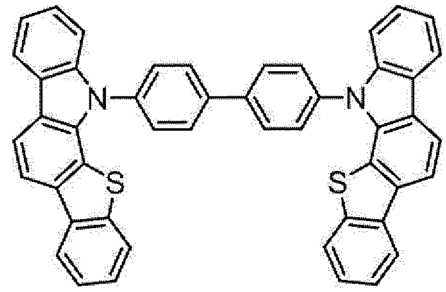

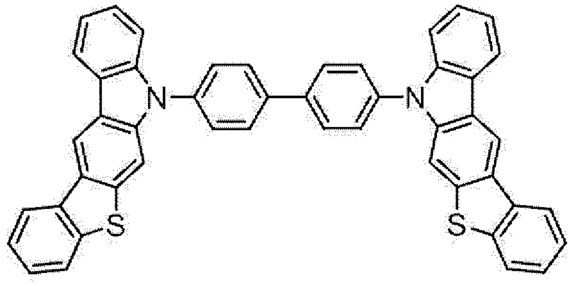

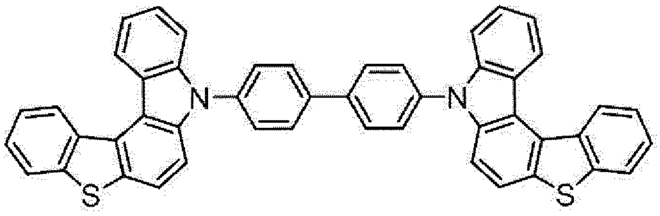

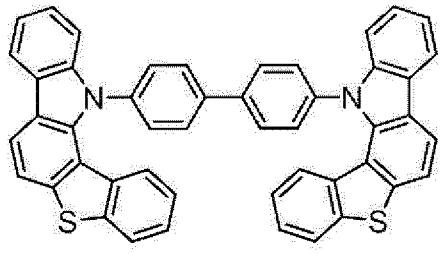

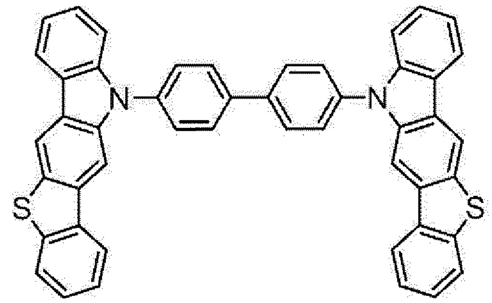

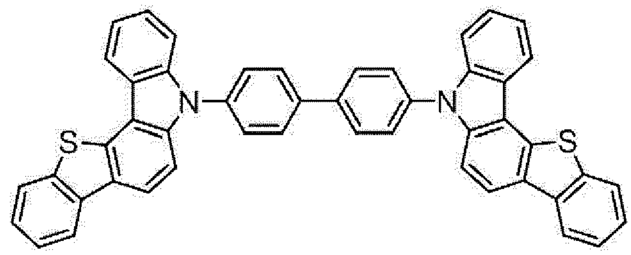

[Formula 59]

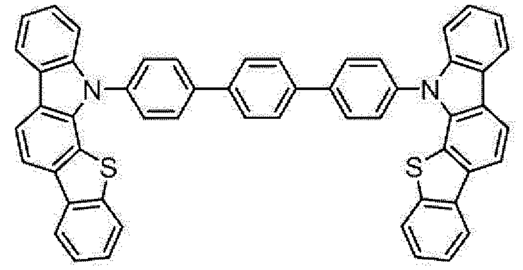

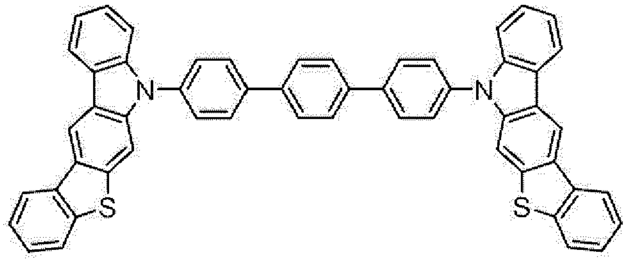

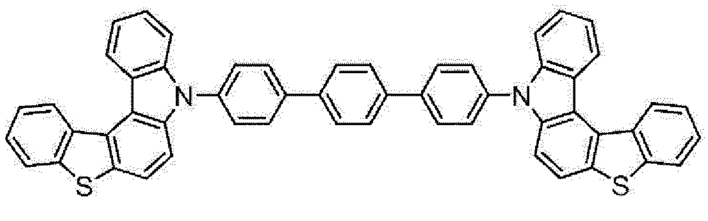

[Formula 60]

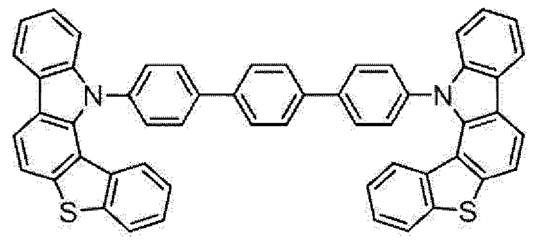

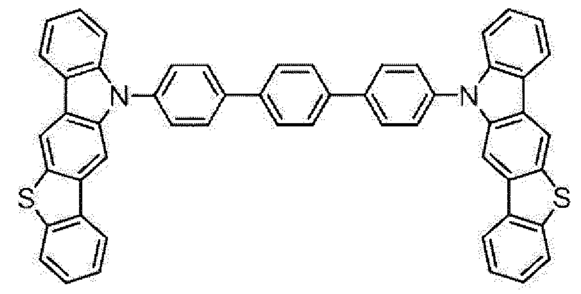

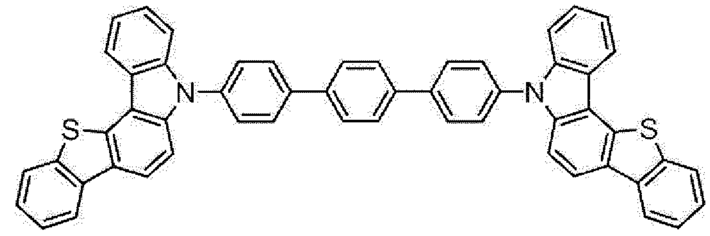

[Formula 61]

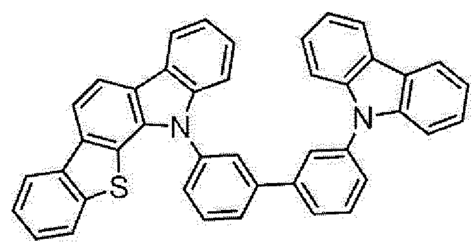

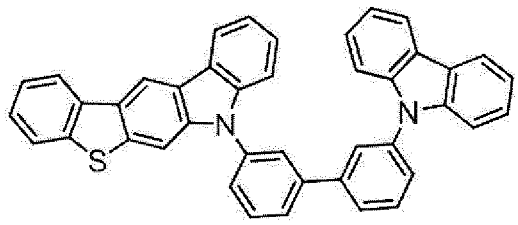

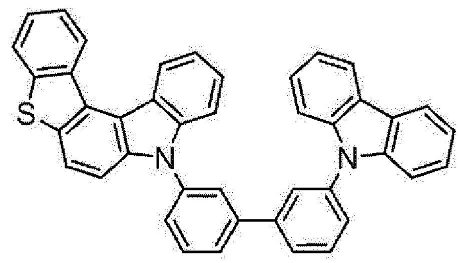

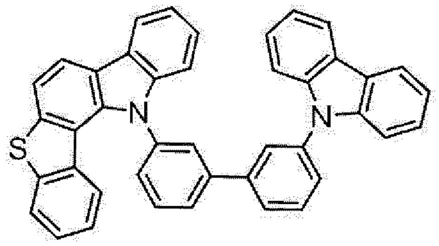

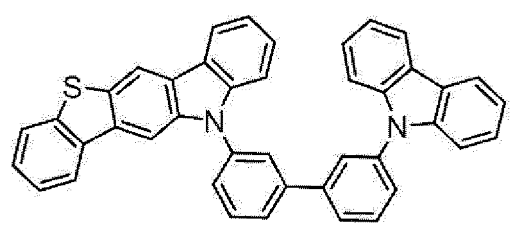

[Formula 62]

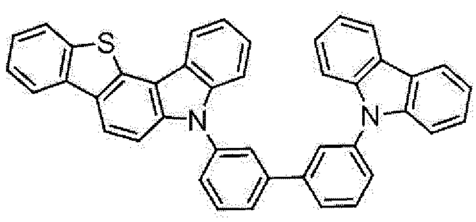

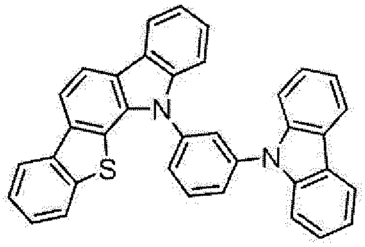

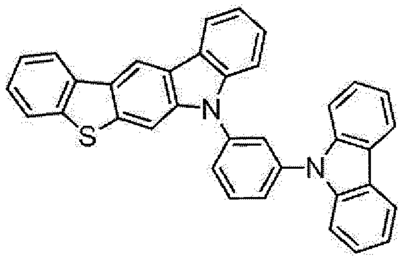

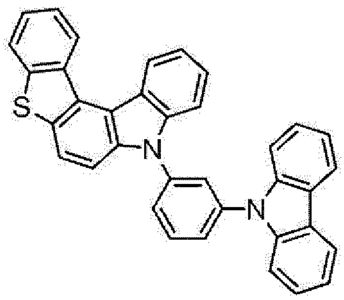

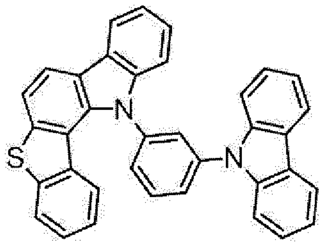

[Formula 63]

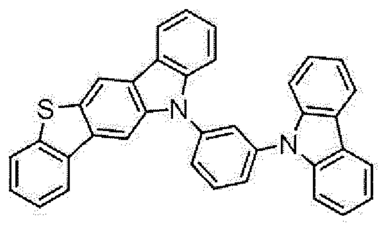

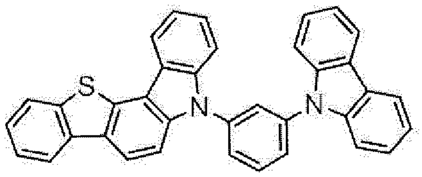

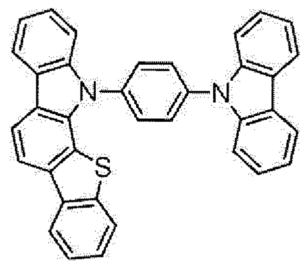

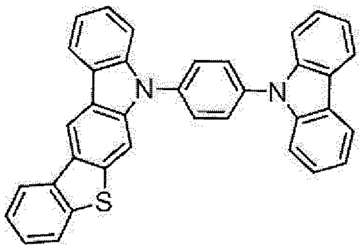

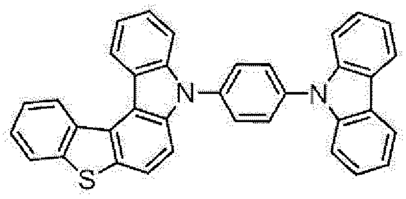

[Formula 64]

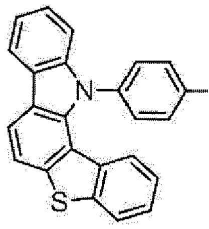
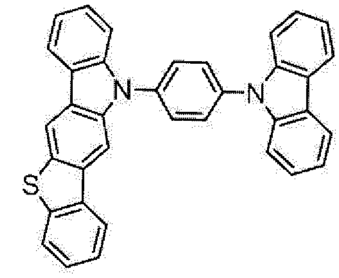
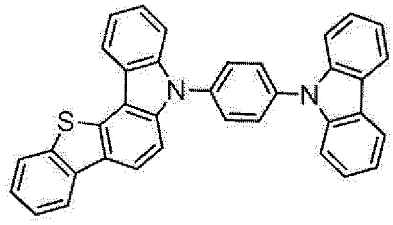
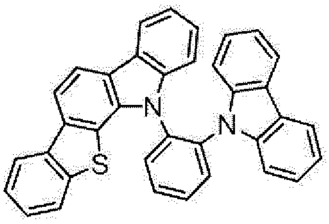
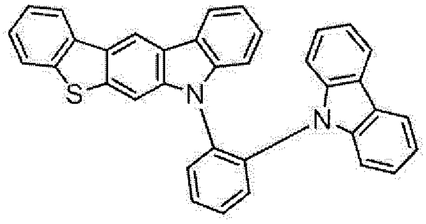
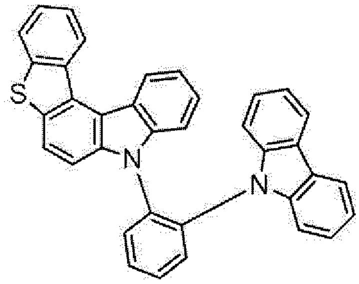

[Formula 65]

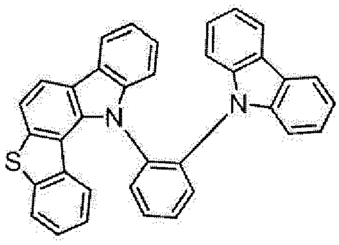
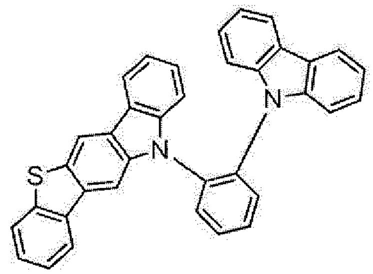
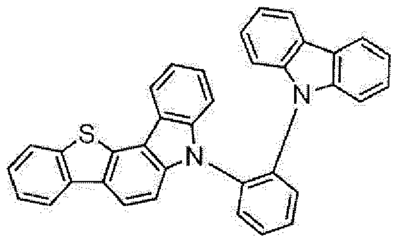

[Formula 66]

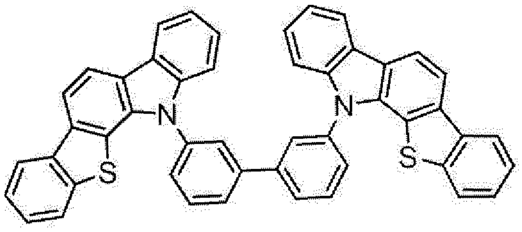
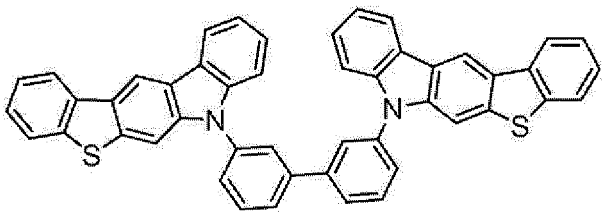
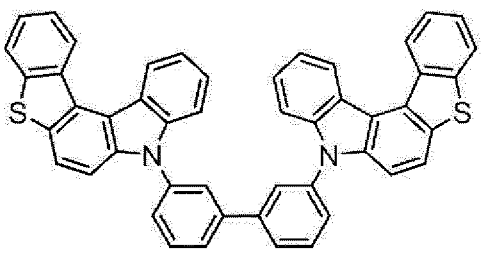
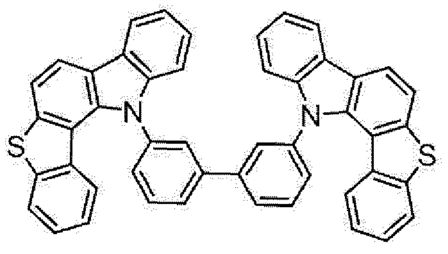

[Formula 67]

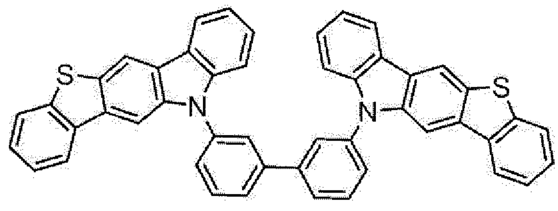

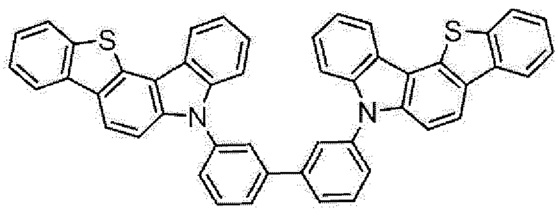

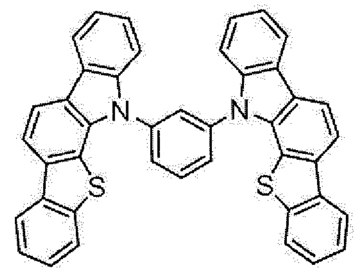

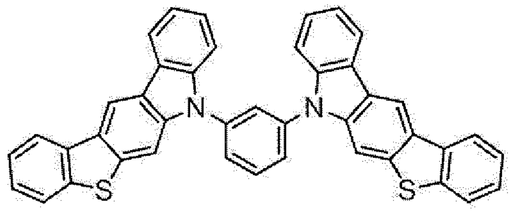

[Formula 68]

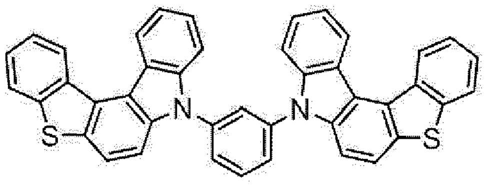

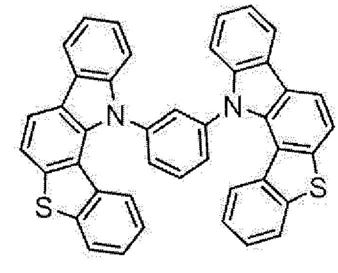

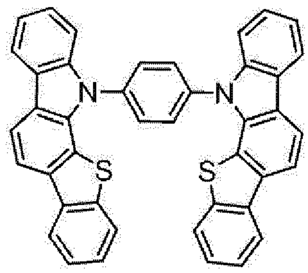

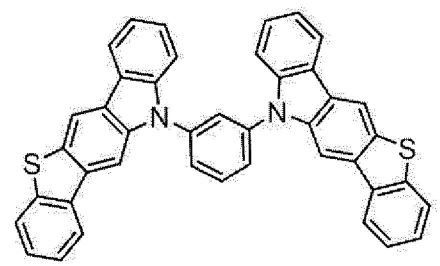

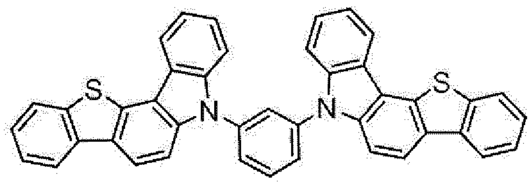

[Formula 69]

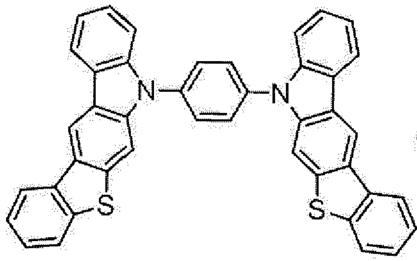
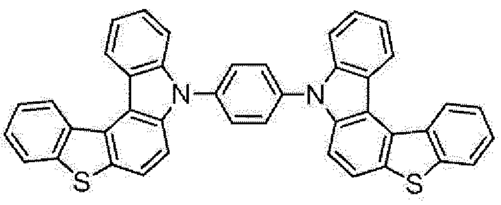
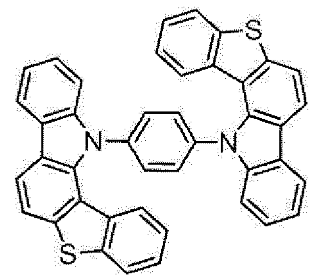
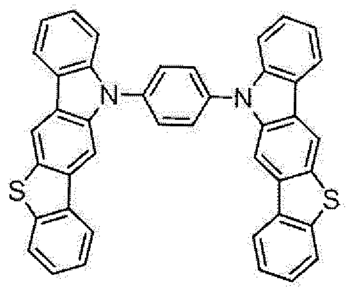
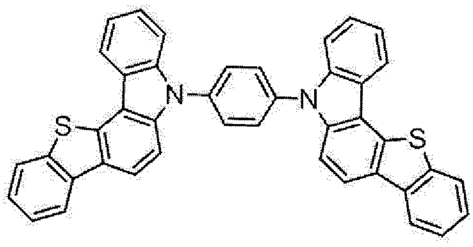

[Formula 70]

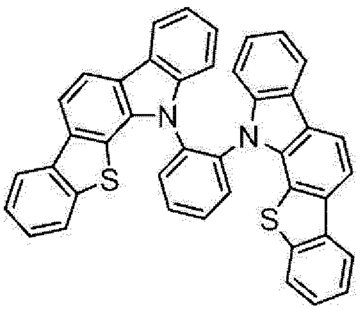
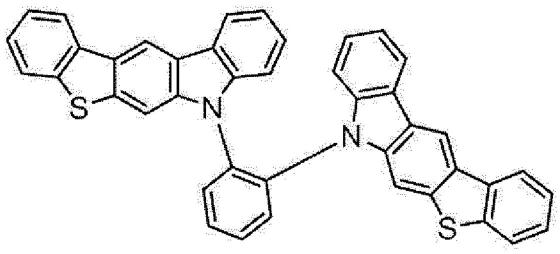
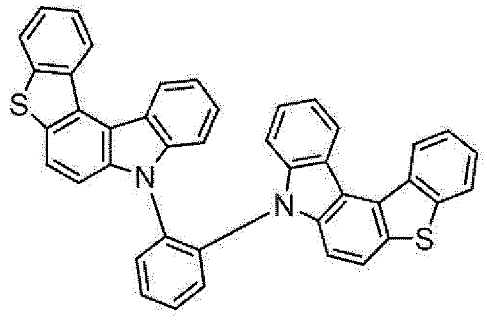
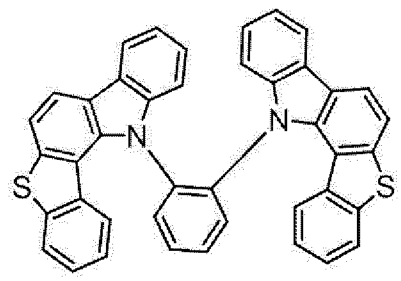
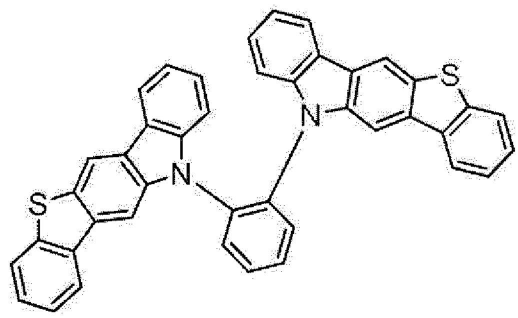
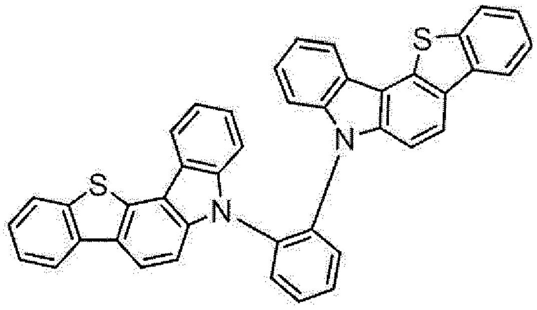

[Formula 71]

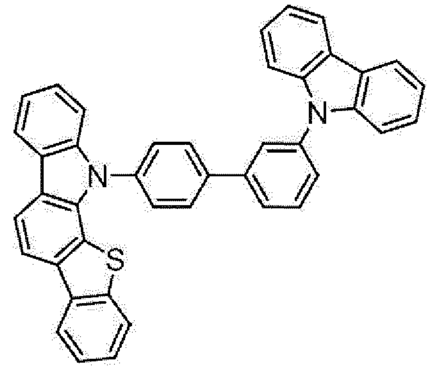

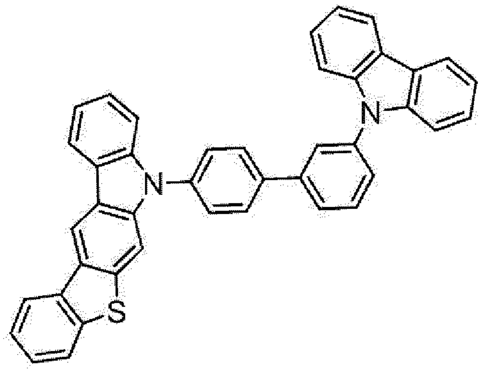

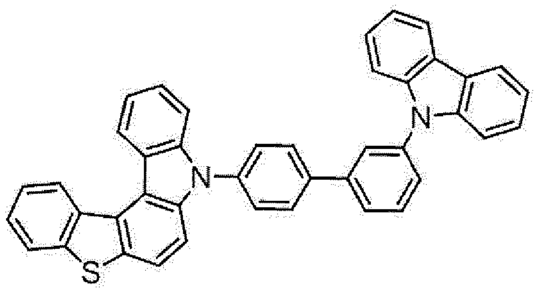

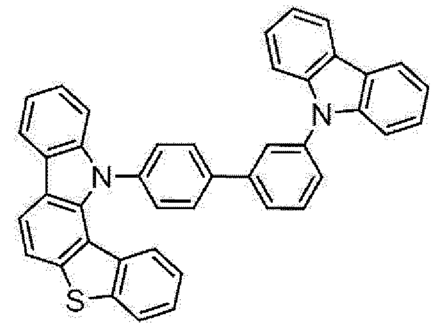

[Formula 72]

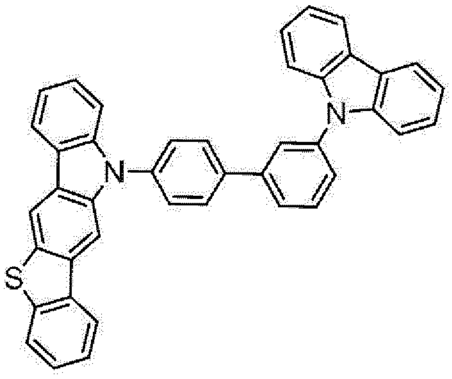

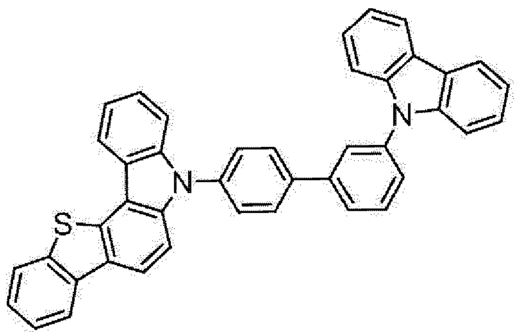

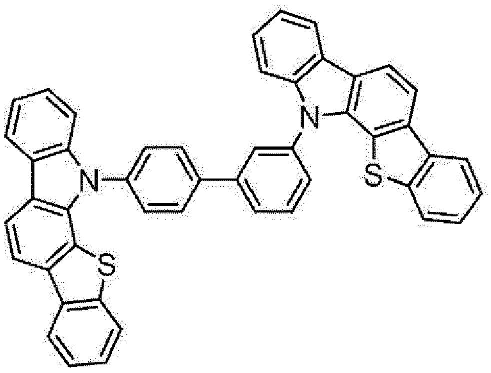

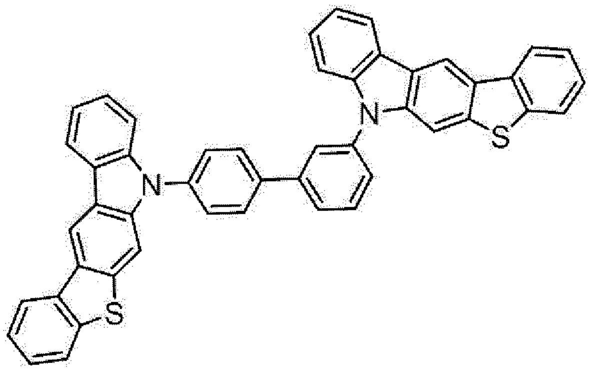

[Formula 73]

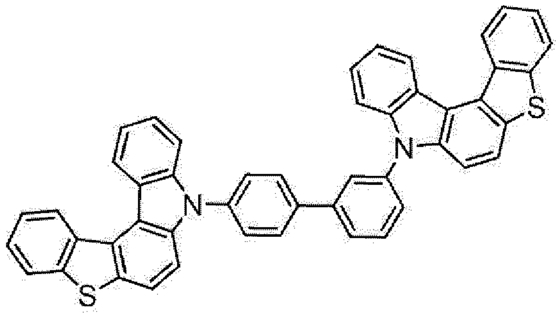

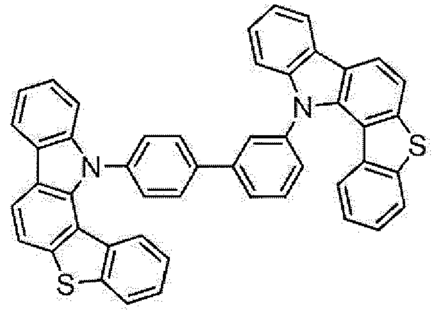

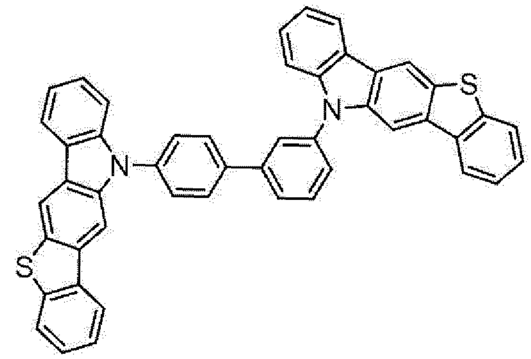

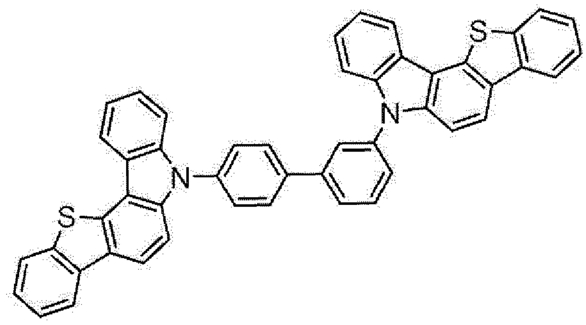

[Formula 74]

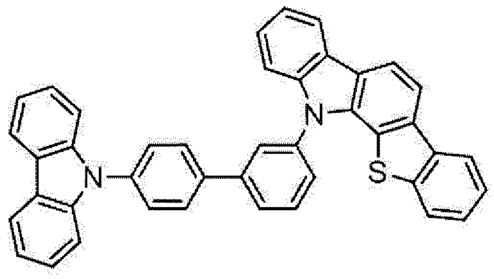

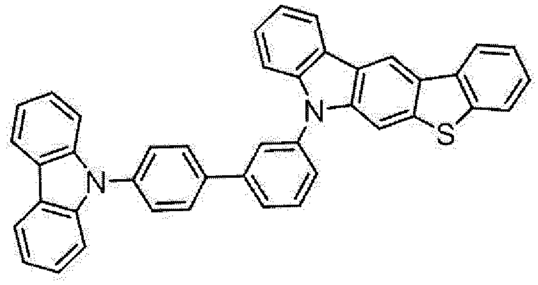

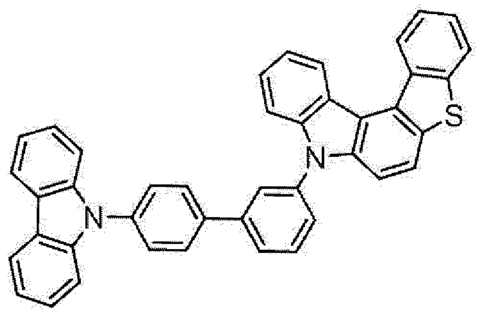

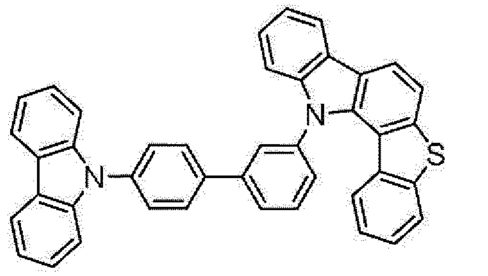

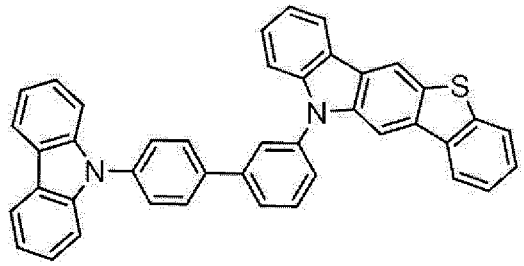

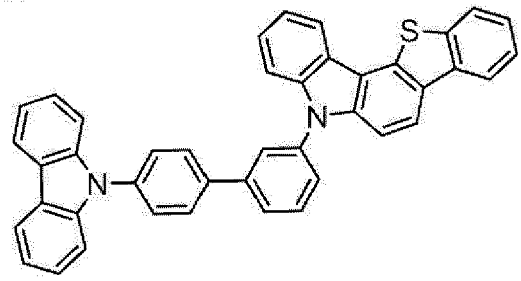

[Formula 75]

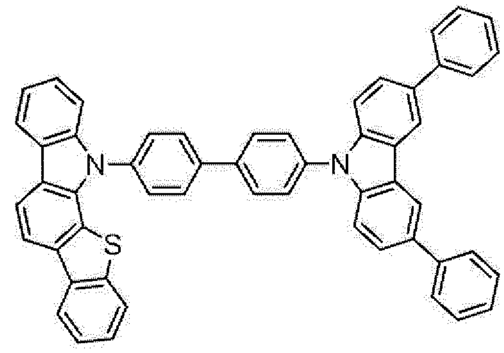

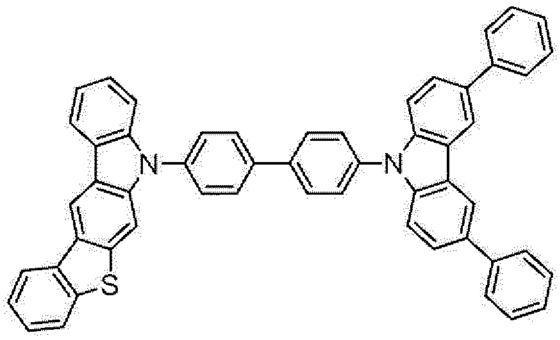

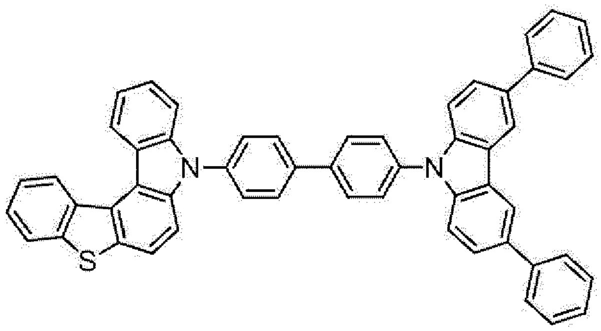

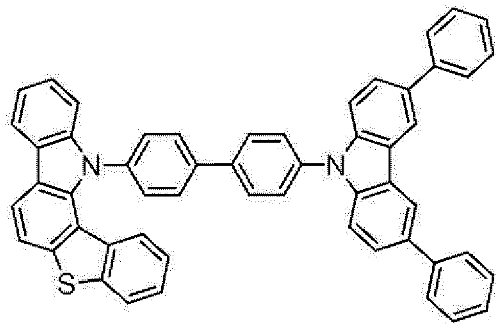

[Formula 76]

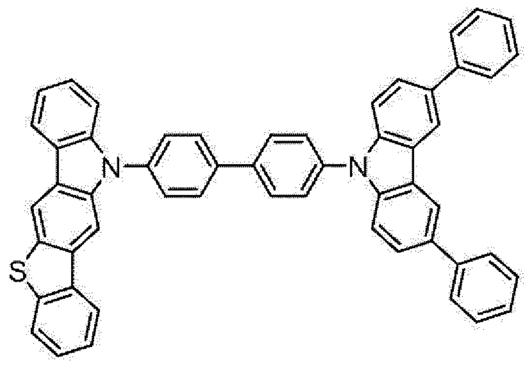

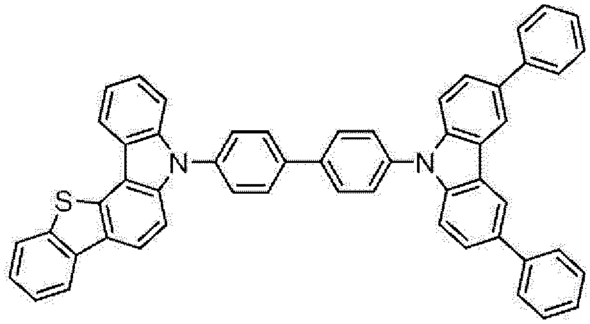

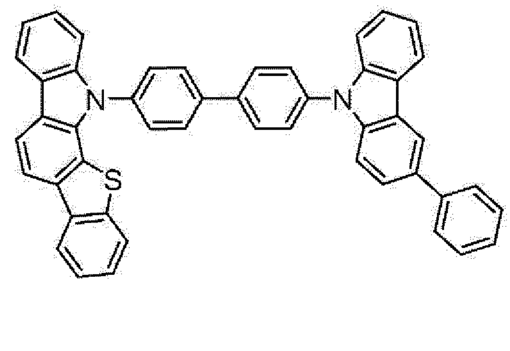

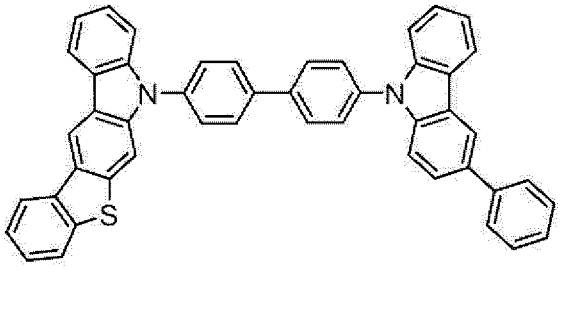

[Formula 77]

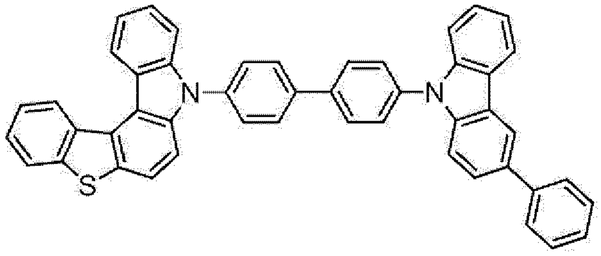

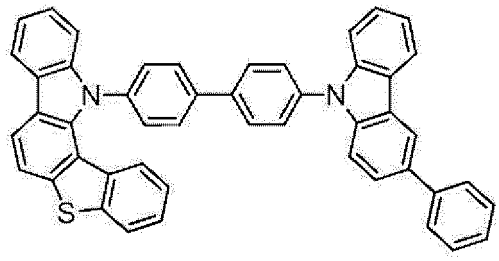

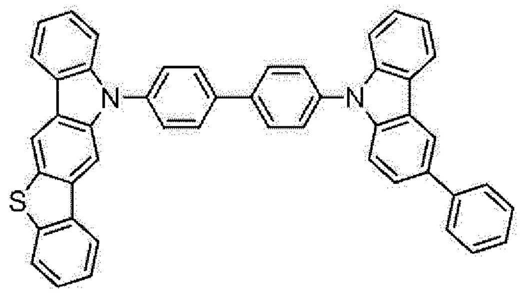

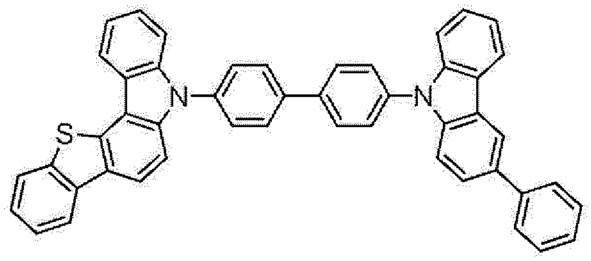

[Formula 78]

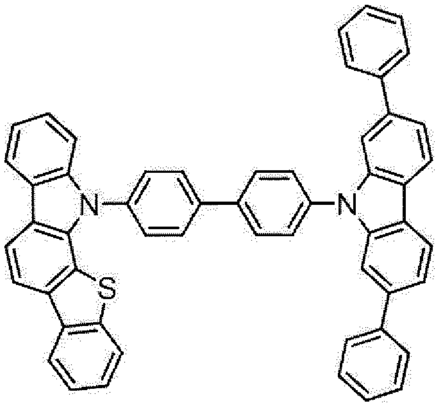

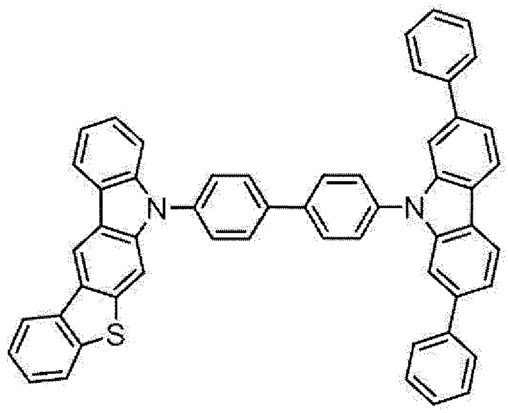

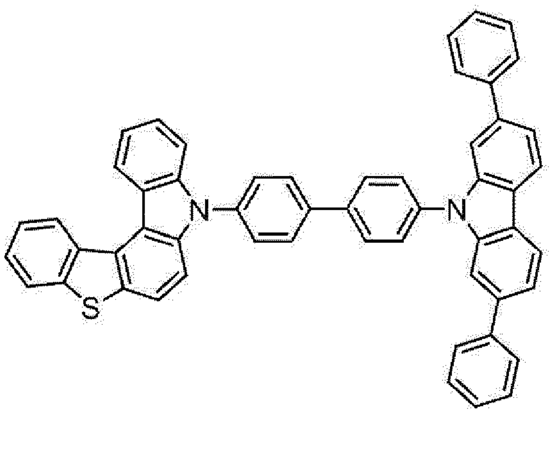

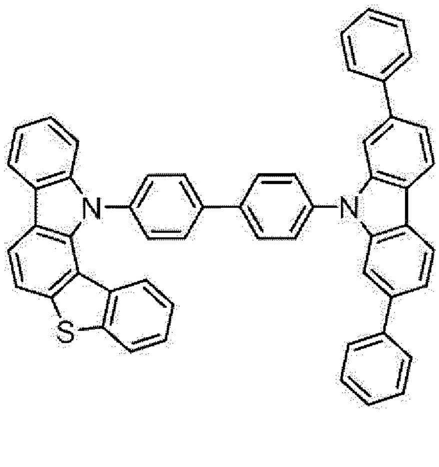

[Formula 79]

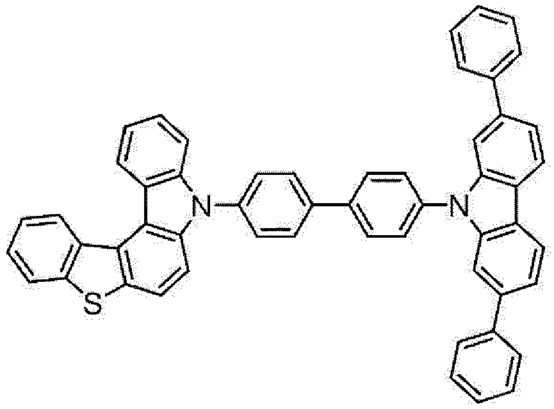

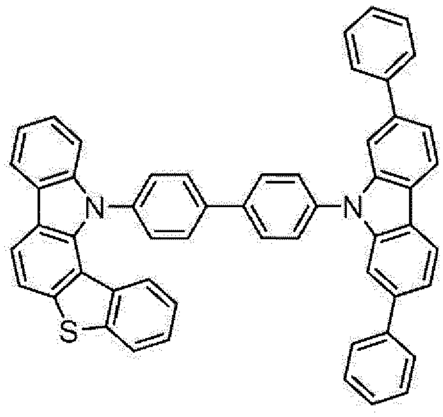

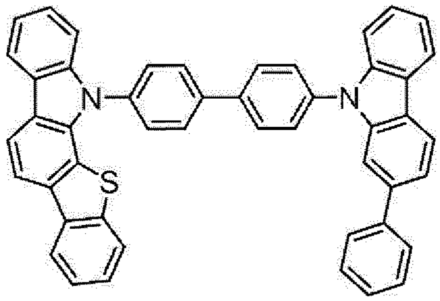

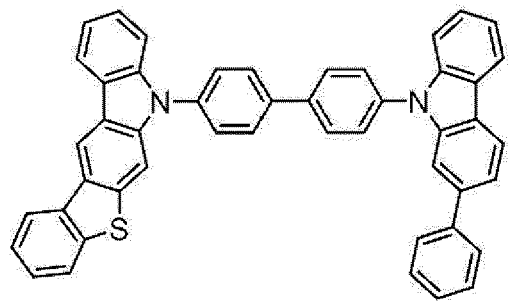

[Formula 80]

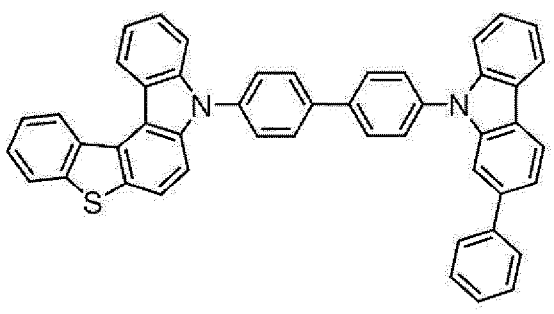

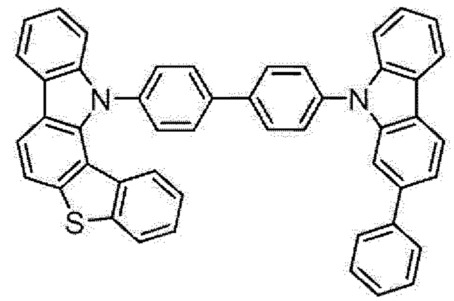

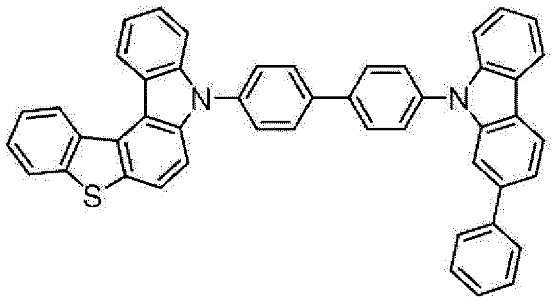

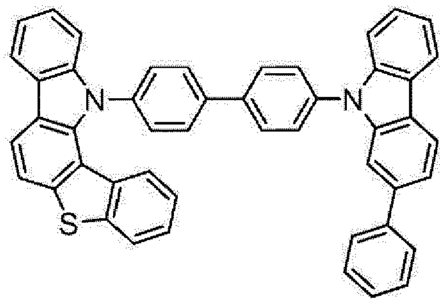

[Formula 81]

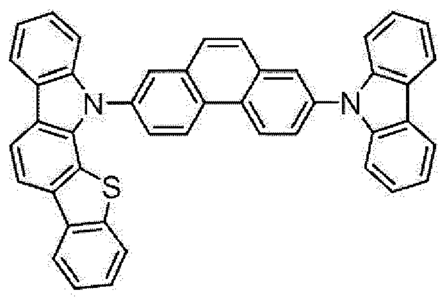

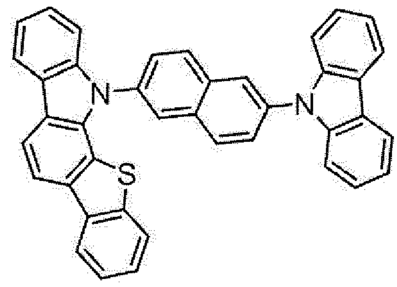

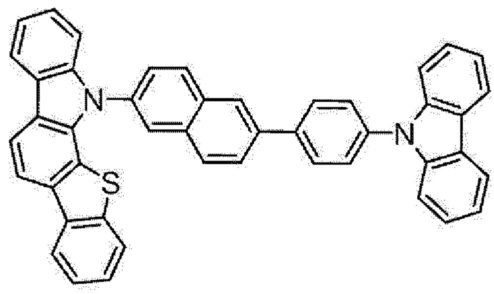

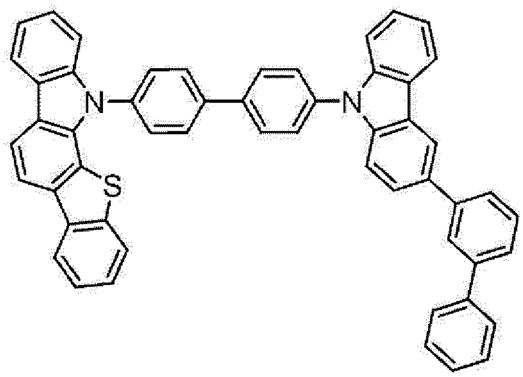

[Formula 82]

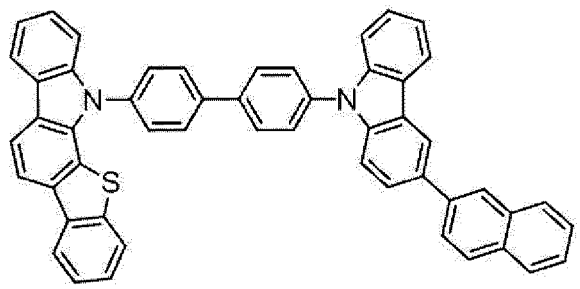

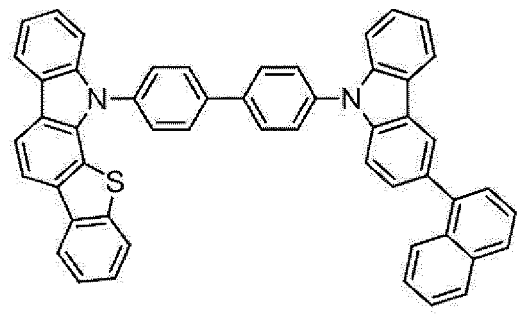

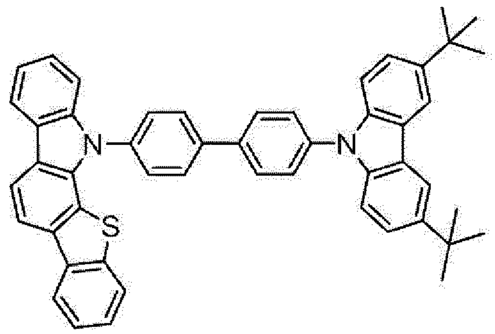

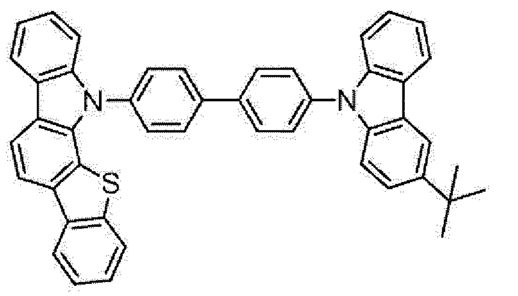

[Formula 83]

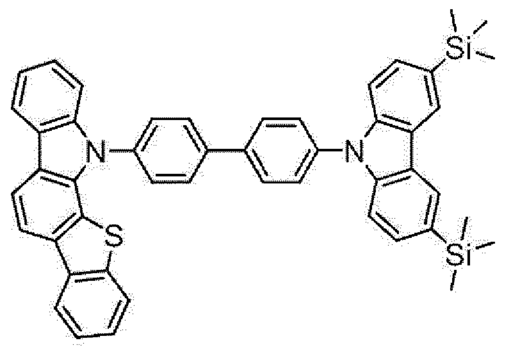

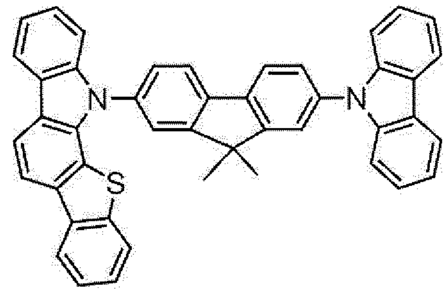

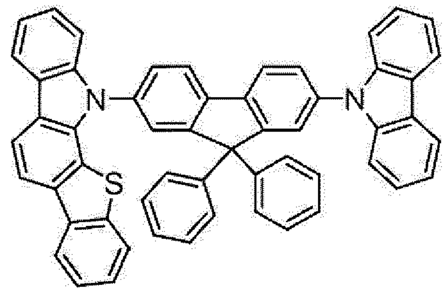

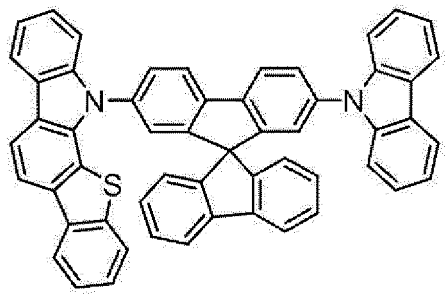

[Formula 84]

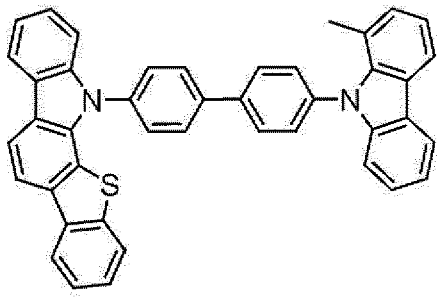

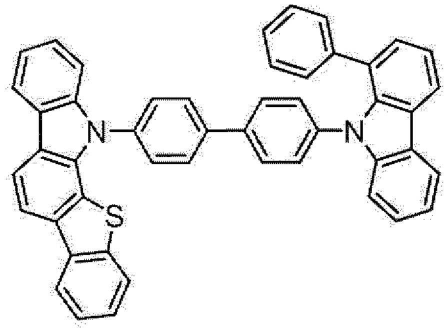

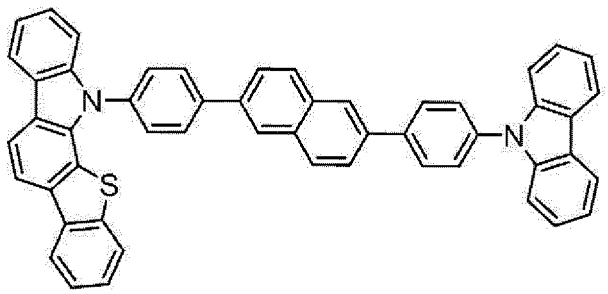

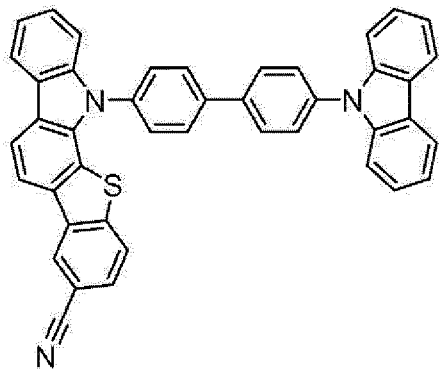

[Formula 85]

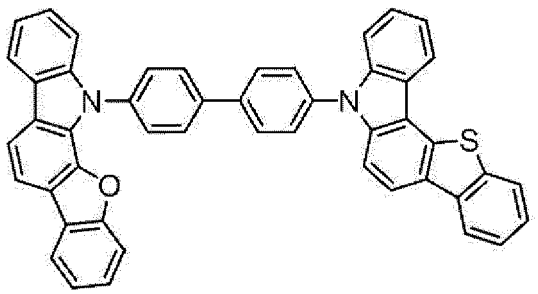

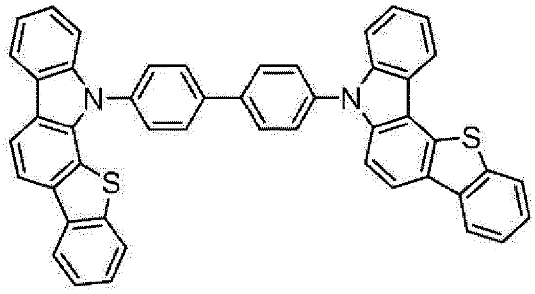

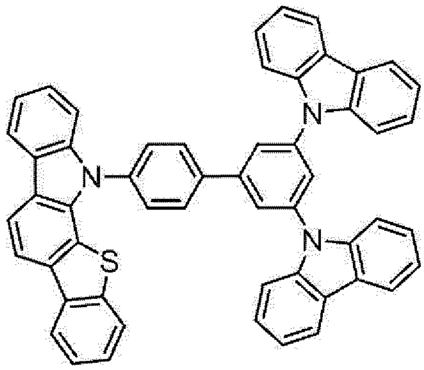

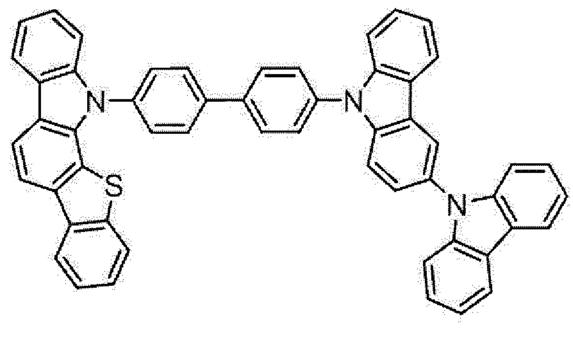

[Formula 86]

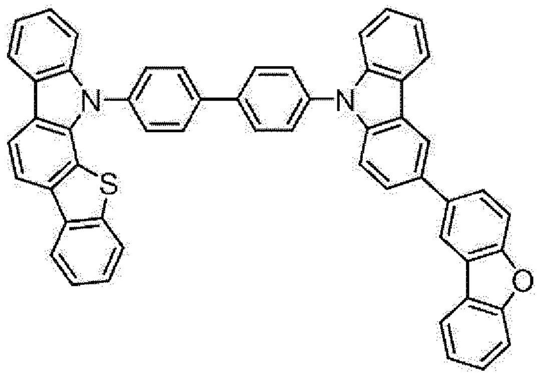

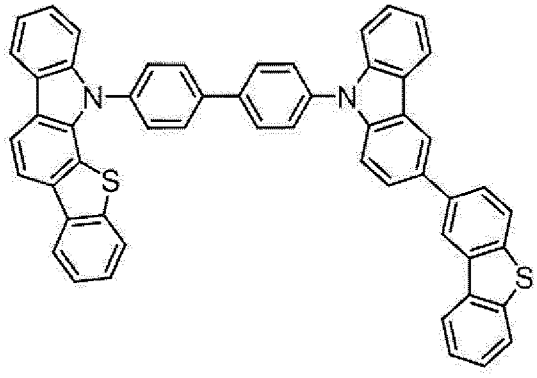

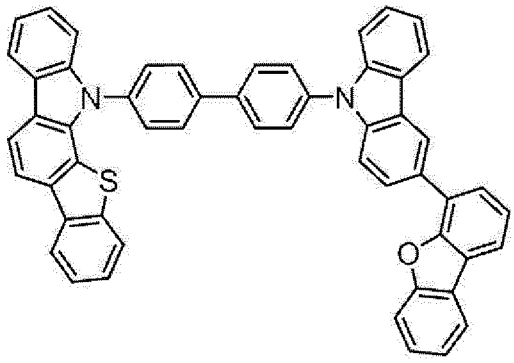

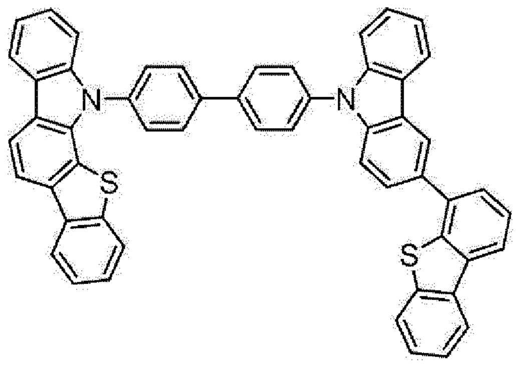

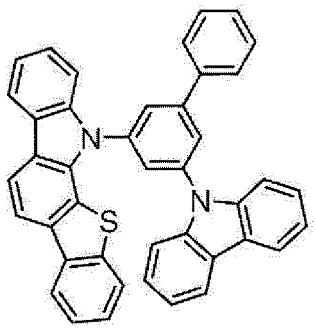

[Formula 87]

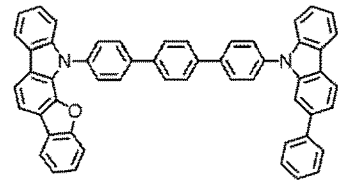 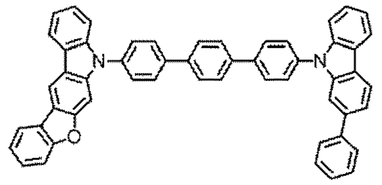 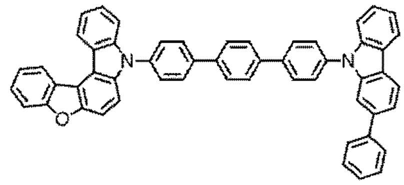

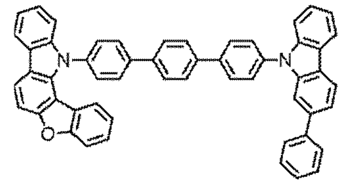 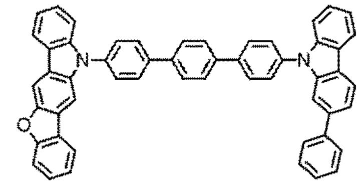 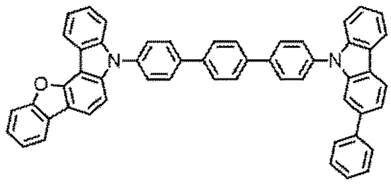

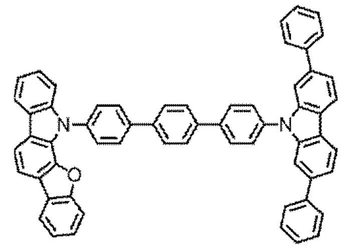 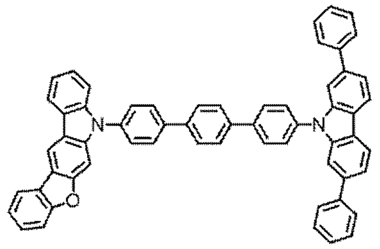 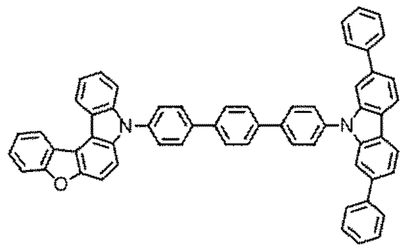

[Formula 88]

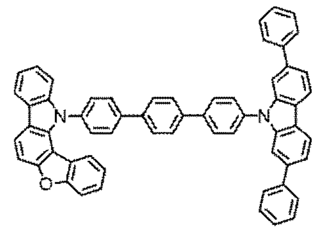 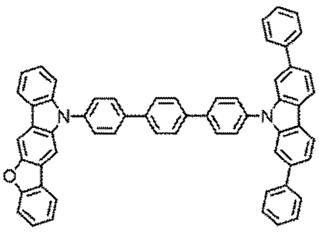 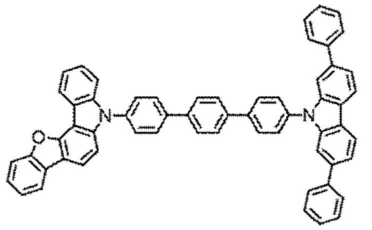

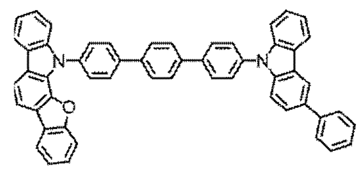 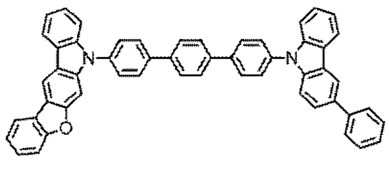 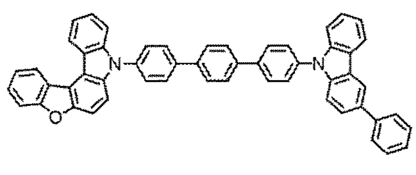

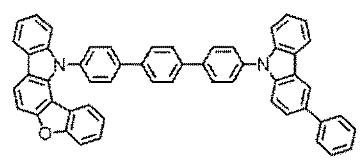 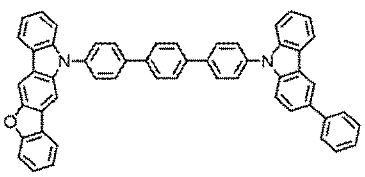 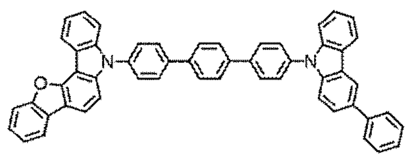

[Formula 89]

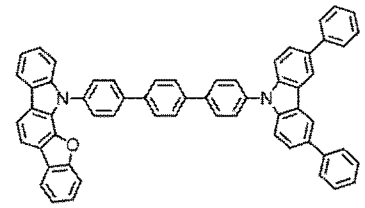
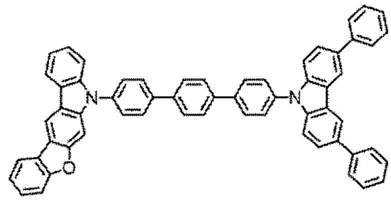
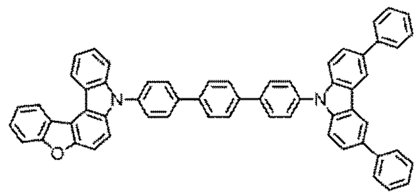
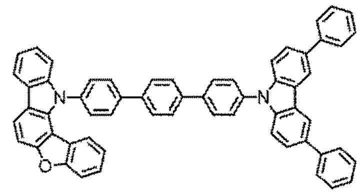
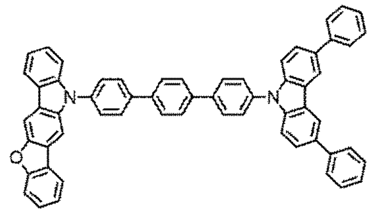
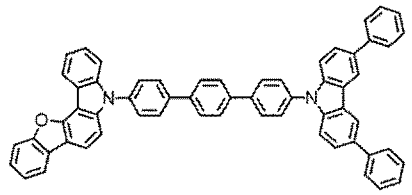

[Formula 90]

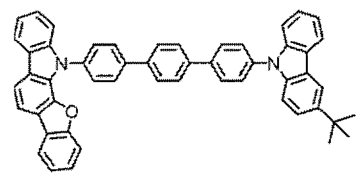
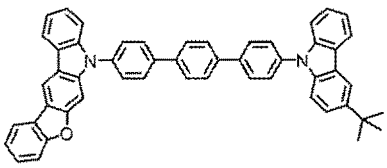
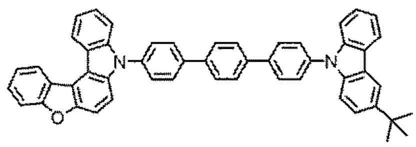
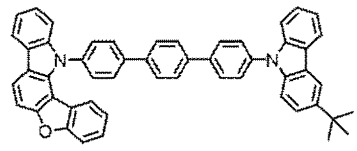
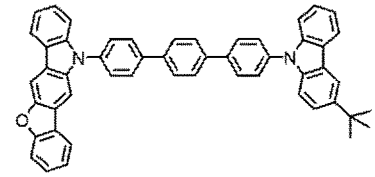
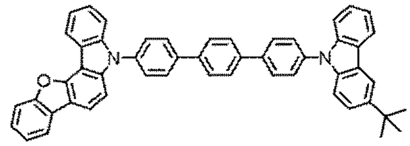
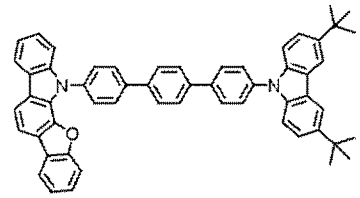
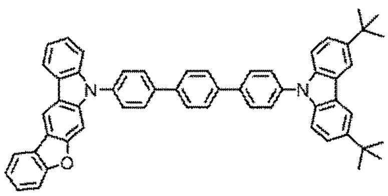
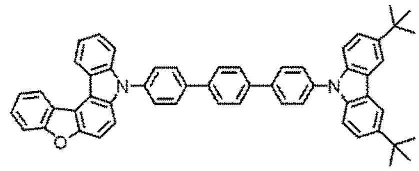

[Formula 91]

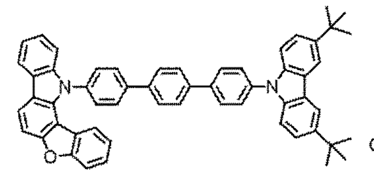
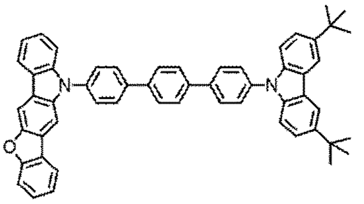
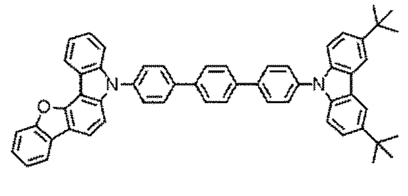
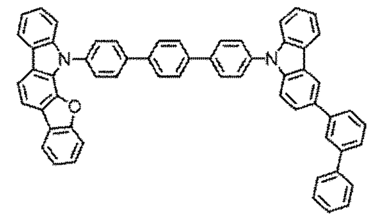
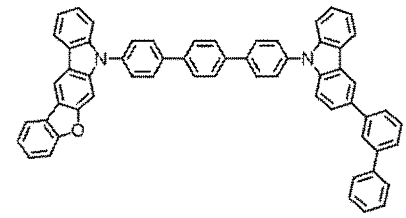
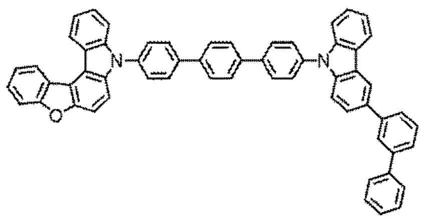
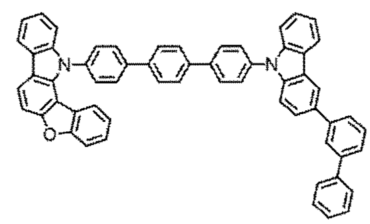
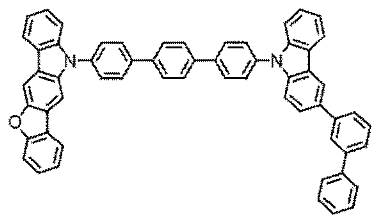
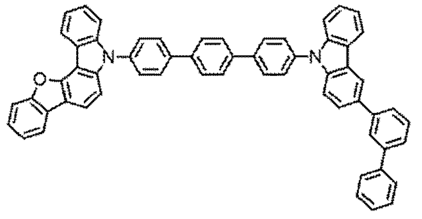

[Formula 92]

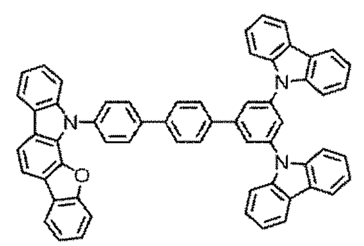
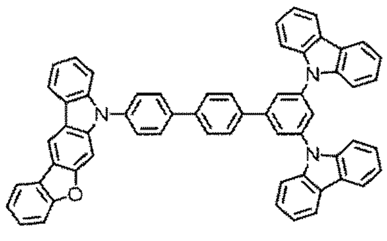
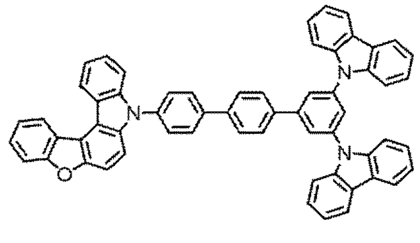
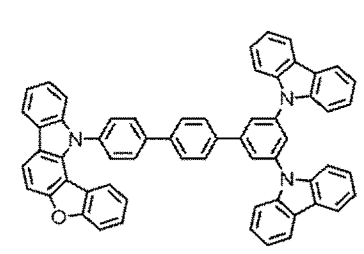
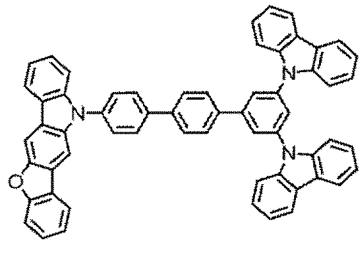
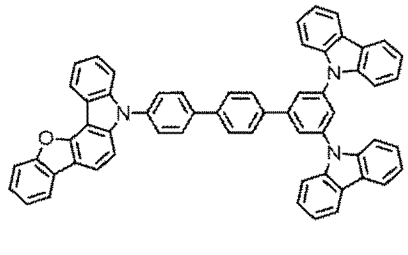

[Formula 93]

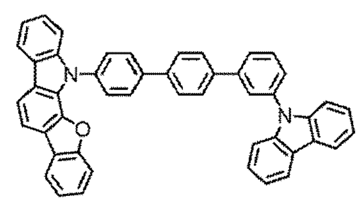
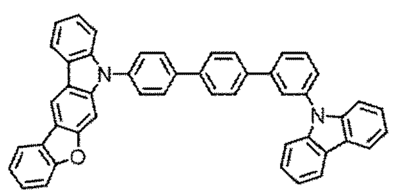
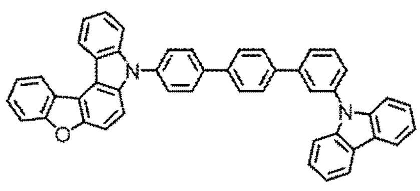
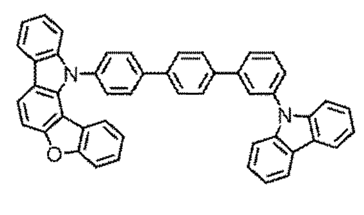
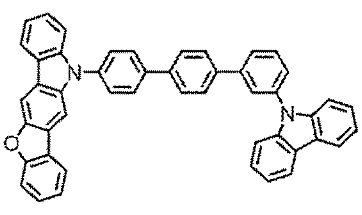
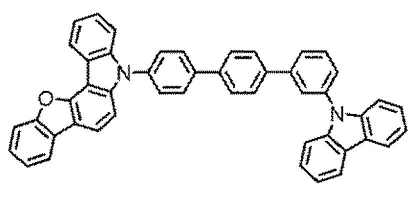

[Formula 94]

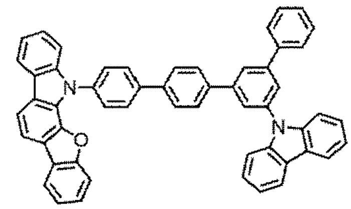
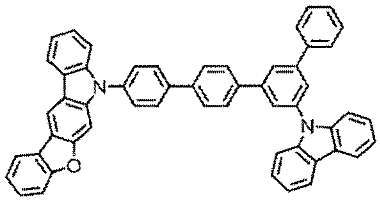
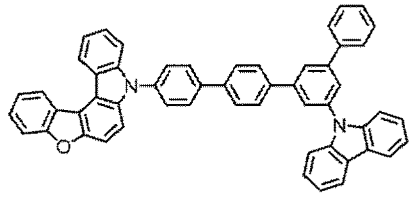
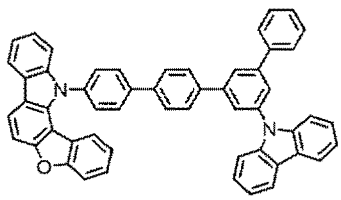
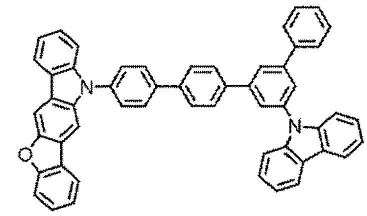
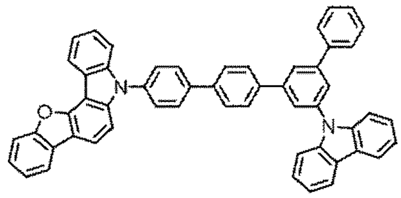

[Formula 95]

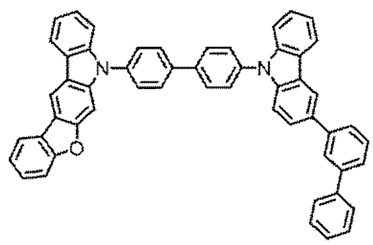
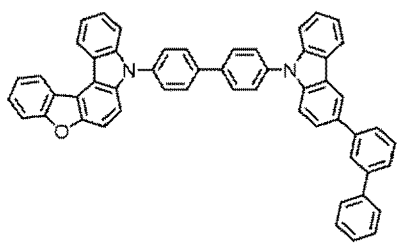
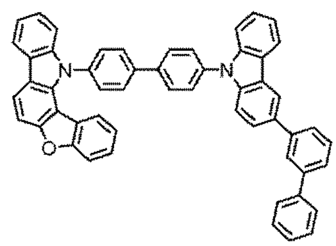
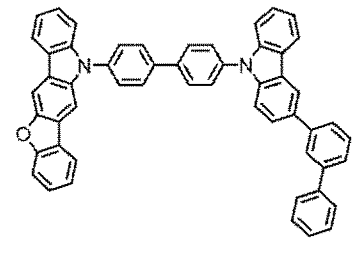
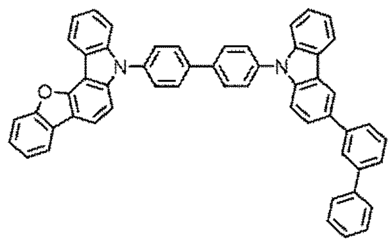
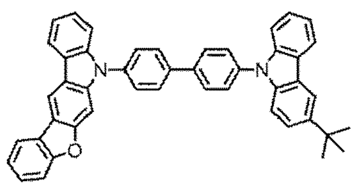
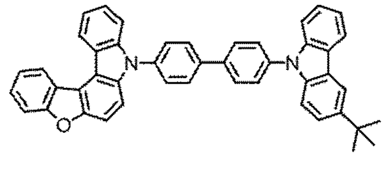
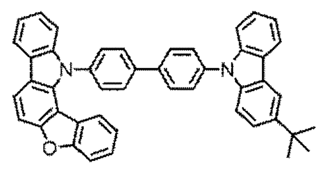

[Formula 96]

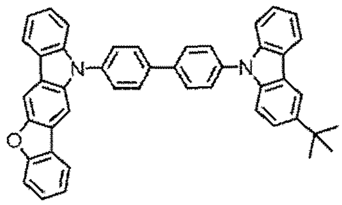
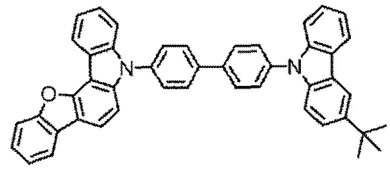
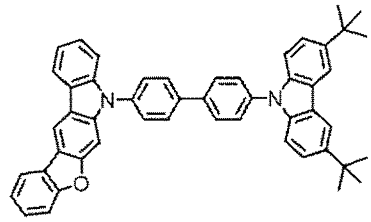
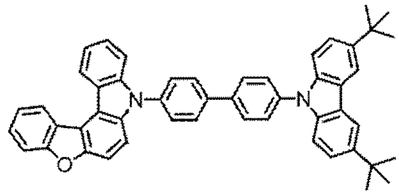
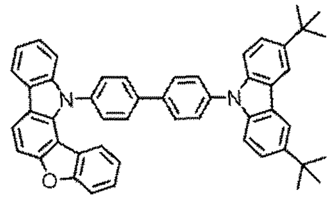
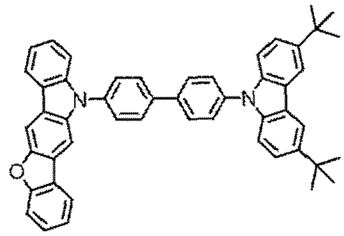
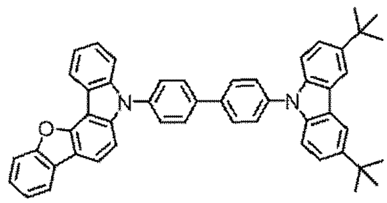
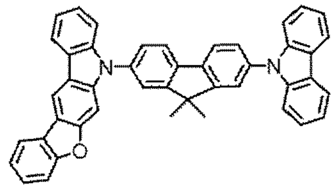

[Formula 97]

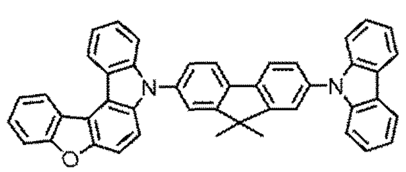
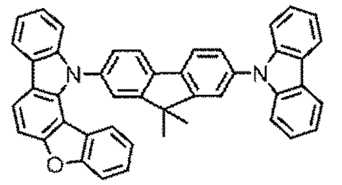
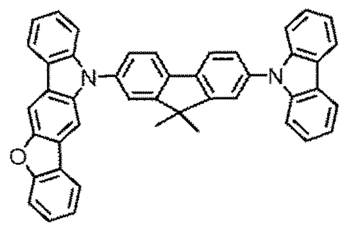
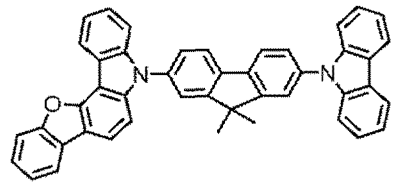
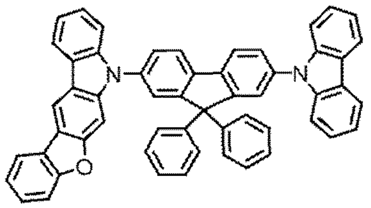
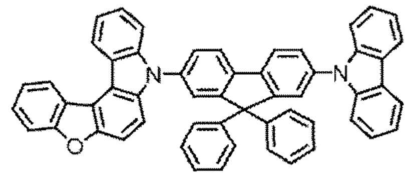
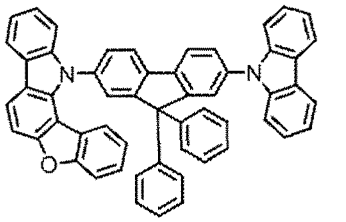
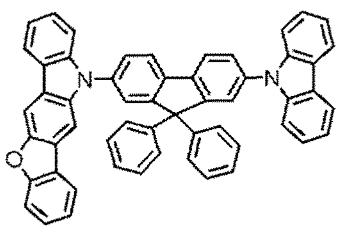

[Formula 98]

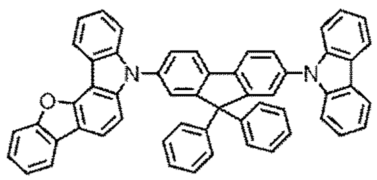
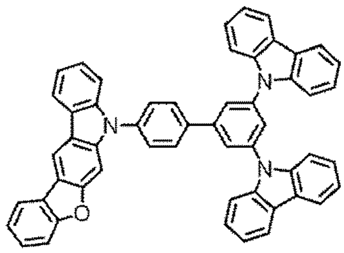
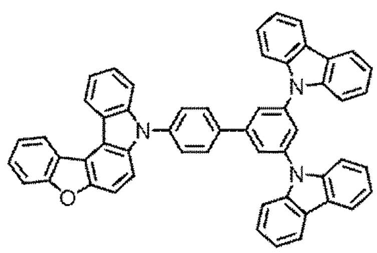
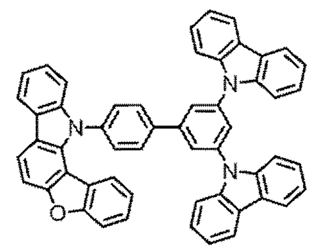
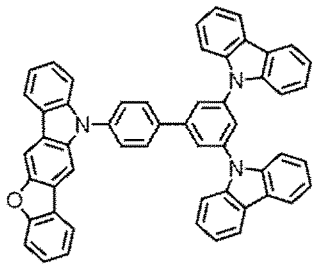
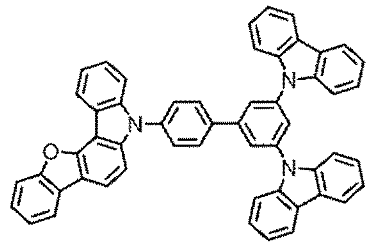

[Formula 99]

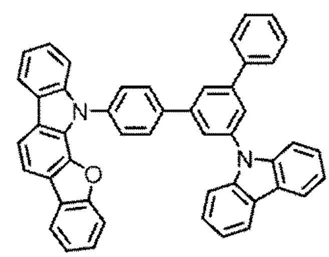
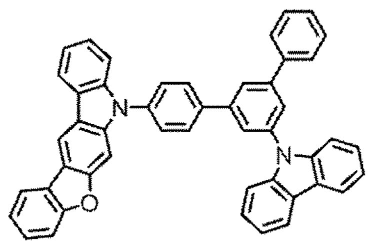
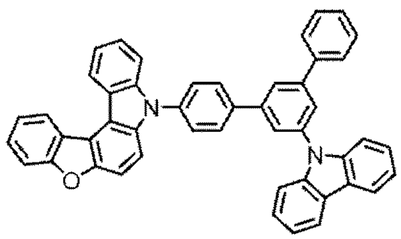
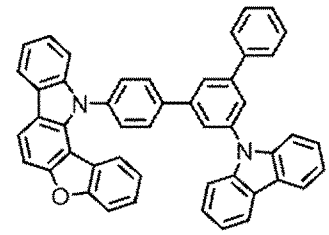
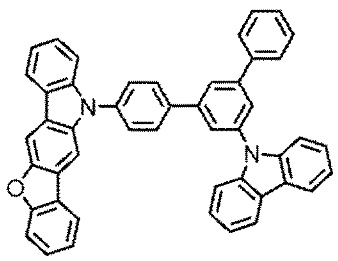

[Formula 100]

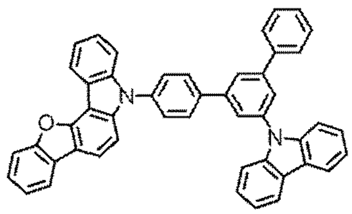
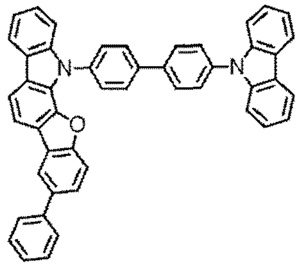
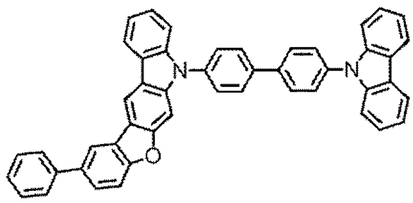
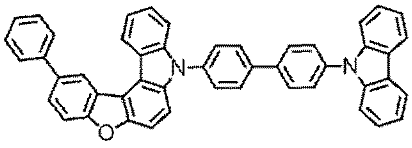
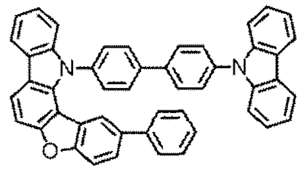
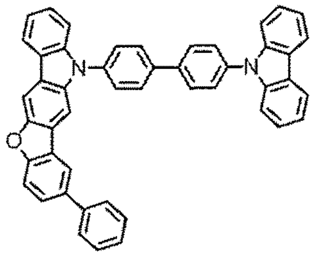
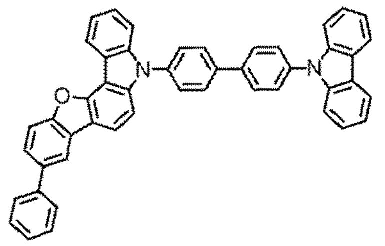
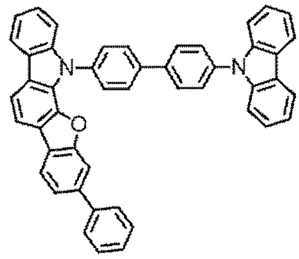

[Formula 101]

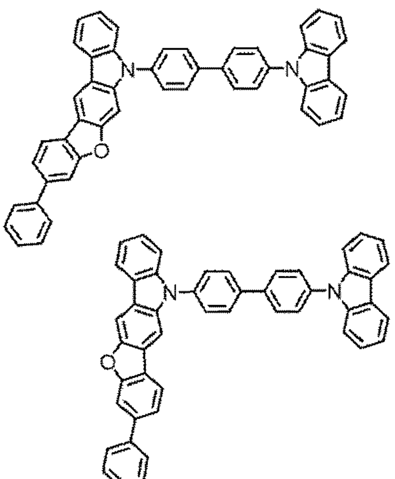
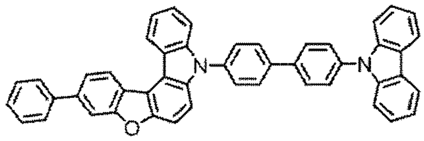
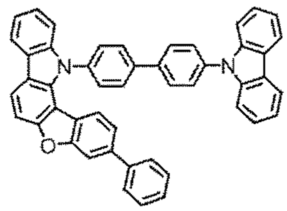
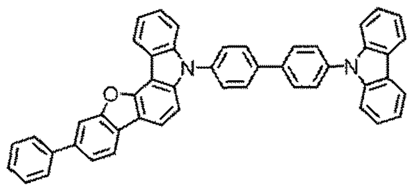
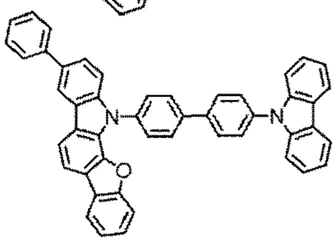
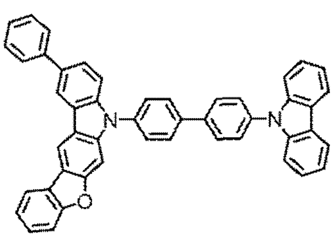
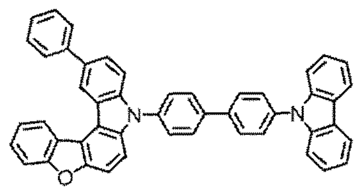

[Formula 102]

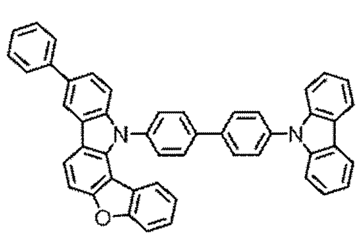
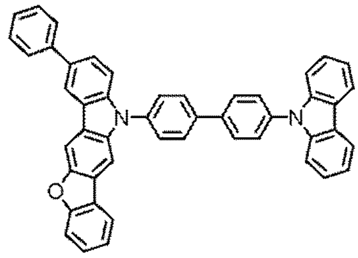
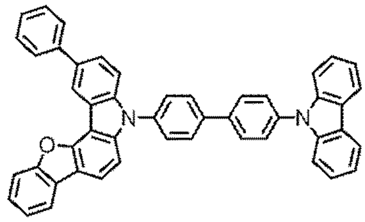
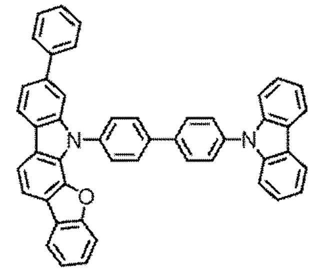
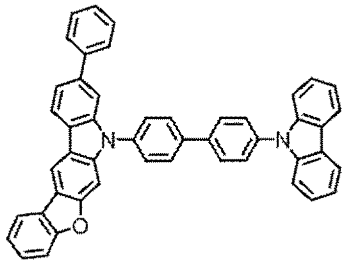
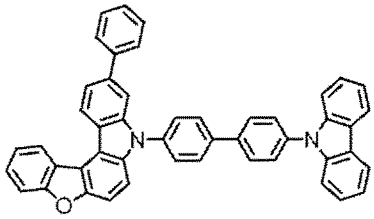
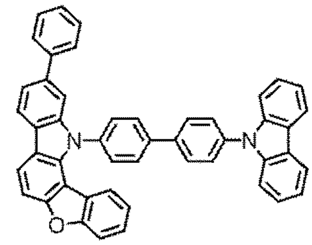
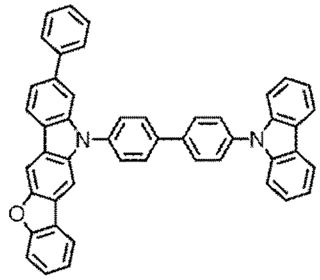
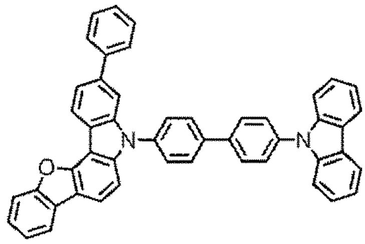

[Formula 103]

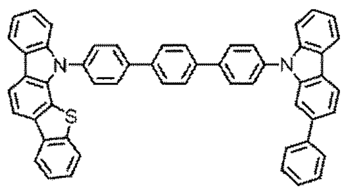
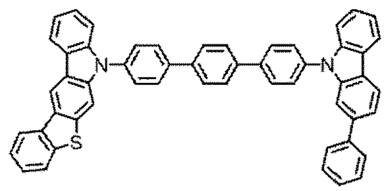
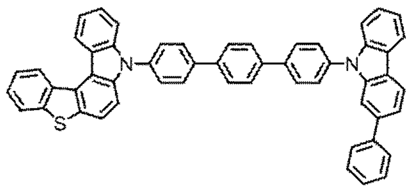
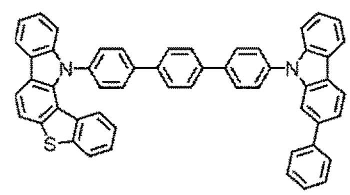
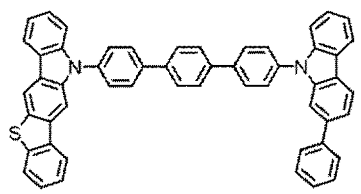
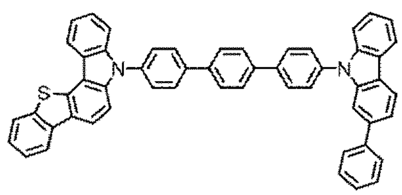

[Formula 104]

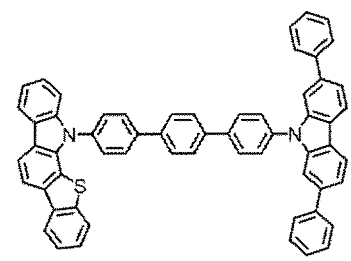 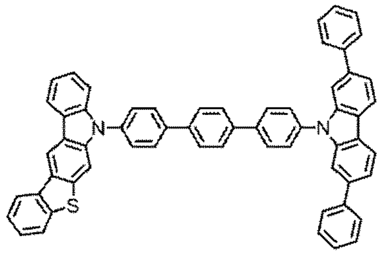 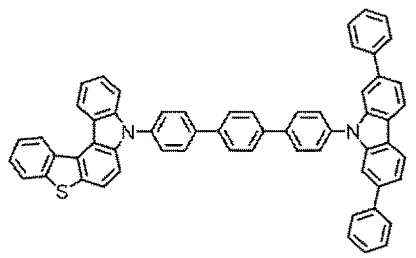

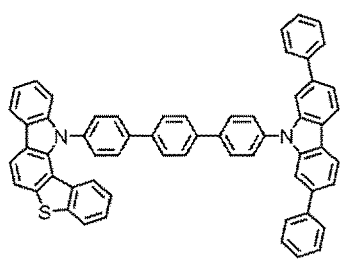 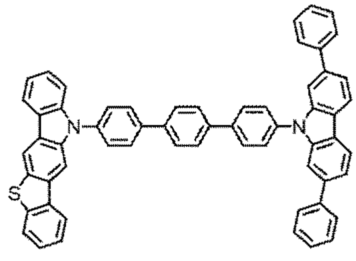 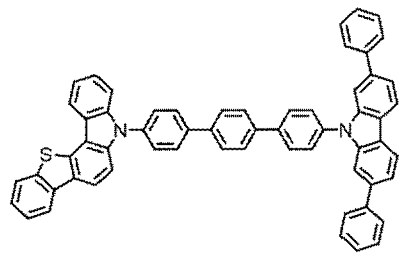

[Formula 105]

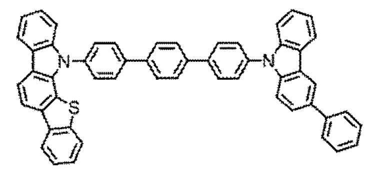 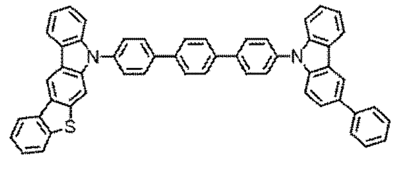 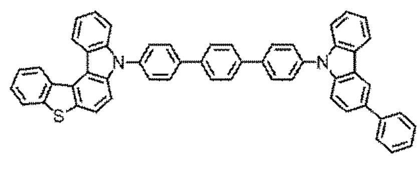

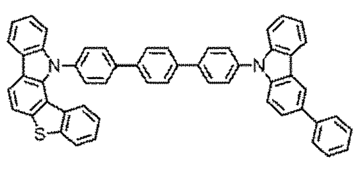 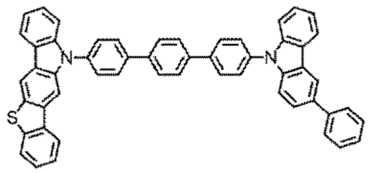 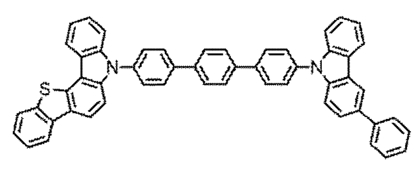

[Formula 106]

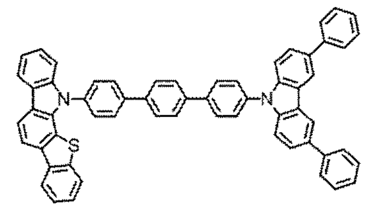 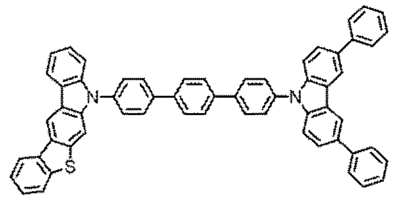 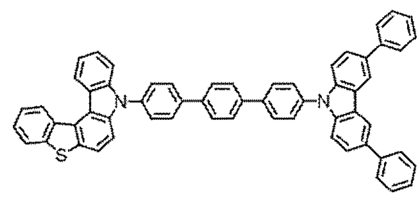

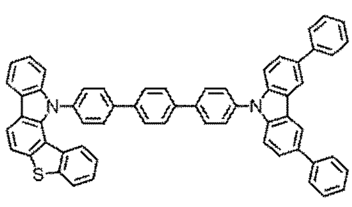 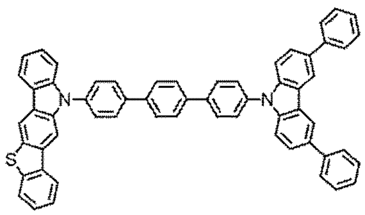 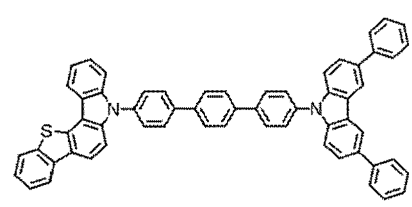

[Formula 107]

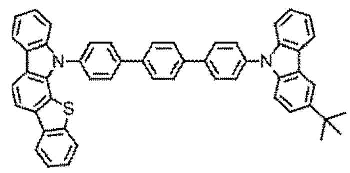
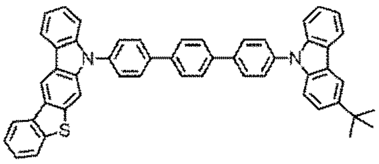
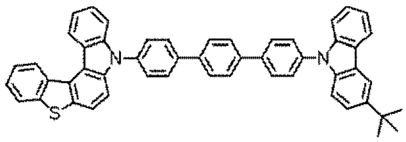
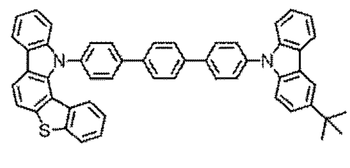
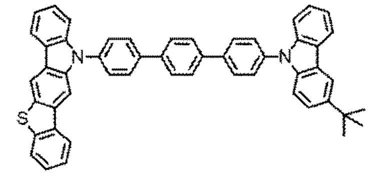
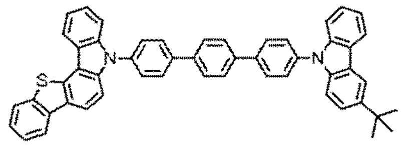
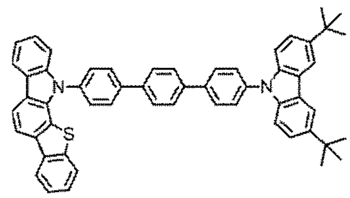
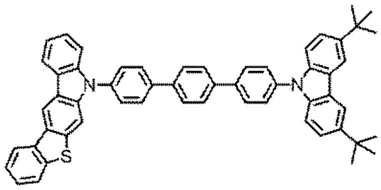
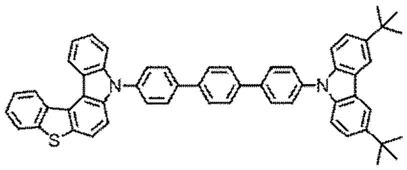

[Formula 108]

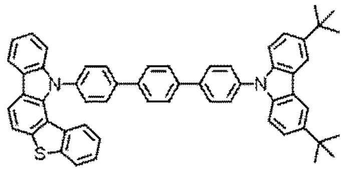
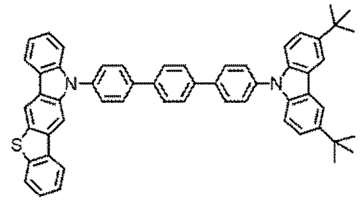
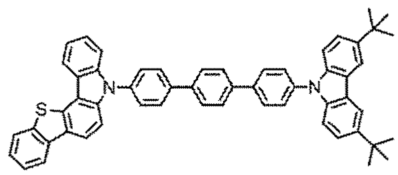
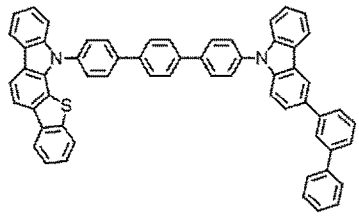
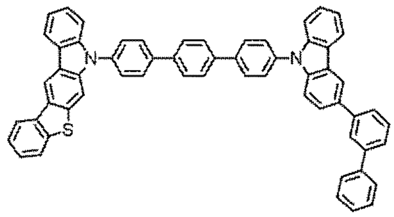
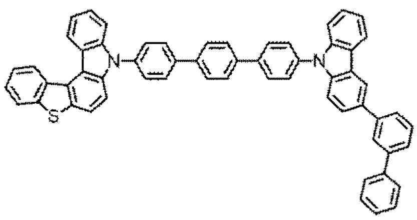
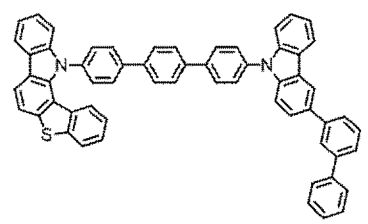
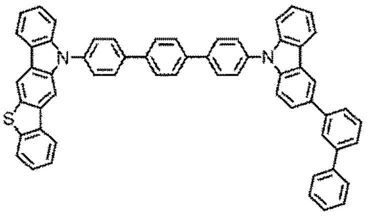
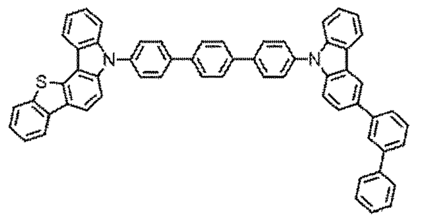

[Formula 109]

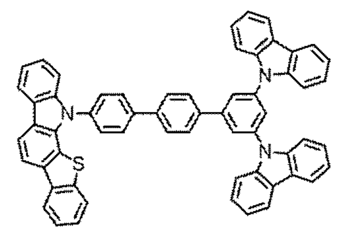
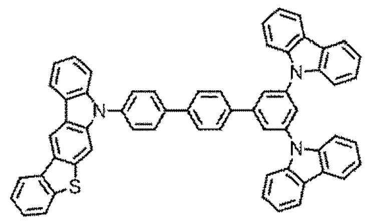
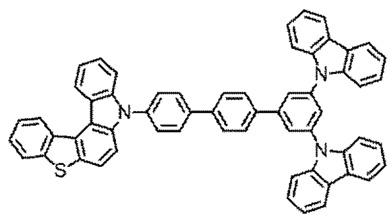

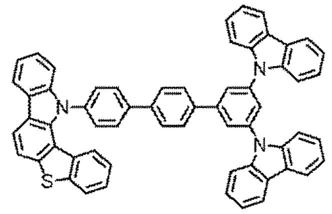
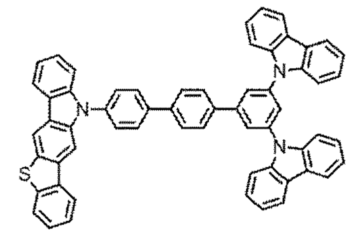
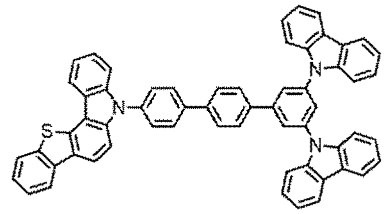

[Formula 110]

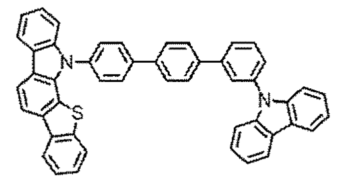
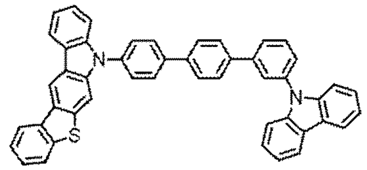
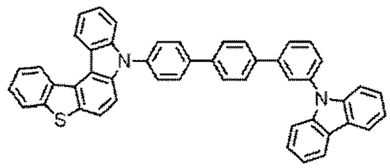

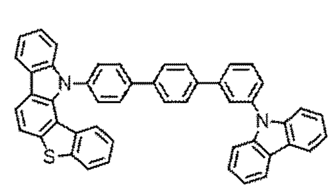
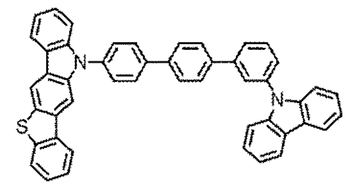
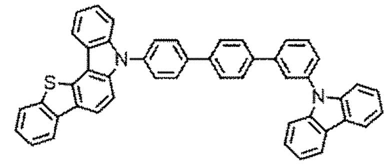

[Formula 111]

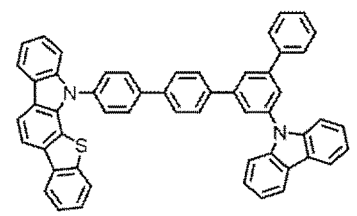
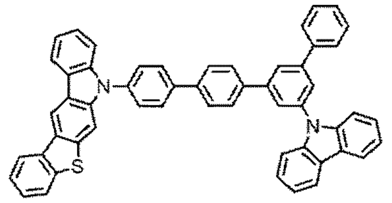
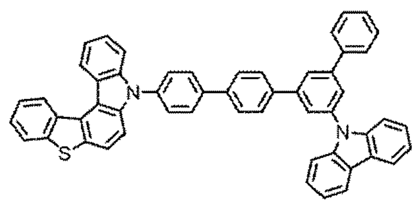

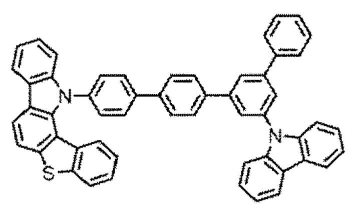
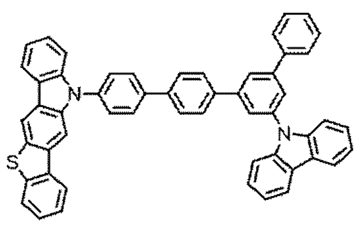
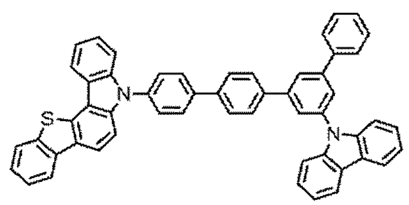

[Formula 112]

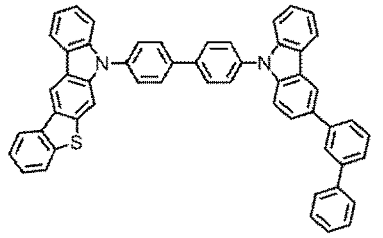
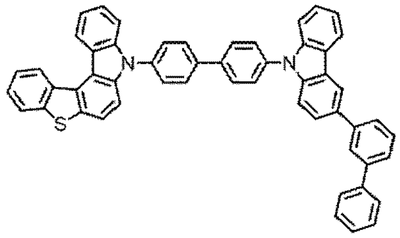
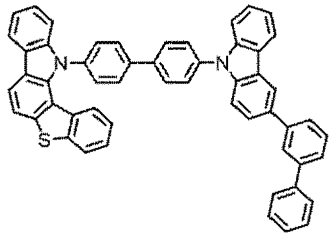
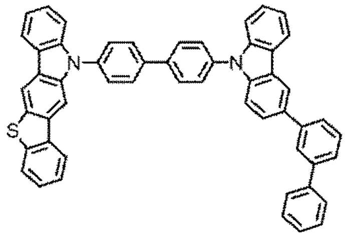
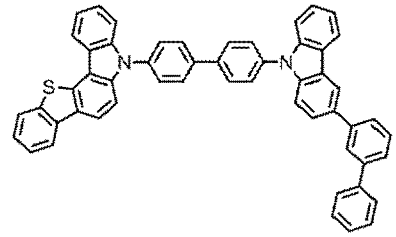
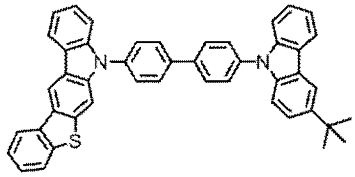
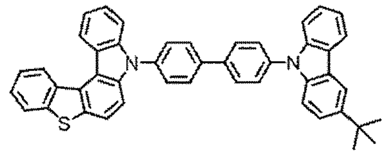
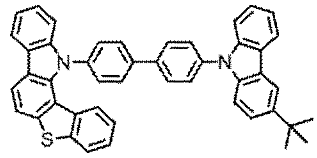

[Formula 113]

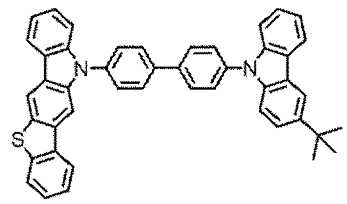
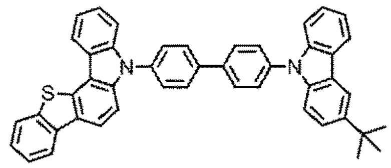
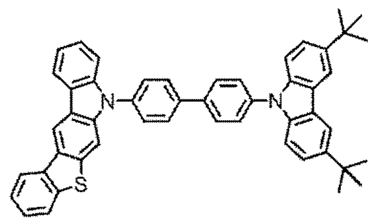
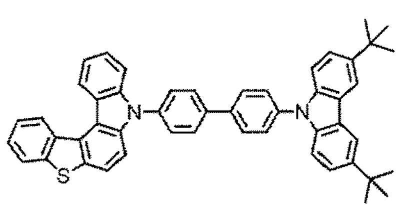
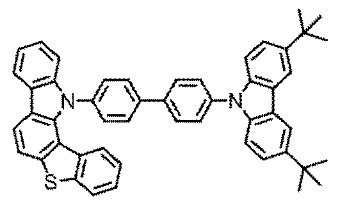
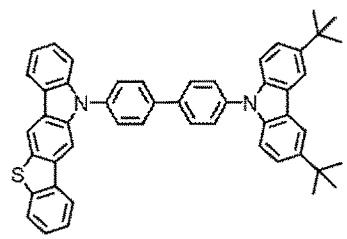
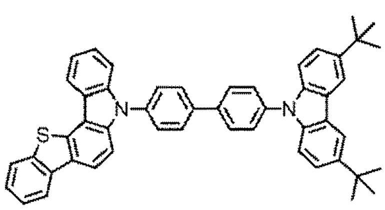
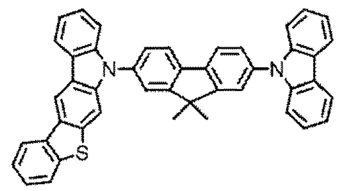

[Formula 114]

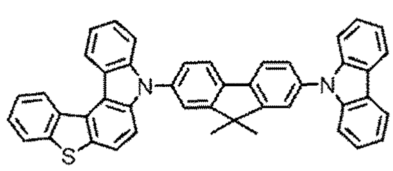 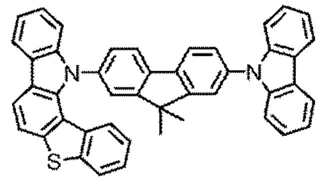 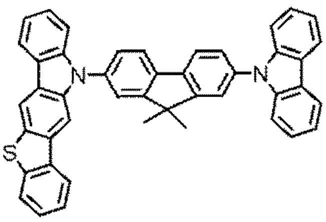

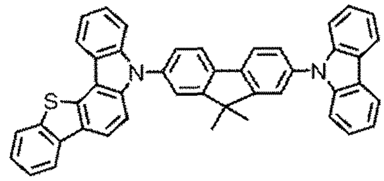 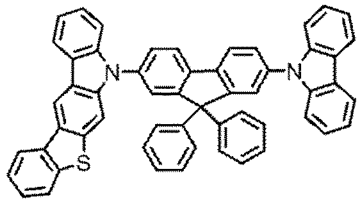

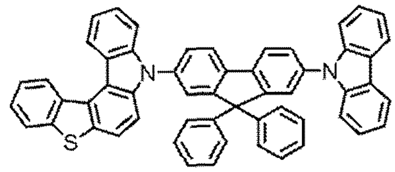 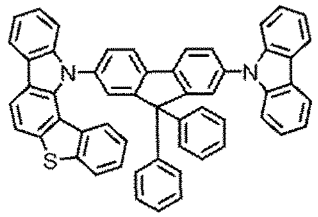 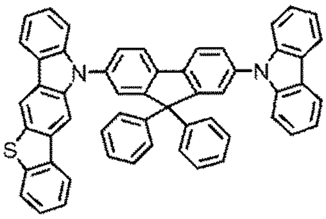

[Formula 115]

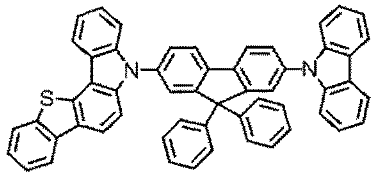 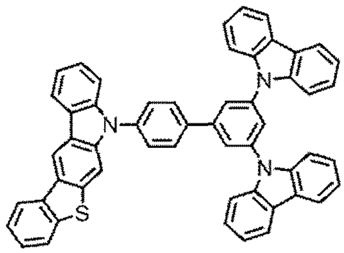 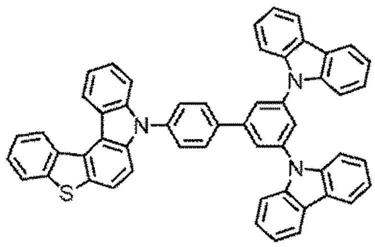

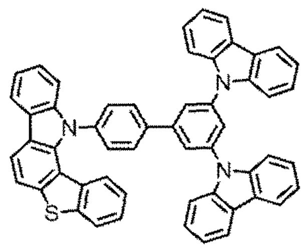 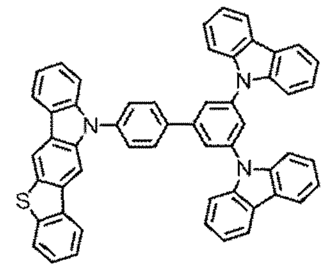 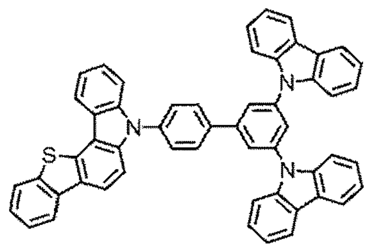

[Formula 116]

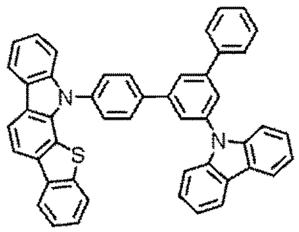
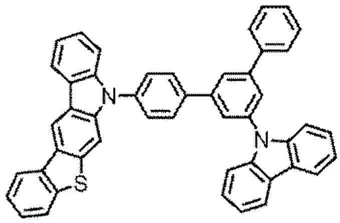
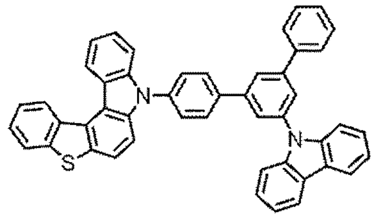
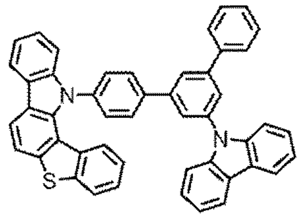
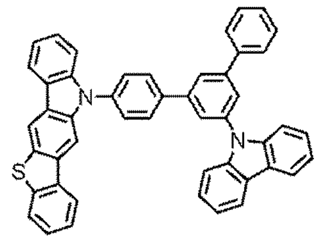
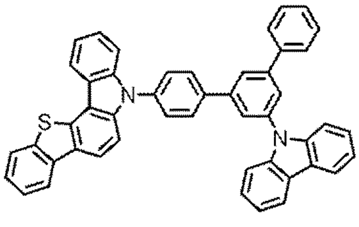
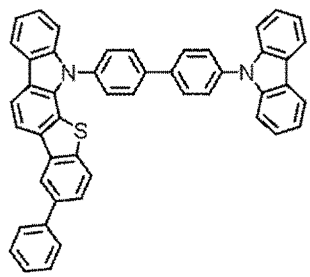
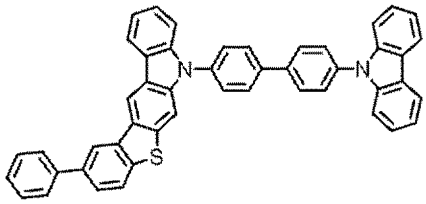
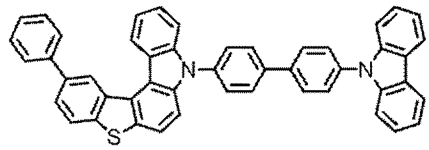

[Formula 117]

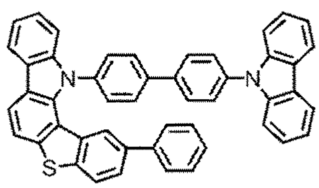
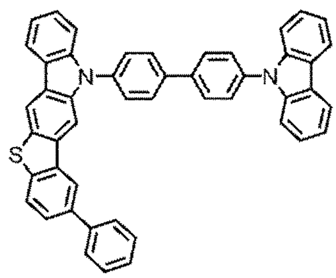
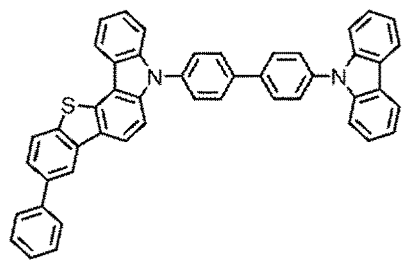
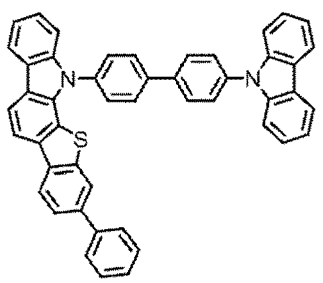
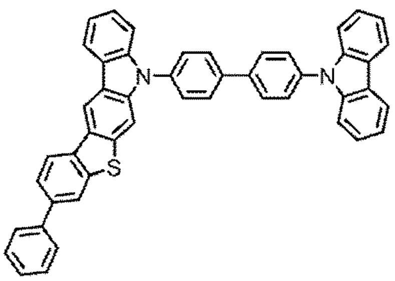
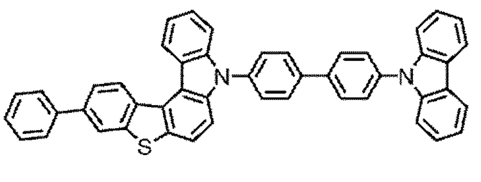
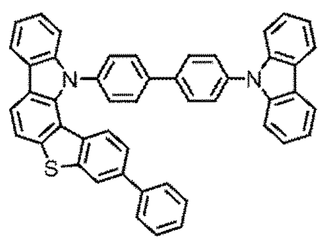
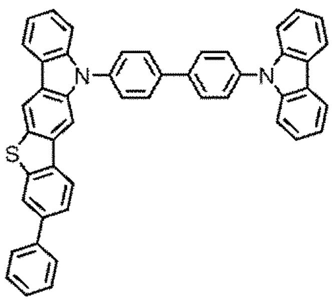

[Formula 118]

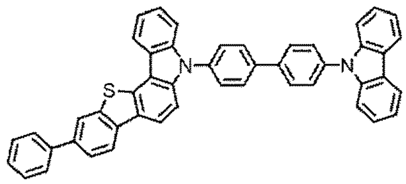
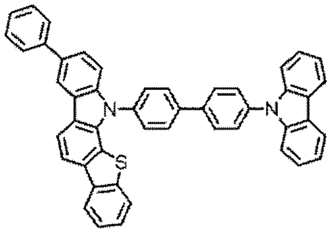
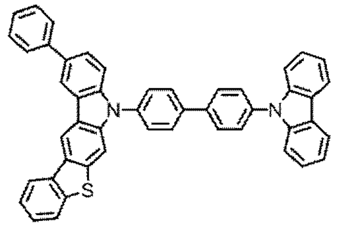
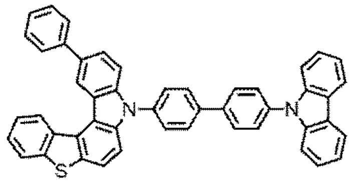
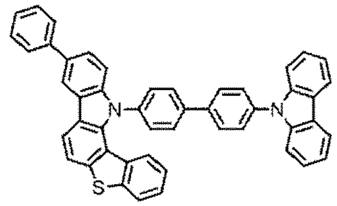
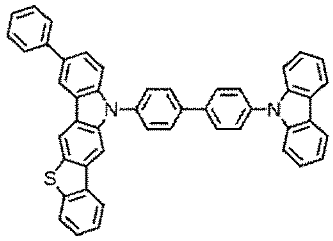
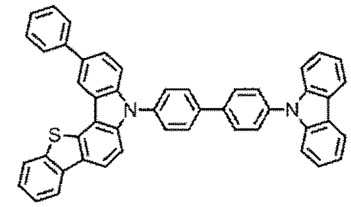
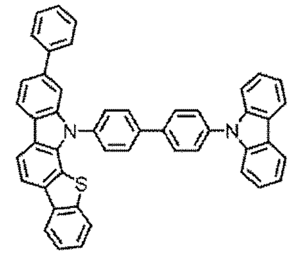

[Formula 119]

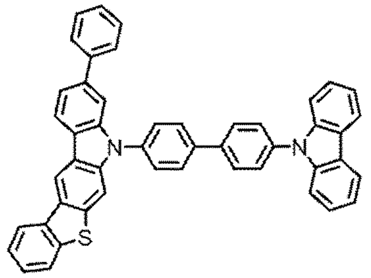
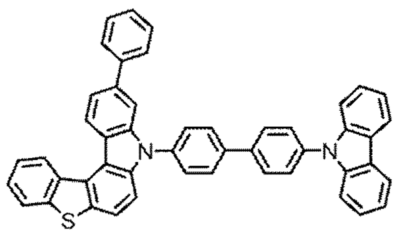
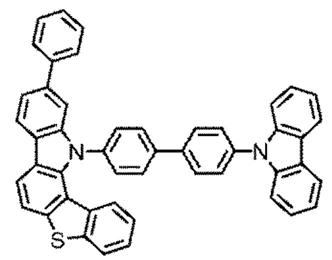
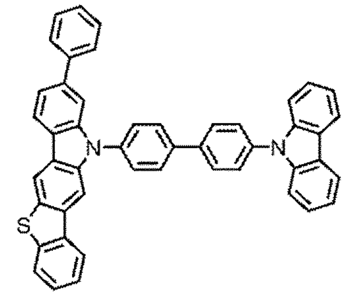
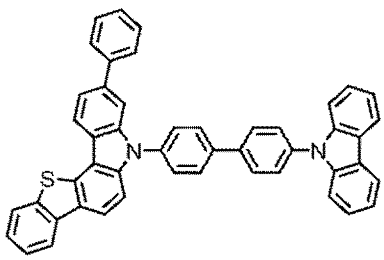

Compound M2

The emitting layer of the exemplary embodiment includes a delayed fluorescent compound M2.

Delayed Fluorescence

Delayed fluorescence is explained in "Yuki Hando-tai no Debaisu Bussei (Device Physics of Organic Semiconductors)" (edited by ADACHI, Chihaya, published by Kodansha, on pages 261-268). This document describes that, if an energy difference $\Delta E_{13}$ of a fluorescent material between a singlet state and a triplet state is reducible, a reverse energy transfer from the triplet state to the singlet state, which usually occurs at a low transition probability, would occur

at a high efficiency to express thermally activated delayed fluorescence (TADF). Further, a mechanism of generating delayed fluorescence is explained in Fig. 10.38 in the document. The compound M2 of the exemplary embodiment is preferably a compound exhibiting thermally activated delayed fluorescence generated by such a mechanism.

In general, emission of delayed fluorescence can be confirmed by measuring the transient PL (Photo Luminescence).

The behavior of delayed fluorescence can also be analyzed based on the decay curve obtained from the transient PL measurement. The transient PL measurement is a method of irradiating a sample with a pulse laser to excite the sample, and measuring the decay behavior (transient characteristics) of PL emission after the irradiation is stopped. PL emission in TADF materials is classified into a light emission component from a singlet exciton generated by the first PL excitation and a light emission component from a singlet exciton generated via a triplet exciton. The lifetime of the singlet exciton generated by the first PL excitation is on the order of nanoseconds and is very short. Therefore, light emission from the singlet exciton rapidly attenuates after irradiation with the pulse laser.

On the other hand, the delayed fluorescence is gradually attenuated due to light emission from a singlet exciton generated via a triplet exciton having a long lifetime. As described above, there is a large temporal difference between the light emission from the singlet exciton generated by the first PL excitation and the light emission from the singlet exciton generated via the triplet exciton. Therefore, the luminous intensity derived from delayed fluorescence can be determined.

Fig. 2 shows a schematic diagram of an exemplary device for measuring the transient PL.

An exemple of a method of measuring a transient PL using Fig. 2 and an example of behavior analysis of delayed fluorescence will be described.

A transient PL measuring device 100 in Fig. 2 includes: a pulse laser 101 capable of radiating a light having a predetermined wavelength; a sample chamber 102 configured to house a measurement sample; a spectrometer 103 configured to divide a light radiated from the measurement sample; a streak camera 104 configured to provide a two-dimensional image; and a personal computer 105 configured to import and analyze the two-dimensional image. A device for measuring the transient PL is not limited to the device described in the exemplary embodiment.

The sample to be housed in the sample chamber 102 is obtained by doping a matrix material with a doping material at a concentration of 12 mass% and forming a thin film on a quartz substrate.

The thin film sample housed in the sample chamber 102 is irradiated with a pulse laser from the pulse laser 101 to excite the doping material. Emission is extracted in a direction of 90 degrees with respect to an irradiation direction of the excitation light. The extracted emission is divided by the spectrometer 103 to form a two-dimensional image in the streak camera 104. As a result, the two-dimensional image is obtainable in which the ordinate axis represents a time, the abscissa axis represents a wavelength, and a bright spot represents a luminous intensity. When this two-dimensional image is taken out at a predetermined time axis, an emission spectrum in which the ordinate axis represents the luminous intensity and the abscissa axis represents the wavelength is obtainable. Moreover, when this two-dimensional image is taken out at the wavelength axis, a decay curve (transient PL) in which the ordinate axis represents a logarithm of the luminous intensity and the abscissa axis represents the time is obtainable.

For instance, a thin film sample A was manufactured as described above from a reference compound H1 as the matrix material and a reference compound D1 as the doping material and was measured in terms of the transient PL.

[Formula 120]

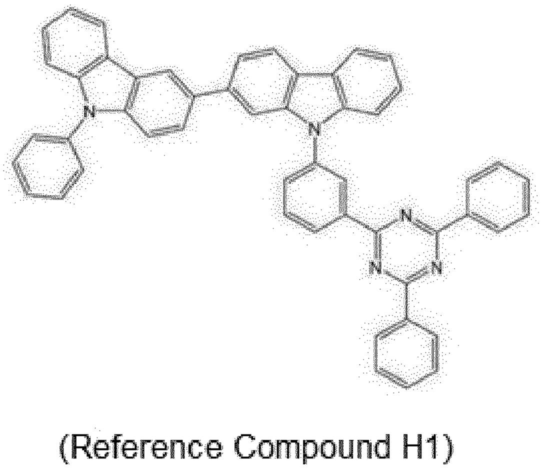

(Reference Compound H1)

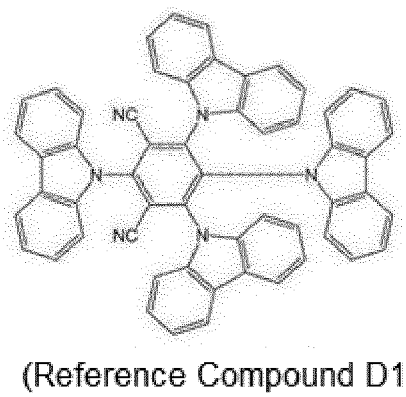

(Reference Compound D1)

Herein, the decay curve was analyzed with respect to the above thin film sample A and a thin film sample B. The thin film sample B was manufactured in the same manner as described above from a reference compound H2 as the matrix material and the reference compound D1 as the doping material.

Fig. 3 shows decay curves obtained from the transient PL obtained by measuring the thin film samples A and B.

[Formula 121]

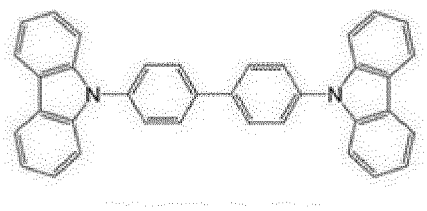

(Reference Compound H2)

As described above, an emission decay curve in which the ordinate axis represents the luminous intensity and the abscissa axis represents the time can be obtained by the transient PL measurement. Based on the emission decay curve, a fluorescence intensity ratio between fluorescence emitted from a singlet state generated by photo-excitation and delayed fluorescence emitted from a singlet state generated by inverse energy transfer via a triplet state can be estimated. In a delayed fluorescent material, a ratio of the intensity of the slowly decaying delayed fluorescence to the intensity of the promptly decaying fluorescence is relatively large.

Specifically, Prompt emission and Delay emission are present as emission from the delayed fluorescent material. Prompt emission is observed promptly when the excited state is achieved by exciting the compound of the exemplary embodiment with a pulse beam (i.e., a beam emitted from a pulse laser) having a wavelength absorbable by the delayed fluorescent material. Delay emission is observed not promptly when the excited state is achieved but after the excited state is achieved.

An amount of Prompt emission, an amount of Delay emission and a ratio between the amounts thereof can be obtained according to the method as described in "Nature 492, 234-238, 2012" (Reference Document 1). The amount of Prompt emission and the amount of Delay emission may be calculated using a device different from one described in Reference Document 1 or one shown in Fig. 2.

In the exemplary embodiment, a sample manufactured by a method shown below is used for measuring delayed fluorescence of the compound M2. For instance, the compound M2 is dissolved in toluene to prepare a dilute solution with an absorbance of 0.05 or less at the excitation wavelength to eliminate the contribution of self-absorption. In order to prevent quenching due to oxygen, the sample solution is frozen and degassed and then sealed in a cell with a lid under an argon atmosphere to obtain an oxygen-free sample solution saturated with argon.

The fluorescence spectrum of the sample solution is measured with a spectrofluorometer FP-8600 (manufactured by JASCO Corporation), and the fluorescence spectrum of a 9,10-diphenylanthracene ethanol solution is measured under the same conditions. Using the fluorescence area intensities of both spectra, the total fluorescence quantum yield is calculated by an equation (1) in Morris et al. J. Phys. Chem. 80 (1976) 969.

An amount of Prompt emission, an amount of Delay emission and a ratio between the amounts thereof can be obtained according to the method as described in "Nature 492, 234-238, 2012" (Reference Document 1). The amount of Prompt emission and the amount of Delay emission may be calculated using a device different from one described in Reference Document 1 or one shown in Fig. 2.

In the exemplary embodiment, provided that an amount of Prompt emission of a measurement target compound (compound M2) is denoted by $X_P$ and the amount of Delay emission is denoted by $X_D$, a value of $X_D/X_P$ is preferably 0.05 or more.

The amounts of Prompt emission and Delay emission and a ratio of the amounts thereof in compounds other than the compound M2 herein are measured in the same manner as those of the compound M2.

The compound M2 is exemplified by a compound represented by a formula (2) below.

[Formula 122]

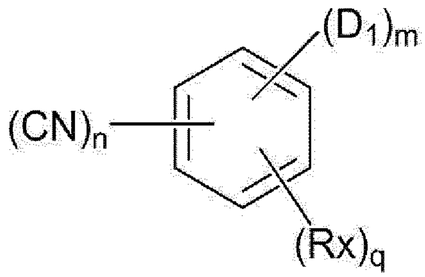

(2)

In the formula (2):

n is 1, 2, 3 or 4;
m is 1, 2, 3 or 4;
q is 0, 1, 2, 3 or 4;
m+n+q=6 is satisfied;
CN is a cyano group;
$D_1$ is a group represented by a formula (2a), (2b) or (2c) below, and when a plurality of $D_1$ are present, the plurality of $D_1$ are mutually the same or different;
Rx is a hydrogen atom or a substituent, or a pair of adjacent ones of Rx are mutually bonded to form a ring, and when a plurality of Rx are present, the plurality of Rx are mutually the same or different;
Rx as a substituent is each independently a halogen atom, a substituted or unsubstituted aryl group having 6 to 30 ring carbon atoms, a substituted or unsubstituted heterocyclic group having 5 to 30 ring atoms, a substituted or unsubstituted amino group, a substituted or unsubstituted carbonyl group, a substituted or unsubstituted alkyl group having 1 to 30 carbon atoms, a substituted or unsubstituted alkyl halide group having 1 to 30 carbon atoms, a substituted or unsubstituted alkylsilyl group having 3 to 30 carbon atoms, or a substituted or unsubstituted arylsilyl group having 6 to 60 ring carbon atoms; and
CN, $D_1$ and Rx are bonded to respective carbon atoms of a six-membered ring.

[Formula 123]

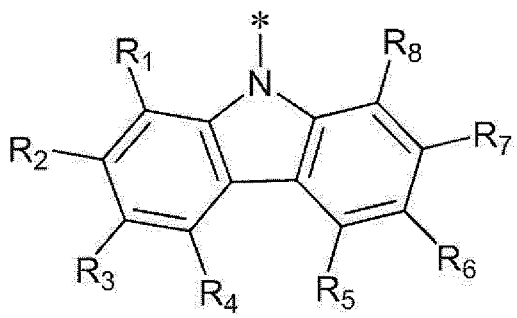

(2a)

In the formula (2a):

$R_1$ to $R_8$ are each independently a hydrogen atom or a substituent, or at least one pair of a pair of $R_1$ and $R_2$, a pair of $R_2$ and $R_3$, a pair of $R_3$ and $R_4$, a pair of $R_5$ and $R_6$, a pair of $R_6$ and $R_7$, or a pair of $R_7$ and $R_8$ are mutually bonded to form a ring;
$R_1$ to $R_8$ as a substituent are each independently a halogen atom, a substituted or unsubstituted aryl group having 6 to 30 ring carbon atoms, a substituted or unsubstituted heterocyclic group having 5 to 30 ring atoms, a substituted or unsubstituted alkyl group having 1 to 30 carbon atoms, a substituted or unsubstituted alkyl halide group having 1 to 30 carbon atoms, a substituted or unsubstituted alkylsilyl group having 3 to 30 carbon atoms, a substituted or unsubstituted arylsilyl group having 6 to 60 ring carbon atoms, a hydroxy group, a substituted or unsubstituted alkoxy group having 1 to 30 carbon atoms, a substituted or unsubstituted alkoxy halide group having 1 to 30 carbon atoms, a substituted or unsubstituted aryloxy group having 6 to 30 ring carbon atoms, a substituted or unsubstituted alkylamino group having 2 to 30 carbon atoms, a substituted or unsubstituted arylamino group having 6 to 60 ring carbon

atoms, a thiol group, a substituted or unsubstituted alkylthio group having 1 to 30 carbon atoms, or a substituted or unsubstituted arylthio group having 6 to 30 ring carbon atoms; and

* represents a bonding position to a carbon atom of a benzene ring in the formula (2).

[Formula 124]

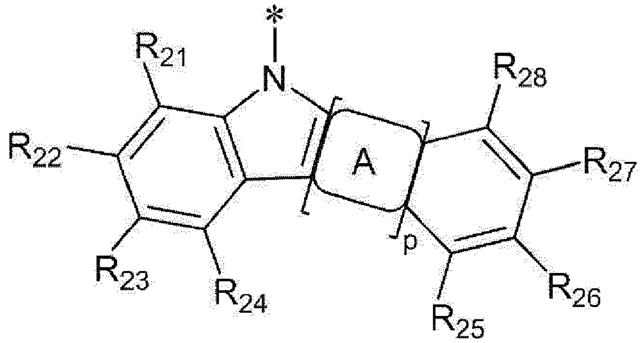

(2b)

In the formula (2b):

$R_{21}$ to $R_{28}$ are each independently a hydrogen atom or a substituent, or at least one pair of a pair of $R_{21}$ and $R_{22}$, a pair of $R_{22}$ and $R_{23}$, a pair of $R_{23}$ and $R_{24}$, a pair of $R_{25}$ and $R_{26}$, a pair of $R_{26}$ and $R_{27}$, or a pair of $R_{27}$ and $R_{28}$ are mutually bonded to form a ring;

$R_{21}$ to $R_{28}$ as a substituent each independently represent the same as $R_1$ to $R_8$ in the formula (2a);

A represents a cyclic structure represented by a formula (211) or (212) below, and the cyclic structure A is fused with any position(s) of adjacent cyclic structure(s);

p is 1, 2, 3 or 4;

when p is 2, 3 or 4, a plurality of cyclic structures A are mutually the same or different; and

* represents a bonding position to a carbon atom of a benzene ring in the formula (2).

[Formula 125]

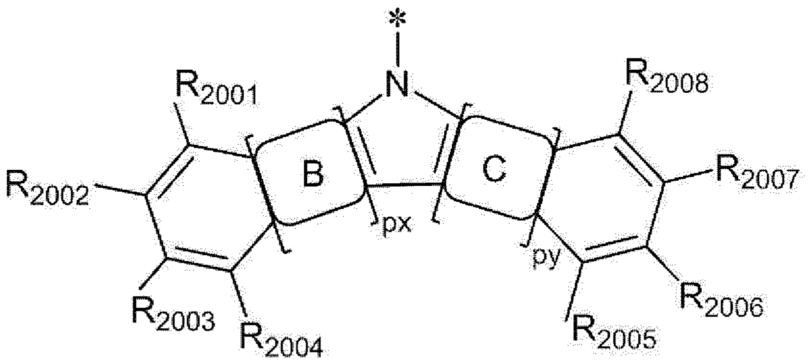

(2c)

In the formula (2c):

$R_{2001}$ to $R_{2008}$ are each independently a hydrogen atom or a substituent, or at least one pair of a pair of $R_{200}$, and $R_{2002}$, a pair of $R_{2002}$ and $R_{2003}$, a pair of $R_{2003}$ and $R_{2004}$, a pair of $R_{2005}$ and $R_{2006}$, a pair of $R_{2006}$ and $R_{2007}$, or a pair of $R_{2007}$ and $R_{2008}$ are mutually bonded to form a ring;

$R_{2001}$ to $R_{2008}$ as a substituent each independently represent the same as $R_1$ to $R_8$ as a substitutent in the formula (2a);

B represents a cyclic structure represented by the formula (211) or (212), and the cyclic structure B is fused with any position(s) of adjacent cyclic structure(s);

px is 1, 2, 3 or 4;

when px is 2, 3 or 4, a plurality of cyclic structures B are mutually the same or different;

C represents a cyclic structure represented by the formula (211) or (212), and the cyclic structure C is fused with any position(s) of adjacent cyclic structure(s);

py is 1, 2, 3 or 4;

when py is 2, 3 or 4, a plurality of cyclic structures C are mutually the same or different; and

* represents a bonding position to a carbon atom of a benzene ring in the formula (2).

[Formula 126]

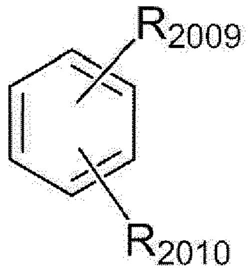

(211)

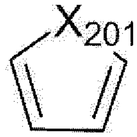

(212)

In the formula (211), $R_{2009}$ and $R_{2010}$ are each independently a hydrogen atom or a substituent, or bonded to a part of an adjacent cyclic structure, or a pair of $R_{2009}$ and $R_{2010}$ are mutually bonded to form a ring;

in the formula (212), $X_{201}$ is $CR_{2011}R_{2012}$, $NR_{2013}$, a sulfur atom, or an oxygen atom, and $R_{2011}$, $R_{2012}$ and $R_{2013}$ are each independently a hydrogen atom or a substituent, or $R_{2011}$ and $R_{2012}$ are mutually bonded to form a ring; and $R_{2009}$, $R_{2010}$, $R_{2011}$, $R_{2012}$ and $R_{2013}$ as a substituent each independently represent the same as $R_1$ to $R_8$ as a substituent in the formula (2a).

In the formula (211), $R_{2009}$ and $R_{2010}$ are each independently bonded to a part of an adjacent cyclic structure, which specifically means any of (I) to (IV) below.

In the formula (211), a pair of $R_{2009}$ and $R_{2010}$ are mutually bonded to form a ring, which specifically means (V) below.

(I) When the cyclic structures represented by the formula (211) are adjacent to each other, between the two adjacent rings, at least one pair of the following are mutually bonded to form a ring: $R_{2009}$ of one of the rings and $R_{2009}$ of the other of the rings; $R_{2009}$ of one of the rings and $R_{2010}$ of the other of the rings; or $R_{2010}$ of one of the rings and $R_{2010}$ of the other of the rings.

(II) When the cyclic structure represented by the formula (211) and the benzene ring having $R_{25}$ to $R_{28}$ in the formula (2b) are adjacent to each other, between the two adjacent rings, at least one pair of the following are mutually bonded to form a ring: $R_{2009}$ of one of the rings and $R_{25}$ of the other of the rings; $R_{2009}$ of one of the rings and $R_{28}$ of the other of the rings; $R_{2010}$ of one of the rings and $R_{25}$ of the other of the rings; or $R_{2010}$ of one of the rings and $R_{28}$ of the other of the rings.

(III) When the cyclic structure represented by the formula (211) and the benzene ring having $R_{2001}$ to $R_{2004}$ in the formula (2c) are adjacent to each other, between the two adjacent rings, at least one pair of the following are mutually bonded to form a ring: $R_{2009}$ of one of the rings and $R_{2001}$ of the other of the rings; $R_{2009}$ of one of the rings and $R_{2004}$ of the other of the rings; $R_{2010}$ of one of the rings and $R_{2001}$ of the other of the rings; or $R_{2010}$ of one of the rings and $R_{2004}$ of the other of the rings.

(IV) When the cyclic structure represented by the formula (211) and the benzene ring having $R_{2005}$ to $R_{2008}$ in the formula (2c) are adjacent to each other, between the two adjacent rings, at least one pair of the following are mutually bonded to form a ring: $R_{2009}$ of one of the rings and $R_{2005}$ of the other of the rings; $R_{2009}$ of one of the rings and $R_{2008}$ of the other of the rings; $R_{2010}$ of one of the rings and $R_{2005}$ of the other of the rings; or $R_{2010}$ of one of the rings and $R_{2008}$ of the other of the rings.

(V) The pair of $R_{2009}$ and $R_{2010}$ of the cyclic structure represented by the formula (211) are mutually bonded to form a ring. In other words, (V) means that the pair of $R_{2009}$ and $R_{2010}$, which are bonded to the same ring, are mutually bonded to form a ring.

In the compound M2 of the exemplary embodiment, it is preferable that: Rx is a hydrogen atom, an unsubstituted aryl group having 6 to 30 ring carbon atoms, an unsubstituted heterocyclic group having 5 to 30 ring atoms, or an unsubstituted alkyl group having 1 to 30 carbon atoms; and when Rx is an unsubstituted heterocyclic group having 5 to 30 ring atoms, Rx as the unsubstituted heterocyclic group having 5 to 30 ring atoms is a pyridyl group, pyrimidinyl group, triazinyl group, dibenzofuranyl group, or dibenzothienyl group.

Herein, the triazinyl group refers to a group obtained by excluding one hydrogen atom from 1,3,5-triazine, 1,2,4-triazine, or 1,2,3-triazine.

The triazinyl group is preferably a group obtained by excluding one hydrogen atom from 1,3,5-triazine.

In the compound M2 of the exemplary embodiment, Rx is more preferably a hydrogen atom, an unsubstituted aryl group having 6 to 30 ring carbon atoms, an unsubstituted dibenzofuranyl group, or an unsubstituted dibenzothienyl group.

In the compound M2 of the exemplary embodiment, Rx is further preferably a hydrogen atom.

In the compound M2 of the exemplary embodiment, it is preferable that $R_1$ to $R_8$, $R_{21}$ to $R_{28}$, $R_{2001}$ to $R_{2998}$, $R_{2009}$ to $R_{2010}$ and $R_{2011}$ to $R_{2013}$ as a substituent are each independently an unsubstituted aryl group having 6 to 30 ring carbon atoms, an unsubstituted heterocyclic group having 5 to 30 ring atoms, or an unsubstituted alkyl group having 1 to 30 carbon atoms.

In the compound M2 of the exemplary embodiment, $D_1$ is also preferably a group represented by a formula (D-21), (D-22), (D-23) or (D-24) below.

[Formula 127]

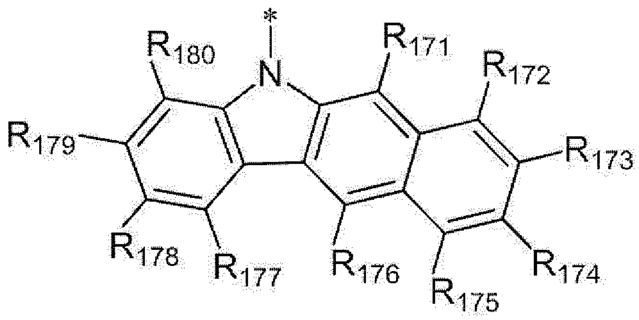

(D-21)

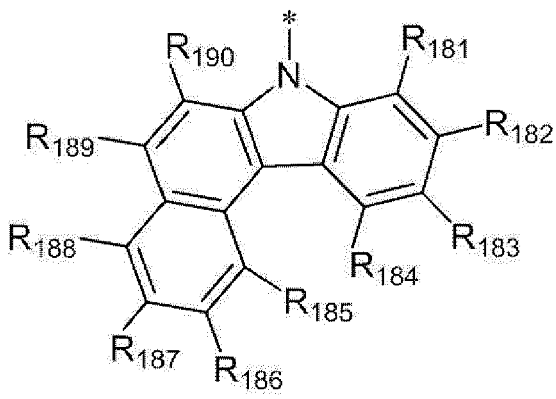

(D-22)

[Formula 128]

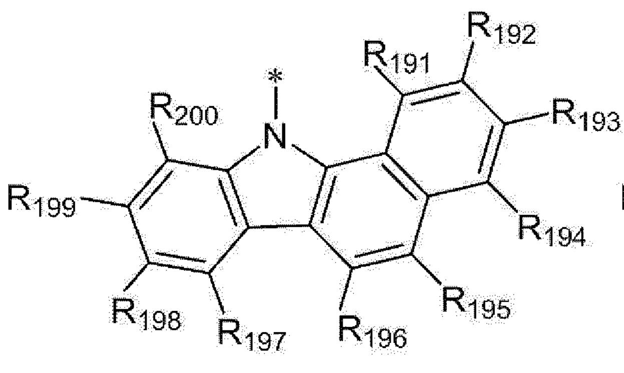

(D-23)

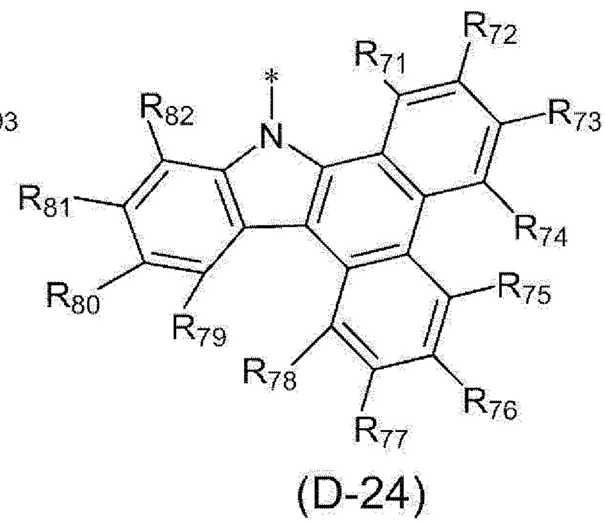

(D-24)

In the formulae (D-21) to (D-24): $R_{171}$ to $R_{200}$ and $R_{71}$ to $R_{82}$ are each independently a hydrogen atom or a substituent, or at least one pair of a pair of $R_{171}$ and $R_{172}$, a pair of $R_{172}$ and $R_{173}$, a pair of $R_{173}$ and $R_{174}$, a pair of $R_{174}$ and $R_{175}$, a pair of $R_{175}$ and $R_{176}$, a pair of $R_{177}$ and $R_{178}$, a pair of $R_{178}$ and $R_{179}$, a pair of $R_{179}$ and $R_{180}$, a pair of

$R_{181}$ and $R_{182}$, a pair of $R_{182}$ and $R_{183}$, a pair of $R_{183}$ and $R_{184}$, a pair of $R_{185}$ and $R_{186}$, a pair of $R_{186}$ and $R_{187}$, a pair of $R_{187}$ and $R_{188}$, a pair of $R_{188}$ and $R_{189}$, a pair of $R_{189}$ and $R_{190}$, a pair of $R_{191}$ and $R_{192}$, a pair of $R_{192}$ and $R_{193}$, a pair of $R_{193}$ and $R_{194}$, a pair of $R_{194}$ and $R_{195}$, a pair of $R_{195}$ and $R_{196}$, a pair of $R_{197}$ and $R_{198}$, a pair of $R_{198}$ and $R_{199}$, a pair of $R_{199}$ and $R_{200}$, a pair of $R_{71}$ and $R_{72}$, a pair of $R_{72}$ and $R_{73}$, a pair of $R_{73}$ and $R_{74}$, a pair of $R_{75}$ and $R_{76}$, a pair of $R_{76}$ and $R_{77}$, a pair of $R_{77}$ and $R_{78}$, a pair of $R_{79}$ and $R_{89}$, a pair of $R_{80}$ and $R_{81}$, or a pair of $R_8$, and $R_{82}$ are mutually bonded to form a ring;

$R_{171}$ to $R_{200}$ and $R_{71}$ to $R_{82}$ as a substituent are each independently a halogen atom, a substituted or unsubstituted aryl group having 6 to 14 ring carbon atoms, a substituted or unsubstituted heterocyclic group having 5 to 14 ring atoms, a substituted or unsubstituted alkyl group having 1 to 6 carbon atoms, a substituted or unsubstituted alkyl halide group having 1 to 30 carbon atoms, a substituted or unsubstituted alkylsilyl group having 3 to 6 carbon atoms, a hydroxy group, a substituted or unsubstituted alkoxy group having 1 to 6 carbon atoms, a substituted or unsubstituted alkoxy halide group having 1 to 6 carbon atoms, a substituted or unsubstituted aryloxy group having 6 to 14 ring carbon atoms, a substituted or unsubstituted alkylamino group having 2 to 12 carbon atoms, a thiol group, a substituted or unsubstituted alkylthio group having 1 to 6 carbon atoms, or a substituted or unsubstituted arylthio group having 6 to 14 ring carbon atoms; and

* represents a bonding position to a carbon atom of a benzene ring in the formula (2).

In the compound M2 of the exemplary embodiment, $D_1$ is also more preferably a group represented by the formula (D-21), (D-23) or (D-24).

In the compound M2 of the exemplary embodiment, $D_1$ is also further preferably a group represented by the formula (D-21) or (D-23).

It is preferable that $R_{171}$ to $R_{200}$ and $R_{71}$ to $R_{82}$ as a substituent in the compound M2 of the exemplary embodiment are each independently an unsubstituted aryl group having 6 to 14 ring carbon atoms, an unsubstituted heterocyclic group having 5 to 14 ring atoms, or an unsubstituted alkyl group having 1 to 6 carbon atoms.

It is also preferable that $R_{171}$ to $R_{200}$ and $R_{71}$ to $R_{82}$ in the compound M2 of the exemplary embodiment are hydrogen atoms.

The compound M2 is exemplified by a compound represented by a formula (22) below.

[Formula 129]

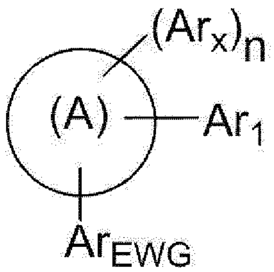

(22)

In the formula (22), $Ar_1$ is a group selected from the group consisting of a substituted or unsubstituted aryl group having 6 to 30 ring carbon atoms, a substituted or unsubstituted heteroaryl group having 5 to 30 ring atoms, a substituted or unsubstituted alkyl group having 1 to 30 carbon atoms, a substituted or unsubstituted fluoroalkyl group having 1 to 30 carbon atoms, a substituted or unsubstituted cycloalkyl group having 3 to 30 ring carbon atoms, a substituted or unsubstituted aralkyl group having 7 to 30 carbon atoms, a substituted phosphoryl group, a substituted silyl group, a cyano group, a nitro group, a carboxy group, and groups represented by formulae (1a) to (1j) below.

In the formula (22), $Ar_{EWG}$ is a substituted or unsubstituted heteroaryl group having 5 to 30 ring atoms that includes at least one nitrogen atom in a ring, or an aryl group having 6 to 30 ring carbon atoms and substituted by at least one cyano group.

In the formula (22), Arx is each independently a hydrogen atom or a substituent, and Arx as a substituent is a group selected from the group consisting of a substituted or unsubstituted aryl group having 6 to 30 ring carbon atoms, a substituted or unsubstituted heteroaryl group having 5 to 30 ring atoms, a substituted or unsubstituted alkyl group having 1 to 30 carbon atoms, a substituted or unsubstituted fluoroalkyl group having 1 to 30 carbon atoms, a substituted or unsubstituted cycloalkyl group having 3 to 30 ring carbon atoms, a substituted or unsubstituted aralkyl group having 7 to 30 carbon atoms, a substituted phosphoryl group, a substituted silyl group, a cyano group, a nitro group, a carboxy group, and groups represented by the formulae (1a) to (1j).

In the formula (22), n is 0, 1, 2, 3, 4 or 5, and when n is 2, 3, 4 or 5, a plurality of Arx are mutually the same or

different.

In the formula (22), a ring (A) is a five-membered ring, a six-membered ring, or a seven-membered ring. The ring (A) may be an aromatic hydrocarbon ring or a heterocycle. $Ar_1$ and Arx are bonded to respective ones of elements forming the ring (A).

In the formula (22), at least one of $Ar_1$ or Arx is a group selected from the group consisting of groups represented by the formulae (1a) to (1j).

[Formula 130]

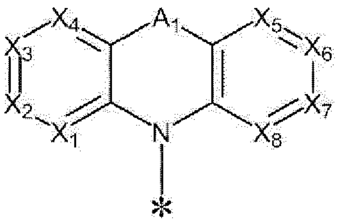

(1a)

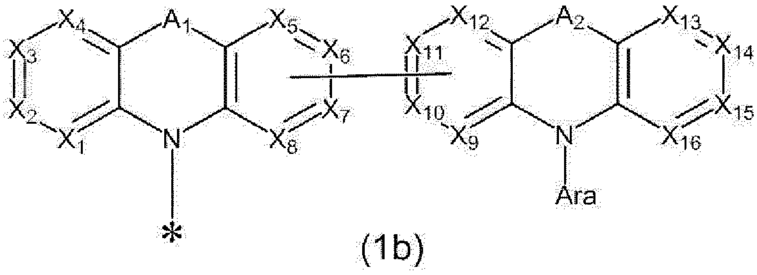

(1b)

[Formula 131]

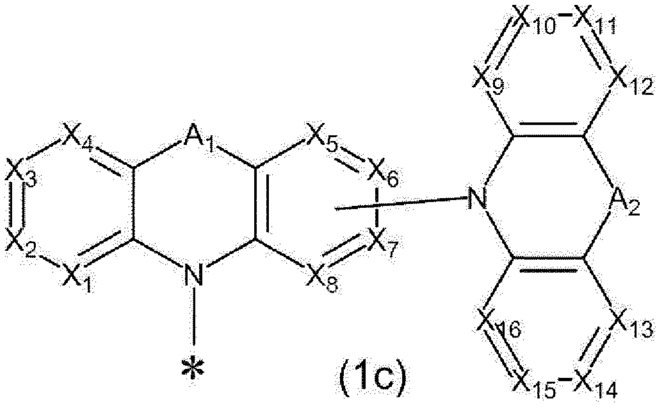

(1c)

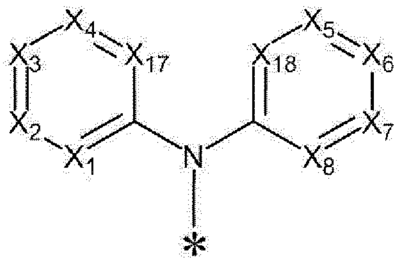

(1d)

[Formula 132]

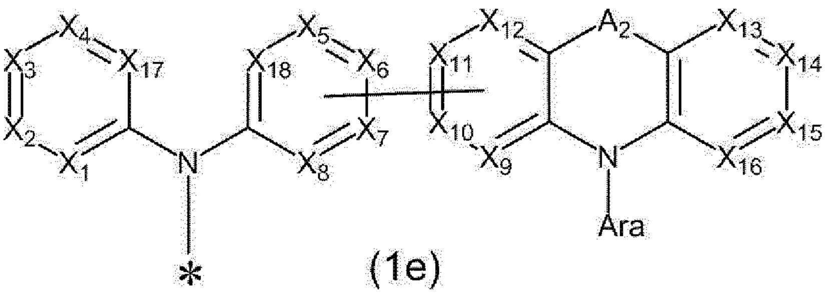

(1e)

[Formula 133]

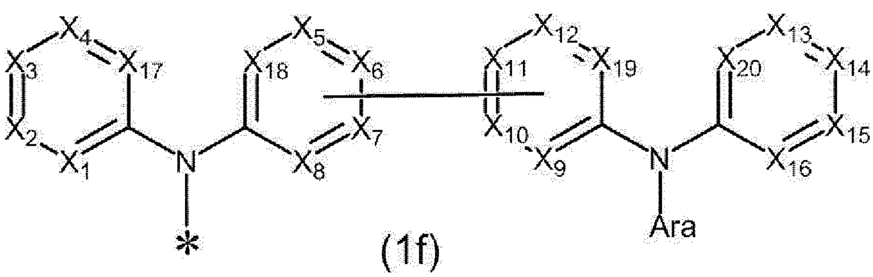

(1f)

[Formula 134]

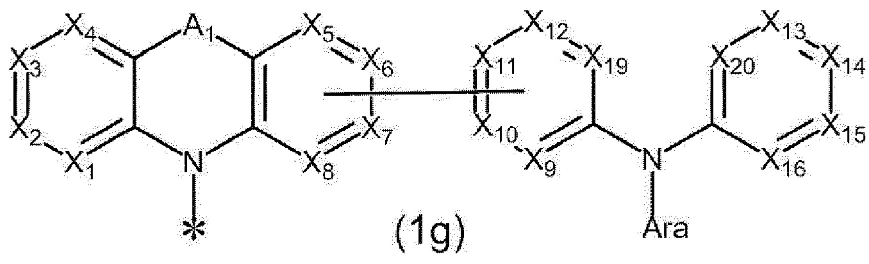

(1g)

[Formula 135]

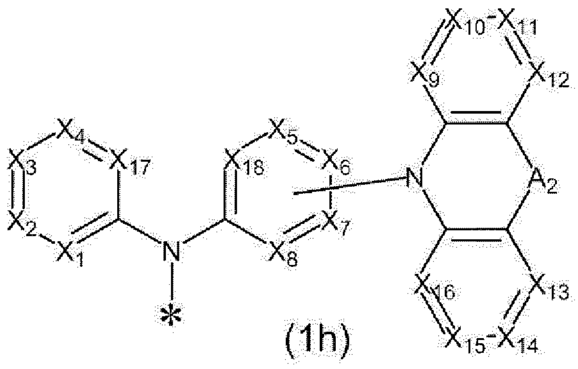

(1h)

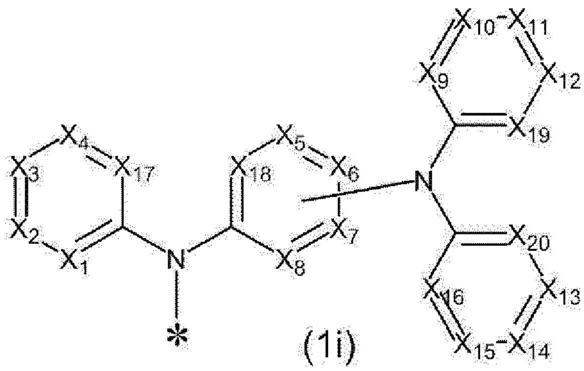

(1i)

[Formula 136]

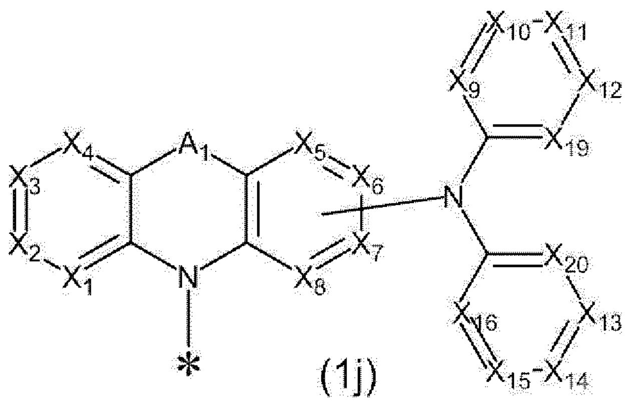

(1j)

In the formulae (1a) to (1j), $X_1$ to $X_{20}$ are each independently a nitrogen atom (N) or a carbon atom bonded with $R_{A1}$ (C-$R_{A1}$).

In the formula (1b), one of $X_5$ to $X_8$ is a carbon atom bonded to one of $X_9$ to $X_{12}$, and one of $X_9$ to $X_{12}$ is a carbon atom bonded to one of $X_5$ to $X_8$.

In the formula (1c), one of $X_5$ to $X_8$ is a carbon atom bonded to a nitrogen atom in a ring including $A_2$.

In the formula (1e), one of $X_5$ to $X_8$ and $X_{18}$ is a carbon atom bonded to one of $X_9$ to $X_{12}$, and one of $X_9$ to $X_{12}$ is a carbon atom bonded to one of $X_5$ to $X_8$ and $X_{18}$.

In the formula (1f), one of $X_5$ to $X_8$ and $X_{18}$ is a carbon atom bonded to one of $X_9$ to $X_{12}$ and $X_{19}$, and one of $X_9$ to $X_{12}$ and $X_{19}$ is a carbon atom bonded to one of $X_5$ to $X_8$ and $X_{18}$.

In the formula (1g), one of $X_5$ to $X_8$ is a carbon atom bonded to one of $X_9$ to $X_{12}$ and $X_{19}$, and one of $X_9$ to $X_{12}$ and $X_{19}$ is a carbon atom bonded to one of $X_5$ to $X_8$.

In the formula (1h), one of $X_5$ to $X_8$ and $X_{18}$ is a carbon atom bonded to a nitrogen atom in a ring including $A_2$.

In the formula (1i), one of $X_5$ to $X_8$ and $X_{18}$ is a carbon atom bonded to a nitrogen atom linking a ring including $X_9$ to $X_{12}$ and $X_{19}$ with a ring including $X_{13}$ to $X_{16}$ and $X_{20}$.

In the formula (1j), one of $X_5$ to $X_8$ is a carbon atom bonded to a nitrogen atom linking a ring including $X_9$ to $X_{12}$ and $X_{19}$ with a ring including $X_{13}$ to $X_{16}$ and $X_{20}$.

$R_{A1}$ is each independently a hydrogen atom or a substituent, or at least one pair of pairs among a plurality of $R_{A1}$ are mutually directly bonded to form a ring or bonded via a hetero atom to form a ring; and $R_{A1}$ as a substituent is a group selected from the group consisting of a substituted or unsubstituted aryl group having 6 to 30 ring carbon atoms, a substituted or unsubstituted heteroaryl group having 5 to 30 ring atoms, a substituted or unsubstituted alkyl group having 1 to 30 carbon atoms, a substituted or unsubstituted fluoroalkyl group having 1 to 30 carbon atoms, a substituted or unsubstituted cycloalkyl group having 3 to 30 ring carbon atoms, a substituted or unsubstituted aralkyl group having 7 to 30 carbon atoms, a substituted phosphoryl group, a substituted silyl group, a cyano group, a nitro group, and a carboxy group.

When a plurality of $R_{A1}$ as a substituent are present, the plurality of $R_{A1}$ are mutually the same or different.

In the formula (1a), when $X_1$ to $X_8$ are a carbon atom bonded with $R_{A1}$ ($C\text{-}R_{A1}$), a plurality of $R_{A1}$ preferably form no ring.

In the formulae (1a) to (1j), * represents a bonding position to the ring (A).

In the formulae (1a) to (1j), $A_1$ and $A_2$ are each independently a single bond, an oxygen atom (O), a sulfur atom (S), $C(R_{2021})(R_{2022})$, $Si(R_{2023})(R_{2024})$, C(=O), S(=O), $SO_2$ or $N(R_{2025})$. $R_{2021}$ to $R_{2025}$ are each independently a hydrogen atom or a substituent, and $R_{2021}$ to $R_{2025}$ as a substituent are each independently a group selected from the group consisting of a substituted or unsubstituted aryl group having 6 to 30 ring carbon atoms, a substituted or unsubstituted heteroaryl group having 5 to 30 ring atoms, a substituted or unsubstituted alkyl group having 1 to 30 carbon atoms, a substituted or unsubstituted fluoroalkyl group having 1 to 30 carbon atoms, a substituted or unsubstituted cycloalkyl group having 3 to 30 ring carbon atoms, a substituted or unsubstituted aralkyl group having 7 to 30 carbon atoms, a substituted phosphoryl group, a substituted silyl group, a cyano group, a nitro group, and a carboxy group.

In the formulae (1a) to (1j), Ara is a group selected from the group consisting of a substituted or unsubstituted aryl group having 6 to 30 ring carbon atoms, a substituted or unsubstituted heteroaryl group having 5 to 30 ring atoms, a substituted or unsubstituted alkyl group having 1 to 30 carbon atoms, a substituted or unsubstituted fluoroalkyl group having 1 to 30 carbon atoms, a substituted or unsubstituted cycloalkyl group having 3 to 30 ring carbon atoms, a substituted or unsubstituted aralkyl group having 7 to 30 carbon atoms, a substituted phosphoryl group, and a substituted silyl group. Ara is preferably a substituted or unsubstituted aryl group having 6 to 30 ring carbon atoms or a substituted or unsubstituted heteroaryl group having 5 to 30 ring atoms.

The formula (1a) is represented by a formula (1aa) below when $A_1$ is a single bond, represented by a formula (1ab) below when $A_1$ is O, represented by a formula (1ac) below when $A_1$ is S, represented by a formula (1ad) below when $A_1$ is $C(R_{2021})(R_{2022})$, represented by a formula (1ae) below when $A_1$ is $Si(R_{2023})(R_{2024})$, represented by a formula (1af) below when $A_1$ is C(=O), represented by a formula (1ag) below when $A_1$ is S(=O), represented by a formula (1ah) below when $A_1$ is $SO_2$, and represented by a formula (1ai) below when $A_1$ is $N(R_{2025})$. In the formulae (1aa) to (1ai), $X_1$ to $X_8$ and $R_{2021}$ to $R_{2925}$ represent the same as described above. Linkages between rings via $A_1$ and $A_2$ in the formulae (1b), (1c), (1e) and (1g) to (1j) are the same as those in the formulae (1aa) to (1ai). In the formula (1aa), when $X_1$ to $X_8$ are a carbon atom bonded with $R_{A1}$ ($C\text{-}R_{A1}$), a plurality of $R_{A1}$ as a substituent preferably form no ring.

[Formula 137]

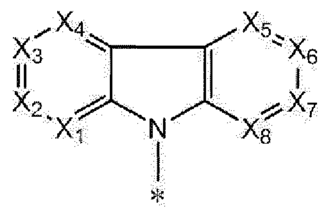

(1aa)

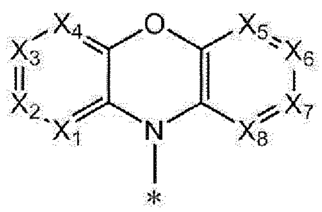

(1ab)

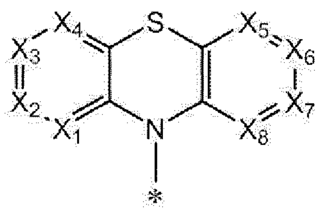

(1ac)

[Formula 138]

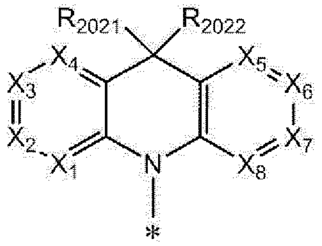

(1ad)

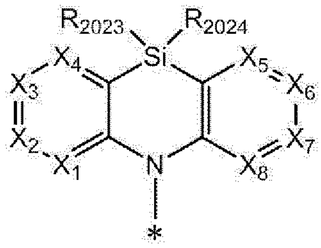

(1ae)

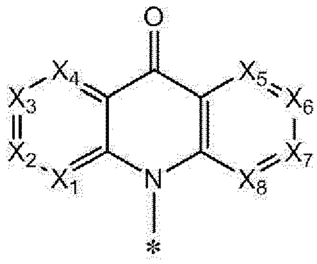

(1af)

[Formula 139]

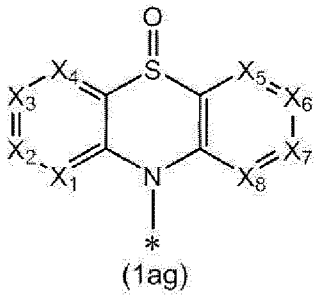

(1ag)

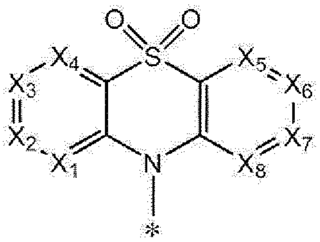

(1ah)

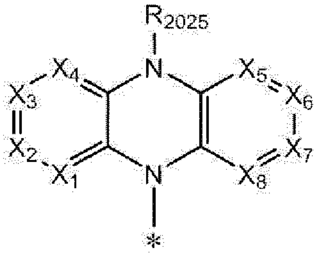

(1ai)

The compound M2 is preferably represented by a formula (221) below.

[Formula 140]

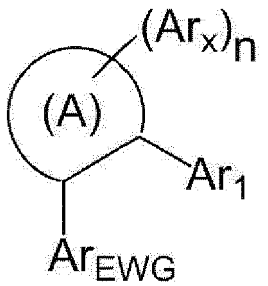

(221)

$Ar_1$, $Ar_{EWG}$, $Ar_x$, n and a ring (A) in the formula (221) respectively represent the same as $Ar_1$, $Ar_{EWG}$, $Ar_x$, n and the ring (A) in the formula (22).

The compound M2 is also preferably represented by a formula (222) below.

[Formula 141]

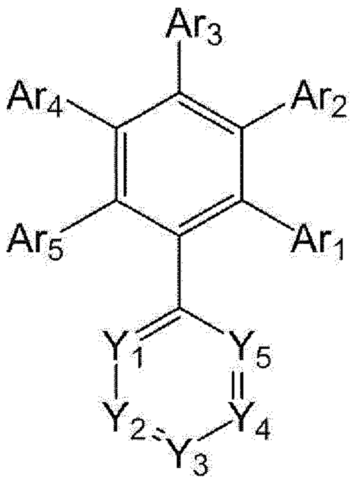

(222)

In the formula (222), $Y_1$ to $Y_5$ are each independently a nitrogen atom (N), a carbon atom bonded with a cyano group (C-CN), or a carbon atom bonded with $R_{A2}$ (C-$R_{A2}$), and at least one of $Y_1$ to $Y_5$ is N or C-CN. A plurality of $R_{A2}$ are mutually the same or different. $R_{A2}$ is each independently a hydrogen atom or a substituent, and $R_{A2}$ as a substituent is a group selected from the group consisting of a substituted or unsubstituted aryl group having 6 to 30 ring carbon atoms, a substituted or unsubstituted heteroaryl group having 5 to 30 ring atoms, a substituted or unsubstituted alkyl group having 1 to 30 carbon atoms, a substituted or unsubstituted fluoroalkyl group having 1 to 30 carbon atoms, a substituted or unsubstituted cycloalkyl group having 3 to 30 ring carbon atoms, a substituted or unsubstituted aralkyl group having 7 to 30 carbon atoms, a substituted phosphoryl group, a substituted silyl group, a cyano group, a nitro group, and a carboxy group; and a plurality of $R_{A2}$ are mutually the same or different.

In the formula (222), $Ar_1$ represents the same as $Ar_1$ in the formula (22).

In the formula (222), $Ar_2$ to $Ar_5$ are each independently a hydrogen atom or a substituent, and $Ar_2$ to $Ar_5$ as a substituent are each independently a group selected from the group consisting of a substituted or unsubstituted aryl group having 6 to 30 ring carbon atoms, a substituted or unsubstituted heteroaryl group having 5 to 30 ring atoms, a substituted or unsubstituted alkyl group having 1 to 30 carbon atoms, a substituted or unsubstituted fluoroalkyl group having 1 to 30 carbon atoms, a substituted or unsubstituted cycloalkyl group having 3 to 30 ring carbon atoms, a substituted or unsubstituted aralkyl group having 7 to 30 carbon atoms, a substituted phosphoryl group, a substituted silyl group, a cyano group, a nitro group, a carboxy group, and groups represented by the formulae (1a) to (1c).

In the formula (222), at least one of $Ar_1$ to $Ar_5$ is a group selected from the group consisting of groups represented by the formulae (1a) to (1c).

The compound M2 is also preferably a compound represented by a formula (11aa), (11bb) or (11cc) below.

[Formula 142]

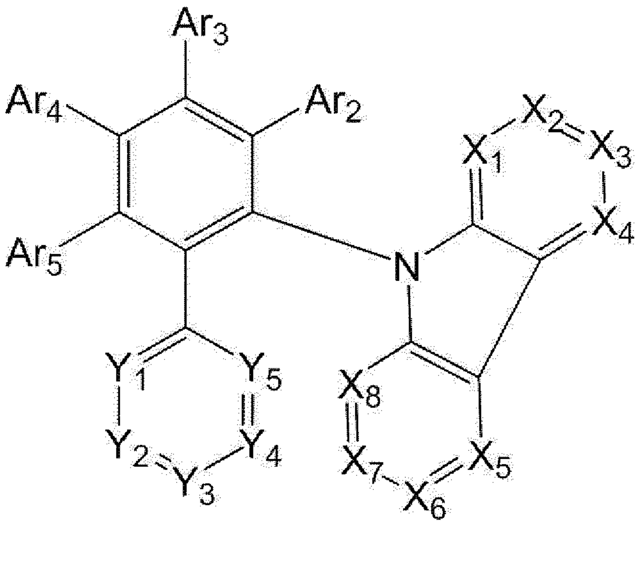

(11aa)

[Formula 143]

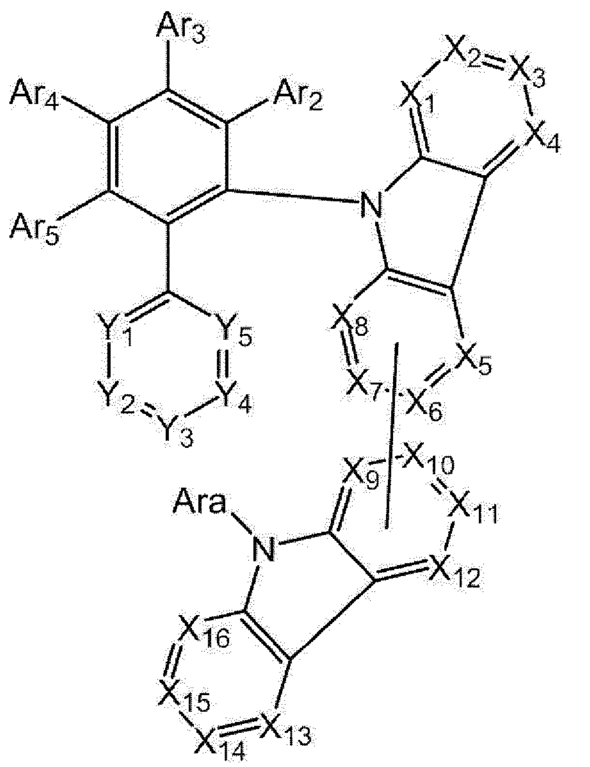

(11bb)

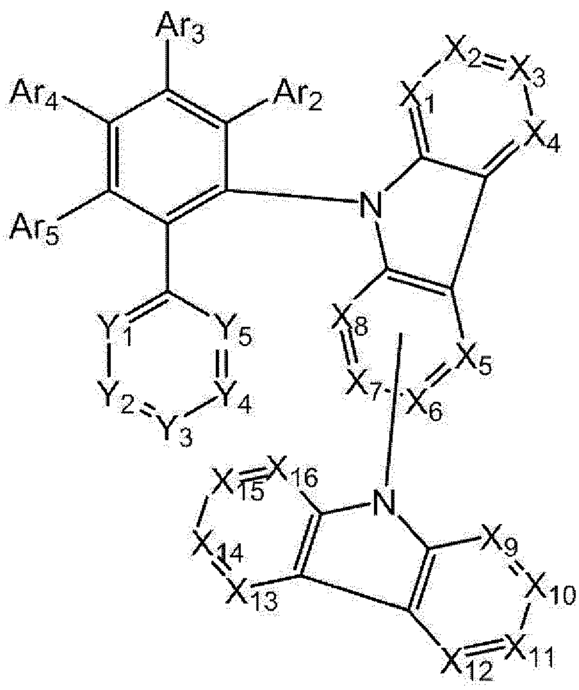

(11cc)

In the formulae (11aa), (11bb) and (11cc), $Y_1$ to $Y_5$, $R_{A2}$, $Ar_2$ to $Ar_5$, $X_1$ to $X_{16}$, $R_{A1}$ and Ara respectively represent the same as the above-described $Y_1$ to $Y_5$, $R_{A2}$, $Ar_2$ to $Ar_5$, $X_1$ to $X_{16}$, $R_{A1}$ and Ara.

The compound M2 is exemplified by a compound represented by a formula (23) below.

[Formula 144]

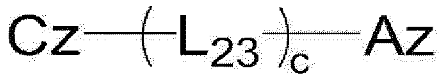

(23)

In the formula (23):

Az is a cyclic structure selected from the group consisting of a substituted or unsubstituted pyridine ring, a substituted or unsubstituted pyrimidine ring, a substituted or unsubstituted triazine ring, and a substituted or unsubstituted pyrazine ring;
c is 0, 1, 2, 3, 4 or 5;
when c is 0, Cz and Az are bonded by a single bond;
when c is 1, 2, 3, 4 or 5, $L_{23}$ is a linking group selected from the group consisting of a substituted or unsubstituted arylene group having 6 to 30 ring carbon atoms, and a substituted or unsubstituted heteroarylene group having 5 to 30 ring atoms;
when c is 2, 3, 4 or 5, a plurality of $L_{23}$ are mutually the same or different;
the plurality of $L_{23}$ are mutually bonded to form a ring or not bonded to form no ring; and
Cz is represented by a formula (23a) below.

[Formula 145]

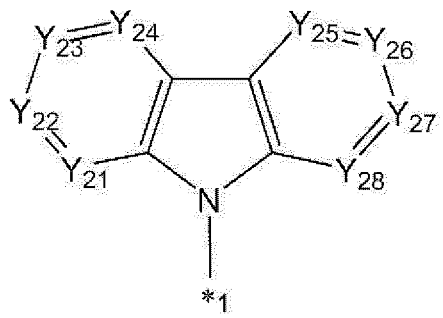

(23a)

In the formula (23a):

$Y_{21}$ to $Y_{28}$ are each independently a nitrogen atom or $CR_{A3}$;
$R_{A3}$ is each independently a hydrogen atom or a substituent, or at least one pair of pairs among a plurality of $R_{A3}$ are mutually bonded to form a ring;
$R_{A3}$ as a substituent is each independently a group selected from the group consisting of a substituted or unsubstituted aryl group having 6 to 30 ring carbon atoms, a substituted or unsubstituted heteroaryl group having 5 to 30 ring atoms, a substituted or unsubstituted alkyl group having 1 to 30 carbon atoms, a substituted or unsubstituted fluoroalkyl group having 1 to 30 carbon atoms, a substituted or unsubstituted cycloalkyl group having 3 to 30 ring carbon atoms, a substituted or unsubstituted aralkyl group having 7 to 30 carbon atoms, a substituted phosphoryl group, a substituted silyl group, a cyano group, a nitro group, and a carboxy group;
a plurality of $R_{A3}$ are mutually the same or different;
*1 represents a bonding position to a carbon atom in a structure of a linking group represented by $L_{23}$, or a bonding position to a carbon atom in a cyclic structure represented by Az.

$Y_{21}$ to $Y_{28}$ are also preferably $CR_{A3}$.
c in the formula (23) is preferably 0 or 1.
Cz is also preferably represented by a formula (23b), (23c) or (23d) below.

[Formula 146]

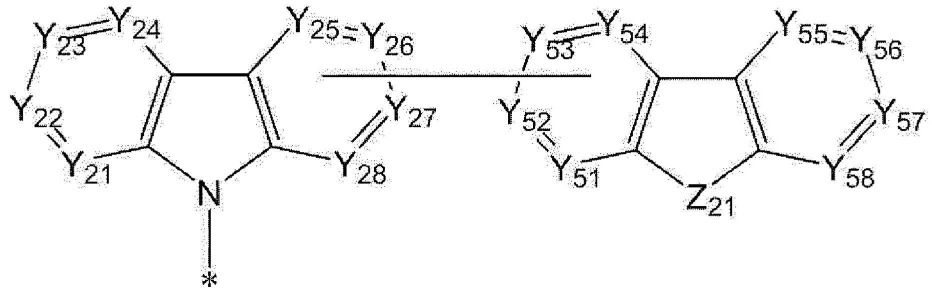

(23b)

[Formula 147]

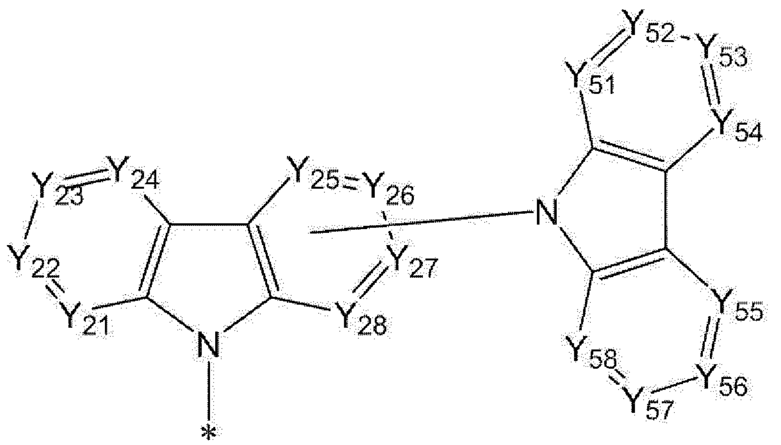

(23c)

[Formula 148]

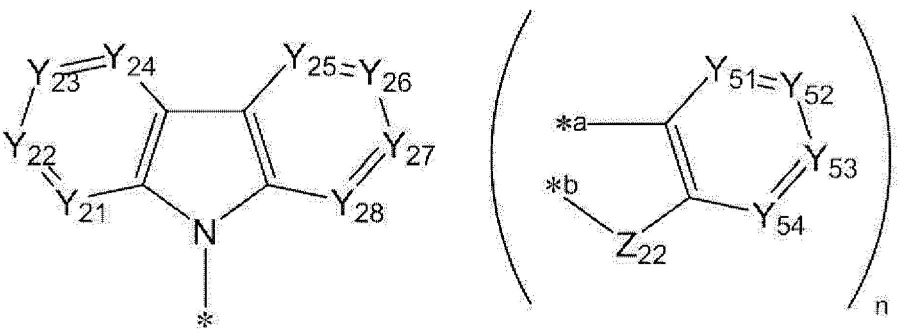

(23d)

In the formulae (23b), (23c) and (23d), $Y_{21}$ to $Y_{28}$ and $Y_{51}$ to $Y_{58}$ are each independently a nitrogen atom or $CR_{A4}$;

in the formula (23b), at least one of $Y_{25}$ to $Y_{28}$ is a carbon atom bonded to one of $Y_{51}$ to $Y_{54}$, and at least one of $Y_{51}$ to $Y_{54}$ is a carbon atom bonded to one of $Y_{25}$ to $Y_{28}$;
in the formula (23c), at least one of $Y_{25}$ to $Y_{28}$ is a carbon atom bonded to a nitrogen atom in a five-membered ring of a nitrogen-containing fused ring including $Y_{51}$ to $Y_{58}$;
in the formula (23d), *a and *b each represent a bonding position to one of $Y_{21}$ to $Y_{28}$, at least one of $Y_{25}$ to $Y_{28}$ is the bonding position represented by *a, and at least one of $Y_{25}$ to $Y_{28}$ is the bonding position represented by *b;
n is 1, 2, 3 or 4;
$R_{A4}$ is each independently a hydrogen atom or a substituent, or at least one pair of pairs of a plurality of $R_{A4}$ are mutually bonded to form a ring;
$R_{A4}$ as a substituent is each independently a substituent selected from the group consisting of a substituted or

unsubstituted aryl group having 6 to 30 ring carbon atoms, a substituted or unsubstituted heteroaryl group having 5 to 30 ring atoms, a substituted or unsubstituted alkyl group having 1 to 30 carbon atoms, a substituted or unsubstituted fluoroalkyl group having 1 to 30 carbon atoms, a substituted or unsubstituted cycloalkyl group having 3 to 30 ring carbon atoms, a substituted or unsubstituted aralkyl group having 7 to 30 carbon atoms, a substituted phosphoryl group, a substituted silyl group, a cyano group, a nitro group, and a carboxy group, a plurality of $R_{A4}$ are mutually the same or different;

$Z_{21}$ and $Z_{22}$ are each independently one selected from the group consisting of an oxygen atom, a sulfur atom, $NR_{45}$ and $CR_{46}R_{47}$;

$R_{45}$ is a hydrogen atom or a substituent;

$R_{46}$ and $R_{47}$ are each independently a hydrogen atom or a substituent, or a pair of $R_{46}$ and $R_{47}$ are mutually bonded to form a ring;

$R_{45}$, $R_{46}$ and $R_{47}$ as a substituent are each independently a substituent selected from the group consisting of a substituted or unsubstituted aryl group having 6 to 30 ring carbon atoms, a substituted or unsubstituted heteroaryl group having 5 to 30 ring atoms, a substituted or unsubstituted alkyl group having 1 to 30 carbon atoms, a substituted or unsubstituted fluoroalkyl group having 1 to 30 carbon atoms, a substituted or unsubstituted cycloalkyl group having 3 to 30 ring carbon atoms, a substituted or unsubstituted aralkyl group having 7 to 30 carbon atoms, a substituted phosphoryl group, a substituted silyl group, a cyano group, a nitro group, and a carboxy group; a plurality of $R_{45}$ are mutually the same or different;

a plurality of $R_{46}$ are mutually the same or different;

a plurality of $R_{47}$ are mutually the same or different; and

* represents a bonding position to a carbon atom in a cyclic structure represented by Az.

$Z_{21}$ is preferably $NR_{45}$.

When $Z_{21}$ is $NR_{45}$, $R_{45}$ is preferably a substituted or unsubstituted aryl group having 6 to 30 ring carbon atoms.

$Z_{22}$ is preferably $NR_{45}$.

When $Z_{22}$ is $NR_{45}$, $R_{45}$ is preferably a substituted or unsubstituted aryl group having 6 to 30 ring carbon atoms.

$Y_{51}$ to $Y_{58}$ are preferably $CR_{A4}$, provided that at least one of $Y_{51}$ to $Y_{58}$ is a carbon atom bonded to a cyclic structure represented by the formula (23a).

It is also preferable that Cz is represented by the formula (23d) and n is 1.

Az is preferably a cyclic structure selected from the group consisting of a substituted or unsubstituted pyrimidine group and a substituted or unsubstituted triazine group.

Az is a cyclic structure selected from the group consisting of a substituted pyrimidine ring and a substituted triazine ring, in which a substituent of each of the substituted pyrimidine ring and the substituted triazine ring is more preferably a group selected from the group consisting of a substituted or unsubstituted aryl group having 6 to 30 ring carbon atoms and a substituted or unsubstituted heteroaryl group having 5 to 30 ring atoms, further preferably a substituted or unsubstituted aryl group having 6 to 30 ring carbon atoms.

When the pyrimidine ring and the triazine ring as Az have a substituted or unsubstituted aryl group as a substituent, the aryl group preferably has 6 to 20 ring carbon atoms, more preferably 6 to 14 ring carbon atoms, further preferably 6 to 12 ring carbon atoms.

When Az has a substituted or unsubstituted aryl group as a substituent, the substituent is preferably a group selected from the group consisting of a substituted or unsubstituted phenyl group, a substituted or unsubstituted biphenyl group, a substituted or unsubstituted naphthyl group, a substituted or unsubstituted phenanthryl group, a substituted or unsubstituted terphenyl group, and a substituted or unsubstituted fluorenyl group, more preferably a group selected from the group consisting of a substituted or unsubstituted phenyl group, a substituted or unsubstituted biphenyl group, and a substituted or unsubstituted naphthyl group.

When Az has a substituted or unsubstituted heteroaryl group as a substituent, the substituent is preferably a substituent selected from the group consisting of a substituted or unsubstituted carbazolyl group, a substituted or unsubstituted dibenzofuranyl group, and a substituted or unsubstituted dibenzothiophenyl group.

$R_{A4}$ is each independently a hydrogen atom or a substituent. $R_{A4}$ as a substituent is preferably a substituent selected from the group consisting of a substituted or unsubstituted aryl group having 6 to 30 ring carbon atoms and a substituted or unsubstituted heteroaryl group having 5 to 30 ring atoms.

When $R_{A4}$ as a substituent is a substituted or unsubstituted aryl group having 6 to 30 ring carbon atoms, $R_{A4}$ as a substituent is preferably a substituent selected from the group consisting of a substituted or unsubstituted phenyl group, a substituted or unsubstituted biphenyl group, a substituted or unsubstituted naphthyl group, a substituted or unsubstituted phenanthryl group, a substituted or unsubstituted terphenyl group, and a substituted or unsubstituted fluorenyl group, more preferably a substituent selected from the group consisting of a substituted or unsubstituted phenyl group, a substituted or unsubstituted biphenyl group, and a substituted or unsubstituted naphthyl group.

When $R_{A4}$ as a substituent is a substituted or unsubstituted heteroaryl group having 5 to 30 ring atoms, $R_{A4}$

as a substituent is preferably a substituent selected from the group consisting of a substituted or unsubstituted carbazolyl group, a substituted or unsubstituted dibenzofuranyl group, and a substituted or unsubstituted dibenzothiophenyl group.

$R_{45}$, $R_{46}$ and $R_{47}$ as a substituent are preferably each independently a substituent selected from the group consisting of a substituted or unsubstituted aryl group having 6 to 30 ring carbon atoms, a substituted or unsubstituted heteroaryl group having 5 to 30 ring atoms, and a substituted or unsubstituted alkyl group having 1 to 30 carbon atoms.

Manufacturing Method of Compound M2 According to Exemplary Embodiment

The compound M2 can be manufactured by a known method.

Specific Examples of Compound M2

Specific examples of the compound M2 of the exemplary embodiment include compounds below. It should however be noted that the invention is not limited to the specific examples of the compound.

[Formula 149]

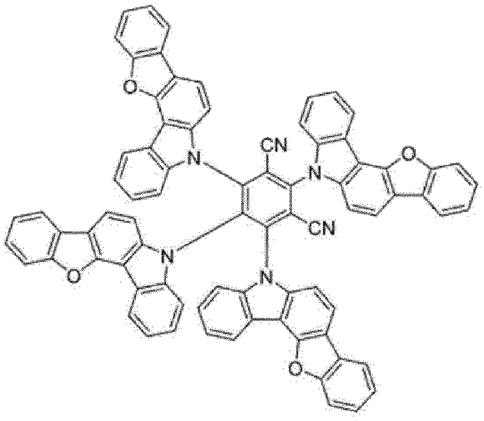

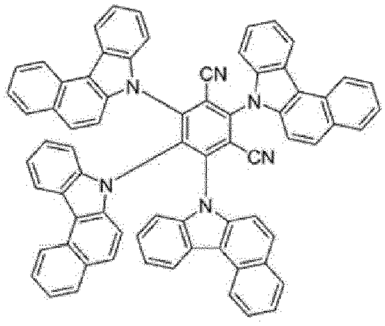

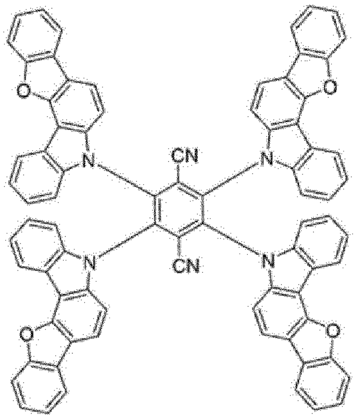

[Formula 150]

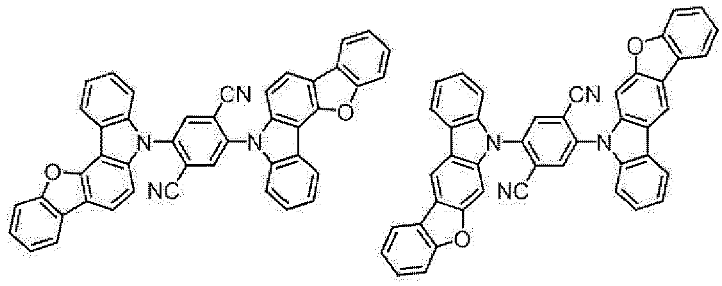

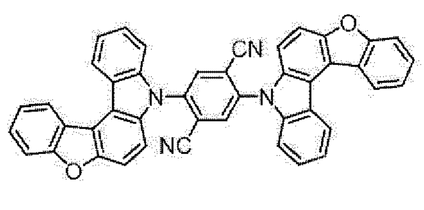

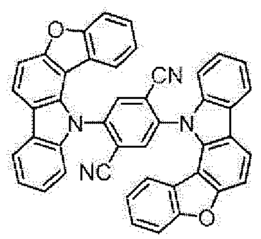

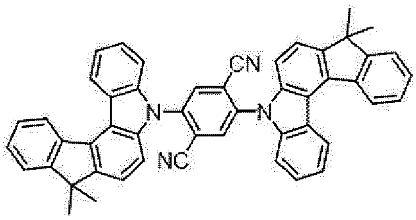

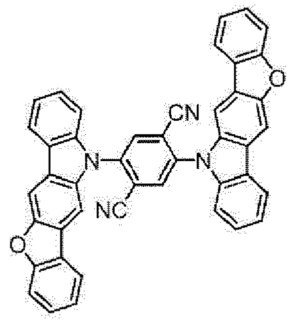

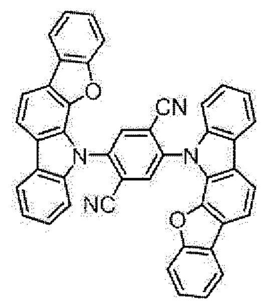

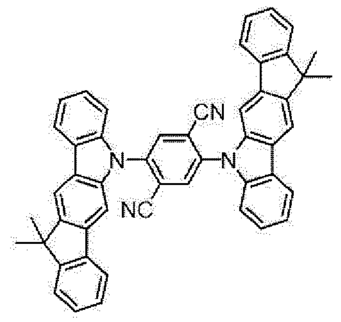

[Formula 151]

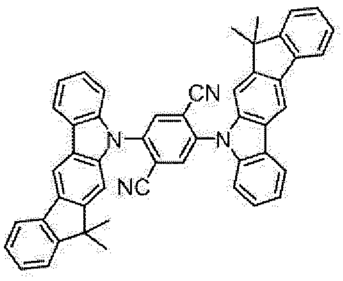
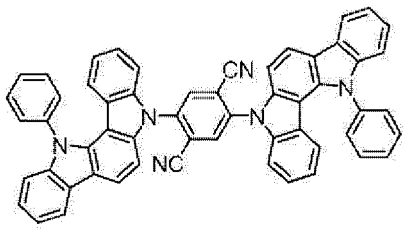
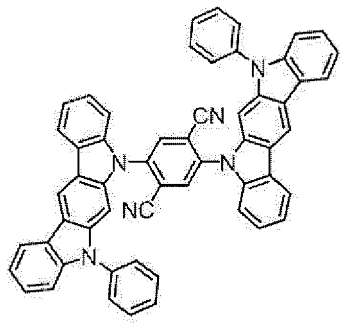
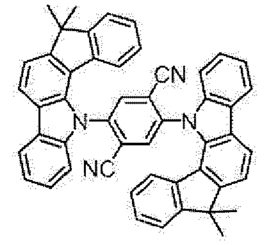
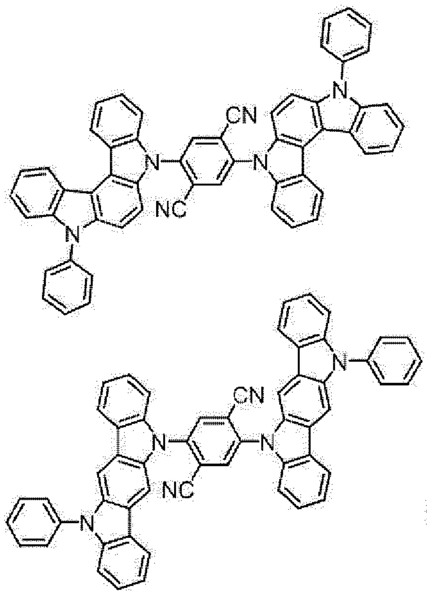
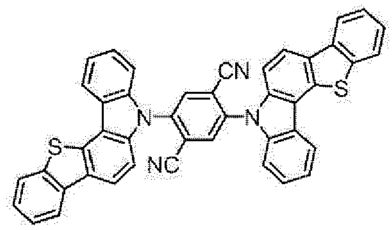
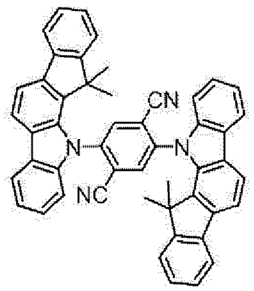
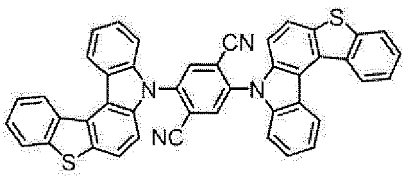

[Formula 152]

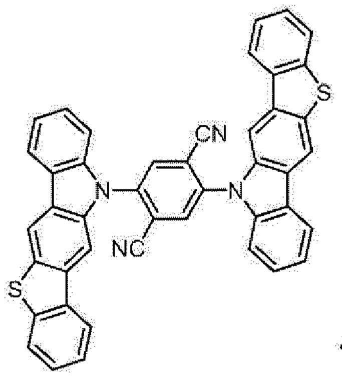
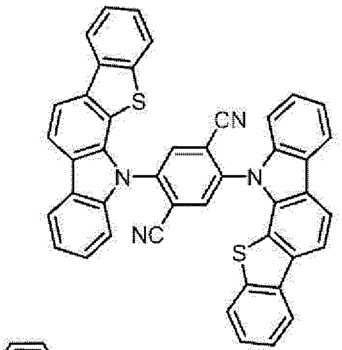
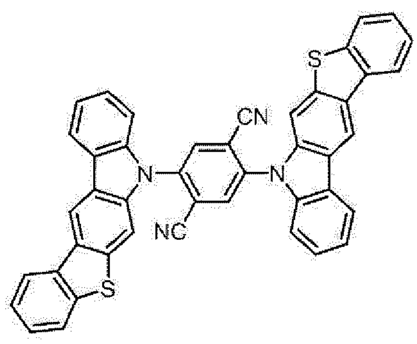
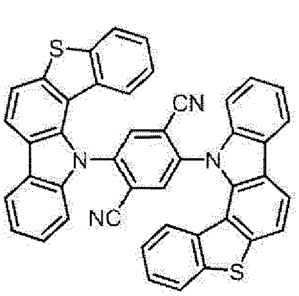
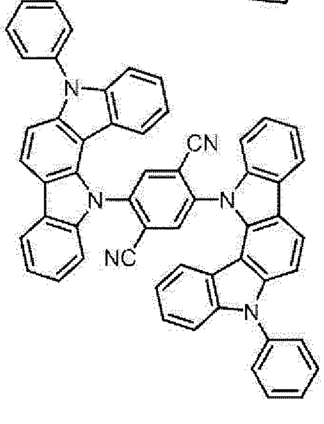
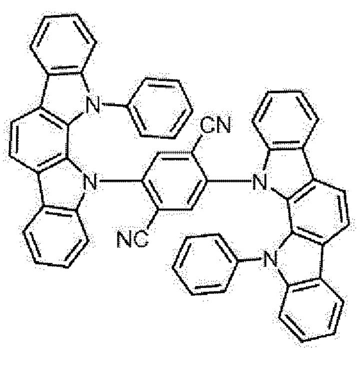

[Formula 153]

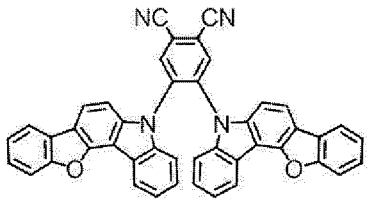
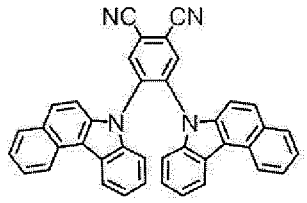
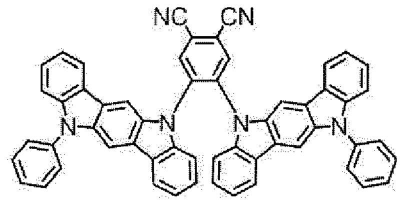
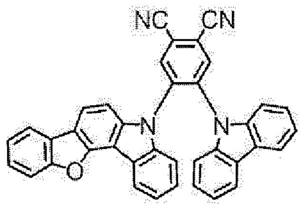
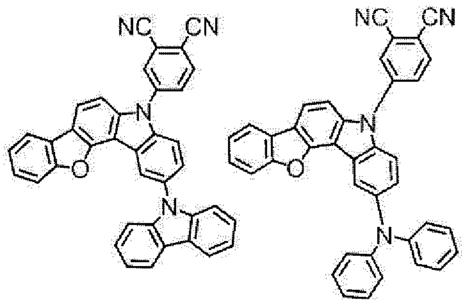
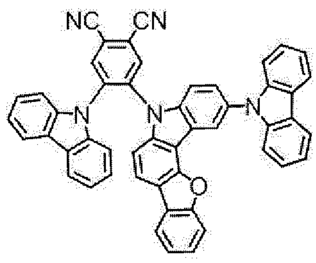

[Formula 154]

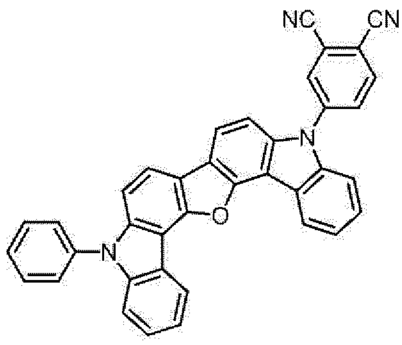
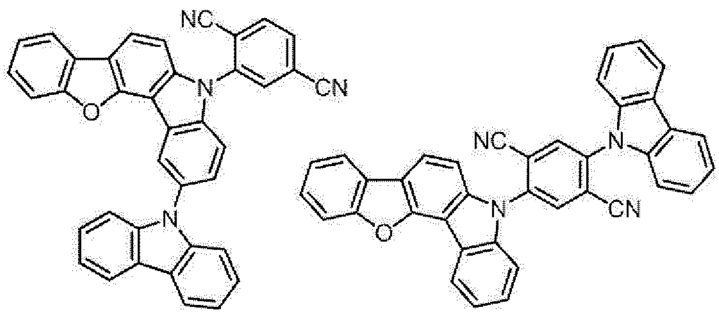
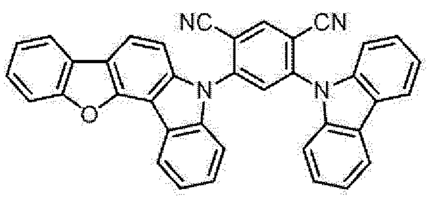
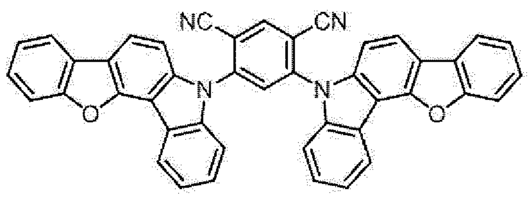
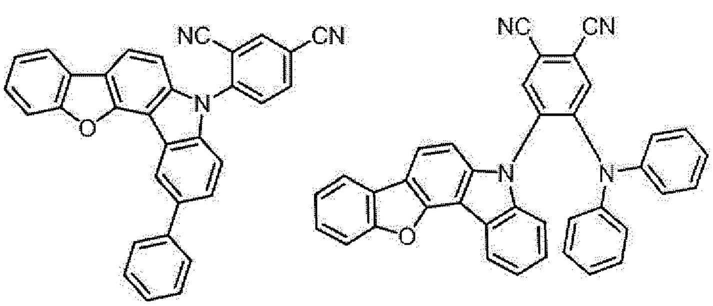
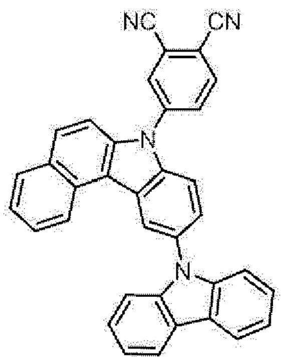

[Formula 155]

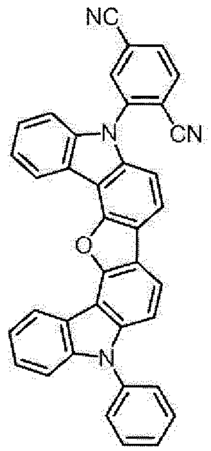
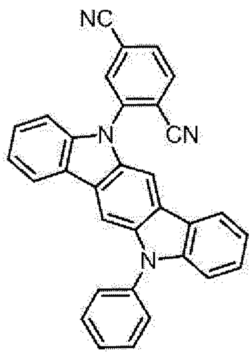

[Formula 156]

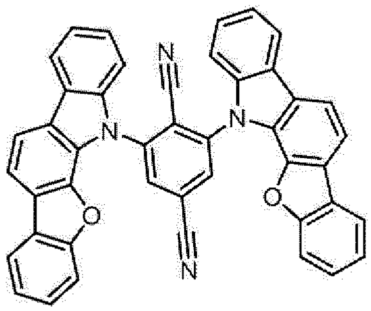
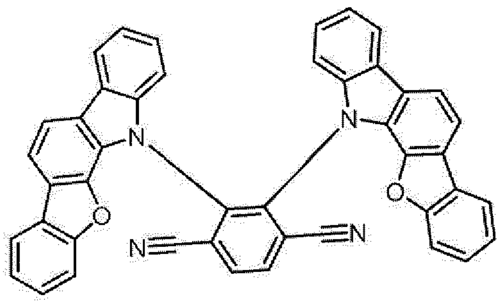

[Formula 157]

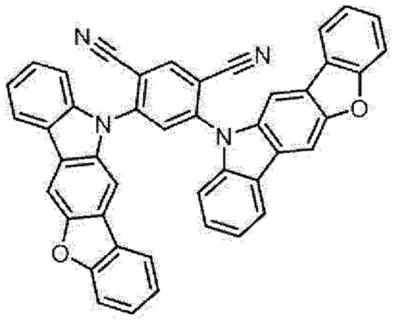
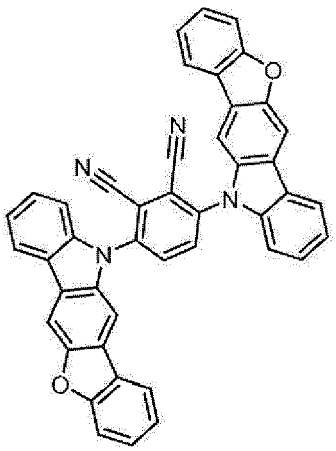
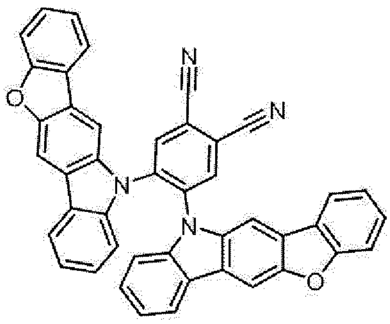

[Formula 158]

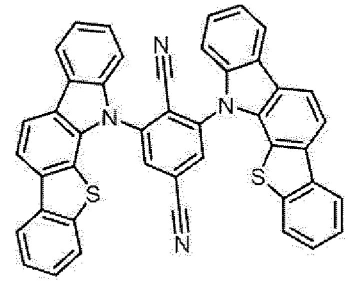
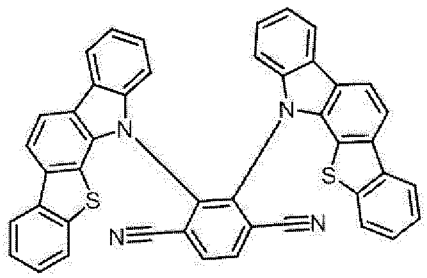
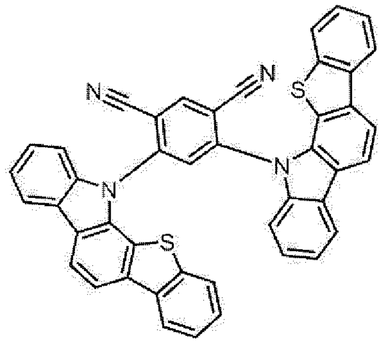

[Formula 159]

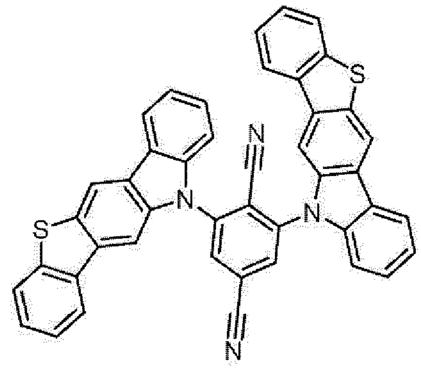
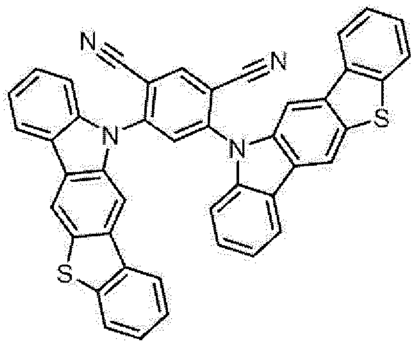

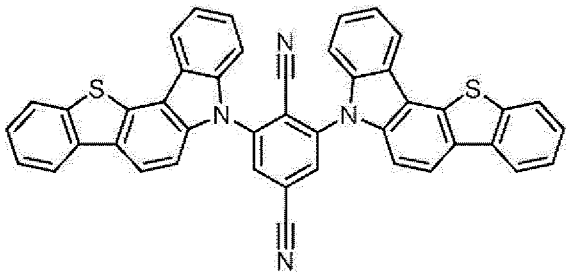

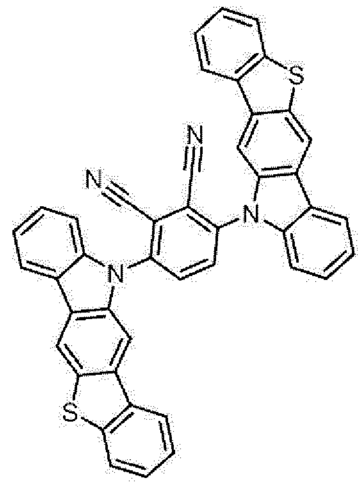
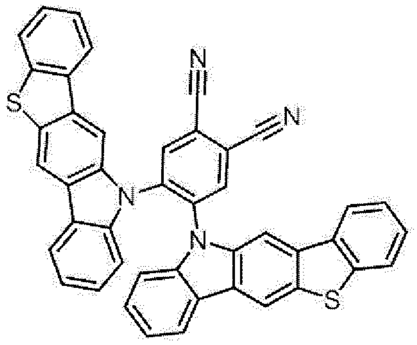

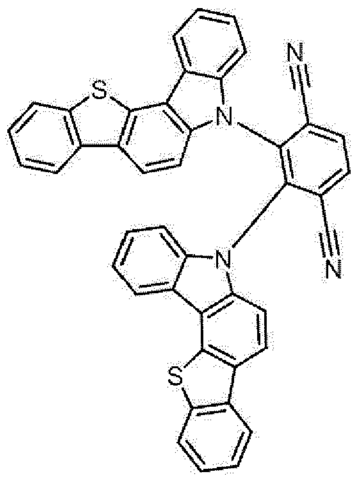
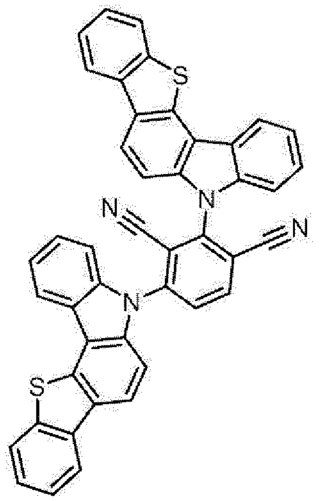

[Formula 160]

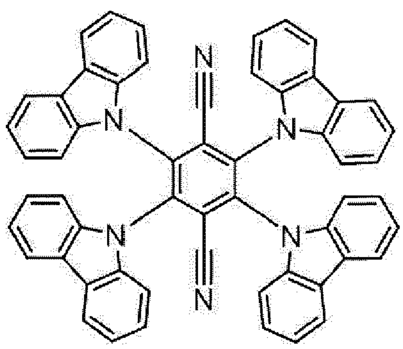

[Formula 161]

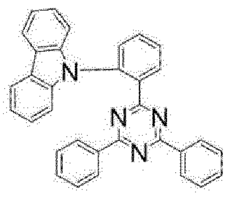

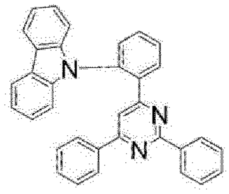

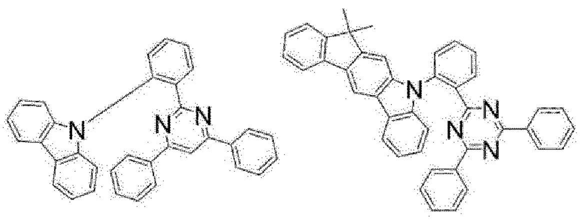

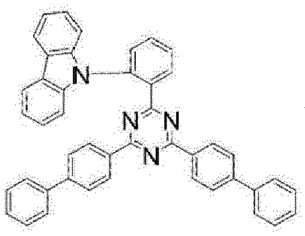

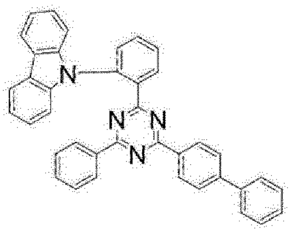

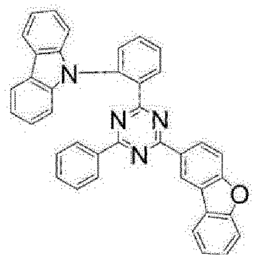

[Formula 162]

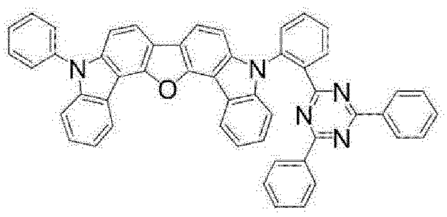

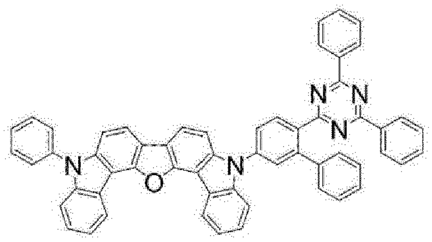

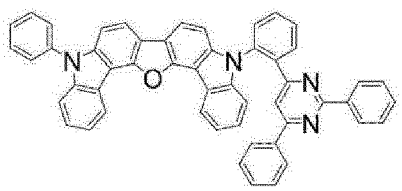

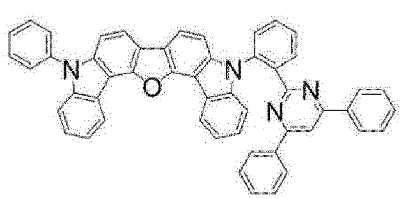

[Formula 163]

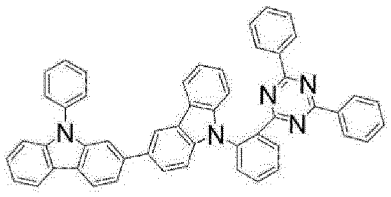

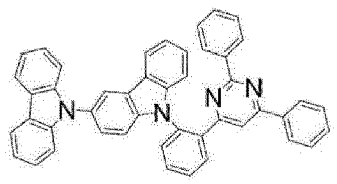

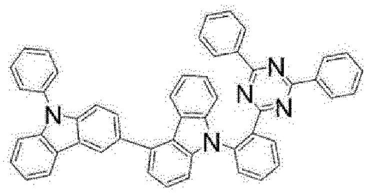

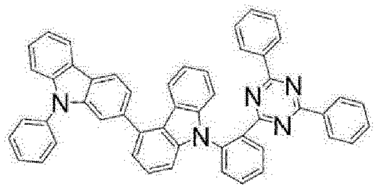

[Formula 164]

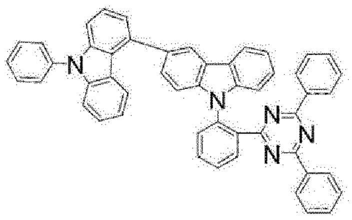

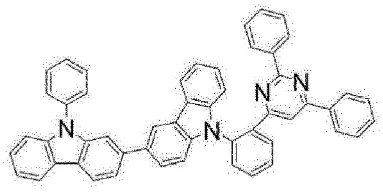

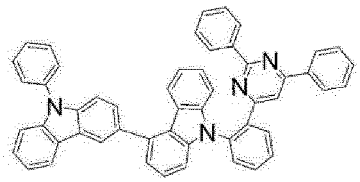

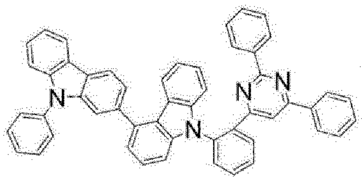

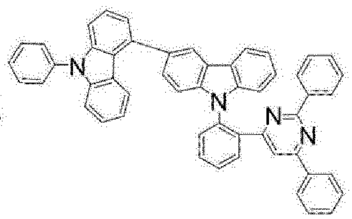

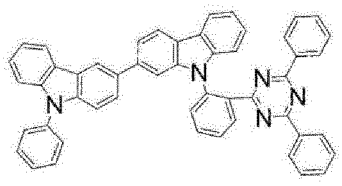

[Formula 165]

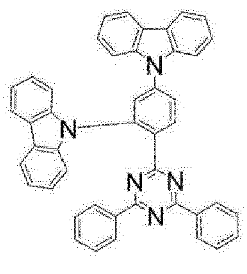

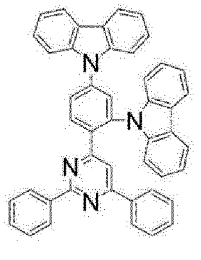

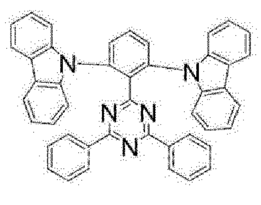

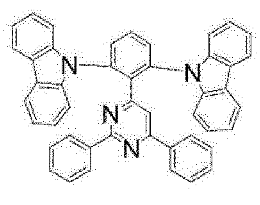

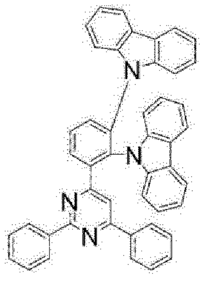

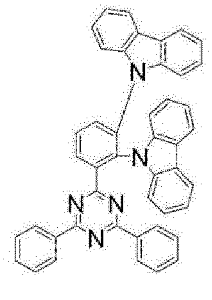

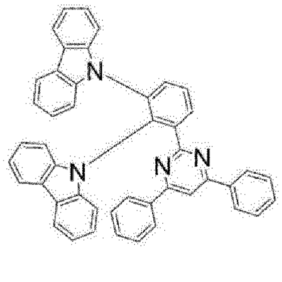

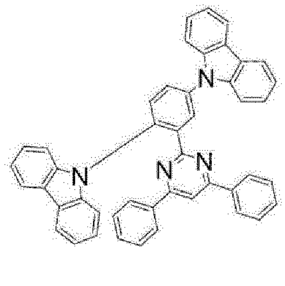

[Formula 166]

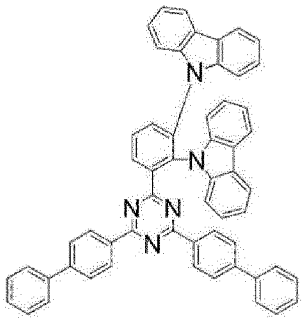
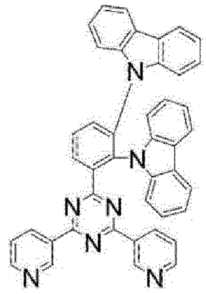
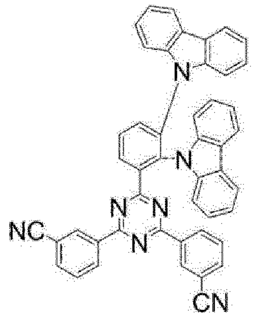

[Formula 167]

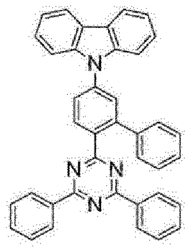
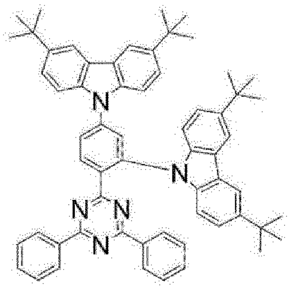
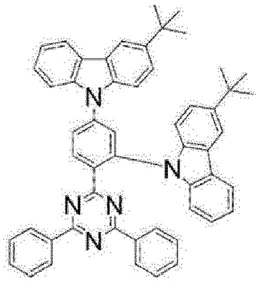

[Formula 168]

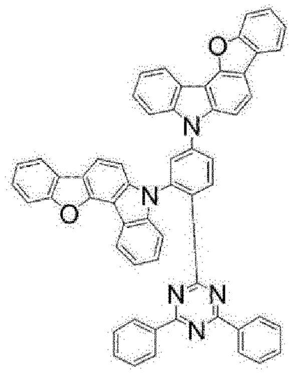
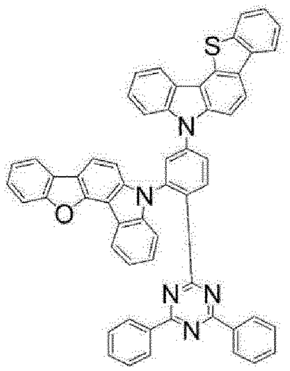

[Formula 169]

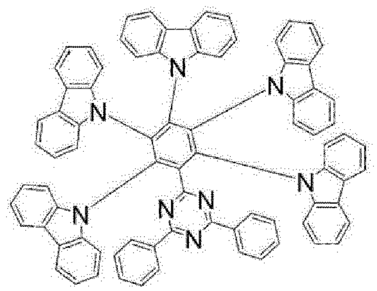

[Formula 170]

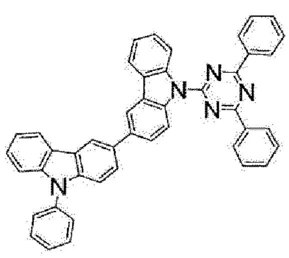

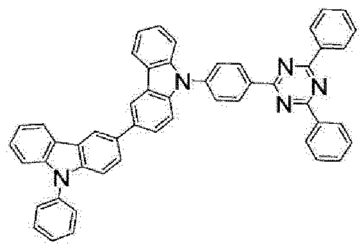

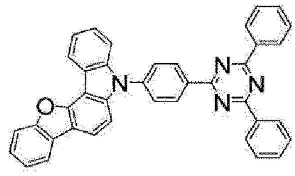

[Formula 171]

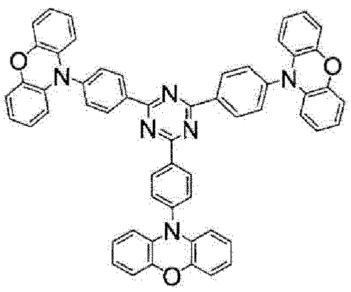

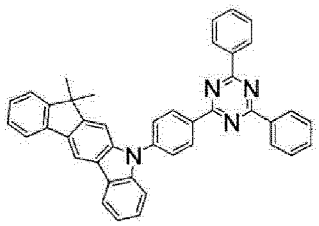

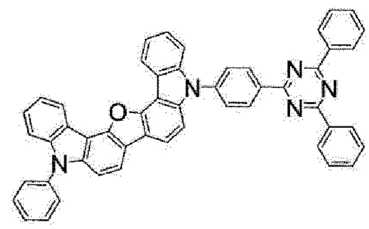

[Formula 172]

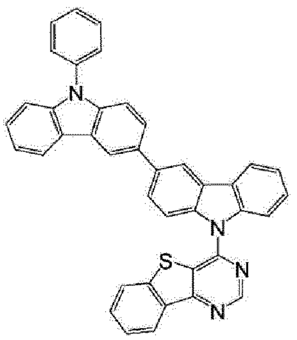

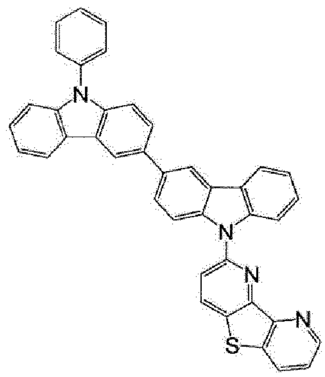

[Formula 173]

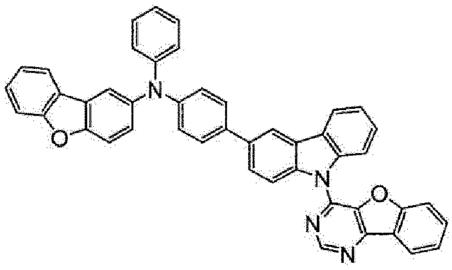

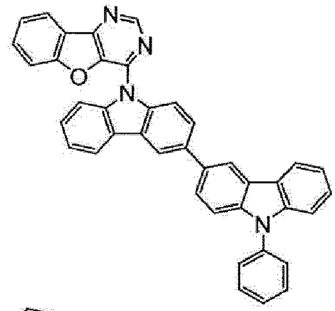

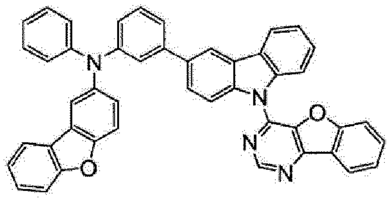

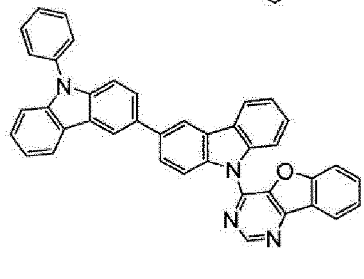

[Formula 174]

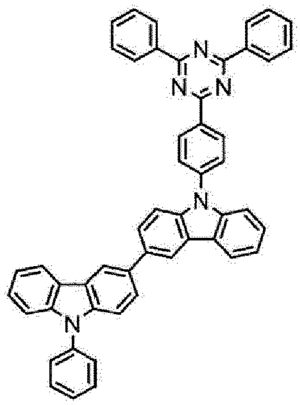

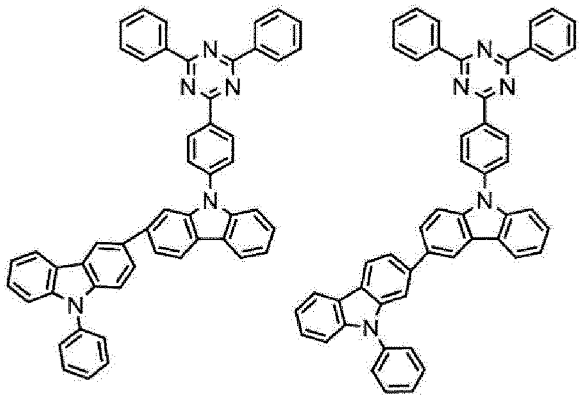

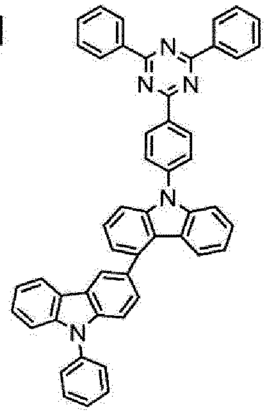

[Formula 175]

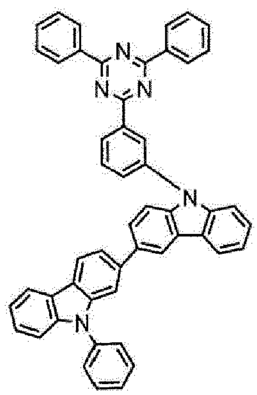

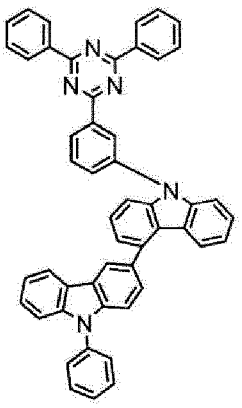

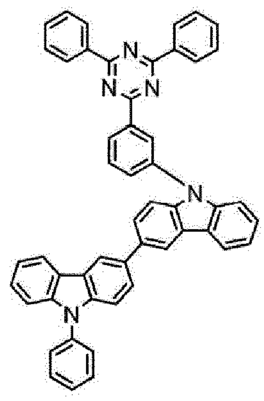

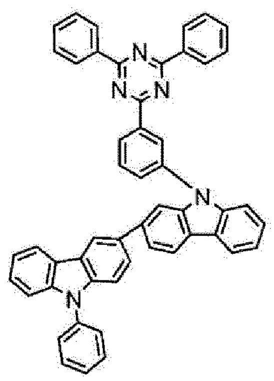

[Formula 176]

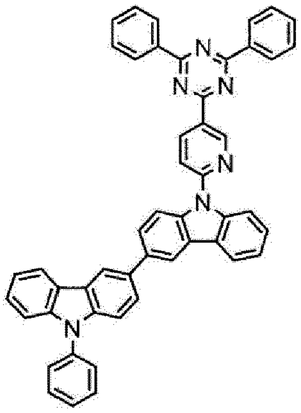

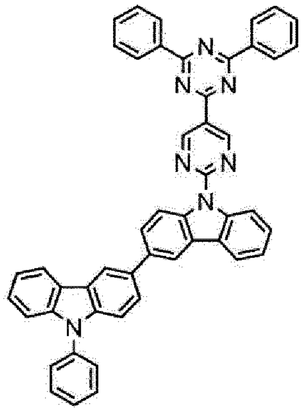

[Formula 177]

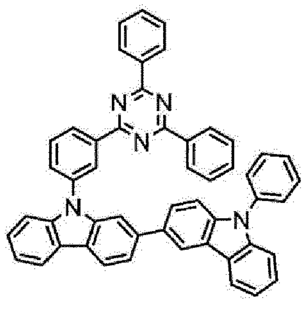

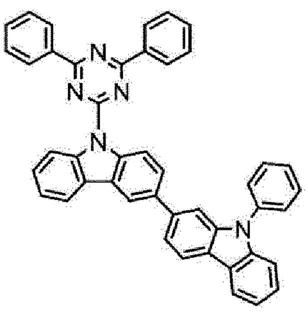

[Formula 178]

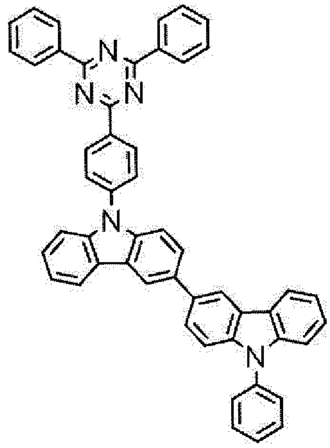

[Formula 179]

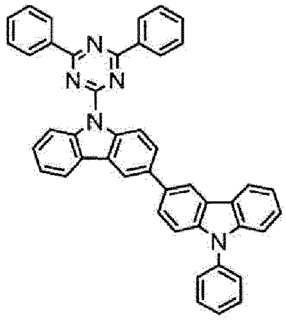 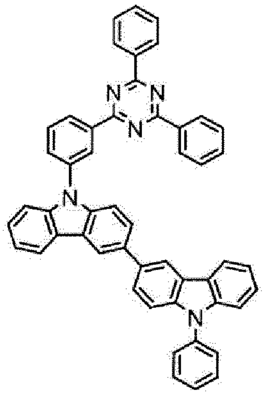 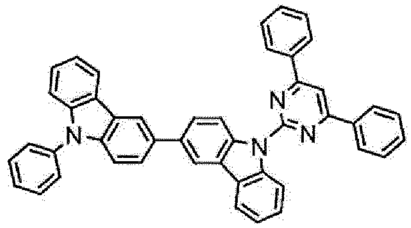

Relationship between Compound M3 and Compound M2 in Emitting Layer

In the organic EL device according to the exemplary embodiment, the singlet energy $S_1$(M2) of the compound M2 and a singlet energy $S_1$(M3) of the compound M3 satisfy a relationship of a numerical formula (Numerical Formula 1) below.

$$S_1(M3) > S_1(M2) \ldots \text{(Numerical Formula 1)}$$

An energy gap $T_{77K}$(M3) at 77K of the compound M3 is preferably larger than an energy gap $T_{77K}$(M2) at 77K of the compound M2. In other words, a relationship of the following numerical formula (Numerical Formula 11) is preferably satisfied.

$$T_{77K}(M3) > T_{77K}(M2) \ldots \text{(Numerical Formula 11)}$$

When the organic EL device according to the exemplary embodiment emits light, it is preferable that the compound M3 does not mainly emit light in the emitting layer.

Relationship between Triplet Energy and Energy Gap at 77K

Here, a relationship between a triplet energy and an energy gap at 77K will be described. In the exemplary embodiment, the energy gap at 77K is different from a typical triplet energy in some aspects.

The triplet energy is measured as follows. Firstly, a solution in which a compound (measurement target) is dissolved in an appropriate solvent is encapsulated in a quartz glass tube to prepare a sample. A phosphorescence spectrum (ordinate axis: phosphorescent luminous intensity, abscissa axis: wavelength) of the sample is measured at a low temperature (77K). A tangent is drawn to the rise of the phosphorescence spectrum close to the short-wavelength region. The triplet energy is calculated by a predetermined equation on a basis of a wavelength value at an intersection of the tangent and the abscissa axis.

Here, the thermally activated delayed fluorescent compound among the compounds of the exemplary embodiment is preferably a compound having a small $\Delta$ST. When $\Delta$ST is small, intersystem crossing and inverse intersystem crossing are likely to occur even at a low temperature (77K), so that the singlet state and the triplet state coexist. As a result, the spectrum to be measured in the same manner as the above includes emission from both the singlet state and the triplet state. Although it is difficult to distinguish the emission from the singlet state from the emission from the triplet state, the value of the triplet energy is basically considered dominant.

Accordingly, in the exemplary embodiment, the triplet energy is measured by the same method as a typical triplet energy T, but a value measured in the following manner is referred to as an energy gap $T_{77K}$ in order to differentiate the measured energy from the typical triplet energy in a strict meaning. The measurement target compound is dissolved in EPA (diethylether: isopentane : ethanol = 5 : 5 : 2 in volume ratio) at a concentration of 10 $\mu$mol/L, and the obtained solution is encapsulated in a quartz cell to provide a measurement sample. A phosphorescence spectrum (ordinate axis: phosphorescent luminous intensity, abscissa axis: wavelength) of the measurement sample is measured at a low temperature (77K). A tangent is drawn to the rise of the phosphorescence spectrum close to the short-wavelength region. An energy amount is calculated by a conversion equation (F1) below on a basis of a wavelength value $\lambda_{edge}$ [nm] at an intersection of the tangent and the abscissa axis. The calculated energy amount is defined as an energy gap $T_{77K}$ at 77K.

$$\text{Conversion Equation (F1): } T_{77K}\ [eV] = 1239.85/\lambda_{edge}$$

The tangent to the rise of the phosphorescence spectrum close to the short-wavelength region is drawn as follows. While moving on a curve of the phosphorescence spectrum from the short-wavelength region to the local maximum value closest to the short-wavelength region among the local maximum values of the phosphorescence spectrum, a tangent is checked at each point on the curve toward the long-wavelength region of the phosphorescence spectrum. An inclination of the tangent is increased along the rise of the curve (i.e., a value of the ordinate axis is increased). A tangent drawn at a point of the local maximum inclination (i.e., a tangent at an inflection point) is defined as the tangent to the rise of the phosphorescence spectrum close to the short-wavelength region.

A local maximum point where a peak intensity is 15% or less of the maximum peak intensity of the spectrum is not counted as the above-mentioned local maximum peak intensity closest to the short-wavelength region. The tangent drawn at a point that is closest to the local maximum peak intensity closest to the short-wavelength region and where the inclination of the curve is the local maximum is defined as a tangent to the rise of the phosphorescence spectrum close to the short-wavelength region.

For phosphorescence measurement, a spectrophotofluorometer body F-4500 (manufactured by Hitachi High-Technologies Corporation) is usable. Any device for phosphorescence measurement is usable. A combination of a cooling unit, a low temperature container, an excitation light source and a light-receiving unit may be used for phosphorescence measurement.

Singlet Energy S1

A method of measuring a singlet energy $S_1$ with use of a solution (occasionally referred to as a solution method) is exemplified by a method below.

A toluene solution of a measurement target compound at a concentration of 10 μmol/L is prepared and put in a quartz cell. An absorption spectrum (ordinate axis: absorption intensity, abscissa axis: wavelength) of the thus-obtained sample is measured at a normal temperature (300K). A tangent is drawn to the fall of the absorption spectrum close to the long-wavelength region, and a wavelength value λedge (nm) at an intersection of the tangent and the abscissa axis is assigned to a conversion equation (F2) below to calculate singlet energy.

$$\text{Conversion Equation (F2): } S_1\ [eV] = 1239.85/\lambda edge$$

Any device for measuring absorption spectrum is usable. For instance, a spectrophotometer (U3310 manufactured by Hitachi, Ltd.) is usable.

The tangent to the fall of the absorption spectrum close to the long-wavelength region is drawn as follows. While moving on a curve of the absorption spectrum from the local maximum value closest to the long-wavelength region, among the local maximum values of the absorption spectrum, in a long-wavelength direction, a tangent at each point on the curve is checked. An inclination of the tangent is decreased and increased in a repeated manner as the curve falls (i.e., a value of the ordinate axis is decreased). A tangent drawn at a point where the inclination of the curve is the local minimum closest to the long-wavelength region (except when absorbance is 0.1 or less) is defined as the tangent to the fall of the absorption spectrum close to the long-wavelength region.

The local maximum absorbance of 0.2 or less is not counted as the above-mentioned local maximum absorbance closest to the long-wavelength region.

In the exemplary embodiment, a difference ($S_1$-$T_{77K}$) between the singlet energy $S_1$ and the energy gap $T_{77K}$ at 77K is defined as ΔST.

In the exemplary embodiment, a difference ΔST(M2) between the singlet energy $S_1$(M2) of the compound M2 and the energy gap $T_{77K}$(M2) at 77K of the compound M2 is preferably less than 0.3 eV, more preferably less than 0.2 eV, further preferably less than 0.1 eV, more further preferably less than 0.01 eV. In other words, ΔST(M2) preferably satisfies a relationship of one of numerical formulae (Numerical Formula 1A to Numerical Formula 1D).

$$\Delta ST(M2) = S_1(M2) - T_{77K}(M2) < 0.3\ eV \ldots \text{(Numerical Formula 1A)}$$

$$\Delta ST(M2) = S_1(M2) - T_{77K}(M2) < 0.2\ eV \ldots \text{(Numerical Formula 1B)}$$

$$\Delta ST(M2) = S_1(M2) - T_{77K}(M2) < 0.1\ eV \ldots \text{(Numerical Formula 1C)}$$

$$\Delta ST(M2) = S_1(M2) - T_{77K}(M2) < 0.01\ eV \ldots \text{(Numerical Formula 1D)}$$

Film Thickness of Emitting Layer

A film thickness of the emitting layer of the organic EL device in the exemplary embodiment is preferably in a range from 5 nm to 50 nm, more preferably in a range from 7 nm to 50 nm, most preferably in a range from 10 nm to 50 nm. When the film thickness of the emitting layer is 5 nm or more, the formation of the emitting layer and the adjustment of the chromaticity are easy. When the film thickness of the emitting layer is 50 nm or less, an increase in the drive voltage is likely to be reducible.

Content Ratios of Compounds in Emitting Layer

Content ratios of the compounds M2 and M3 in the emitting layer preferably fall, for instance, within a range below.
The content ratio of the compound M2 is preferably in a range from 10 mass% to 80 mass%, more preferably in a range from 10 mass% to 60 mass%, further preferably in a range from 20 mass% to 60 mass%.
The content ratio of the compound M3 is preferably in a range from 20 mass% to 90 mass%, more preferably in a range from 40 mass% to 90 mass%, further preferably in a range from 40 mass% to 80 mass%.
It should be noted that the emitting layer of the exemplary embodiment may further contain material(s) other than the compounds M2 and M3.
The emitting layer may include a single type of the compound M2 or may include two or more types of the compound M2. The emitting layer may include a single type of the compound M3 or may include two or more types of the compound M3.
Fig. 4 shows a relationship in energy level and energy transfer between the compound M3 and the compound M2 in the emitting layer.
In Fig. 4, S0 represents a ground state. $S_1$ (M2) represents the lowest singlet state of the compound M2. T1(M2) represents the lowest triplet state of the compound M2. S1(M3) represents the lowest singlet state of the compound M3. T1(M3) represents the lowest triplet state of the compound M3.
Dashed arrows in Fig. 4 show energy transfer between the excited states. An energy transfer occurs by Förster transfer from the lowest singlet state S1 of the compound M3 to the lowest singlet state S1 of the compound M2 or an energy transfer occurs by Dexter transfer from the lowest triplet state T1 of the compound M3 to the lowest triplet state T1 of the compound M2.
Further, when a material having a small ΔST(M2) is used as the compound M2, inverse intersystem crossing can be caused by a heat energy from the lowest triplet state T1 to the lowest singlet state S1 in the compound M2. Consequently, fluorescence from the lowest singlet state S1 of the compound M2 can be observed. It is inferred that the internal quantum efficiency can be theoretically raised up to 100% also by using delayed fluorescence by the TADF mechanism.
The organic EL device according to the exemplary embodiment contains the delayed fluorescent compound M2 and the compound M3 having the singlet energy larger than that of the compound M2 in the emitting layer.
According to the exemplary embodiment, a high-performance organic EL device such as an organic EL device emitting light with a high efficiency is achievable.
The organic EL device according to the exemplary embodiment is applicable to an electronic device such as a display device and a light-emitting device.
An arrangement of an organic EL device will be further described below.

Substrate

The substrate is used as a support for the organic EL device. For instance, glass, quartz, plastics and the like are usable for the substrate. A flexible substrate is also usable. The flexible substrate is a bendable substrate, which is exemplified by a plastic substrate. Examples of the material for the plastic substrate include polycarbonate, polyarylate, polyethersulfone, polypropylene, polyester, polyvinyl fluoride, polyvinyl chloride, polyimide, and polyethylene naphthalate. Moreover, an inorganic vapor deposition film is also usable.

Anode

Metal, an alloy, an electrically conductive compound, a mixture thereof, or the like having a large work function (specifically, 4.0 eV or more) is preferably used as the anode formed on the substrate. Specific examples of the material include ITO (Indium Tin Oxide), indium oxide-tin oxide containing silicon or silicon oxide, indium oxide-zinc oxide, indium oxide containing tungsten oxide and zinc oxide, and graphene. In addition, gold (Au), platinum (Pt), nickel (Ni), tungsten (W), chrome (Cr), molybdenum (Mo), iron (Fe), cobalt (Co), copper (Cu), palladium (Pd), titanium (Ti), and nitrides of a metal material (e.g., titanium nitride) are usable.

The material is typically formed into a film by a sputtering method. For instance, the indium oxide-zinc oxide can be formed into a film by the sputtering method using a target in which zinc oxide in a range from 1 mass% to 10 mass% is added to indium oxide. Moreover, for instance, the indium oxide containing tungsten oxide and zinc oxide can be formed by the sputtering method using a target in which tungsten oxide in a range from 0.5 mass% to 5 mass% and zinc oxide in a range from 0.1 mass% to 1 mass% are added to indium oxide. In addition, the anode may be formed by a vacuum deposition method, a coating method, an inkjet method, a spin coating method or the like.

Among the EL layers formed on the anode, since the hole injecting layer adjacent to the anode is formed of a composite material into which holes are easily injectable irrespective of the work function of the anode, a material usable as an electrode material (e.g., metal, an alloy, an electrically conductive compound, a mixture thereof, and the elements belonging to the group 1 or 2 of the periodic table) is also usable for the anode.

A material having a small work function such as elements belonging to Groups 1 and 2 in the periodic table of the elements, specifically, an alkali metal such as lithium (Li) and cesium (Cs), an alkaline earth metal such as magnesium (Mg), calcium (Ca) and strontium (Sr), alloys (e.g., MgAg and AlLi) including the alkali metal or the alkaline earth metal, a rare earth metal such as europium (Eu) and ytterbium (Yb), and alloys including the rare earth metal are also usable for the anode. It should be noted that the vacuum deposition method and the sputtering method are usable for forming the anode using the alkali metal, alkaline earth metal and the alloy thereof. Further, when a silver paste is used for the anode, the coating method and the inkjet method are usable.

Cathode

It is preferable to use metal, an alloy, an electroconductive compound, a mixture thereof, or the like having a small work function (specifically, 3.8 eV or less) for the cathode. Examples of the material for the cathode include elements belonging to Groups 1 and 2 in the periodic table of the elements, specifically, the alkali metal such as lithium (Li) and cesium (Cs), the alkaline earth metal such as magnesium (Mg), calcium (Ca) and strontium (Sr), alloys (e.g., MgAg and AlLi) including the alkali metal or the alkaline earth metal, the rare earth metal such as europium (Eu) and ytterbium (Yb), and alloys including the rare earth metal.

It should be noted that the vacuum deposition method and the sputtering method are usable for forming the cathode using the alkali metal, alkaline earth metal and the alloy thereof. Further, when a silver paste is used for the cathode, the coating method and the inkjet method are usable.

By providing the electron injecting layer, various conductive materials such as Al, Ag, ITO, graphene, and indium oxide-tin oxide containing silicon or silicon oxide may be used for forming the cathode regardless of the work function. The conductive materials can be formed into a film using the sputtering method, inkjet method, spin coating method and the like.

Hole Injecting Layer

The hole injecting layer is a layer containing a substance exhibiting a high hole injectability. Examples of the substance exhibiting a high hole injectability include molybdenum oxide, titanium oxide, vanadium oxide, rhenium oxide, ruthenium oxide, chrome oxide, zirconium oxide, hafnium oxide, tantalum oxide, silver oxide, tungsten oxide, and manganese oxide.

In addition, the examples of the highly hole-injectable substance further include: an aromatic amine compound, which is a low-molecule organic compound, such as 4,4',4"-tris(N,N-diphenylamino)triphenylamine (abbreviation: TDATA), 4,4',4"-tris[N-(3-methylphenyl)-N-phenylamino]triphenylamine (abbreviation: MTDATA), 4,4'-bis[N-(4-diphenylaminophenyl)-N-phenylamino]biphenyl (abbreviation: DPAB), 4,4'-bis(N-{4-[N'-(3-methylphenyl)-N'-phenylamino]phenyl}-N-phenylamino)biphenyl (abbreviation: DNTPD), 1,3,5-tris[N-(4-diphenylaminophenyl)-N-phenylamino]benzene (abbreviation: DPA3B), 3-[N-(9-phenylcarbazole-3-yl)-N-phenylamino]-9-phenylcarbazole (abbreviation: PCzPCA1), 3,6-bis[N-(9-phenylcarbazole-3-yl)-N-phenylamino]-9-phenylcarbazole (abbreviation: PCzPCA2), and 3-[N-(1-naphthyl)-N-(9-phenylcarbazole-3-yl)amino]-9-phenylcarbazole (abbreviation: PCzPCN1); and dipyrazino[2,3-f:20,30-h]quinoxaline-2,3,6,7,10,11-hexacarbonitrile (HAT-CN).

In addition, a high polymer compound (e.g., oligomer, dendrimer and polymer) is usable as the substance

exhibiting a high hole injectability. Examples of the high-molecule compound include poly(N-vinylcarbazole) (abbreviation: PVK), poly(4-vinyltriphenylamine) (abbreviation: PVTPA), poly[N-(4-{N'-[4-(4-diphenylamino)phenyl]phenyl-N'-phenylamino}phenyl)methacrylamide] (abbreviation: PTPDMA), and poly[N,N'-bis(4-butylphenyl)-N,N'-bis(phenyl)benzidine] (abbreviation: Poly-TPD). Moreover, an acid-added high polymer compound such as poly(3,4-ethylenedioxythiophene)/poly(styrene sulfonic acid) (PEDOT/PSS) and polyaniline/poly(styrene sulfonic acid) (PAni/PSS) are also usable.

Hole Transporting Layer

The hole transporting layer is a layer containing a highly hole-transporting substance. An aromatic amine compound, carbazole derivative, anthracene derivative and the like are usable for the hole transporting layer. Specific examples of a material for the hole transporting layer include an aromatic amine compound such as 4,4'-bis[N-(1-naphthyl)-N-phenylamino]biphenyl (abbreviation: NPB), N,N'-bis(3-methylphenyl)-N,N'-diphenyl-[1,1'-biphenyl]-4,4'-diamine (abbreviation: TPD), 4-phenyl-4'-(9-phenylfluorene-9-yl)triphenylamine (abbreviation: BAFLP), 4,4'-bis[N-(9,9-dimethylfluorene-2-yl)-N-phenylamino]biphenyl (abbreviation: DFLDPBi), 4,4',4"-tris(N,N-diphenylamino)triphenylamine (abbreviation: TDATA), 4,4',4"-tris[N-(3-methylphenyl)-N-phenylamino]triphenylamine (abbreviation: MTDATA), and 4,4'-bis[N-(spiro-9,9'-bifluorene-2-yl)-N-phenylamino]biphenyl (abbreviation: BSPB). The above-described substances mostly have a hole mobility of $10^{-6}$ $cm^2/(V{\cdot}s)$ or more.

For the hole transporting layer, a carbazole derivative such as CBP, 9-[4-(N-carbazolyl)]phenyl-10-phenylanthracene (CzPA), and 9-phenyl-3-[4-(10-phenyl-9-anthryl)phenyl]-9H-carbazole (PCzPA) and an anthracene derivative such as t-BuDNA, DNA, and DPAnth may be used. A high polymer compound such as poly(N-vinylcarbazole) (abbreviation: PVK) and poly(4-vinyltriphenylamine) (abbreviation: PVTPA) is also usable.

However, in addition to the above substances, any substance exhibiting a higher hole transportability than an electron transportability may be used. It should be noted that the layer containing the substance exhibiting a high hole transportability may be not only a single layer but also a laminate of two or more layers formed of the above substance(s).

When the hole transporting layer includes two or more layers, one of the layers with a larger energy gap is preferably provided closer to the emitting layer. Examples of the material with a larger energy gap include compounds EBL and EBL-2 used in later-described Examples.

Electron Transporting Layer

The electron transporting layer is a layer containing a highly electron-transporting substance. For the electron transporting layer, 1) a metal complex such as an aluminum complex, beryllium complex, and zinc complex, 2) a hetero aromatic compound such as imidazole derivative, benzimidazole derivative, azine derivative, carbazole derivative, and phenanthroline derivative, and 3) a high polymer compound are usable. Specifically, as a low-molecule organic compound, a metal complex such as Alq, tris(4-methyl-8-quinolinato)aluminum (abbreviation: $Almq_3$), bis(10-hydroxybenzo[h]quinolinato)beryllium (abbreviation: $BeBq_2$), BAlq, Znq, ZnPBO and ZnBTZ is usable. In addition to the metal complex, a heteroaromatic compound such as 2-(4-biphenylyl)-5-(4-tert-butylphenyl)-1,3,4-oxadiazole (abbreviation: PBD), 1,3-bis[5-(ptert-butylphenyl)-1,3,4-oxadiazole-2-yl]benzene (abbreviation: OXD-7), 3-(4-tert-butylphenyl)-4-phenyl-5-(4-biphenylyl)-1,2,4-triazole (abbreviation: TAZ), 3-(4-tert-butylphenyl)-4-(4-ethylphenyl)-5-(4-biphenylyl)-1,2,4-triazole (abbreviation: p-EtTAZ), bathophenanthroline (abbreviation: BPhen), bathocuproine (abbreviation: BCP), and 4,4'-bis(5-methylbenzoxazole-2-yl)stilbene (abbreviation: BzOs) is usable. In the exemplary embodiment, a benzimidazole compound is preferably usable. The above-described substances mostly have an electron mobility of $10^{-6}$ $cm^2/Vs$ or more. It should be noted that any substance other than the above substance may be used for the electron transporting layer as long as the substance exhibits a higher electron transportability than the hole transportability. The electron transporting layer may be provided in the form of a single layer or a laminate of two or more layers of the above substance(s).

Further, a high polymer compound is usable for the electron transporting layer. For instance, poly[(9,9-dihexylfluorene-2,7-diyl)-co-(pyridine-3,5-diyl)] (abbreviation: PF-Py), poly[(9,9-dioctylfluorene-2,7-diyl)-co-(2,2'-bipyridine-6,6'-diyl)] (abbreviation: PF-BPy) and the like are usable.

Electron Injecting Layer

The electron injecting layer is a layer containing a highly electron-injectable substance. Examples of a material for the electron injecting layer include an alkali metal, alkaline earth metal and a compound thereof, examples of which include lithium (Li), cesium (Cs), calcium (Ca), lithium fluoride (LiF), cesium fluoride (CsF), calcium fluoride ($CaF_2$), and lithium oxide (LiOx). In addition, the alkali metal, alkaline earth metal or the compound thereof may be added to the substance exhibiting the electron transportability in use. Specifically, for instance, magnesium (Mg) added to Alq may

be used. In this case, the electrons can be more efficiently injected from the cathode.

Alternatively, the electron injecting layer may be provided by a composite material in a form of a mixture of the organic compound and the electron donor. Such a composite material exhibits excellent electron injectability and electron transportability since electrons are generated in the organic compound by the electron donor. In this case, the organic compound is preferably a material excellent in transporting the generated electrons. Specifically, the above examples (e.g., the metal complex and the hetero aromatic compound) of the substance forming the electron transporting layer are usable. As the electron donor, any substance exhibiting electron donating property to the organic compound is usable. Specifically, the electron donor is preferably alkali metal, alkaline earth metal and rare earth metal such as lithium, cesium, magnesium, calcium, erbium and ytterbium. The electron donor is also preferably alkali metal oxide and alkaline earth metal oxide such as lithium oxide, calcium oxide, and barium oxide. Moreover, a Lewis base such as magnesium oxide is usable. Further, the organic compound such as tetrathiafulvalene (abbreviation: TTF) is usable.

Layer Formation Method

A method for forming each layer of the organic EL device in the exemplary embodiment is subject to no limitation except for the above particular description. However, known methods of dry film-forming such as vacuum deposition, sputtering, plasma or ion plating and wet film-forming such as spin coating, dipping, flow coating or ink-jet are applicable.

Film Thickness

A thickness of each of the organic layers in the organic EL device according to the exemplary embodiment is not limited except for the above particular description. In general, the thickness preferably ranges from several nanometers to 1 $\mu$m because excessively small film thickness is likely to cause defects (e.g. pin holes) and excessively large thickness leads to the necessity of applying high voltage and consequent reduction in efficiency.

The inventors have found that a high-performance organic EL device is achievable by using the compound M3 represented by the formula (3) with the delayed fluorescent compound M2.

Since the compound M3 has both carbazole being at least one of a hole-injectable moiety or a hole transporting moiety and $A_{30}$ (preferably benzofurocarbazole) being higher in at least one of hole injectability or hole transportability than carbazole, the compound M3 is suitably higher in at least one of the hole injectability or hole transportability than a compound having only carbazole and no $A_{30}$ (e.g., CBP).

In the exemplary embodiment, it is considered that by containing, in the emitting layer, the compound M3 having at least one of hole injectability or hole transportability that is appropriately enhanced, an appropriate amount of holes can be supplied to the emitting layer to bring the amount of holes in line with an amount of electrons. As a result, it is considered that the device is highly improved in efficiency. In general, a moiety having a large absolute value of an energy level of LUMO (lowest unoccupied molecular orbital) is often introduced into a molecule of a delayed fluorescent compound to reduce $\Delta$ST, which would also often increase an absolute value of an energy level of HOMO (highest occupied molecular orbital) of the entire molecule thereof. However, having a large absolute value of an energy level of HOMO may hinder hole injection into the emitting layer, failing to supply an appropriate amount of holes to the emitting layer.

In the exemplary embodiment, it is considered that since the emitting layer contains the compound M3 having at least one of hole injectability or hole transportability that is appropriately enhanced and the delayed fluorescent compound M2, hole injection into the emitting layer is prevented from being hindered to supply an appropriate amount of holes to the emitting layer, thus highly improving the device in efficiency.

Accordingly, according to the exemplary embodiment, a high-performance organic EL device is achievable.

"High performance" in the exemplary embodiment means at least one of light emission with a long lifetime, an improvement in a luminous efficiency, or a decrease in a drive voltage.

Second Exemplary Embodiment

An arrangement of an organic EL device according to a second exemplary embodiment will be described below. In the description of the second exemplary embodiment, the same components as those in the first exemplary embodiment are denoted by the same reference signs and names to simplify or omit an explanation of the components. In the second exemplary embodiment, any materials and compounds that are not specified may be the same as those in the first exemplary embodiment.

The organic EL device according to the second exemplary embodiment is different from the organic EL device according to the first exemplary embodiment in that the emitting layer further includes a fluorescent compound M1. The second exemplary embodiment is the same as the first exemplary embodiment in other respects.

In other words, in the second exemplary embodiment, the emitting layer contains the compound M3 represented

by the formula (3), the delayed fluorescent compound M2, and the fluorescent compound M1.

In this arrangement, the compound M1 is preferably a dopant material, the compound M2 is preferably a host material, and the compound M3 is preferably not a dopant material.

Compound M1

The emitting layer of the exemplary embodiment includes the fluorescent compound M1.

The compound M1 of the exemplary embodiment is not a phosphorescent metal complex. The compound M1 of the exemplary embodiment is preferably not a heavy-metal complex. The compound M1 of the exemplary embodiment is preferably not a metal complex.

A fluorescent material is usable as the compound M1 of the exemplary embodiment. Specific examples of the fluorescent material include a bisarylaminonaphthalene derivative, aryl-substituted naphthalene derivative, bisarylaminoanthracene derivative, aryl-substituted anthracene derivative, bisarylaminopyrene derivative, aryl-substituted pyrene derivative, bisarylamino chrysene derivative, aryl-substituted chrysene derivative, bisarylaminofluoranthene derivative, aryl-substituted fluoranthene derivative, indenoperylene derivative, acenaphthofluoranthene derivative, compound including a boron atom, pyromethene boron complex compound, compound having a pyromethene skeleton, metal complex of the compound having a pyrromethene skeleton, diketopyrrolopyrrole derivative, perylene derivative, and naphthacene derivative.

The compound M1 of the exemplary embodiment is preferably a compound represented by a formula (10) below.

[Formula 180]

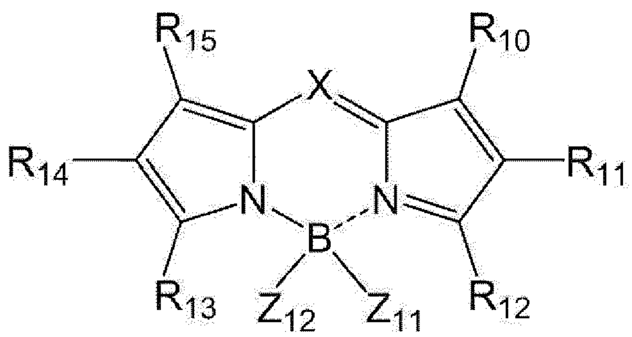

(10)

In the formula (10):

X is a nitrogen atom, or a carbon atom bonded to Y;
Y is a hydrogen atom or a substituent;
$R_{10}$ to $R_{15}$ are each independently a hydrogen atom or a substituent, or at least one pair of a pair of $R_{10}$ and $R_{11}$, a pair of $R_{11}$ and $R_{12}$, a pair of $R_{13}$ and $R_{14}$, or a pair of $R_{14}$ and $R_{15}$ are mutually bonded to form a ring;
Y and $R_{10}$ to $R_{15}$ as a substituent are each independently selected from the group consisting of a substituted or unsubstituted alkyl group having 1 to 30 carbon atoms, a substituted or unsubstituted alkyl halide group having 1 to 30 carbon atoms, a substituted or unsubstituted cycloalkyl group having 3 to 30 ring carbon atoms, a substituted or unsubstituted aryl group having 6 to 30 ring carbon atoms, a substituted or unsubstituted alkoxy group having 1 to 30 carbon atoms, a substituted or unsubstituted alkoxy halide group having 1 to 30 carbon atoms, a substituted or unsubstituted alkylthio group having 1 to 30 carbon atoms, a substituted or unsubstituted aryloxy group having 6 to 30 ring carbon atoms, a substituted or unsubstituted arylthio group having 6 to 30 ring carbon atoms, a substituted or unsubstituted alkenyl group having 2 to 30 carbon atoms, a substituted or unsubstituted aralkyl group having 7 to 30 carbon atoms, a substituted or unsubstituted heteroaryl group having 5 to 30 ring atoms, a halogen atom, a carboxy group, a substituted or unsubstituted ester group, a substituted or unsubstituted carbamoyl group, a substituted or unsubstituted amino group, a nitro group, a cyano group, a substituted or unsubstituted silyl group, and a substituted or unsubstituted siloxanyl group;
$Z_{11}$ and $Z_{12}$ are each independently a substituent, or $Z_{11}$ and $Z_{12}$ are mutually bonded to form a ring; and
$Z_{11}$ and $Z_{12}$ as a substituent are each independently selected from the group consisting of a halogen atom, a substituted or unsubstituted alkyl group having 1 to 30 carbon atoms, a substituted or unsubstituted alkyl halide group having 1 to 30 carbon atoms, a substituted or unsubstituted aryl group having 6 to 30 ring carbon atoms, a substituted or unsubstituted alkoxy group having 1 to 30 carbon atoms, a substituted or unsubstituted alkoxy halide group having 1 to 30 carbon atoms, and a substituted or unsubstituted aryloxy group having 6 to 30 ring carbon atoms.

In the formula (10), for instance, when a pair of $R_{14}$ and $R_{15}$ are mutually bonded to form a ring, the compound M1 is represented by a formula (11) below.

[Formula 181]

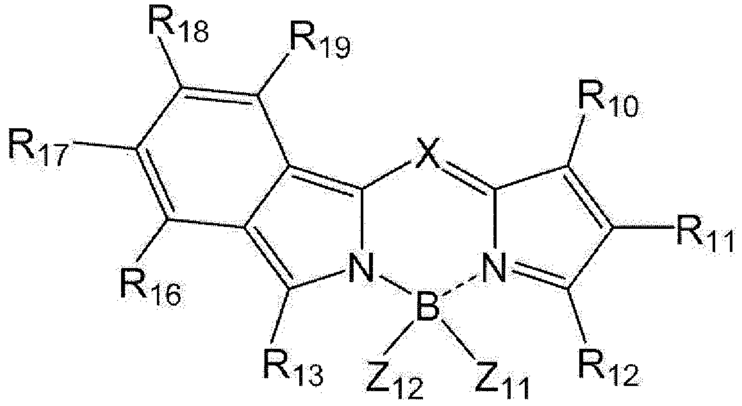

(11)

In the formula (11): X, Y, $R_{10}$ to $R_{13}$, $Z_{11}$ and $Z_{12}$ respectively represent the same as X, Y, $R_{10}$ to $R_{13}$, $Z_{11}$ and $Z_{12}$ in the formula (10); $R_{16}$ to $R_{19}$ are each independently a hydrogen atom or a substituent; and $R_{16}$ to $R_{19}$ as a substituent each independently represent the same as $R_{10}$ to $R_{13}$ as a substituent.

In the formula (10), when $Z_{11}$ and $Z_{12}$ are mutually bonded to form a ring, the compound M1 is represented by, for instance, a formula (10A) or (10B) below. However, a structure of the compound M1 is not limited to structures below.

[Formula 182]

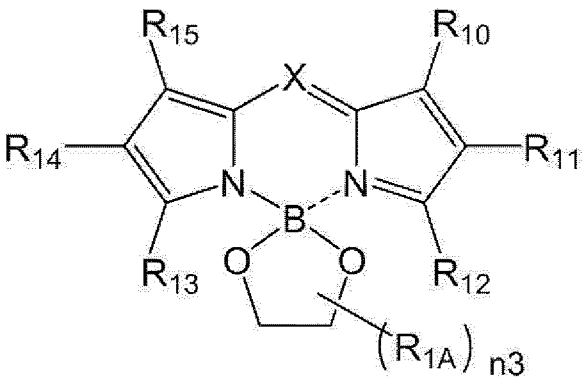

(10A)

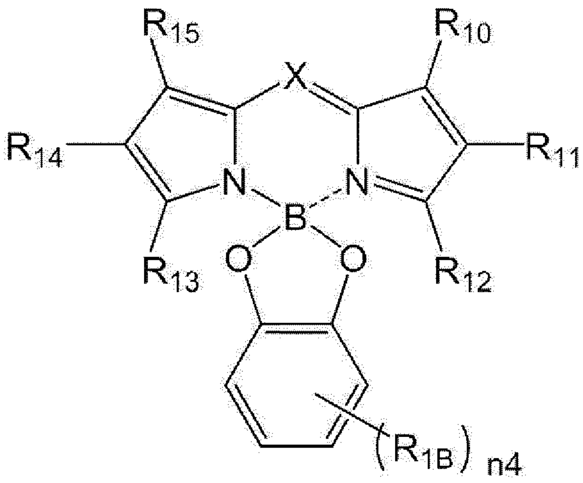

(10B)

In the formula (10A): X, Y and $R_{10}$ to $R_{15}$ respectively represent the same as X, Y and $R_{10}$ to $R_{15}$ in the formula (10); $R_{1A}$ is each independently a hydrogen atom or a substituent; $R_{1A}$ as a substituent represents the same as $R_{10}$ to $R_{15}$ as a substituent; and n3 is 4.

In the formula (10B): X, Y and $R_{10}$ to $R_{15}$ respectively represent the same as X, Y and $R_{10}$ to $R_{15}$ in the formula (10); $R_{1B}$ is each independently a hydrogen atom or a substituent; $R_{1B}$ as a substituent represents the same as $R_{10}$ to $R_{15}$ as a substituent; and n4 is 4.

It is preferable that at least one of $Z_{11}$ or $Z_{12}$ (preferably both of $Z_{11}$ and $Z_{12}$) is a group selected from the group consisting of a substituted or unsubstituted alkyl group having 1 to 30 carbon atoms, a substituted or unsubstituted alkyl halide group having 1 to 30 carbon atoms, a substituted or unsubstituted aryl group having 6 to 30 ring carbon atoms, a substituted or unsubstituted alkoxy group having 1 to 30 carbon atoms, a substituted or unsubstituted alkoxy halide group having 1 to 30 carbon atoms, and a substituted or unsubstituted aryloxy group having 6 to 30 ring carbon atoms.

It is more preferable that at least one of $Z_{11}$ or $Z_{12}$ is a group selected from the group consisting of a fluorine-substituted alkoxy group having 1 to 30 carbon atoms, a fluorine-substituted aryloxy group having 6 to 30 ring carbon atoms, and an aryloxy group having 6 to 30 ring carbon atoms and substituted with a fluoroalkyl group having 1 to 30 carbon atoms.

Further preferably, at least one of $Z_{11}$ or $Z_{12}$ is a fluorine-substituted alkoxy group having 1 to 30 carbon atoms. Further more preferably, both of $Z_{11}$ and $Z_{12}$ are a fluorine-substituted alkoxy group having 1 to 30 carbon atoms.

It is also preferable that both of $Z_{11}$ and $Z_{12}$ are the same.

Meanwhile, it is also preferable that at least one of $Z_{11}$ or $Z_{12}$ is a fluorine atom. It is also more preferable that both of $Z_{11}$ and $Z_{12}$ are fluorine atoms.

It is also preferable that at least one of $Z_{11}$ or $Z_{12}$ is a group represented by a formula (10a) below.

[Formula 183]

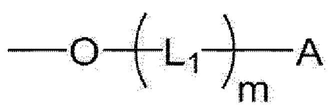

(10a)

In the formula (10a): A is a substituted or unsubstituted alkyl group having 1 to 6 carbon atoms, a substituted or unsubstituted alkyl halide group having 1 to 6 carbon atoms, or a substituted or unsubstituted aryl group having 6 to 12 ring carbon atoms; $L_1$ is a substituted or unsubstituted alkylene group having 1 to 6 carbon atoms, or a substituted or unsubstituted arylene group having 6 to 12 ring carbon atoms; m is 0, 1, 2, 3, 4, 5, 6 or 7; and when m is 2, 3, 4, 5, 6 or 7, a plurality of $L_1$ are mutually the same or different. m is preferably 0, 1 or 2. When m is 0, A is directly bonded to O (oxygen atom).

When $Z_{11}$ and $Z_{12}$ in the formula (10) are each the group represented by the formula (10a), the compound M1 is a compound represented by a formula (12) below.

The compound M1 is also preferably the compound represented by the formula (12) below.

[Formula 184]

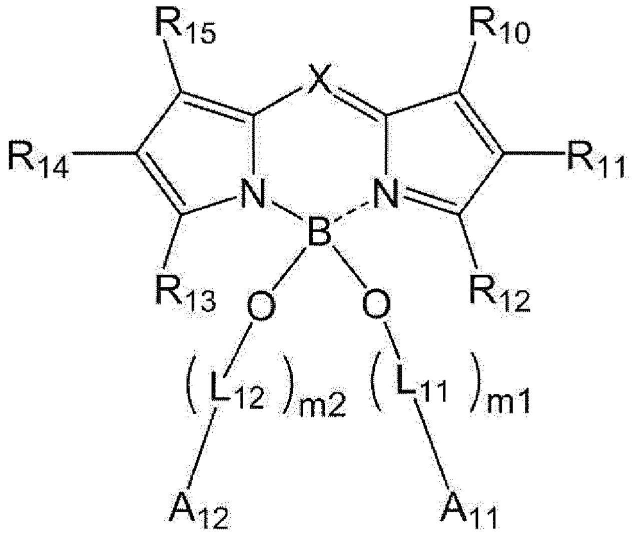

(12)

In the formula (12), X, Y bonded with a carbon atom as X, and $R_{10}$ to $R_{15}$ respectively represent the same as X, Y and $R_{10}$ to $R_{15}$ in the formula (10). $A_{11}$ and $A_{12}$ represent the same as A in the formula (10a) and may be mutually the same or different. $L_{11}$ and $L_{12}$ represent the same as $L_1$ in the formula (10a) and may be mutually the same or different. m1 and m2 are each independently 0, 1, 2, 3, 4, 5, 6 or 7, preferably 0, 1 or 2. When m1 is 2, 3, 4, 5, 6 or 7, a plurality of $L_{11}$ are mutually the same or different. When m2 is 2, 3, 4, 5, 6 or 7, a plurality of $L_{12}$ are mutually the same or different. When m1 is 0, $A_{11}$ is directly bonded to O (oxygen atom). When m2 is 0, $A_{12}$ is directly bonded to O (oxygen atom).

At least one of A or $L_1$ in the formula (10a) is preferably substituted with a halogen atom, more preferably substituted with a fluorine atom.

A in the formula (10a) is more preferably a perfluoroalkyl group having 1 to 6 carbon atoms or a perfluoroaryl group having 6 to 12 ring carbon atoms, further preferably a perfluoroalkyl group having 1 to 6 carbon atoms.

$L_1$ in the formula (10a) is more preferably a perfluoroalkylene group having 1 to 6 carbon atoms or a perfluoroarylene group having 6 to 12 ring carbon atoms, further preferably a perfluoroalkylene group having 1 to 6 carbon atoms.

In other words, the compound M1 is also preferably a compound represented by a formula (12a) below.

[Formula 185]

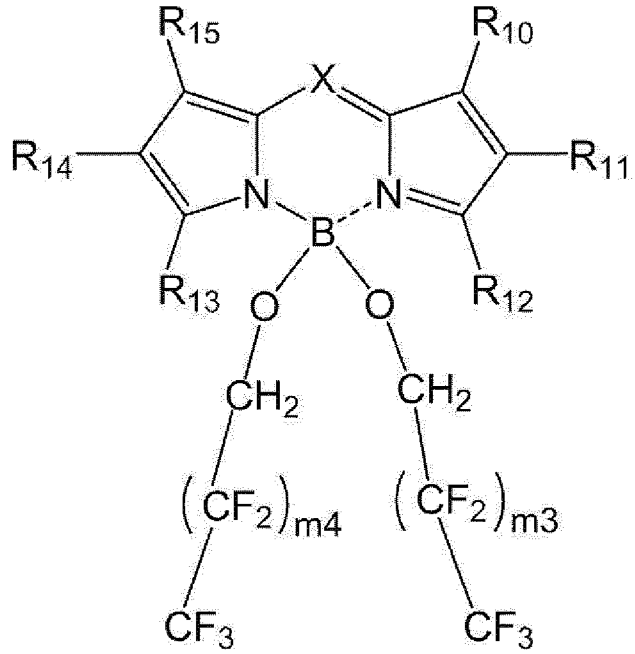

(12a)

In the formula (12a):

X represents the same as X in the formula (10);
Y bonded with a carbon atom as X represents the same as Y in the formula (10);
$R_{10}$ to $R_{15}$ each independently represent the same as $R_{10}$ to $R_{15}$ in the formula (10);
m3 is 0, 1, 2, 3 or 4;
m4 is 0, 1, 2, 3 or 4; and
m3 and m4 are mutually the same or different.

In the formulae (10), (11), (12) and (12a):

X is a carbon atom bonded to Y; and Y is a hydrogen atom or a substituent.
Y as a substituent is preferably a substituent selected from the group consisting of a substituted or unsubstituted alkyl group having 1 to 30 carbon atoms, a substituted or unsubstituted alkyl halide group having 1 to 30 carbon atoms and a substituted or unsubstituted aryl group having 6 to 30 ring carbon atoms, more preferably a substituted or unsubstituted aryl group having 6 to 30 ring carbon atoms.

In the formulae (10), (11), (12) and (12a), it is more preferable that: X is a carbon atom bonded to Y; Y is a hydrogen atom or a substituent; Y as a substituent is a substituted or unsubstituted aryl group having 6 to 30 ring carbon atoms; and when Y as a substituent is an aryl group having 6 to 30 ring carbon atoms having a substituent, the substituent is a substituted or unsubstituted alkyl group having 1 to 30 carbon atoms, a substituted or unsubstituted alkyl halide group having 1 to 30 carbon atoms, a substituted or unsubstituted alkoxy group having 1 to 30 carbon atoms, a substituted or unsubstituted alkoxy halide group having 1 to 30 carbon atoms, or an aryl group having 6 to 30 ring carbon atoms and substituted by an alkyl group having 1 to 30 carbon atoms.

In the compound M1, $Z_{11}$ and $Z_{12}$ may be mutually bonded to form a ring. However, it is preferable that $Z_{11}$ and $Z_{12}$ are not mutually bonded to form no ring.

In the formulae (10), (12) and (12a), at least one of $R_{10}$, $R_{12}$, $R_{13}$ or $R_{15}$ is preferably a substituted or unsubstituted alkyl group having 1 to 30 carbon atoms or a substituted or unsubstituted alkyl halide group having 1 to 30 carbon atoms.

In the formulae (10), (12) and (12a), $R_{10}$, $R_{12}$, $R_{13}$ and $R_{15}$ are more preferably a substituted or unsubstituted alkyl group having 1 to 30 carbon atoms or a substituted or unsubstituted alkyl halide group having 1 to 30 carbon atoms. In this case, $R_{11}$ and $R_{14}$ are preferably hydrogen atoms.

In the formulae (10), (12) and (12a), at least one of $R_{10}$, $R_{12}$, $R_{13}$ or $R_{15}$ is preferably a substituted or unsubstituted aryl group having 6 to 30 ring carbon atoms.

In the formulae (10), (12) and (12a), $R_{10}$, $R_{12}$, $R_{13}$ and $R_{15}$ are more preferably a substituted or unsubstituted aryl group having 6 to 30 ring carbon atoms. In this case, $R_{11}$ and $R_{14}$ are preferably hydrogen atoms.

In the formulae (10), (12) and (12a), it is more preferable that: $R_{10}$, $R_{12}$, $R_{13}$ and $R_{15}$ are each independently a substituted or unsubstituted alkyl group having 1 to 30 carbon atoms (preferably 1 to 6 carbon atoms), a substituted or unsubstituted alkyl halide group having 1 to 30 carbon atoms (preferably 1 to 6 carbon atoms), or an aryl group having 6 to 30 ring carbon atoms (preferably 6 to 12 ring carbon atoms) and substituted with an alkyl group having 1 to 30 carbon atoms; and $R_{11}$ and $R_{14}$ are hydrogen atoms.

In the formula (11), at least one of $R_{10}$, $R_{12}$ or $R_{13}$ is preferably a substituted or unsubstituted alkyl group having 1 to 30 carbon atoms or a substituted or unsubstituted alkyl halide group having 1 to 30 carbon atoms.

In the formula (11), $R_{10}$, $R_{12}$ and $R_{13}$ are more preferably a substituted or unsubstituted alkyl group having 1 to 30 carbon atoms or a substituted or unsubstituted alkyl halide group having 1 to 30 carbon atoms. In this case, $R_{11}$ is preferably a hydrogen atom.

In the formula (11), at least one of $R_{10}$, $R_{12}$ or $R_{13}$ is preferably a substituted or unsubstituted aryl group having 6 to 30 ring carbon atoms.

In the formula (11), $R_{10}$, $R_{12}$ and $R_{13}$ are more preferably a substituted or unsubstituted aryl group having 6 to 30 ring carbon atoms. In this case, $R_{11}$ is preferably a hydrogen atom.

In the formula (11), it is more preferable that: $R_{10}$, $R_{12}$ and $R_{13}$ are each independently a substituted or unsubstituted alkyl group having 1 to 30 carbon atoms (preferably 1 to 6 carbon atoms), a substituted or unsubstituted alkyl halide group having 1 to 30 carbon atoms (preferably 1 to 6 carbon atoms), or an aryl group having 6 to 30 ring carbon atoms (preferably 6 to 12 ring carbon atoms) and substituted with an alkyl group having 1 to 30 carbon atoms; and $R_{11}$ is a hydrogen atom.

In the compound M1, examples of the fluorine-substituted alkoxy group include 2,2,2-trifluoroethoxy group, 2,2-difluoroethoxy group, 2,2,3,3,3-pentafluoro-1-propoxy group, 2,2,3,3-tetrafluoro-1-propoxy group, 1,1,1,3,3,3-hexafluoro-2-propoxy group, 2,2,3,3,4,4,4-heptafluoro-1-butyloxy group, 2,2,3,3,4,4-hexafluoro-1-butyloxy group, nonafluoro-tertiary-butyloxy group, 2,2,3,3,4,4,5,5,5-nonafluoropentanoxy group, 2,2,3,3,4,4,5,5,6,6,6-undecafluorohexanoxy group, 2,3-bis(trifluoromethyl)-2,3-butanedioxy group, 1,1,2,2-tetra(trifluoromethyl)ethylene glycoxy group, 4,4,5,5,6,6,6-heptafluorohexane-1,2-dioxy group, and 4,4,5,5,6,6,7,7,8,8,9,9,9-tridecafluorononane-1,2-dioxy group.

In the compound M1, examples of the fluorine-substituted aryloxy group or the aryloxy group substituted with a fluoroalkyl group include a pentafluorophenoxy group, 3,4,5-trifluorophenoxy group, 4-trifluoromethylphenoxy group, 3,5-bistrifluoromethylphenoxy group, 3-fluoro-4-trifluoromethylphenoxy group, 2,3,5,6-tetrafluoro-4-trifluoromethylphenoxy group, 4-fluorocatecholato group, 4-trifluoromethylcatecholato group, and 3,5-bistrifluoromethylcatecholato group.

When the compound M1 is a fluorescent compound, the compound M1 preferably emits light having a main peak wavelength in a range from 400 nm to 700 nm.

Herein, the main peak wavelength means a peak wavelength of an emission spectrum exhibiting a maximum luminous intensity among fluorescence spectra measured in a toluene solution in which a measurement target compound is dissolved at a concentration ranging from $10^{-6}$ mol/l to $10^{-5}$ mol/l. A spectrophotofluorometer (F-7000 manufactured by Hitachi High-Tech Science Corporation) is used as a measurement device.

The compound M1 preferably exhibits red or green light emission.

Herein, the red light emission refers to a light emission in which a main peak wavelength of fluorescence spectrum is in a range from 600 nm to 660 nm.

When the compound M1 is a red fluorescent compound, the main peak wavelength of the compound M1 is preferably in a range from 600 nm to 660 nm, more preferably in a range from 600 nm to 640 nm, further preferably in a range from 610 nm to 630 nm.

Herein, the green light emission refers to a light emission in which a main peak wavelength of fluorescence spectrum is in a range from 500 nm to 560 nm.

When the compound M1 is a green fluorescent compound, the main peak wavelength of the compound M1 is preferably in a range from 500 nm to 560 nm, more preferably in a range from 500 nm to 540 nm, further preferably in a range from 510 nm to 540 nm.

Herein, the blue light emission refers to a light emission in which a main peak wavelength of fluorescence spectrum is in a range from 430 nm to 480 nm.

When the compound M1 is a blue fluorescent compound, the main peak wavelength of the compound M1 is preferably in a range from 430 nm to 480 nm, more preferably in a range from 440 nm to 480 nm.

A main peak wavelength of the light emitted from the organic EL device is measured as follows.

Voltage is applied on the organic EL devices such that a current density becomes 10 $mA/cm^2$, where spectral radiance spectrum is measured by a spectroradiometer CS-2000 (manufactured by Konica Minolta, Inc.).

A peak wavelength of an emission spectrum, at which the luminous intensity of the resultant spectral radiance

spectrum is at the maximum, is measured and defined as the main peak wavelength (unit: nm).

Manufacturing Method of Compound M1 According to Exemplary Embodiment

The compound M1 can be manufactured by a known method.

Specific Examples of Compound M1

Specific examples of the compound M1 according to the exemplary embodiment are shown below. It should however be noted that the invention is not limited to the specific examples of the compound.

A coordinate bond between a boron atom and a nitrogen atom in a pyrromethene skeleton is shown by various means such as a solid line, a broken line, an arrow, and omission. Herein, the coordinate bond is shown by a solid line or a broken line, or the description of the coordinate bond is omitted.

[Formula 186]

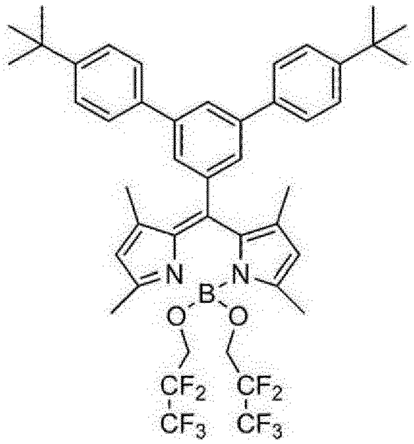
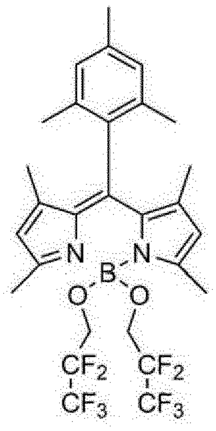
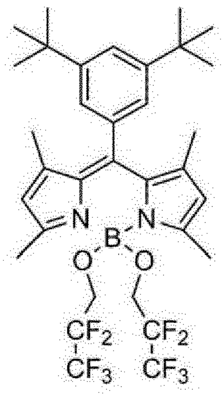

[Formula 187]

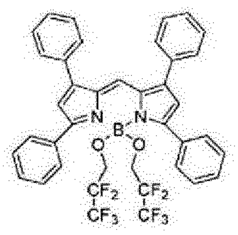
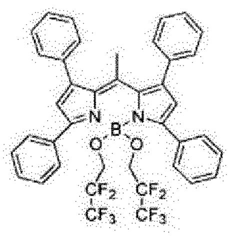
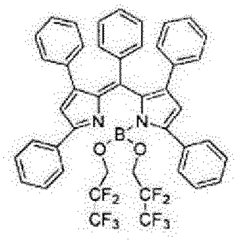
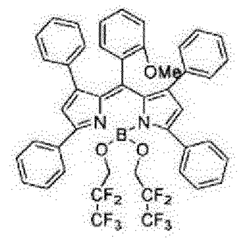
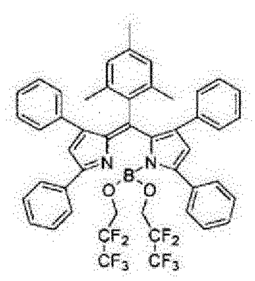
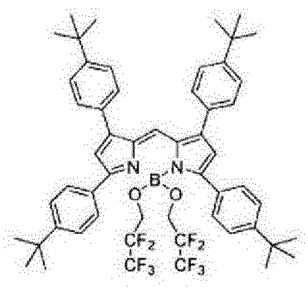
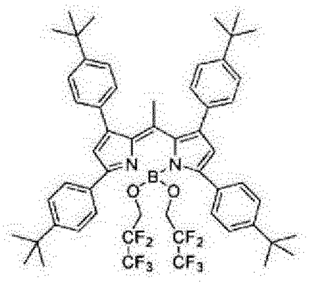
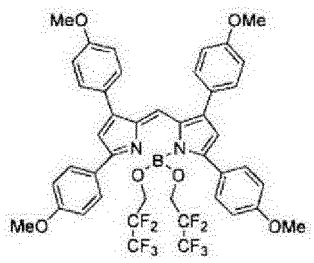

[Formula 188]

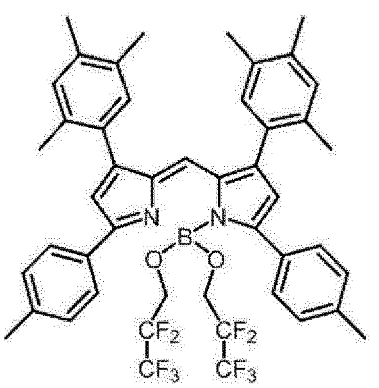

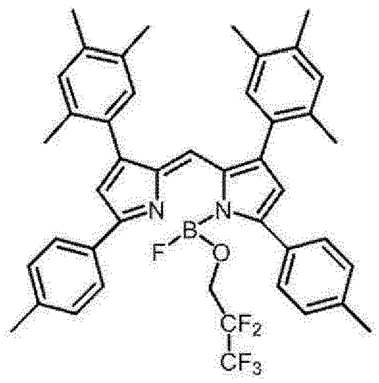

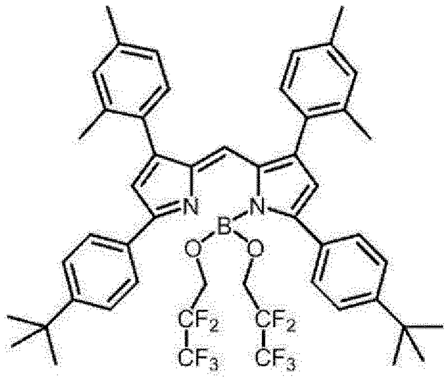

[Formula 189]

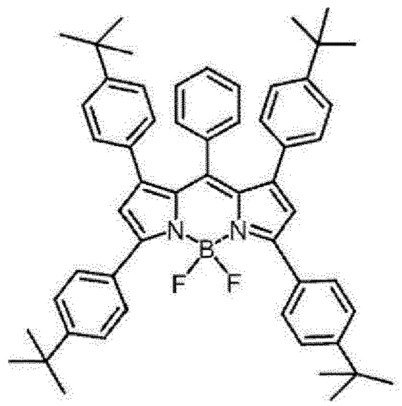

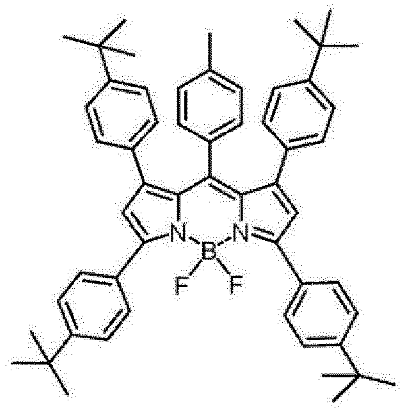

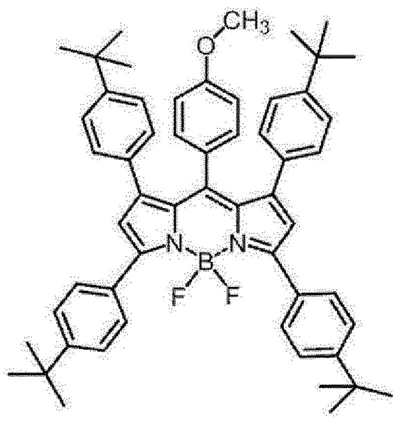

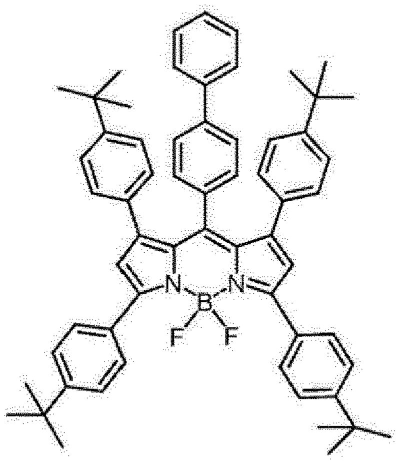

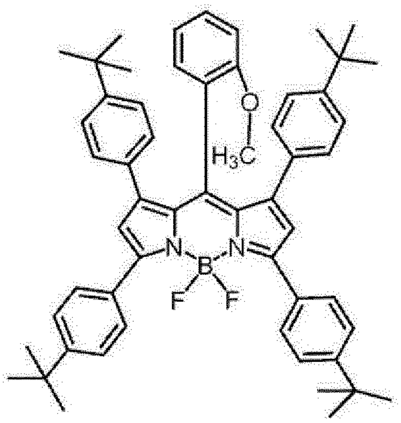

[Formula 190]

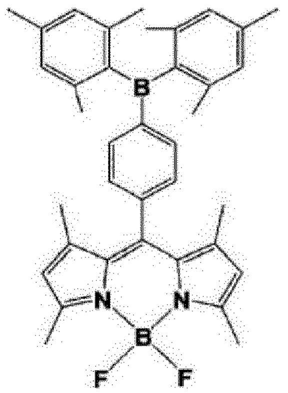
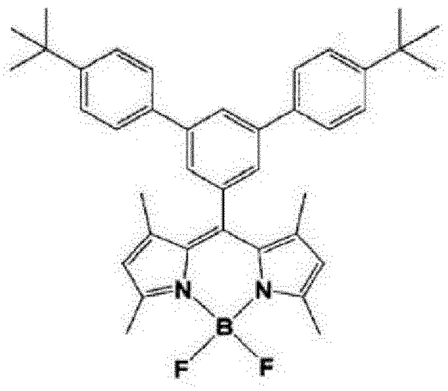
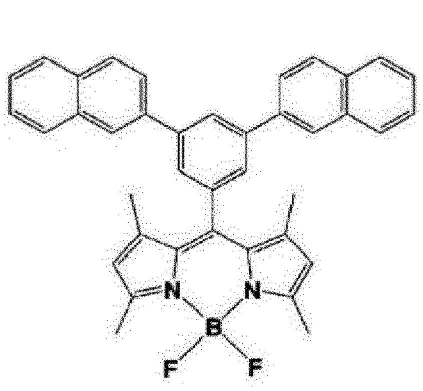

[Formula 191]

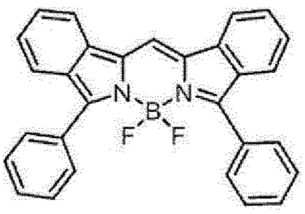
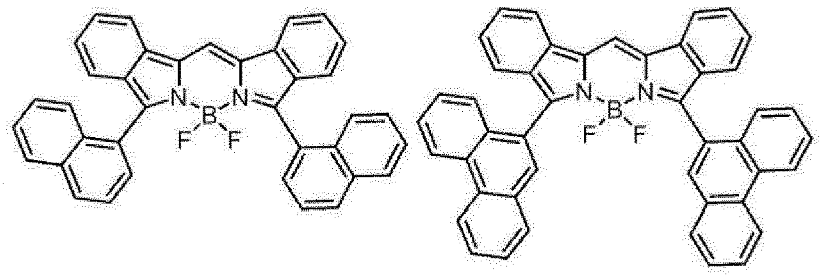

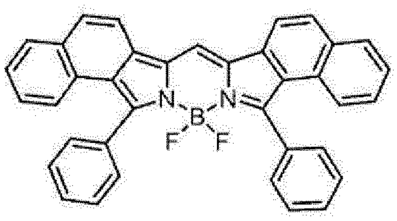
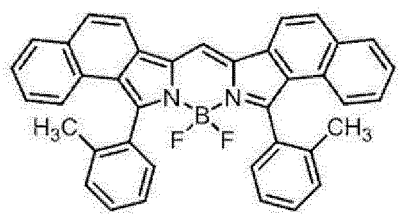
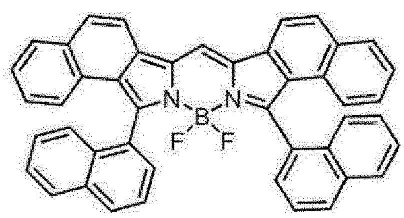

[Formula 192]

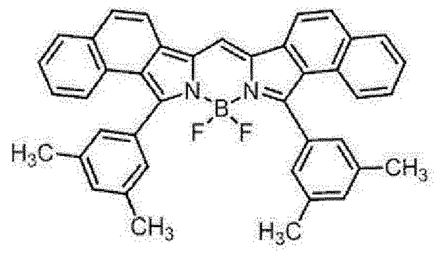
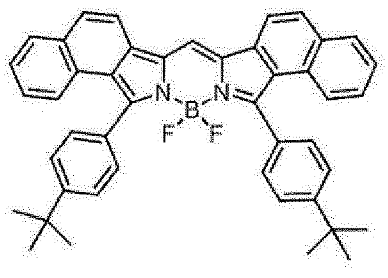
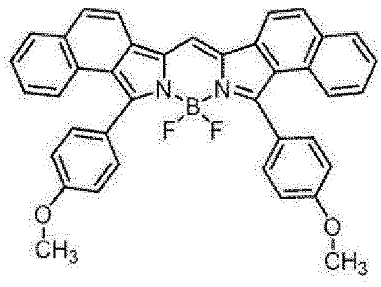
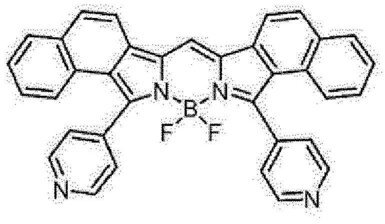
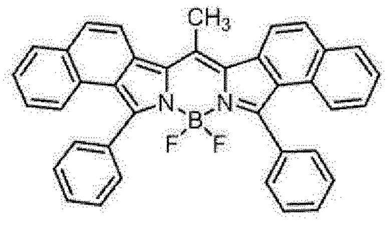
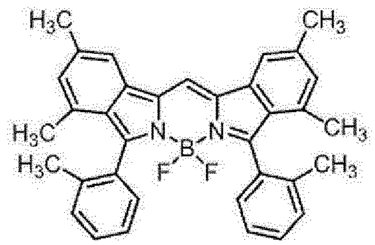

Relationship between Compound M3, Compound M2 and Compound M1 in Emitting Layer

In the organic EL device according to the exemplary embodiment, the singlet energy $S_1(M2)$ of the compound

M2 and a singlet energy $S_1(M1)$ of the compound M1 preferably satisfy a relationship of a numerical formula (Numerical Formula 2) below.

$$S_1(M2) > S_1(M1)\ldots\text{(Numerical Formula 2)}$$

The singlet energy $S_1(M3)$ of the compound M3 is preferably larger than the singlet energy $S_1(M1)$ of the compound M1.

$$S_1(M3) > S_1(M1)\ldots\text{(Numerical Formula 2A)}$$

The singlet energy $S_1(M3)$ of the compound M3, the singlet energy $S_1(M2)$ of the compound M2, and the singlet energy $S_1(M1)$ of the compound M1 preferably satisfy a relationship of a numerical formula (Numerical Formula 2B) below.

$$S_1(M3) > S_1(M2) > S_1(M1)\ldots\text{(Numerical Formula 2B)}$$

When the organic EL device according to the exemplary embodiment emits light, it is preferable that the fluorescent compound M1 in the emitting layer mainly emits light.

The organic EL device according to the exemplary embodiment preferably emits red light or green light.

Content Ratios of Compounds in Emitting Layer

Content ratios of the compounds M3, M2 and M1 in the emitting layer are preferably fall within, for instance, the following range.

The content ratio of the compound M3 is preferably in a range from 10 mass% to 80 mass%.

The content ratio of the compound M2 is preferably in a range from 10 mass% to 80 mass%, more preferably in a range from 10 mass% to 60 mass%, further preferably in a range from 20 mass% to 60 mass%.

The content ratio of the compound M1 is preferably in a range from 0.01 mass% to 10 mass%, more preferably in a range from 0.01 mass% to 5 mass%, further preferably in a range from 0.01 mass% to 1 mass%.

An upper limit of the total of the respective content ratios of the compounds M3, M2 and M1 in the emitting layer is 100 mass%.It should be noted that the emitting layer of the exemplary embodiment may further contain material(s) other than the compounds M3, M2 and M1.

The emitting layer may include a single type of the compound M3 or may include two or more types of the compound M3. The emitting layer may include a single type of the compound M2 or may include two or more types of the compound M2. The emitting layer may include a single type of the compound M1 or may include two or more types of the compound M1.

Fig. 5 shows an example of a relationship between energy levels of the compounds M3, M2 and M1 in the emitting layer. In Fig. 5, S0 represents a ground state. S1(M1) represents the lowest singlet state of the compound M1. T1(M1) represents the lowest triplet state of the compound M1. S1(M2) represents the lowest singlet state of the compound M2. T1(M2) represents the lowest triplet state of the compound M1. S1(M3) represents the lowest singlet state of the compound M3. T1(M3) represents the lowest triplet state of the compound M3. A dashed arrow directed from S1(M2) to S1(M1) in Fig. 5 represents Förster energy transfer from the lowest singlet state of the compound M2 to the lowest singlet state of the compound M1.

As shown in Fig. 5, when a compound having a small ΔST(M2) is used as the compound M2, inverse intersystem crossing from the lowest triplet state T1(M2) to the lowest singlet state S1(M2) can be caused by a heat energy. Subsequently, Förster energy transfer from the lowest singlet state S1(M2) of the compound M2 to the compound M1 occurs to generate the lowest singlet state S1(M1). Consequently, fluorescence from the lowest singlet state S1(M1) of the compound M1 can be observed. It is inferred that the internal quantum efficiency can be theoretically raised up to 100% also by using delayed fluorescence by the TADF mechanism.

The organic EL device according to the second exemplary embodiment contains the delayed fluorescent compound M2, the compound M3 having the singlet energy larger than that of the compound M2, and the compound M1 having the singlet energy smaller than that of the delayed fluorescent compound M2 in the emitting layer.

According to the second exemplary embodiment, a high-performance organic EL device such as an organic EL device emitting light with a high efficiency can be achieved.

The organic EL device according to the second exemplary embodiment is applicable to an electronic device such as a display device and a light-emitting device.

Third Exemplary Embodiment

Compound

A compound of a third exemplary embodiment is a compound represented by a formula (301) below. A compound falling under the formula (301) among the specific examples of M3 of the first embodiment is also used as a specific example of the compound of the third exemplary embodiment.

[Formula 193]

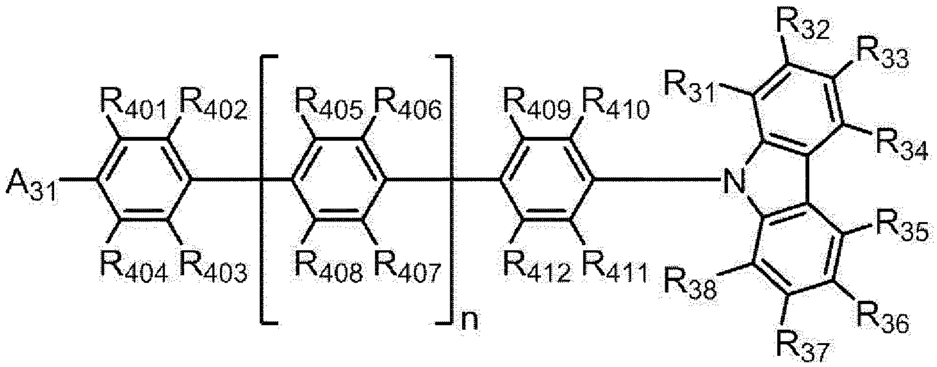

(301)

In the formula (301), $A_{31}$ is a group represented by a formula (31a), (31b), (31c), (31d), (31e) or (31f) below.

[Formula 194]

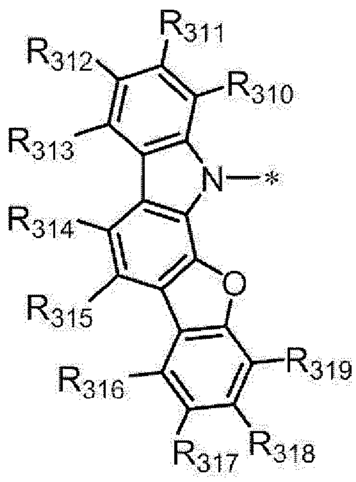

(31a)

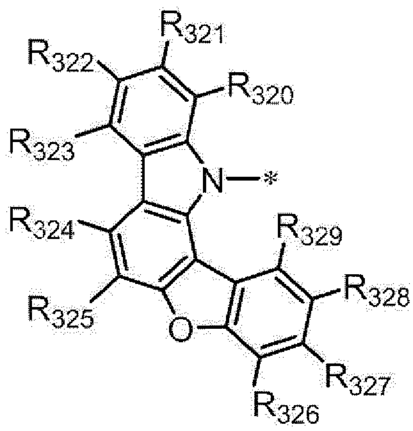

(31b)

[Formula 195]

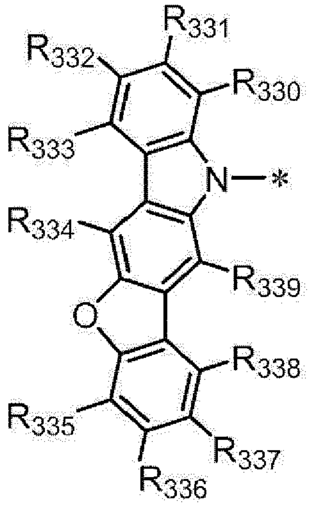

(31c)

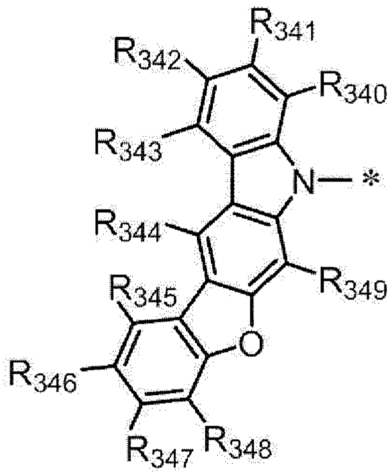

(31d)

[Formula 196]

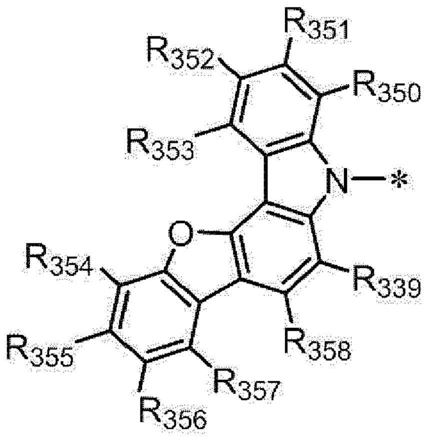

(31e)

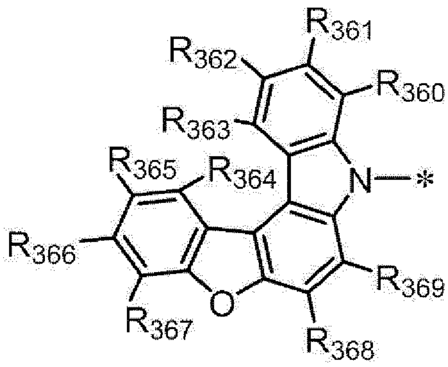

(31f)

In the formulae (31a), (31b), (31c), (31d), (31e) and (31f):

$R_{310}$ to $R_{319}$ are each independently a hydrogen atom or a substituent;
$R_{320}$ to $R_{329}$ are each independently a hydrogen atom or a substituent;
$R_{330}$ to $R_{339}$ are each independently a hydrogen atom or a substituent;
$R_{340}$ to $R_{349}$ are each independently a hydrogen atom or a substituent;
$R_{350}$ to $R_{359}$ are each independently a hydrogen atom or a substituent;
$R_{369}$ to $R_{369}$ are each independently a hydrogen atom or a substituent;
$R_{310}$ to $R_{319}$, $R_{320}$ to $R_{329}$, $R_{330}$ to $R_{339}$, $R_{340}$ to $R_{349}$, $R_{350}$ to $R_{359}$ and $R_{360}$ to $R_{369}$ as a substituent are each independently a halogen atom, a substituted or unsubstituted aryl group having 6 to 30 ring carbon atoms, a substituted or unsubstituted heterocyclic group having 5 to 30 ring atoms, a substituted or unsubstituted alkyl group having 1 to 30 carbon atoms, a substituted or unsubstituted alkyl halide group having 1 to 30 carbon atoms, a substituted or unsubstituted alkenyl group having 2 to 30 carbon atoms, a substituted or unsubstituted alkynyl group having 2 to 30 carbon atoms, a substituted or unsubstituted alkylsilyl group having 3 to 30 carbon atoms, a substituted or unsubstituted arylsilyl group having 6 to 60 ring carbon atoms, a substituted or unsubstituted arylphosphoryl group having 6 to 60 ring carbon atoms, a hydroxy group, a substituted or unsubstituted alkoxy group having 1 to

30 carbon atoms, a substituted or unsubstituted aryloxy group having 6 to 30 ring carbon atoms, an amino group, a substituted or unsubstituted alkylamino group having 2 to 30 carbon atoms, a substituted or unsubstituted arylamino group having 6 to 60 ring carbon atoms, a thiol group, a substituted or unsubstituted alkylthio group having 1 to 30 carbon atoms, or a substituted or unsubstituted arylthio group having 6 to 30 ring carbon atoms; and
* represents a bonding position to a benzene ring bonded with $R_{401}$ to $R_{404}$.

In the formula (301):

$R_{31}$ to $R_{38}$ are each independently a hydrogen atom or a substituent;
$R_{401}$ to $R_{412}$ are each independently a hydrogen atom or a substituent;
$R_{31}$ to $R_{38}$ as a substituent are each independently a halogen atom, a substituted or unsubstituted aryl group having 6 to 30 ring carbon atoms, a substituted or unsubstituted heterocyclic group having 5 to 30 ring atoms, a substituted or unsubstituted alkyl group having 1 to 30 carbon atoms, a substituted or unsubstituted alkyl halide group having 1 to 30 carbon atoms, a substituted or unsubstituted alkenyl group having 2 to 30 carbon atoms, a substituted or unsubstituted alkynyl group having 2 to 30 carbon atoms, a substituted or unsubstituted alkylsilyl group having 3 to 30 carbon atoms, a substituted or unsubstituted arylsilyl group having 6 to 60 ring carbon atoms, a substituted or unsubstituted arylphosphoryl group having 6 to 60 ring carbon atoms, a hydroxy group, a substituted or unsubstituted alkoxy group having 1 to 30 carbon atoms, a substituted or unsubstituted aryloxy group having 6 to 30 ring carbon atoms, an amino group, a substituted or unsubstituted alkylamino group having 2 to 30 carbon atoms, a substituted or unsubstituted arylamino group having 6 to 60 ring carbon atoms, a thiol group, a substituted or unsubstituted alkylthio group having 1 to 30 carbon atoms, or a substituted or unsubstituted arylthio group having 6 to 30 ring carbon atoms; and
$R_{401}$ to $R_{412}$ as a substituent are each independently a halogen atom, a substituted or unsubstituted aryl group having 6 to 30 ring carbon atoms, a substituted or unsubstituted heterocyclic group having 5 to 30 ring atoms, a substituted or unsubstituted alkyl group having 1 to 30 carbon atoms, a substituted or unsubstituted alkyl halide group having 1 to 30 carbon atoms, a substituted or unsubstituted alkenyl group having 2 to 30 carbon atoms, a substituted or unsubstituted alkynyl group having 2 to 30 carbon atoms, a substituted or unsubstituted alkylsilyl group having 3 to 30 carbon atoms, a substituted or unsubstituted arylsilyl group having 6 to 60 ring carbon atoms, a substituted or unsubstituted arylphosphoryl group having 6 to 60 ring carbon atoms, a hydroxy group, a substituted or unsubstituted alkoxy group having 1 to 30 carbon atoms, a substituted or unsubstituted aryloxy group having 6 to 30 ring carbon atoms, a thiol group, a substituted or unsubstituted alkylthio group having 1 to 30 carbon atoms, or a substituted or unsubstituted arylthio group having 6 to 30 ring carbon atoms;
n is 0, 1, 2 or 3;
when a plurality of $R_{405}$ are present, the plurality of $R_{405}$ are mutually the same or different;
when a plurality of $R_{406}$ are present, the plurality of $R_{406}$ are mutually the same or different;
when a plurality of $R_{407}$ are present, the plurality of $R_{407}$ are mutually the same or different; and
when a plurality of $R_{408}$ are present, the plurality of $R_{408}$ are mutually the same or different.

In the formula (31a), a pair of $R_{310}$ and $R_{311}$, a pair of $R_{311}$ and $R_{312}$, a pair of $R_{312}$ and $R_{313}$, a pair of $R_{314}$ and $R_{315}$, a pair of $R_{316}$ and $R_{317}$, a pair of $R_{317}$ and $R_{318}$, and a pair of $R_{318}$ and $R_{319}$ are not mutually bonded.
In the formula (31b), a pair of $R_{320}$ and $R_{321}$, a pair of $R_{321}$ and $R_{322}$, a pair of $R_{322}$ and $R_{323}$, a pair of $R_{324}$ and $R_{325}$, a pair of $R_{326}$ and $R_{327}$, a pair of $R_{327}$ and $R_{328}$, and a pair of $R_{328}$ and $R_{329}$ are not mutually bonded.
In the formula (31c), a pair of $R_{330}$ and $R_{331}$, a pair of $R_{331}$ and $R_{332}$, a pair of $R_{332}$ and $R_{333}$, a pair of $R_{335}$ and $R_{336}$, a pair of $R_{336}$ and $R_{337}$, and a pair of $R_{337}$ and $R_{338}$ are not mutually bonded.
In the formula (31d), a pair of $R_{340}$ and $R_{341}$, a pair of $R_{341}$ and $R_{342}$, a pair of $R_{342}$ and $R_{343}$, a pair of $R_{345}$ and $R_{346}$, a pair of $R_{346}$ and $R_{347}$, and a pair of $R_{347}$ and $R_{348}$ are not mutually bonded.
In the formula (31e), a pair of $R_{350}$ and $R_{351}$, a pair of $R_{351}$ and $R_{352}$, a pair of $R_{352}$ and $R_{353}$, a pair of $R_{354}$ and $R_{355}$, a pair of $R_{355}$ and $R_{356}$, a pair of $R_{356}$ and $R_{357}$, and a pair of $R_{358}$ and $R_{359}$ are not mutually bonded.
In the formula (31f), a pair of $R_{360}$ and $R_{361}$, a pair of $R_{361}$ and $R_{362}$, a pair of $R_{362}$ and $R_{363}$, a pair of $R_{364}$ and $R_{365}$, a pair of $R_{365}$ and $R_{366}$, a pair of $R_{366}$ and $R_{367}$, and a pair of $R_{368}$ and $R_{369}$ are not mutually bonded.
In the compound of the third exemplary embodiment, n is preferably 0 or 1.
The compound of the third exemplary embodiment is preferably represented by a formula (302) below.

[Formula 197]

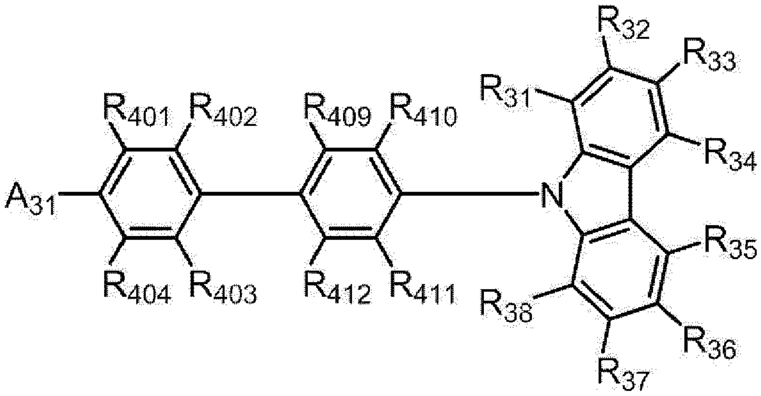

(302)

$A_{31}$, $R_{401}$ to $R_{404}$, $R_{409}$ to $R_{412}$ and $R_{31}$ to $R_{38}$ in the formula (302) respectively represent the same as $A_{31}$, $R_{401}$ to $R_{404}$, $R_{409}$ to $R_{412}$ and $R_{31}$ to $R_{38}$ in the formula (301).

In the compound of the third exemplary embodiment, $A_{30}$ is preferably a group represented by the formula (31a) or (31d).

The compound of the third exemplary embodiment is preferably represented by a formula (303) below.

[Formula 198]

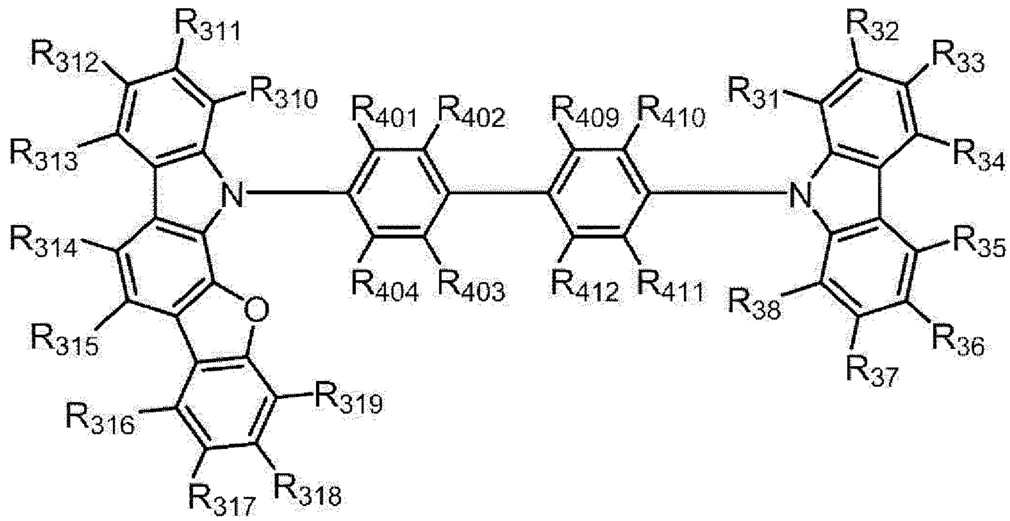

(303)

$R_{401}$ to $R_{404}$, $R_{409}$ to $R_{412}$ and $R_{31}$ to $R_{38}$ in the formula (303) respectively represent the same as $R_{401}$ to $R_{404}$, $R_{409}$ to $R_{412}$ and $R_{31}$ to $R_{38}$ in the formula (301).

In the compound of the third exemplary embodiment, $R_{31}$ to $R_{38}$ are preferably each independently a hydrogen atom, a substituted or unsubstituted aryl group having 6 to 30 ring carbon atoms, a substituted or unsubstituted heterocyclic group having 5 to 30 ring atoms, or a substituted or unsubstituted alkyl group having 1 to 30 carbon atoms.

In the compound of the third exemplary embodiment, $R_{31}$ to $R_{38}$ are more preferably each independently a hydrogen atom, a substituted or unsubstituted aryl group having 6 to 30 ring carbon atoms, or a substituted or unsubstituted heterocyclic group having 5 to 30 ring atoms.

In the compound of the third exemplary embodiment, $R_{31}$ to $R_{38}$ are further preferably each independently a hydrogen atom, or a substituted or unsubstituted aryl group having 6 to 30 ring carbon atoms.

In the compound of the third exemplary embodiment, $R_{31}$ to $R_{38}$ are further more preferably a hydrogen atom.

In the compound of the third exemplary embodiment, $R_{401}$ to $R_{412}$ are preferably each independently a hydrogen atom, a substituted or unsubstituted aryl group having 6 to 30 ring carbon atoms, a substituted or unsubstituted heterocyclic group having 5 to 30 ring atoms, or a substituted or unsubstituted alkyl group having 1 to 30 carbon atoms.

In the compound of the third exemplary embodiment, $R_{401}$ to $R_{412}$ are more preferably each independently a

hydrogen atom, a substituted or unsubstituted aryl group having 6 to 30 ring carbon atoms, or a substituted or unsubstituted heterocyclic group having 5 to 30 ring atoms.

In the compound of the third exemplary embodiment, $R_{401}$ to $R_{412}$ are further preferably each independently a hydrogen atom, or a substituted or unsubstituted aryl group having 6 to 30 ring carbon atoms.

In the compound of the third exemplary embodiment, $R_{401}$ to $R_{412}$ are further more preferably a hydrogen atom.

In the compound of the third exemplary embodiment, it is preferable that $R_{310}$ to $R_{319}$, $R_{320}$ to $R_{329}$, $R_{330}$ to $R_{339}$, $R_{340}$ to $R_{349}$, $R_{350}$ to $R_{359}$ and $R_{360}$ to $R_{369}$ are each independently a hydrogen atom, a substituted or unsubstituted aryl group having 6 to 30 ring carbon atoms, a substituted or unsubstituted heterocyclic group having 5 to 30 ring atoms, or a substituted or unsubstituted alkyl group having 1 to 30 carbon atoms.

In the compound of the third exemplary embodiment, $R_{401}$ to $R_{412}$ and $R_{31}$ to $R_{38}$ are preferably each independently a hydrogen atom, a substituted or unsubstituted aryl group having 6 to 30 ring carbon atoms, a substituted or unsubstituted heterocyclic group having 5 to 30 ring atoms, or a substituted or unsubstituted alkyl group having 1 to 30 carbon atoms.

In the compound of the third exemplary embodiment, $R_{401}$ to $R_{412}$ and $R_{31}$ to $R_{38}$ are more preferably each independently a hydrogen atom, a substituted or unsubstituted aryl group having 6 to 30 ring carbon atoms, or a substituted or unsubstituted heterocyclic group having 5 to 30 ring atoms.

In the compound of the third exemplary embodiment, $R_{401}$ to $R_{412}$ and $R_{31}$ to $R_{38}$ are further preferably each independently a hydrogen atom or a substituted or unsubstituted aryl group having 6 to 30 ring carbon atoms.

In the compound of the third exemplary embodiment, $R_{401}$ to $R_{412}$ and $R_{31}$ to $R_{38}$ are further more preferably a hydrogen atom.

In the compound of the third exemplary embodiment, it is more preferable that $R_{310}$ to $R_{319}$, $R_{320}$ to $R_{329}$, $R_{330}$ to $R_{339}$, $R_{340}$ to $R_{349}$, $R_{350}$ to $R_{359}$ and $R_{360}$ to $R_{369}$ are each independently a hydrogen atom, a substituted or unsubstituted aryl group having 6 to 30 ring carbon atoms, or a substituted or unsubstituted heterocyclic group having 5 to 30 ring atoms.

In the compound of the third exemplary embodiment, it is further preferable that $R_{310}$ to $R_{319}$, $R_{320}$ to $R_{329}$, $R_{330}$ to $R_{339}$, $R_{340}$ to $R_{349}$, $R_{350}$ to $R_{359}$ and $R_{360}$ to $R_{369}$ are each independently a hydrogen atom or a substituted or unsubstituted aryl group having 6 to 30 ring carbon atoms.

In the compound of the third exemplary embodiment, it is further more preferable that $R_{310}$ to $R_{319}$, $R_{320}$ to $R_{329}$, $R_{330}$ to $R_{339}$, $R_{340}$ to $R_{349}$, $R_{350}$ to $R_{359}$ and $R_{360}$ to $R_{369}$ are a hydrogen atom.

It should be noted that the compound of the third exemplary embodiment also corresponds to a compound with an exemplary arrangement of the compound M3 described in the first exemplary embodiment. Accordingly, specific examples of the compound of the third exemplary embodiment are also shown in the specific examples of the compound M3 described in the first exemplary embodiment.

## Organic EL Device

An organic EL device in an exemplary arrangement according to the third exemplary embodiment is an organic EL device in which the compound M3 in the organic EL device according to the first exemplary embodiment is replaced by the compound of the third exemplary embodiment.

The compound of the third exemplary embodiment allows an organice EL device to have higher performance such as improved luminous efficiency.

Accordingly, an organic EL device in an exemplary arrangement of the third exemplary embodiment also has high-performance such as high luminous effeciency.

## Fourth Exemplary Embodiment

### Compound

A compound of a fourth exemplary embodiment is a compound represented by a formula (310) below. A compound falling under the formula (310) among the specific examples of M3 of the first embodiment is also used as a specific example of the compound of the fourth exemplary embodiment.

[Formula 199]

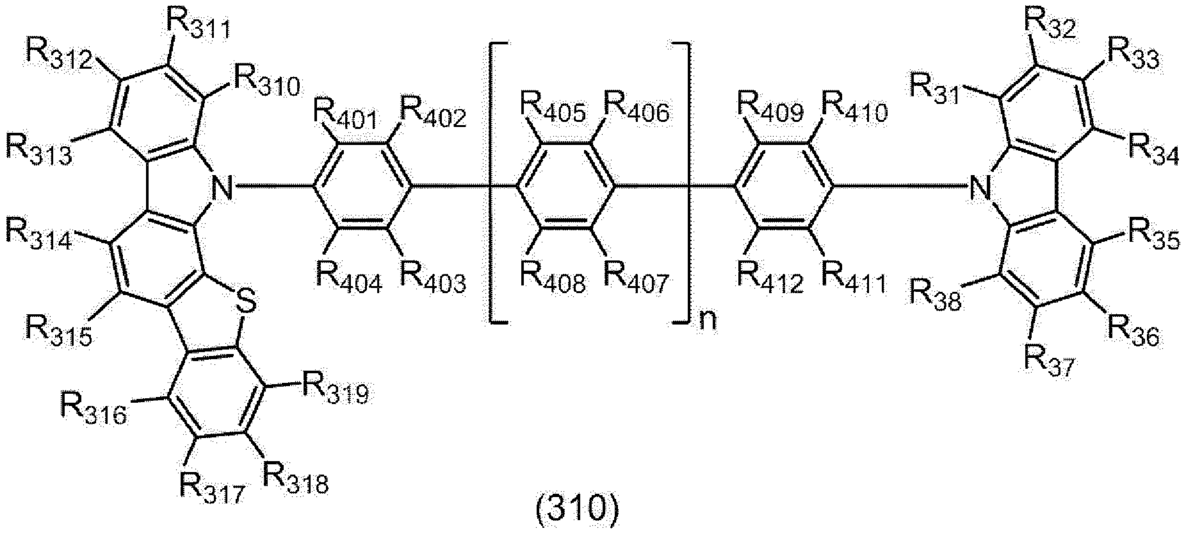

(310)

In the formula (310):

$R_{310}$ to $R_{319}$ are each independently a hydrogen atom or a substituent;
$R_{31}$ to $R_{38}$ are each independently a hydrogen atom or a substituent;
$R_{401}$ to $R_{412}$ are each independently a hydrogen atom or a substituent;
$R_{310}$ to $R_{319}$ as a substituent, $R_{31}$ to $R_{38}$ as a substituent and $R_{401}$ to $R_{412}$ as a substituent are each independently a halogen atom, a substituted or unsubstituted aryl group having 6 to 30 ring carbon atoms, a substituted or unsubstituted heterocyclic group having 5 to 30 ring atoms, a substituted or unsubstituted alkyl group having 1 to 30 carbon atoms, a substituted or unsubstituted alkyl halide group having 1 to 30 carbon atoms, a substituted or unsubstituted alkenyl group having 2 to 30 carbon atoms, a substituted or unsubstituted alkynyl group having 2 to 30 carbon atoms, a substituted or unsubstituted alkylsilyl group having 3 to 30 carbon atoms, a substituted or unsubstituted arylsilyl group having 6 to 60 ring carbon atoms, a substituted or unsubstituted arylphosphoryl group having 6 to 60 ring carbon atoms, a hydroxy group, a substituted or unsubstituted alkoxy group having 1 to 30 carbon atoms, a substituted or unsubstituted aryloxy group having 6 to 30 ring carbon atoms, an amino group, a substituted or unsubstituted alkylamino group having 2 to 30 carbon atoms, a substituted or unsubstituted arylamino group having 6 to 60 ring carbon atoms, a thiol group, a substituted or unsubstituted alkylthio group having 1 to 30 carbon atoms, or a substituted or unsubstituted arylthio group having 6 to 30 ring carbon atoms;
n is 0, 1, 2 or 3;
when a plurality of $R_{405}$ are present, the plurality of $R_{405}$ are mutually the same or different;
when a plurality of $R_{406}$ are present, the plurality of $R_{406}$ are mutually the same or different;
when a plurality of $R_{407}$ are present, the plurality of $R_{407}$ are mutually the same or different; and
when a plurality of $R_{408}$ are present, the plurality of $R_{408}$ are mutually the same or different.

In the compound of the fourth exemplary embodiment, a pair of $R_{310}$ and $R_{311}$, a pair of $R_{311}$ and $R_{312}$, a pair of $R_{312}$ and $R_{313}$, a pair of $R_{313}$ and $R_{314}$, a pair of $R_{314}$ and $R_{315}$, a pair of $R_{315}$ and $R_{316}$, a pair of $R_{316}$ and $R_{317}$, a pair of $R_{317}$ and $R_{318}$, and a pair of $R_{318}$ and $R_{319}$ are not mutually bonded.

In the compound of the fourth exemplary embodiment, n is preferably 0 or 1.

The compound of the fourth exemplary embodiment is preferably represented by a formula (311) below.

[Formula 200]

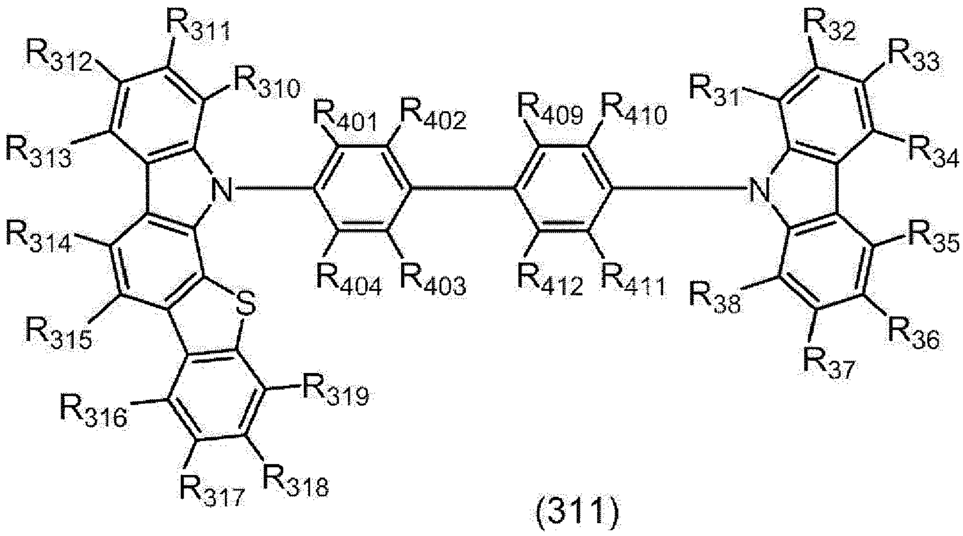

(311)

$R_{310}$ to $R_{319}$, $R_{401}$ to $R_{404}$, $R_{409}$ to $R_{412}$ and $R_{31}$ to $R_{38}$ in the formula (311) respectively represent the same as $R_{310}$ to $R_{319}$, $R_{401}$ to $R_{404}$, $R_{409}$ to $R_{412}$ and $R_{31}$ to $R_{38}$ in the formula (310).

In the compound of the fourth exemplary embodiment, $R_{31}$ to $R_{38}$ are preferably each independently a hydrogen atom, a substituted or unsubstituted aryl group having 6 to 30 ring carbon atoms, a substituted or unsubstituted heterocyclic group having 5 to 30 ring atoms, or a substituted or unsubstituted alkyl group having 1 to 30 carbon atoms.

In the compound of the fourth exemplary embodiment, $R_{31}$ to $R_{38}$ are more preferably each independently a hydrogen atom, a substituted or unsubstituted aryl group having 6 to 30 ring carbon atoms, or a substituted or unsubstituted heterocyclic group having 5 to 30 ring atoms.

In the compound of the fourth exemplary embodiment, $R_{31}$ to $R_{38}$ are further preferably each independently a hydrogen atom or a substituted or unsubstituted aryl group having 6 to 30 ring carbon atoms.

In the compound of the fourth exemplary embodiment, $R_{31}$ to $R_{38}$ are further more preferably a hydrogen atom.

In the compound of the fourth exemplary embodiment, $R_{401}$ to $R_{412}$ are preferably each independently a hydrogen atom, a substituted or unsubstituted aryl group having 6 to 30 ring carbon atoms, a substituted or unsubstituted heterocyclic group having 5 to 30 ring atoms, or a substituted or unsubstituted alkyl group having 1 to 30 carbon atoms.

In the compound of the fourth exemplary embodiment, $R_{401}$ to $R_{412}$ are more preferably each independently a hydrogen atom, a substituted or unsubstituted aryl group having 6 to 30 ring carbon atoms, or a substituted or unsubstituted heterocyclic group having 5 to 30 ring atoms.

In the compound of the fourth exemplary embodiment, $R_{401}$ to $R_{412}$ are further preferably each independently a hydrogen atom or a substituted or unsubstituted aryl group having 6 to 30 ring carbon atoms.

In the compound of the fourth exemplary embodiment, $R_{401}$ to $R_{412}$ are further more preferably a hydrogen atom.

In the compound of the fourth exemplary embodiment, $R_{401}$ to $R_{412}$ and $R_{31}$ to $R_{38}$ are preferably each independently a hydrogen atom, a substituted or unsubstituted aryl group having 6 to 30 ring carbon atoms, a substituted or unsubstituted heterocyclic group having 5 to 30 ring atoms, or a substituted or unsubstituted alkyl group having 1 to 30 carbon atoms.

In the compound of the fourth exemplary embodiment, $R_{401}$ to $R_{412}$ and $R_{31}$ to $R_{38}$ are more preferably each independently a hydrogen atom, a substituted or unsubstituted aryl group having 6 to 30 ring carbon atoms, or a substituted or unsubstituted heterocyclic group having 5 to 30 ring atoms.

In the compound of the fourth exemplary embodiment, $R_{401}$ to $R_{412}$ and $R_{31}$ to $R_{38}$ are further preferably each independently a hydrogen atom or a substituted or unsubstituted aryl group having 6 to 30 ring carbon atoms.

In the compound of the fourth exemplary embodiment, $R_{401}$ to $R_{412}$ and $R_{31}$ to $R_{38}$ are further more preferably a hydrogen atom.

In the compound of the fourth exemplary embodiment, $R_{310}$ to $R_{319}$ are further preferably each independently a hydrogen atom, a substituted or unsubstituted aryl group having 6 to 30 ring carbon atoms, a substituted or unsubstituted heterocyclic group having 5 to 30 ring atoms, or a substituted or unsubstituted alkyl group having 1 to 30 carbon atoms.

In the compound of the fourth exemplary embodiment, $R_{310}$ to $R_{319}$ are more preferably each independently a hydrogen atom, a substituted or unsubstituted aryl group having 6 to 30 ring carbon atoms, or a substituted or unsubstituted heterocyclic group having 5 to 30 ring atoms.

In the compound of the fourth exemplary embodiment, $R_{310}$ to $R_{319}$ are further preferably each independently

a hydrogen atom or a substituted or unsubstituted aryl group having 6 to 30 ring carbon atoms.

In the compound of the fourth exemplary embodiment, $R_{310}$ to $R_{319}$ are further more preferably a hydrogen atom.

It should be noted that the compound of the fourth exemplary embodiment also corresponds to a compound with an exemplary arrangement of the compound M3 described in the first exemplary embodiment. Accordingly, specific examples of the compound of the fourth exemplary embodiment are also shown in the specific examples of the compound M3 described in the first exemplary embodiment.

Organic EL Device

An organic EL device in an exemplary arrangement according to the fourth exemplary embodiment is an organic EL device in which the compound M3 in the organic EL device according to the first exemplary embodiment is replaced by the compound of the fourth exemplary embodiment.

The compound of the fourth exemplary embodiment allows an organice EL device to have higher performance such as improved luminous efficiency.

Accordingly, an organic EL device in an exemplary arrangement of the fourth exemplary embodiment also has high-performance such as high luminous effeciency.

Fifth Exemplary Embodiment (This embodiment is not encompassed by the wording of the claims but is considered as useful for understanding the invention)

Electronic Device

An electronic device according to a fifth exemplary embodiment is installed with any one of the organic EL devices according to the above exemplary embodiments. Examples of the electronic device include a display device and a light-emitting device. Examples of the display device include a display component (e.g., an organic EL panel module), TV, mobile phone, tablet and personal computer. Examples of the light-emitting device include an illuminator and a vehicle light.

Sixth Exemplary Embodiment

Organic-EL-Device Material

An organic-EL-device material of a sixth exemplary embodiment contains at least one of the compound of the third exemplary embodiment or the compound of the fourth exemplary embodiment.

The organic-EL-device material of the sixth exemplary embodiment allows an organice EL device to have higher performance such as improved luminous efficiency.

The organic-EL-device material according to the sixth exemplary embodiment may further contain an additional compound. When the organic-EL-device material according to the sixth exemplary embodiment further contains the additional compound, the additional compound may be solid or liquid.

Modification of Embodiment(s)

The scope of the invention is not limited by the above-described exemplary embodiments but includes any modification and improvement as long as such modification and improvement are compatible with the invention.

For instance, the emitting layer is not limited to a single layer, but may be provided by laminating a plurality of emitting layers. When the organic EL device has a plurality of emitting layers, it is only required that at least one of the emitting layers satisfies the conditions described in the above exemplary embodiments. For instance, the rest of the emitting layers may be a fluorescent emitting layer or a phosphorescent emitting layer with use of emission caused by electron transfer from the triplet excited state directly to the ground state.

When the organic EL device includes a plurality of emitting layers, these emitting layers may be mutually adjacently provided, or may form a so-called tandem organic EL device, in which a plurality of emitting units are laminated via an intermediate layer.

For instance, a blocking layer may be provided adjacent to at least one of a side of the emitting layer close to the anode or a side of the emitting layer close to the cathode. The blocking layer is preferably provided in contact with the emitting layer to block at least any of holes, electrons, and excitons.

For instance, when the blocking layer is provided in contact with the side of the emitting layer close to the cathode, the blocking layer permits transport of electrons and blocks holes from reaching a layer provided closer to the cathode (e.g., the electron transporting layer) than the blocking layer. When the organic EL device includes the electron

transporting layer, the blocking layer is preferably interposed between the emitting layer and the electron transporting layer.

When the blocking layer is provided in contact with the side of the emitting layer close to the anode, the blocking layer permits transport of holes and blocks electrons from reaching a layer provided closer to the anode (e.g., the hole transporting layer) than the blocking layer. When the organic EL device includes the hole transporting layer, the blocking layer is preferably interposed between the emitting layer and the hole transporting layer.

Alternatively, the blocking layer may be provided adjacent to the emitting layer so that excitation energy does not leak out from the emitting layer toward neighboring layer(s). The blocking layer blocks excitons generated in the emitting layer from being transferred to a layer(s) (e.g., the electron transporting layer and the hole transporting layer) closer to the electrode(s) than the blocking layer.

The emitting layer is preferably bonded with the blocking layer.

Specific structure, shape and the like of the components in the invention may be designed in any manner as long as an object of the invention can be achieved.

Other Explanations

Herein, numerical ranges represented by "x to y" represents a range whose lower limit is the value (x) recited before "to" and whose upper limit is the value (y) recited after "to."

Rx and Ry are mutually bonded to form a ring, which means herein, for instance, that Rx and Ry contain a carbon atom, a nitrogen atom, an oxygen atom, a sulfur atom or a silicon atom, the atom (a carbon atom, a nitrogen atom, an oxygen atom, a sulfur atom or a silicon atom) contained in Rx and the atom (a carbon atom, a nitrogen atom, an oxygen atom, a sulfur atom or a silicon atom) contained in Ry are mutually bonded via a single bond, a double bond, a triple bond or a divalent linking group to form a ring having 5 or more ring atoms (specifically, a heterocyclic ring or an aromatic hydrocarbon ring). x represents a number, a character or a combination of a number and a character. y represents a number, a character or a combination of a number and a character.

The divalent linking group is not particularly limited and is exemplified by -O-, -CO-, $-CO_2-$, -S-, -SO-, $-SO_2-$, -NH-, -NRa-, and a group obtained by combining two or more linking groups of these linking group.

Specific examples of the heterocyclic ring include a cyclic structure (heterocyclic ring) obtained by removing a bond from a "heteroaryl group $Sub_2$" exemplarily shown in the later-described "Description of Each Substituent in Formula." The heterocyclic ring may have a substituent.

Specific examples of the aromatic hydrocarbon ring include cyclic structures (aromatic hydrocarbon rings) obtained by removing a bond from an "aryl group $Sub_1$" exemplarily shown in the later-described "Description of Each Substituent in Formula." The aromatic hydrocarbon ring may have a substituent.

Examples of Ra include a substituted or unsubstituted alkyl group $Sub_3$ having 1 to 30 carbon atoms, a substituted or unsubstituted aryl group $Sub_1$ having 6 to 30 ring carbon atoms, and a substituted or unsubstituted heteroaryl group $Sub_2$ having 5 to 30 ring atoms, which are exemplarily shown in the later-described "Description of Each Substituent in Formula."

Rx and Ry are mutually bonded to form a ring, which means, for instance, that: an atom contained in $Rx_1$ and an atom contained in $Ry_1$ in a molecular structure represented by a formula (E1) below form a ring (cyclic structure) E represented by a formula (E2); an atom contained in $Rx_1$ and an atom contained in $Ry_1$ in a molecular structure represented by a formula (F1) below form a ring (cyclic structure) F represented by a formula (F2); an atom contained in $Rx_1$ and an atom contained in $Ry_1$ in a molecular structure represented by a formula (G1) below form a ring (cyclic structure) G represented by a formula (G2); an atom contained in $Rx_1$ and an atom contained in $Ry_1$ in a molecular structure represented by a formula (H1) below form a ring (cyclic structure) H represented by a formula (H2); and an atom contained in $Rx_1$ and an atom contained in $Ry_1$ in a molecular structure represented by a formula (11) below form a ring (cyclic structure) I represented by a formula (I2).

In the formulae (E1) to (11), * each independently represents a bonding position to another atom in a molecule. Two * in the formula (E1) correspond one-to-one to two * in the formula (E2). Two * in the formula (F1) correspond one-to-one to two * in the formula (F2). Two * in the formula (G1) correspond one-to-one to two * in the formula (G2). Two * in the formula (H1) correspond one-to-one to two * in the formula (H2). Two * in the formula (11) correspond one-to-one to two * in the formula (I2).

[Formula 201]

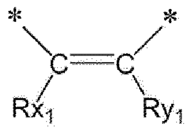

(E1)

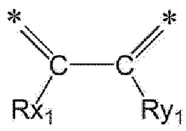

(F1)

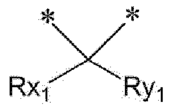

(G1)

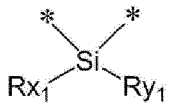

(H1)

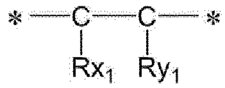

(I1)

[Formula 202]

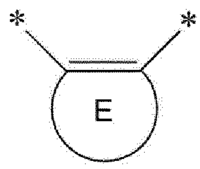

(E2)

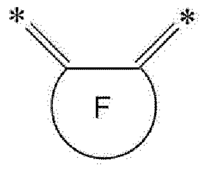

(F2)

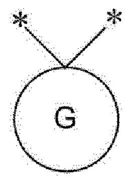

(G2)

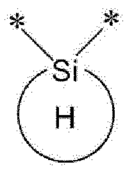

(H2)

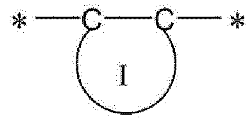

(I2)

In the molecular structures represented by the respective formulae (E2) to (I2), E to I each represent a cyclic structure (the ring having 5 or more ring atoms). In the formulae (E2) to (I2), * each independently represents a bonding position to another atom in a molecule. Two * in the formula (E2) correspond one-to-one to two * in the formula (E1). Similarly, two * in each of the formulae (F2) to (I2) correspond one-to-one to two * in in each of the formulae (F1) to (11).

For instance, when in the formula (E1), $Rx_1$ and $Ry_1$ are mutually bonded to form the ring E in the formula (E2) and the ring E is an unsubstituted benzene ring, the molecular structure represented by the formula (E1) is a molecular structure represented by a formula (E3) below. Herein, two * in the formula (E3) correspond one-to-one to two * in each of the formulae (E2) and (E1).

For instance, when in the formula (E1), $Rx_1$ and $Ry_1$ are mutually bonded to form the ring E in the formula (E2) and the ring E is an unsubstituted pyrrole ring, the molecular structure represented by the formula (E1) is a molecular structure represented by a formula (E4) below. Herein, two * in the formula (E4) correspond one-to-one to two * in each of the formulae (E2) and (E1). In the formulae (E3) and (E4), * each independently represents a bonding position to another atom in a molecule.

[Formula 203]

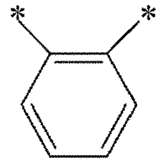

(E3)

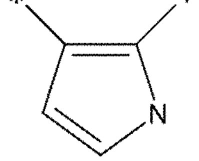

(E4)

Herein, the ring carbon atoms refer to the number of carbon atoms among atoms forming a ring of a compound

(e.g., a monocyclic compound, fused-ring compound, crosslinking compound, carbon ring compound, and heterocyclic compound) in which the atoms are bonded to each other to form the ring. When the ring is substituted by a substituent(s), carbon atom(s) contained in the substituent(s) is not counted in the ring carbon atoms. Unless specifically described, the same applies to the "ring carbon atoms" described later. For instance, a benzene ring has 6 ring carbon atoms, a naphthalene ring has 10 ring carbon atoms, a pyridinyl group has 5 ring carbon atoms, and a furanyl group has 4 ring carbon atoms. When a benzene ring and/or a naphthalene ring is substituted by a substituent (e.g., an alkyl group), the number of carbon atoms of the alkyl group is not counted in the number of the ring carbon atoms. When a fluorene ring is substituted by a substituent (e.g., a fluorene ring) (i.e., a spirofluorene ring is included), the number of carbon atoms of the fluorene ring as the substituent is not counted in the number of the ring carbon atoms of the fluorene ring.

Herein, the ring atoms refer to the number of atoms forming a ring of a compound (e.g., a monocyclic compound, fused-ring compound, crosslinking compound, carbon ring compound, and heterocyclic compound) in which the atoms are bonded to each other to form the ring (e.g., monocyclic ring, fused ring, ring assembly). Atom(s) not forming a ring and atom(s) included in a substituent when the ring is substituted by the substituent are not counted in the number of the ring atoms. Unless specifically described, the same applies to the "ring atoms" described later. For instance, a pyridine ring has six ring atoms, a quinazoline ring has ten ring atoms, and a furan ring has five ring atoms. A hydrogen atom(s) and/or an atom(s) of a substituent which are bonded to carbon atoms of a pyridine ring and/or quinazoline ring are not counted in the ring atoms. When a fluorene ring is substituted by a substituent (e.g., a fluorene ring) (i.e., a spirofluorene ring is included), the number of atoms of the fluorene ring as the substituent is not counted in the number of the ring atoms of the fluorene ring.

Description of Each Substituent in Formula Herein

The aryl group (occasionally referred to as an aromatic hydrocarbon group) herein is exemplified by an aryl group $Sub_1$. The aryl group $Sub_1$ is at least one group selected from the group consisting of a phenyl group, biphenyl group, terphenyl group, naphthyl group, anthryl group, phenanthryl group, fluorenyl group, pyrenyl group, chrysenyl group, fluoranthenyl group, benz[a]anthryl group, benzo[c]phenanthryl group, triphenylenyl group, benzo[k]fluoranthenyl group, benzo[g]chrysenyl group, benzo[b]triphenylenyl group, picenyl group, and perylenyl group.

Herein, the aryl group $Sub_1$ preferably has 6 to 30 ring carbon atoms, more preferably 6 to 22 ring carbon atoms, further preferably 6 to 20 ring carbon atoms, further more preferably 6 to 14 ring carbon atoms, still further preferably 6 to 12 ring carbon atoms. Among the aryl group $Sub_1$, a phenyl group, biphenyl group, naphthyl group, phenanthryl group, terphenyl group and fluorenyl group are preferable. A carbon atom in a position 9 of each of 1-fluorenyl group, 2-fluorenyl group, 3-fluorenyl group and 4-fluorenyl group is preferably substituted by a substituted or unsubstituted alkyl group $Sub_3$ or a substituted or unsubstituted aryl group $Sub_1$ described later herein.

The heteroaryl group (occasionally referred to as a heterocyclic group, heteroaromatic ring group or aromatic heterocyclic group) herein is exemplified by a heterocyclic group $Sub_2$. The heterocyclic group $Sub_2$ is a group containing, as a hetero atom(s), at least one atom selected from the group consisting of nitrogen, sulfur, oxygen, silicon, selenium atom and germanium atom. The heterocyclic group $Sub_2$ preferably contains, as a hetero atom(s), at least one atom selected from the group consisting of nitrogen, sulfur and oxygen.

The heterocyclic group $Sub_2$ herein are, for instance, at least one group selected from the group consisting of a pyridyl group, pyrimidinyl group, pyrazinyl group, pyridazinyl group, triazinyl group, quinolyl group, isoquinolinyl group, naphthyridinyl group, phthalazinyl group, quinoxalinyl group, quinazolinyl group, phenanthridinyl group, acridinyl group, phenanthrolinyl group, pyrrolyl group, imidazolyl group, pyrazolyl group, triazolyl group, tetrazolyl group, indolyl group, benzimidazolyl group, indazolyl group, imidazopyridinyl group, benzotriazolyl group, carbazolyl group, furyl group, thienyl group, oxazolyl group, thiazolyl group, isoxazolyl group, isothiazolyl group, oxadiazolyl group, thiadiazolyl group, benzofuranyl group, benzothienyl group, benzoxazolyl group, benzothiazolyl group, benzisoxazolyl group, benzisothiazolyl group, benzoxadiazolyl group, benzothiadiazolyl group, dibenzofuranyl group, dibenzothienyl group, piperidinyl group, pyrrolidinyl group, piperazinyl group, morpholyl group, phenazinyl group, phenothiazinyl group, and phenoxazinyl group.

Herein, the heterocyclic group $Sub_2$ preferably has 5 to 30 ring atoms, more preferably 5 to 20 ring atoms, further preferably 5 to 14 ring atoms. Among the above heterocyclic group $Sub_2$, a 1-dibenzofuranyl group, 2-dibenzofuranyl group, 3-dibenzofuranyl group, 4-dibenzofuranyl group, 1-dibenzothienyl group, 2-dibenzothienyl group, 3-dibenzothienyl group, 4-dibenzothienyl group, 1-carbazolyl group, 2-carbazolyl group, 3-carbazolyl group, 4-carbazolyl group, and 9-carbazolyl group are further more preferable. A nitrogen atom in position 9 of 1-carbazolyl group, 2-carbazolyl group, 3-carbazolyl group and 4-carbazolyl group is preferably substituted by the substituted or unsubstituted aryl group $Sub_1$ or the substituted or unsubstituted heterocyclic group $Sub_2$ described herein.

Herein, the heterocyclic group $Sub_2$ may be a group derived from any one of partial structures represented by formulae (XY-1) to (XY-18) below.

[Formula 204]

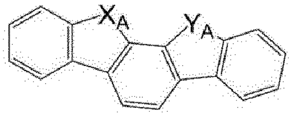
(XY-1)

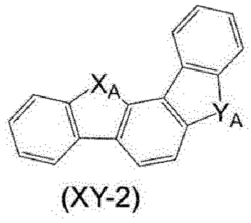
(XY-2)

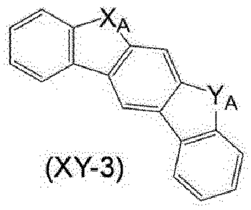
(XY-3)

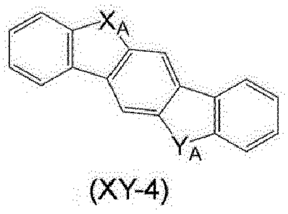
(XY-4)

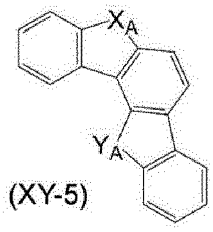
(XY-5)

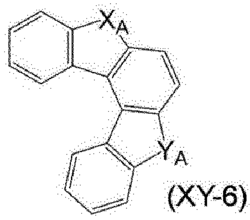
(XY-6)

[Formula 205]

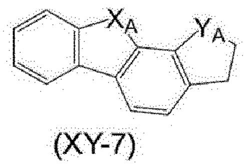
(XY-7)

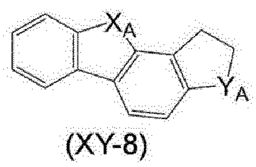
(XY-8)

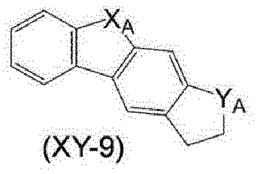
(XY-9)

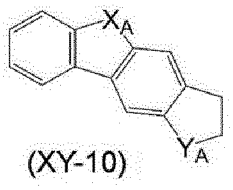
(XY-10)

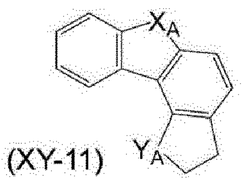
(XY-11)

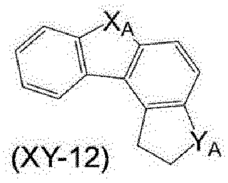
(XY-12)

[Formula 206]

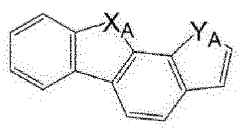
(XY-13)

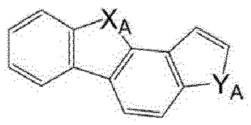
(XY-14)

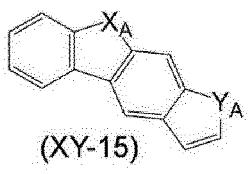
(XY-15)

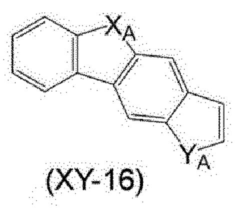
(XY-16)

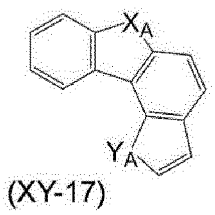
(XY-17)

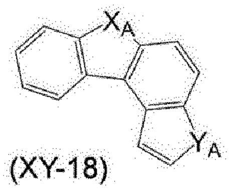
(XY-18)

In the formulae (XY-1) to (XY-18), $X_A$ and $Y_A$ each independently represent a hetero atom, and preferably represent an oxygen atom, sulfur atom, selenium atom, silicon atom or germanium atom. Each of the partial structures represented by the respective formulae (XY-1) to (XY-18) has a bond at any position to provide a heterocyclic group.

The heterocyclic group may be substituted.

Herein, the heterocyclic group $Sub_2$ may be a group represented by one of formulae (XY-19) to (XY-22) below. Moreover, the position of the bond may be changed as needed

[Formula 207]

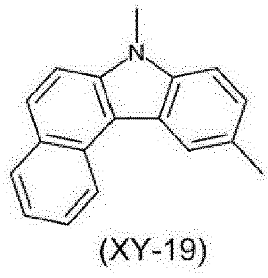
(XY-19)
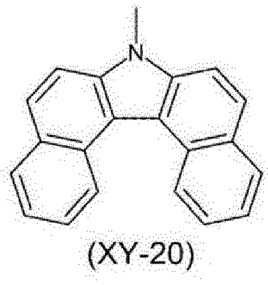
(XY-20)
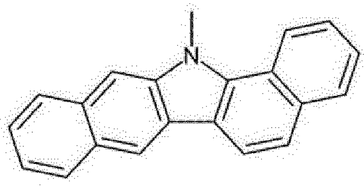
(XY-21)
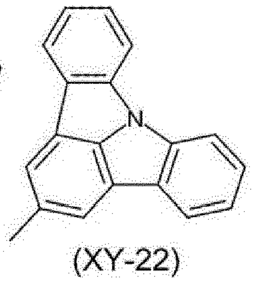
(XY-22)

The alkyl group herein may be any one of a linear alkyl group, branched alkyl group and cyclic alkyl group.

The alkyl group herein is exemplified by an alkyl group $Sub_3$.

The linear alkyl group herein is exemplified by a linear alkyl group $Sub_{31}$.

The branched alkyl group herein is exemplified by a branched alkyl group $Sub_{32}$.

The cyclic alkyl group herein is exemplified by a cyclic alkyl group $Sub_{33}$.

For instance, the alkyl group $Sub_3$ is at least one group selected from the group consisting of the linear alkyl group $Sub_{31}$, branched alkyl group $Sub_{32}$, and cyclic alkyl group $Sub_{33}$.

The linear alkyl group $Sub_{31}$ or branched alkyl group $Sub_{32}$ is exemplified by at least one group selected from the group consisting of a methyl group, ethyl group, n-propyl group, isopropyl group, n-butyl group, s-butyl group, isobutyl group, t-butyl group, n-pentyl group, n-hexyl group, n-heptyl group, n-octyl group, n-nonyl group, n-decyl group, n-undecyl group, n-dodecyl group, n-tridecyl group, n-tetradecyl group, n-pentadecyl group, n-hexadecyl group, n-heptadecyl group, n-octadecyl group, neopentyl group, amyl group, isoamyl group, 1-methylpentyl group, 2-methylpentyl group, 1-pentylhexyl group, 1-butylpentyl group, 1-heptyloctyl group, and 3-methylpentyl group.

Herein, the linear alkyl group $Sub_{31}$ or branched alkyl group $Sub_{32}$ preferably has 1 to 30 carbon atoms, more preferably 1 to 20 carbon atoms, further preferably 1 to 10 carbon atoms, further more preferably 1 to 6 carbon atoms. The linear alkyl group $Sub_{31}$ or branched alkyl group $Sub_{32}$ is further more preferably a methyl group, ethyl group, propyl group, isopropyl group, n-butyl group, s-butyl group, isobutyl group, t-butyl group, n-pentyl group, n-hexyl group, amyl group, isoamyl group and neopentyl group.

Herein, the cyclic alkyl group $Sub_{33}$ is exemplified by a cycloalkyl group $Sub_{331}$.

The cycloalkyl group $Sub_{331}$ herein is exemplified by at least one group selected from the group consisting of a cyclopropyl group, cyclobutyl group, cyclopentyl group, cyclohexyl group, 4-metylcyclohexyl group, adamantyl group and norbornyl group. The cycloalkyl group $Sub_{331}$ preferably has 3 to 30 ring carbon atoms, more preferably 3 to 20 ring carbon atoms, further preferably 3 to 10 ring carbon atoms, further more preferably 5 to 8 ring carbon atoms. Among the cycloalkyl group $Sub_{331}$, a cyclopentyl group and a cyclohexyl group are further more preferable.

Herein, an alkyl halide group is exemplified by an alkyl halide group $Sub_4$. The alkyl halide group $Sub_4$ is provided by substituting the alkyl group $Sub_3$ with at least one halogen atom, preferably at least one fluorine atom.

Herein, the alkyl halide group $Sub_4$ is exemplified by at least one group selected from the group consisting of a fluoromethyl group, difluoromethyl group, trifluoromethyl group, fluoroethyl group, trifluoromethylmethyl group, trifluoroethyl group, and pentafluoroethyl group.

Herein, a substituted silyl group is exemplified by a substituted silyl group Subs. The substituted silyl group $Sub_5$ is exemplified by at least one group selected from the group consisting of an alkylsilyl group $Sub_{51}$ and an arylsilyl group $Sub_{52}$.

Herein, the alkylsilyl group $Sub_{51}$ is exemplified by a trialkylsilyl group $Sub_{511}$ having the above-described alkyl group $Sub_3$.

The trialkylsilyl group $Sub_{511}$ is exemplified by at least one group selected from the group consisting of a trimethylsilyl group, triethylsilyl group, tri-n-butylsilyl group, tri-n-octylsilyl group, triisobutylsilyl group, dimethylethylsilyl group, dimethylisopropylsilyl group, dimethyl-n-propylsilyl group, dimethyl-n-butylsilyl group, dimethyl-t-butylsilyl group, diethylisopropylsilyl group, vinyl dimethylsilyl group, propyldimethylsilyl group, and triisopropylsilyl group. Three alkyl groups $Sub_3$ in the trialkylsilyl group $Sub_{511}$ may be mutually the same or different.

Herein, the arylsilyl group $Sub_{52}$ is exemplified by at least one group selected from the group consisting of a

dialkylarylsilyl group $Sub_{521}$, alkyldiarylsilyl group $Sub_{522}$ and triarylsilyl group $Sub_{523}$.

The dialkylarylsilyl group $Sub_{521}$ is exemplified by a dialkylarylsilyl group including two alkyl groups $Sub_3$ and one aryl group $Sub_1$. The dialkylarylsilyl group $Sub_{521}$ preferably has 8 to 30 carbon atoms.

The alkyldiarylsilyl group $Sub_{522}$ is exemplified by an alkyldiarylsilyl group including one alkyl group $Sub_3$ and two aryl groups $Sub_1$. The alkyldiarylsilyl group $Sub_{522}$ preferably has 13 to 30 carbon atoms.

The triarylsilyl group $Sub_{523}$ is exemplified by a triarylsilyl group including three aryl groups $Sub_1$. The triarylsilyl group $Sub_{523}$ preferably has 18 to 30 carbon atoms.

Herein, a substituted or unsubstituted alkyl sulfonyl group is exemplified by an alkyl sulfonyl group Subs. The alkyl sulfonyl group $Sub_6$ is represented by $-SO_2R_w$. $R_w$ in $-SO_2R_w$ represents a substituted or unsubstituted alkyl group $Sub_3$ described above.

Herein, an aralkyl group (occasionally referred to as an arylalkyl group) is exemplified by an aralkyl group $Sub_7$. An aryl group in the aralkyl group $Sub_7$ includes, for instance, at least one of the above-described aryl group $Sub_1$ or the above-described heteroaryl group $Sub_2$.

The aralkyl group $Sub_7$ herein is preferably a group having the aryl group $Sub_1$ and is represented by $-Z_3-Z_4$. $Z_3$ is exemplified by an alkylene group corresponding to the above alkyl group $Sub_3$. $Z_4$ is exemplified by the above aryl group $Sub_1$. In this aralkyl group $Sub_7$, an aryl moiety has 6 to 30 carbon atoms (preferably 6 to 20 carbon atoms, more preferably 6 to 12 carbon atoms) and an alkyl moiety has 1 to 30 carbon atoms (preferably 1 to 20 carbon atoms, more preferably 1 to 10 carbon atoms, further preferably 1 to 6 carbon atoms). The aralkyl group $Sub_7$ is exemplified by at least one group selected from the group consisting of a benzyl group, 2-phenylpropane-2-yl group, 1-phenylethyl group, 2-phenylethyl group, 1-phenylisopropyl group, 2-phenylisopropyl group, phenyl-t-butyl group, α-naphthylmethyl group, 1-α-naphthylethyl group, 2-α-naphthylethyl group, 1-α-naphthylisopropyl group, 2-α-naphthylisopropyl group, β-naphthylmethyl group, 1-β-naphthylethyl group, 2-β-naphthylethyl group, 1-β-naphthylisopropyl group, and 2-β-naphthylisopropyl group.

The alkoxy group herein is exemplified by an alkoxy group Subs. The alkoxy group Subs is represented by $-OZ_1$. $Z_1$ is exemplified by the above alkyl group $Sub_3$. The alkoxy group Subs is exemplified by at least one group selected from the group consisting of a methoxy group, ethoxy group, propoxy group, butoxy group, pentyloxy group and hexyloxy group. The alkoxy group Subs preferably has 1 to 30 carbon atoms, more preferably 1 to 20 carbon atoms.

Herein, an alkoxy halide group is exemplified by an alkoxy halide group Subs. The alkoxy halide group Subs is provided by substituting the alkoxy group Subs with at least one halogen atom, preferably at least one fluorine atom.

Herein, an aryloxy group (occasionally referred to as an arylalkoxy group) is exemplified by an arylalkoxy group $Sub_{10}$. An aryl group in the arylalkoxy group $Sub_{10}$ includes at least one of the aryl group $Sub_1$ or the heteroaryl group $Sub_2$.

The arylalkoxy group $Sub_{10}$ herein is represented by $-OZ_2$. $Z_2$ is exemplified by the aryl group $Sub_1$ or the heteroaryl group $Sub_2$. The arylalkoxy group $Sub_{10}$ preferably has 6 to 30 ring carbon atoms, more preferably 6 to 20 ring carbon atoms. The arylalkoxy group $Sub_{10}$ is exemplified by a phenoxy group.

Herein, a substituted amino group is exemplified by a substituted amino group $Sub_{11}$. The substituted amino group $Sub_{11}$ is exemplified by at least one group selected from the group consisting of an arylamino group $Sub_{111}$ and an alkylamino group $Sub_{112}$.

The arylamino group $Sub_{111}$ is represented by $-NHR_{V1}$ or $-N(R_{V1})_2$. $R_{V1}$ is exemplified by the aryl group $Sub_1$. Two $R_{V1}$ in $-N(R_{V1})_2$ are mutually the same or different.

The alkylamino group $Sub_{112}$ is represented by $-NHR_{V2}$ or $-N(R_{V2})_2$. $R_{V2}$ is exemplified by the alkyl group Subs. Two $R_{V2}$ in $-N(R_{V2})_2$ are mutually the same or different.

Herein, the alkenyl group is exemplified by an alkenyl group $Sub_{12}$. The alkenyl group $Sub_{12}$, which is linear or branched, is exemplified by at least one group selected from the group consisting of a vinyl group, propenyl group, butenyl group, oleyl group, eicosapentaenyl group, docosahexaenyl group, styryl group, 2,2-diphenylvinyl group, 1,2,2-triphenylvinyl group, and 2-phenyl-2-propenyl group.

The alkynyl group herein is exemplified by an alkynyl group $Sub_{13}$. The alkynyl group $Sub_{13}$ may be linear or branched, and is exemplified by at least one group selected from the group consisting of an ethynyl group, a propynyl group and a 2-phenylethynyl group.

The alkylthio group herein is exemplified by an alkylthio group $Sub_{14}$.

The alkylthio group $Sub_{14}$ is represented by $-SR_{V3}$. $R_{V3}$ is exemplified by the alkyl group $Sub_3$. The alkylthio group $Sub_{14}$ preferably has 1 to 30 carbon atoms, more preferably 1 to 20 carbon atoms.

The arylthio group herein is exemplified by an arylthio group $Sub_{15}$.

The arylthio group $Sub_{15}$ is represented by $-SR_{V4}$. $R_{V4}$ is exemplified by the aryl group $Sub_1$. The arylthio group $Sub_{15}$ preferably has 6 to 30 ring carbon atoms, more preferably 6 to 20 ring carbon atoms.

Herein, examples of the halogen atom include a fluorine atom, a chlorine atom, a bromine atom and an iodine atom, among which a fluorine atom is preferable.

A substituted phosphino group herein is exemplified by a substituted phosphino group $Sub_{16}$. The substituted phosphino group $Sub_{16}$ is exemplified by a phenyl phosphanyl group.

An arylcarbonyl group herein is exemplified by an arylcarbonyl group $Sub_{17}$. The arylcarbonyl group $Sub_{17}$ is represented by -COY'. Y' is exemplified by the aryl group $Sub_1$. Herein, the arylcarbonyl group $Sub_{17}$ is exemplified by at least one group selected from the group consisting of a phenyl carbonyl group, diphenyl carbonyl group, naphthyl carbonyl group, and triphenyl carbonyl group.

An acyl group herein is exemplified by an acyl group $Sub_{18}$. The acyl group $Sub_{18}$ is represented by -COR'. R' is exemplified by the alkyl group $Sub_3$. The acyl group $Sub_{18}$ herein is exemplified by at least one group selected from the group consisting of an acetyl group and a propionyl group.

A substituted phosphoryl group herein is exemplified by a substituted phosphoryl group $Sub_{19}$. The substituted phosphoryl group $Sub_{19}$ is represented by a formula (P) below.

[Formula 208]

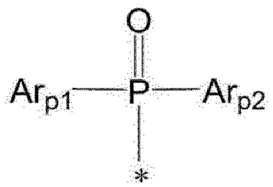

(P)

In the formula (P), $Ar_{P1}$ and $Ar_{P2}$ are any one substituent selected from the group consisting of the above alkyl group Subs and the above aryl group $Sub_1$.

An ester group herein is exemplified by an ester group $Sub_{20}$. The ester group $Sub_{20}$ is exemplified by at least one group selected from the group consisting of an alkyl ester group and an aryl ester group.

An alkyl ester group herein is exemplified by an alkyl ester group $Sub_{201}$. The alkyl ester group $Sub_{201}$ is represented by $-C(=O)OR^E$. $R^E$ is exemplified by a substituted or unsubstituted alkyl group $Sub_3$ described above (preferably having 1 to 10 carbon atoms).

An aryl ester group herein is exemplified by an aryl ester group $Sub_{202}$. The aryl ester group $Sub_{202}$ is represented by $-C(=O)OR^{Ar}$. $R^{Ar}$ is exemplified by a substituted or unsubstituted aryl group $Sub_1$ described above.

A siloxanyl group herein is exemplified by a siloxanyl group $Sub_{21}$. The siloxanyl group $Sub_{21}$ is a silicon compound group through an ether bond. The siloxanyl group $Sub_{21}$ is exemplified by a trimethylsiloxanyl group.

A carbamoyl group herein is represented by $-CONH_2$.

A substituted carbamoyl group herein is exemplified by a carbamoyl group $Sub_{22}$. The carbamoyl group $Sub_{22}$ is represented by $-CONH-Ar^C$ or $-CONH-R^C$. $Ar^C$ is exemplified by at least one group selected from the group consisting of a substituted or unsubstituted aryl group $Sub_1$ (preferably 6 to 10 ring carbon atoms) and a substituted or unsubstituted heteroaryl group $Sub_2$ (preferably 5 to 14 ring atoms). $Ar^C$ may be a group formed by bonding the aryl group $Sub_1$ and the heteroaryl group $Sub_2$.

$R^C$ is exemplified by a substituted or unsubstituted alkyl group $Sub_3$ described above (preferably having 1 to 6 carbon atoms).

Herein, "carbon atoms forming a ring (ring carbon atoms)" mean carbon atoms forming a saturated ring, unsaturated ring, or aromatic ring. "Atoms forming a ring (ring atoms)" mean carbon atoms and hetero atoms forming a ring including a saturated ring, unsaturated ring, or aromatic ring.

Herein, a hydrogen atom includes isotope having different numbers of neutrons, specifically, protium, deuterium and tritium.

Hereinafter, an alkyl group $Sub_3$ means at least one group of a linear alkyl group $Sub_{31}$, a branched alkyl group $Sub_{32}$, or a cyclic alkyl group $Sub_{33}$ described in "Description of Each Substituent."

Similarly, a substituted silyl group Subs means at least one group of an alkylsilyl group $Sub_{51}$ or an arylsilyl group $Sub_{52}$.

Similarly, a substituted amino group $Sub_{11}$ means at least one group of an arylamino group $Sub_{111}$ or an alkylamino group $Sub_{112}$.

Herein, a substituent for a "substituted or unsubstituted" group is exemplified by a substituent $R_{F1}$. The substituent $R_{F1}$ is at least one group selected from the group consisting of an aryl group $Sub_1$, heteroaryl group $Sub_2$, alkyl group Subs, alkyl halide group $Sub_4$, substituted silyl group Subs, alkylsulfonyl group Subs, aralkyl group $Sub_7$, alkoxy group Subs, alkoxy halide group Subs, arylalkoxy group $Sub_{10}$, substituted amino group $Sub_{11}$, alkenyl group $Sub_{12}$, alkynyl group $Sub_{13}$, alkylthio group $Sub_{14}$, arylthio group $Sub_{15}$, substituted phosphino group $Sub_{16}$, arylcarbonyl group $Sub_{17}$, acyl group $Sub_{18}$, substituted phosphoryl group $Sub_{19}$, ester group $Sub_{20}$, siloxanyl group $Sub_{21}$, carbamoyl group $Sub_{22}$, unsubstituted amino group, unsubstituted silyl group, halogen atom, cyano group, hydroxy group, nitro group, and carboxy group.

Herein, the substituent $R_{F1}$ for a "substituted or unsubstituted" group may be a diaryl boron group ($Ar_{B1}Ar_{B2}B$-).$Ar_{B1}$ and $Ar_{B2}$ are exemplified by the above-described aryl group $Sub_1$. $Ar_{B1}$ and $Ar_{B2}$ in $Ar_{B1}Ar_{B2}B$- are the same or different.

Specific examples and preferable examples of the substituent $R_{F1}$ are the same as those of the substituents described in "Description of Each Substituent" (e.g., an aryl group $Sub_1$, heteroaryl group $Sub_2$, alkyl group Subs, alkyl halide group $Sub_4$, substituted silyl group Subs, alkylsulfonyl group $Sub_6$, aralkyl group $Sub_7$, alkoxy group $Sub_8$, alkoxy halide group $Sub_9$, arylalkoxy group $Sub_{10}$, substituted amino group $Sub_{11}$, alkenyl group $Sub_{12}$, alkynyl group $Sub_{13}$, alkylthio group $Sub_{14}$, arylthio group $Sub_{15}$, substituted phosphino group $Sub_{16}$, arylcarbonyl group $Sub_{17}$, acyl group $Sub_{18}$, substituted phosphoryl group $Sub_{19}$, ester group $Sub_{20}$, siloxanyl group $Sub_{21}$, and carbamoyl group $Sub_{22}$).

The substituent $R_{F1}$ for a "substituted or unsubstituted" group may be further substituted by at least one group (hereinafter, also referred to as a substitutent $R_{F2}$) selected from the group consisting of an aryl group $Sub_1$, heteroaryl group $Sub_2$, alkyl group $Sub_3$, alkyl halide group $Sub_4$, substituted silyl group $Sub_5$, alkylsulfonyl group $Sub_6$, aralkyl group $Sub_7$, alkoxy group $Sub_6$, alkoxy halide group $Sub_6$, arylalkoxy group $Sub_{10}$, substituted amino group $Sub_{11}$, alkenyl group $Sub_{12}$, alkynyl group $Sub_{13}$, alkylthio group $Sub_{14}$, arylthio group $Sub_{15}$, substituted phosphino group $Sub_{16}$, arylcarbonyl group $Sub_{17}$, acyl group $Sub_{18}$, substituted phosphoryl group $Sub_{19}$, ester group $Sub_{20}$, siloxanyl group $Sub_{21}$, carbamoyl group $Sub_{22}$, unsubstituted amino group, unsubstituted silyl group, halogen atom, cyano group, hydroxy group, nitro group, and carboxy group. Moreover, a plurality of substituents $R_{F2}$ may be bonded to each other to form a ring.

"Unsubstituted" for a "substituted or unsubstituted" group means that a group is not substituted by the above-described substituent $R_{F1}$ but bonded with a hydrogen atom.

Herein, "XX to YY carbon atoms" in the description of "substituted or unsubstituted ZZ group having XX to YY carbon atoms" represent carbon atoms of an unsubstituted ZZ group and do not include carbon atoms of the substituent $R_{F1}$ of the substituted ZZ group.

Herein, "XX to YY atoms" in the description of "substituted or unsubstituted ZZ group having XX to YY atoms" represent atoms of an unsubstituted ZZ group and do not include atoms of the substituent $R_{F1}$ of the substituted ZZ group.

The same description as the above applies to "substituted or unsubstituted" in compounds or partial structures thereof described herein.

Herein, when the substituents are bonded to each other to form a ring, the ring is structured to be a saturated ring, an unsaturated ring, an aromatic hydrocarbon ring or a hetero ring.

Herein, examples of the aromatic hydrocarbon group in the linking group include a divalent or multivalent group obtained by eliminating one or more atoms from the above monovalent aryl group $Sub_1$.

Herein, examples of the heterocyclic group in the linking group include a divalent or multivalent group obtained by eliminating one or more atoms from the above monovalent heteroaryl group $Sub_2$.

Examples

Compounds

Structures of the compound represented by the formula (3) and used for manufacturing organic EL devices in Examples 1 to 4 are shown below.

[Formula 209]

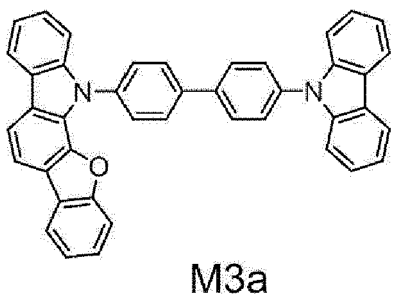

M3a

[Formula 210]

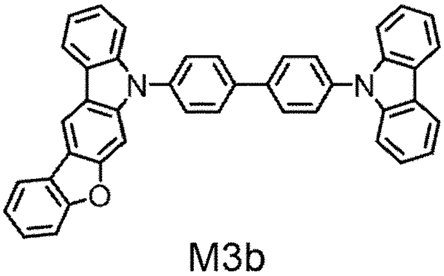

M3b

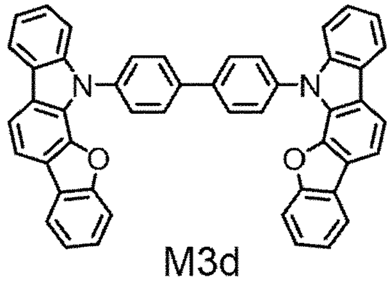

M3d

Structures of comparative compounds used for manufacturing organic EL devices in Comparatives 1 to 3 are shown below.

[Formula 211]

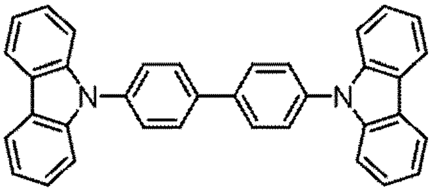

Ref-1

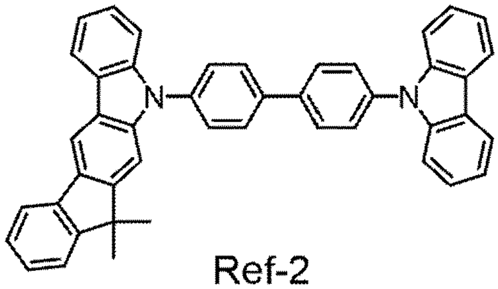

Ref-2

Structures of other compounds used for manufacturing the organic EL devices in Examples 1 to 4 and Comparatives 1 to 3 are shown below.

[Formula 212]

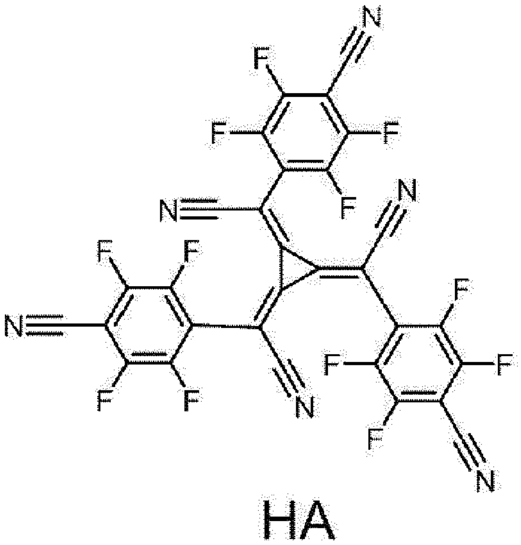

HA

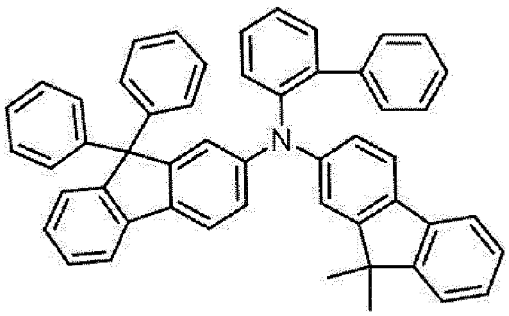

HT

[Formula 213]

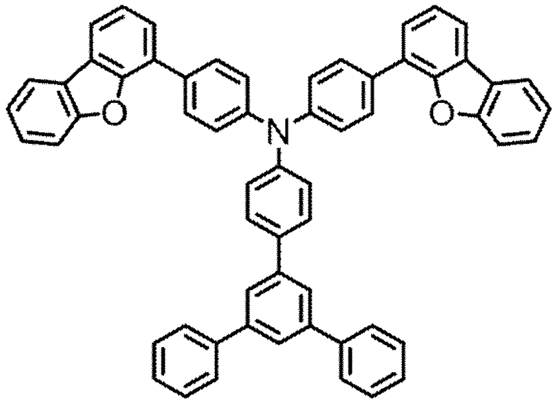

EBL

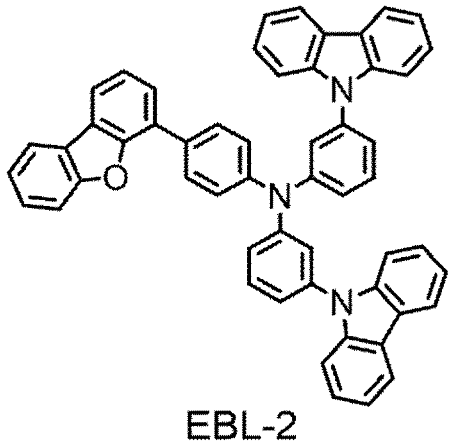

EBL-2

[Formula 214]

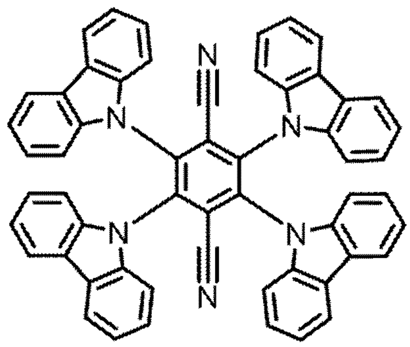

TADF

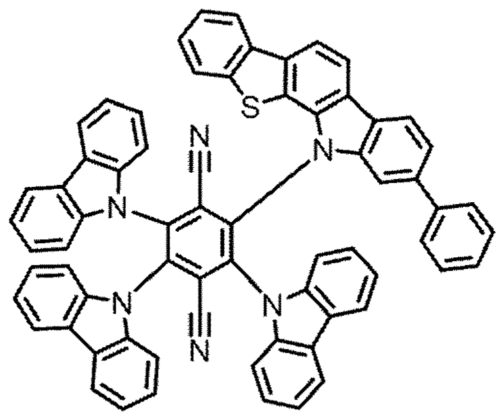

TADF-2

[Formula 215]

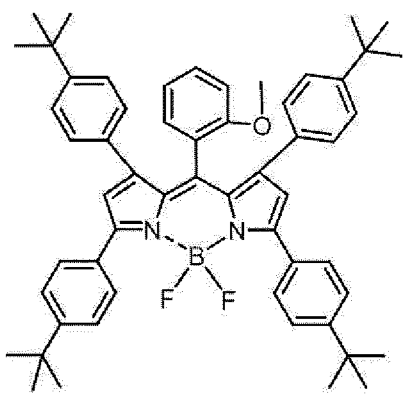

RD

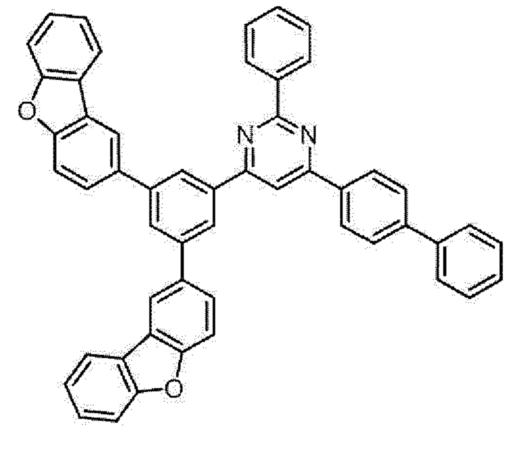

HBL

[Formula 216]

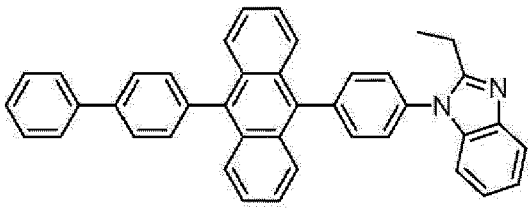

ET

Manufacture of Organic EL Devices

Organic EL devices were manufactured and evaluated as follows.

Example 1

A glass substrate (size: 25 mm × 75 mm × 1.1 mm thick, manufactured by Geomatec Co., Ltd.) having an ITO transparent electrode (anode) was ultrasonic-cleaned in isopropyl alcohol for five minutes, and then UV/ozone-cleaned for one minute. A film of ITO was 130 nm thick.

After the glass substrate having the transparent electrode line was cleaned, the glass substrate was mounted on a substrate holder of a vacuum evaporation apparatus. Firstly, a compound HT and a compound HA were co-deposited on a surface of the glass substrate where the transparent electrode line was provided in a manner to cover the transparent electrode, thereby forming a 10-nm-thick hole injecting layer. The concentrations of the compound HT and the compound HA in the hole injecting layer were 97 mass% and 3 mass%, respectively.

Next, the compound HT was vapor-deposited on the hole injecting layer to form a 200-nm-thick hole transporting layer.

Next, the compound EBL was vapor-deposited on the hole transporting layer to form a 10-nm-thick electron blocking layer.

Next, a compound M3a (the compound M3), a compound TADF (the compound M2) and a compound RD (the compound M1) were co-deposited on the electron blocking layer to form a 25-nm-thick emitting layer. The concentrations of the compound M3a, the compound TADF, and the compound RD in the emitting layer were 74 mass%, 25 mass%, and 1 mass%, respectively.

Next, a compound HBL was vapor-deposited on the emitting layer to form a 10-nm-thick hole blocking layer.

Next, a compound ET was vapor-deposited on the hole blocking layer to form a 30-nm-thick electron transporting layer.

Lithium fluoride (LiF) was vapor-deposited on the electron transporting layer to form a 1-nm-thick electron injectable electrode (cathode).

Subsequently, metal aluminum (Al) was vapor-deposited on the electron injectable electrode to form an 80-nm-thick metal Al cathode.

A device arrangement of the organic EL device of Example 1 is roughly shown as follows.

ITO(130)/HT: HA(10,97%:3%)/HT(200)/EBL(10)/M3a:TADF: RD(25,74%:25%:1 %)/H BL(10)/ET(30)/LiF(1)/Al(80)

Numerals in parentheses represent a film thickness (unit: nm).

The numerals (97%:3%) represented by percentage in the same parentheses indicate a ratio (mass%) between the compound HT and the compound HA in the hole injecting layer, and the numerals (74%:25%:1%) represented by percentage in the same parentheses indicate a ratio (mass%) between the compound M3a, the compound TADF, and the compound RD in the emitting layer. Similar notations apply to the description below.

Comparative 1

The organic EL device in Comparative 1 was manufactured in the same manner as in Example 1 except that a compound shown in Table 1 was used in place of the compound M3a used in the emitting layer of the organic EL device of Example 1.

Evaluation of Organic EL Devices

The organic EL devices manufactured in Examples 1 to 4 and Comparatives 1 to 3 were evaluated as follows.

Evaluation results are shown in Table 1 or 2. It should be noted that a comparative compound Ref-1 used in Comparatives 1 and 2 and a comparative compound Ref-2 used in Comparative 3 are shown in a column of the compound M3 for convenience of explanation.

Main Peak Wavelength (λp)

Voltage was applied on the organic EL devices such that a current density of the organic EL device was 10 mA/cm$^2$, where spectral radiance spectrum was measured by a spectroradiometer CS-2000 (manufactured by Konica Minolta, Inc.). The main peak wavelength $\lambda_p$ (unit: nm) was calculated based on the obtained spectral-radiance spectra.

Drive Voltage

A voltage (unit: V) was measured when current was applied between the anode and the cathode such that a current density was 10 mA/cm$^2$.

External Quantum Efficiency EQE

Voltage was applied on the organic EL devices so that a current density was 10 mA/cm$^2$, where spectral radiance spectrum was measured by a spectroradiometer CS-2000 (manufactured by Konica Minolta, Inc.). The external quantum efficiency EQE (unit: %) was calculated based on the obtained spectral-radiance spectra, assuming that the spectra was provided under a Lambertian radiation.

[Table 1]

| | Compound M3 | | Compound M2 | | Compound M1 | | Evaluation Results of Devices (@10mA/cm$^2$) | | |
|---|---|---|---|---|---|---|---|---|---|
| | Type | S1 [eV] | Type | S1 [eV] | Type | S1 [eV] | λ p [nm] | Voltage V [V] | EQE [%] |
| Example 1 | M3a | 3.42 | TADF | 2.38 | RD | 2.02 | 621 | 4.41 | 18.2 |
| Comparative 1 | Ref-1 | 3.52 | TADF | 2.38 | RD | 2.02 | 621 | 4.41 | 15.6 |

In Tables 1 and 2 above, $S_1$[eV] shows values of physical properties of the compounds used in the emitting layer of each of Examples and Comparatives. For instance, for Example 1, it is shown that $S_1$ of the compound M3a is 3.42 eV and $S_1$ of the compound TADF is 2.38 eV.

Although Ref-1 of Comparative 1 does not fall under the formula of the compound M3, Ref-1 is shown in the same column as M3a used in Example 1 for convenience.

The organic EL device in Example 1 emitted light with a higher efficiency than the organic EL device in Comparative 1.

Manufacture of Organic EL Devices

Example 2

A glass substrate (size: 25 mm × 75 mm × 1.1 mm thick, manufactured by Geomatec Co., Ltd.) having an ITO transparent electrode (anode) was ultrasonic-cleaned in isopropyl alcohol for five minutes, and then UV/ozone-cleaned for one minute. A film of ITO was 130 nm thick.

After the glass substrate having the transparent electrode line was cleaned, the glass substrate was mounted on a substrate holder of a vacuum evaporation apparatus. Firstly, the compound HT and the compound HA were co-deposited on a surface of the glass substrate where the transparent electrode line was provided in a manner to cover the transparent electrode, thereby forming a 10-nm-thick hole injecting layer. The concentrations of the compound HT and the compound HA in the hole injecting layer were 97 mass% and 3 mass%, respectively.

Next, the compound HT was vapor-deposited on the hole injecting layer to form a 200-nm-thick hole transporting layer.

Next, the compound EBL-2 was vapor-deposited on the hole transporting layer to form a 10-nm-thick electron blocking layer.

Next, the compound M3a (the compound M3), a compound TADF-2 (the compound M2) and the compound RD (the compound M1) were co-deposited on the electron blocking layer to form a 25-nm-thick emitting layer. The

concentrations of the compound M3a, the compound TADF-2, and the compound RD in the emitting layer were 74 mass%, 25 mass%, and 1 mass%, respectively.

Next, the compound HBL was vapor-deposited on the emitting layer to form a 10-nm-thick hole blocking layer.

Next, the compound ET was vapor-deposited on the hole blocking layer to form a 30-nm-thick electron transporting layer.

Lithium fluoride (LiF) was vapor-deposited on the electron transporting layer to form a 1-nm-thick electron injectable electrode (cathode).

Subsequently, metal aluminum (Al) was vapor-deposited on the electron injectable electrode to form an 80-nm-thick metal Al cathode.

A device arrangement of the organic EL device of Example 1 is roughly shown as follows.

ITO(130)/HT:HA(10,97%:3%)/HT(200)/EBL-2(10)/M3a:TADF-2:RD(25,74%:25%:1%)/HBL(10)/ET(30)/LiF(1)/Al(80)

Numerals in parentheses represent a film thickness (unit: nm).

The numerals (97%:3%) represented by percentage in the same parentheses indicate a ratio (mass%) between the compound HT and the compound HA in the hole injecting layer, and the numerals (74%:25%:1%) represented by percentage in the same parentheses indicate a ratio (mass%) between the compound M3a, the compound TADF-2, and the compound RD in the emitting layer. Similar notations apply to the description below.

Examples 3 and 4

The organic EL devices in Examples 3 and 4 were each manufactured in the same manner as in Example 2 except that a compound shown in Table 2 was used in place of the compound M3a used in the emitting layer of the organic EL device of Example 2.

Comparatives 2 and 3

The organic EL devices in Comparatives 2 and 3 were each manufactured in the same manner as in Example 2 except that a compound shown in Table 2 was used in place of the compound M3a used in the emitting layer of the organic EL device of Example 2.

Evaluation of Organic EL Devices

The manufactured organic EL devices were evaluated by the same method as described above in terms of the external quantum efficiency EQE and main peak wavelength λp. Evaluation results are shown in Table 2.

[Table 2]

| | Compound M3 | | Compound M2 | | Compound M1 | | Evaluation Results of Devices (@10mA/$cm^2$) | |
|---|---|---|---|---|---|---|---|---|
| | Type | S1 [eV] | Type | S1 [eV] | Type | S1 [eV] | λ p [nm] | EQE [%] |
| Example 2 | M3a | 3.42 | TADF-2 | 2.34 | RD | 2.02 | 621 | 19.5 |
| Example 3 | M3b | 3.39 | TADF-2 | 2.34 | RD | 2.02 | 621 | 18.9 |
| Example 4 | M3d | 3.42 | TADF-2 | 2.34 | RD | 2.02 | 621 | 17.7 |
| Comparative 2 | Ref-1 | 3.52 | TADF-2 | 2.34 | RD | 2.02 | 621 | 16.0 |
| Comparative 3 | Ref-2 | 3.34 | TADF-2 | 2.34 | RD | 2.02 | 621 | 12.4 |

Although Ref-1 of Comparative 2 and Ref-2 of Comparative 3 do not fall under the formula of the compound M3, Ref-1 and Ref-2 are shown in the same column as the compound M3 used in Examples 2 to 4 for convenience.

The organic EL devices in Examples 2 to 4 emitted light with a higher efficiency than the organic EL devices in Comparatives 2 to 3.

Evaluation of Compounds

Values of physical properties of the compounds used in Examples 1 to 4 and Comparatives 1 to 3 were measured by the following method.

Delayed Fluorescence

Delayed Fluorescence of Compound TADF

Delayed fluorescence properties were checked by measuring transient photoluminescence (PL) using a device shown in Fig. 2. The compound TADF was dissolved in toluene to prepare a dilute solution with an absorbance of 0.05 or less at the excitation wavelength to eliminate the contribution of self-absorption. In order to prevent quenching due to oxygen, the sample solution was frozen and degassed and then sealed in a cell with a lid under an argon atmosphere to obtain an oxygen-free sample solution saturated with argon.

The fluorescence spectrum of the above sample solution was measured with a spectrofluorometer FP-8600 (manufactured by JASCO Corporation), and the fluorescence spectrum of a 9,10-diphenylanthracene ethanol solution was measured under the same conditions. Using the fluorescence area intensities of both spectra, the total fluorescence quantum yield was calculated by an equation (1) in Morris et al. J. Phys. Chem. 80 (1976) 969.

Prompt emission was observed immediately when the excited state was achieved by exciting the compound TADF with a pulse beam (i.e., a beam emitted from a pulse laser) having a wavelength to be absorbed by the compound TADF, and Delay emission was observed not immediately when the excited state was achieved but after the excited state was achieved. The delayed fluorescence in Examples means that an amount of Delay Emission is 5% or more with respect to an amount of Prompt Emission. Specifically, provided that the amount of Prompt emission is denoted by $X_P$ and the amount of Delay emission is denoted by $X_D$, the delayed fluorescence means that a value of $X_D/X_P$ is 0.05 or more.

An amount of Prompt emission, an amount of Delay emission and a ratio between the amounts thereof can be obtained according to the method as described in "Nature 492, 234-238, 2012" (Reference Document 1). The amount of Prompt emission and the amount of Delay emission may be calculated using a device different from one described in Reference Document 1 or one shown in Fig. 2.

It was confirmed that the amount of Delay emission was 5% or more with respect to the amount of Prompt emission in the compound TADF.

The compound TADF-2 was measured in the same manner as the compound TADF, so that it was confirmed that the amount of Delay emission was also 5% or more with respect to the amount of Prompt emission in the compound TADF-2.

Specifically, it was found that a value of $X_D/X_P$ was 0.05 or more in both the compounds TADF and TADF-2.

Singlet Energy $S_1$

Singlet energy $S_1$ of each of the compounds M3a, M3b, M3d, TADF, TADF-2 and RD, and the comparative compounds Ref-1 and Ref-2 was measured according to the above-described solution method. Measurement results are shown in Table 1 or 2.

Singlet energies of compounds M3c and M3e of later-described synthesis examples were measured according to the same method, and were 3.35 eV and 3.46 eV, respectively.

Energy Gap at 77K

An energy gap $T_{77K}$ at 77K of each of the compounds TADF and TADF-2 was measured by the above-described method.

$\Delta ST$

$\Delta ST$ was calculated based on the measured singlet energy $S_1$ and energy gap $T_{77K}$ at 77K. It was confirmed that $\Delta ST$ of each of the compounds TADF and TADF-2 was less than 0.01.

Synthesis Example 1: Synthesis of Compound M3a

(1-1) Synthesis of Compound M3a

A synthesis scheme of the compound M3a is shown below.

[Formula 217]

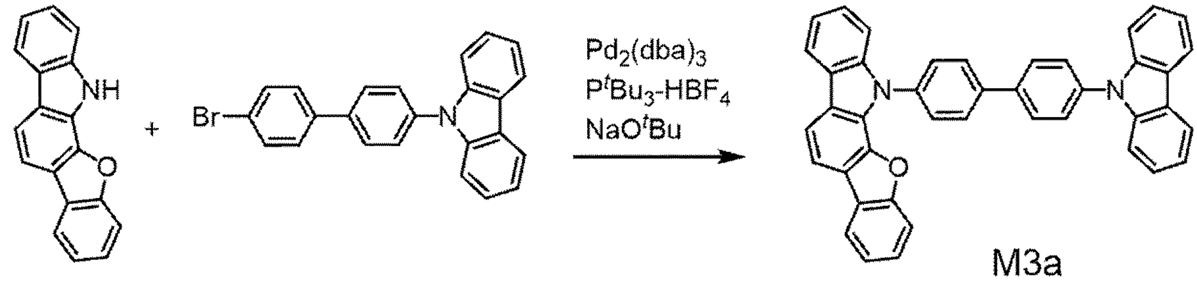


M3a

Under nitrogen atmosphere, xylene (675 mL) was added into a mixture of 12H-benzofuro[2,3-a]carbazole (26.6 g, 103 mmol), 9-(4'-bromo-[1,1'-biphenyl]-4-yl)-9H-carbazole (41.2 g, 103 mmol), tris(dibenzylideneacetone)dipalladium (1.90 g, 2.07 mmol), tri-tert-butylphosphonium tetrafluoroborate (1.20 g, 4.14 mmol), and sodium tert-butoxide (11.9 g, 124 mmol), and stirred at 130 degrees C for eight hours. After the reaction, a solid was collected by filtration. The solid collected by filtration was recrystallized with toluene to obtain the compound M3a (51.5 g, a yield of 87%). The obtained compound was identified as the compound M3a by analysis according to LC-MS (Liquid chromatography mass spectrometry).

Synthesis Example 2: Synthesis of Compound M3b

(1-2) Synthesis of Compound M3b

A synthesis scheme of the compound M3b is shown below.

[Formula 218]

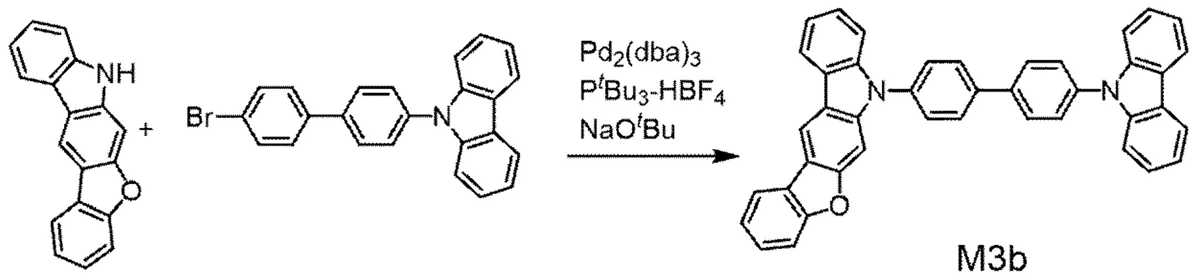


M3b

Under nitrogen atmosphere, xylene (650 mL) was added into a mixture of 7H-benzofuro[2,3-b]carbazole (25.8 g, 100 mmol), 9-(4'-bromo-[1,1'-biphenyl]-4-yl)-9H-carbazole (40.0 g, 100 mmol), tris(dibenzylideneacetone)dipalladium (1.83 g, 2.00 mmol), tri-tert-butylphosphonium tetrafluoroborate (1.16 g, 4.00 mmol), and sodium tert-butoxide (11.5 g, 120 mmol), and stirred at 130 degrees C for eight hours. After the reaction, a solid was collected by filtration. The solid collected by filtration was recrystallized with toluene to obtain the compound M3b (45.7 g, a yield of 79%). The obtained compound was identified as the compound M3b by analysis according to LC-MS (Liquid chromatography mass spectrometry).

Synthesis Example 3: Synthesis of Compound M3c

(1-3) Synthesis of Compound M3c

A synthesis scheme of the compound M3c is shown below.

[Formula 219]

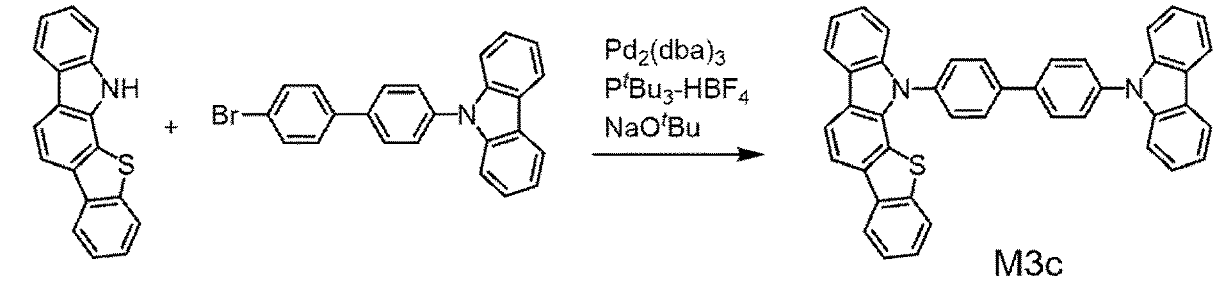


M3c

Under nitrogen atmosphere, xylene (650 mL) was added into a mixture of 12H-[1]benzothieno[2,3-a]carbazole (27.3 g, 100 mmol), 9-(4'-bromo-[1,1'-biphenyl]-4-yl)-9H-carbazole (40.0 g, 100 mmol), tris(dibenzylideneacetone)dipalladium (1.83 g, 2.00 mmol), tri-tert-butylphosphonium tetrafluoroborate (1.16 g, 4.00 mmol), and sodium tert-butoxide (11.5 g, 120 mmol), and stirred at 130 degrees C for eight hours. After the reaction, a solid was collected by filtration. The solid collected by filtration was recrystallized with toluene to obtain the compound M3c (42.5 g, a yield of 72%). The obtained compound was identified as the compound M3c by analysis according to LC-MS (Liquid chromatography mass spectrometry).

Synthesis Example 4: Synthesis of Compound M3d

(1-4) Synthesis of Compound M3e

A synthesis scheme of the compound M3e is shown below.

[Formula 220]

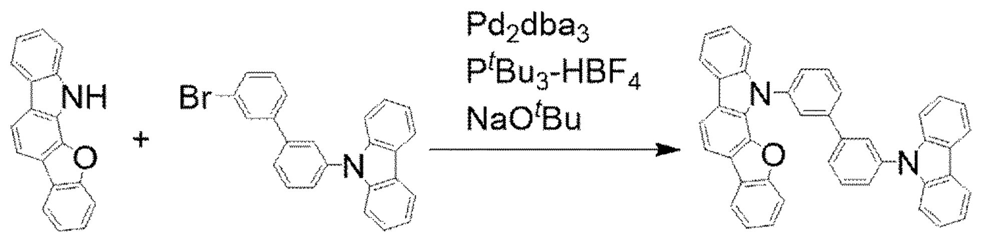


M3e

The compound M3e was obtained in the same manner as in Synthesis Example 1 except for using 9-(3'-bromo-[1,1'-biphenyl]-3-yl)-9H-carbazole in place of 9-(4'-bromo-[1,1'-biphenyl]-4-yl)-9H-carbazole in Synthesis Example 1. A yield was 63%. The obtained compound was identified as the compound M3e by analysis according to LC-MS (Liquid chromatography mass spectrometry).

EXPLANATION OF CODE(S)

1... organic EL device, 2... substrate, 3... anode, 4... cathode, 5... emitting layer, 6... hole injecting layer, 7... hole transporting layer, 8... electron transporting layer, 9... electron injecting layer

## Claims

1. An organic electroluminescence device comprising:

an anode;
a cathode;
an emitting layer provided between the anode and the cathode, wherein
the emitting layer comprises a delayed fluorescent compound M2, and a compound M3 represented by a formula

(3) below, and

a singlet energy $S_1(M2)$ of the compound M2 and a singlet energy $S_1(M3)$ of the compound M3 satisfy a relationship of a numerical formula (Numerical Formula 1) below,

$$S_1(M3) > S_1(M2) \ldots \text{(Numerical Formula 1)},$$

[Formula 1]

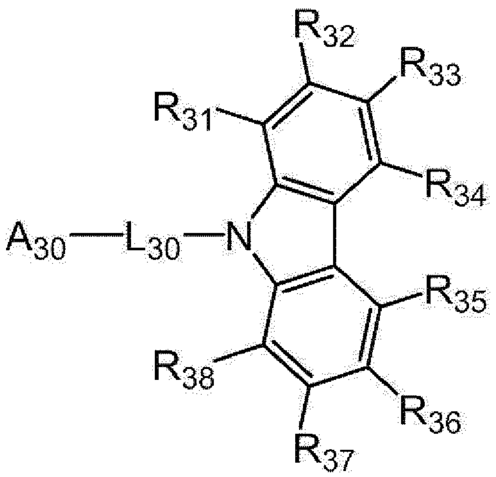

(3)

where, in the formula (3): $A_{30}$ is a group represented by a formula (3a), (3b), (3c), (3d), (3e) or (3f) below,

[Formula 2]

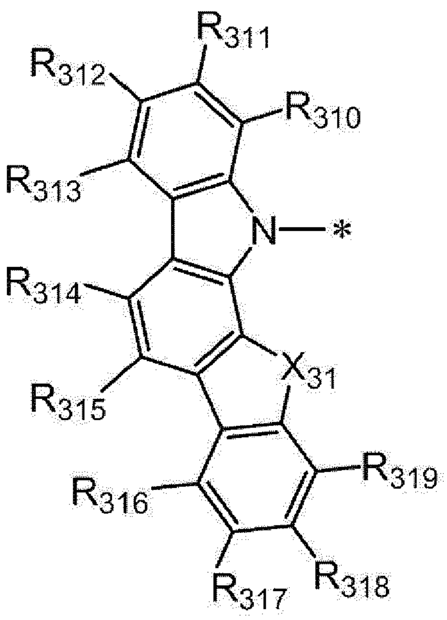

(3a)

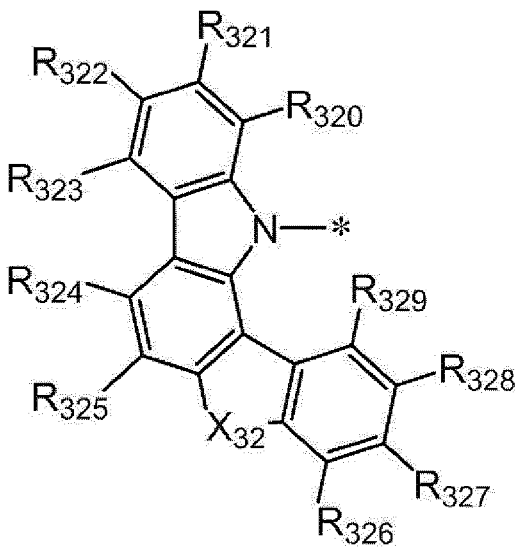

(3b)

[Formula 3]

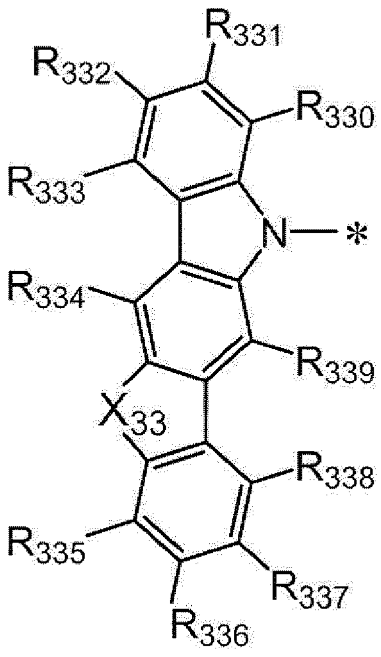

(3c)

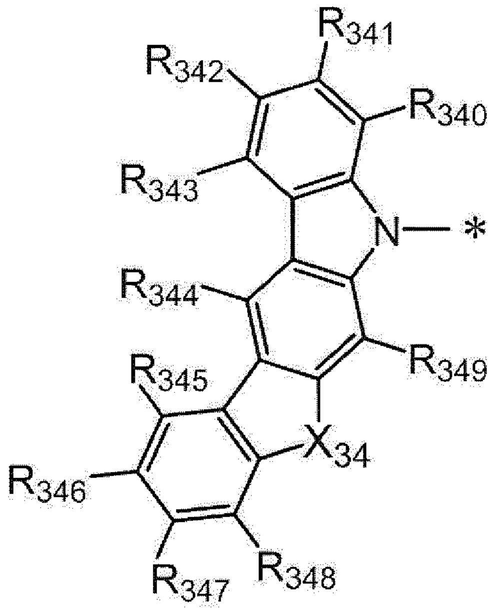

(3d)

[Formula 4]

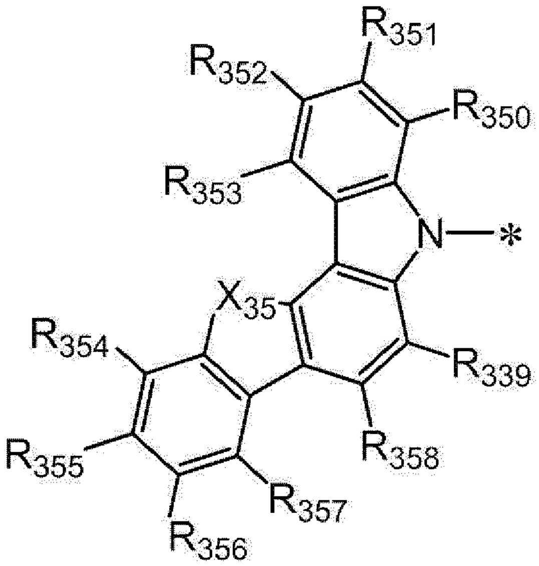

(3e)

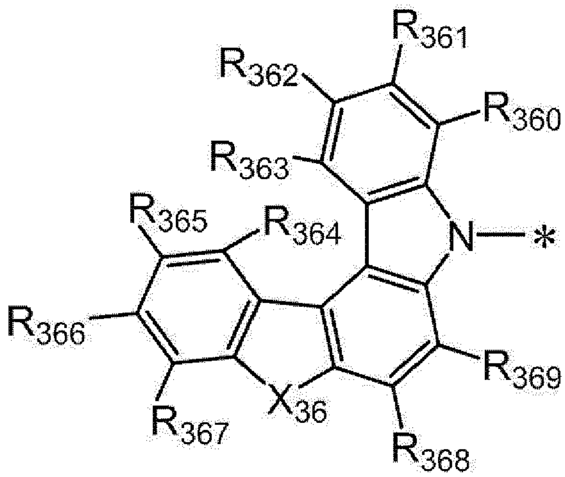

(3f)

where, in the formulae (3a), (3b), (3c), (3d), (3e) and (3f):

$X_{31}$, $X_{32}$, $X_{33}$, $X_{34}$, $X_{35}$ and $X_{36}$ are each independently an oxygen atom, a sulfur atom or $SiR_{372}R_{373}$;
$R_{372}$ and $R_{373}$ are each independently a hydrogen atom or a substituent;
$R_{310}$ to $R_{319}$ are each independently a hydrogen atom or a substituent, or at least one pair of a pair of $R_{310}$ and $R_{311}$, a pair of $R_{311}$ and $R_{312}$, a pair of $R_{312}$ and $R_{313}$, a pair of $R_{314}$ and $R_{315}$, a pair of $R_{316}$ and $R_{317}$, a pair of $R_{317}$ and $R_{318}$, or a pair of $R_{318}$ and $R_{319}$ are mutually bonded to form a ring;

$R_{320}$ to $R_{329}$ are each independently a hydrogen atom or a substituent, or at least one pair of a pair of $R_{320}$ and $R_{321}$, a pair of $R_{321}$ and $R_{322}$, a pair of $R_{322}$ and $R_{323}$, a pair of $R_{324}$ and $R_{325}$, a pair of $R_{326}$ and $R_{327}$, a pair of $R_{327}$ and $R_{328}$, or a pair of $R_{328}$ and $R_{329}$ are mutually bonded to form a ring;
$R_{330}$ to $R_{339}$ are each independently a hydrogen atom or a substituent, or at least one pair of a pair of $R_{330}$ and $R_{331}$, a pair of $R_{331}$ and $R_{332}$, a pair of $R_{332}$ and $R_{333}$, a pair of $R_{335}$ and $R_{336}$, a pair of $R_{336}$ and $R_{337}$, or a pair of $R_{337}$ and $R_{338}$ are mutually bonded to form a ring;
$R_{340}$ to $R_{349}$ are each independently a hydrogen atom or a substituent, or at least one pair of a pair of $R_{340}$ and $R_{341}$, a pair of $R_{341}$ and $R_{342}$, a pair of $R_{342}$ and $R_{343}$, a pair of $R_{345}$ and $R_{346}$, a pair of $R_{346}$ and $R_{347}$, or a pair of $R_{347}$ and $R_{348}$ are mutually bonded to form a ring;
$R_{350}$ to $R_{359}$ are each independently a hydrogen atom or a substituent, or at least one pair of a pair of $R_{350}$ and $R_{351}$, a pair of $R_{351}$ and $R_{352}$, a pair of $R_{352}$ and $R_{353}$, a pair of $R_{354}$ and $R_{355}$, a pair of $R_{355}$ and $R_{356}$, a pair of $R_{356}$ and $R_{357}$, or a pair of $R_{358}$ and $R_{359}$ are mutually bonded to form a ring;
$R_{360}$ to $R_{369}$ are each independently a hydrogen atom or a substituent, or at least one pair of a pair of $R_{360}$ and $R_{361}$, a pair of $R_{361}$ and $R_{362}$, a pair of $R_{362}$ and $R_{363}$, a pair of $R_{364}$ and $R_{365}$, a pair of $R_{365}$ and $R_{366}$, a pair of $R_{366}$ and $R_{367}$, or a pair of $R_{368}$ and $R_{369}$ are mutually bonded to form a ring;
$R_{372}$ and $R_{373}$ as a substituent and $R_{310}$ to $R_{319}$, $R_{320}$ to $R_{329}$, $R_{330}$ to $R_{339}$, $R_{340}$ to $R_{349}$, $R_{350}$ to $R_{359}$ and $R_{360}$ to $R_{369}$ as a substituent are each independently a halogen atom, a cyano group, a substituted or unsubstituted aryl group having 6 to 30 ring carbon atoms, a substituted or unsubstituted heterocyclic group having 5 to 30 ring atoms, a substituted or unsubstituted alkyl group having 1 to 30 carbon atoms, a substituted or unsubstituted alkyl halide group having 1 to 30 carbon atoms, a substituted or unsubstituted alkenyl group having 2 to 30 carbon atoms, a substituted or unsubstituted alkynyl group having 2 to 30 carbon atoms, a substituted or unsubstituted alkylsilyl group having 3 to 30 carbon atoms, a substituted or unsubstituted arylsilyl group having 6 to 60 ring carbon atoms, a substituted or unsubstituted arylphosphoryl group having 6 to 60 ring carbon atoms, a hydroxy group, a substituted or unsubstituted alkoxy group having 1 to 30 carbon atoms, a substituted or unsubstituted aryloxy group having 6 to 30 ring carbon atoms, an amino group, a substituted or unsubstituted alkylamino group having 2 to 30 carbon atoms, a substituted or unsubstituted arylamino group having 6 to 60 ring carbon atoms, a thiol group, a substituted or unsubstituted alkylthio group having 1 to 30 carbon atoms, or a substituted or unsubstituted arylthio group having 6 to 30 ring carbon atoms; and
* represents a bonding position to $L_{30}$, and
where, in the formula (3):

$L_{30}$ is a substituted or unsubstituted arylene group having 6 to 22 ring carbon atoms, a group formed by bonding two substituted or unsubstituted arylene groups having 6 to 22 ring carbon atoms, or a group formed by bonding three substituted or unsubstituted arylene groups having 6 to 22 ring carbon atoms;
$R_{31}$ to $R_{38}$ are each independently a hydrogen atom or a substituent, or at least one pair of a pair of $R_{31}$ and $R_{32}$, a pair of $R_{32}$ and $R_{33}$, a pair of $R_{33}$ and $R_{34}$, a pair of $R_{35}$ and $R_{36}$, a pair of $R_{36}$ and $R_{37}$, or a pair of $R_{37}$ and $R_{38}$ are mutually bonded to form a ring; and
a substituent for substituting $L_{30}$, and $R_{31}$ to $R_{38}$ as a substituent are each independently a halogen atom, a cyano group, a substituted or unsubstituted aryl group having 6 to 30 ring carbon atoms, a substituted or unsubstituted heterocyclic group having 5 to 30 ring atoms, a substituted or unsubstituted alkyl group having 1 to 30 carbon atoms, a substituted or unsubstituted alkyl halide group having 1 to 30 carbon atoms, a substituted or unsubstituted alkenyl group having 2 to 30 carbon atoms, a substituted or unsubstituted alkynyl group having 2 to 30 carbon atoms, a substituted or unsubstituted alkylsilyl group having 3 to 30 carbon atoms, a substituted or unsubstituted arylsilyl group having 6 to 60 ring carbon atoms, a substituted or unsubstituted aryl phosphoryl group having 6 to 60 ring carbon atoms, a hydroxy group, a substituted or unsubstituted alkoxy group having 1 to 30 carbon atoms, a substituted or unsubstituted aryloxy group having 6 to 30 ring carbon atoms, an amino group, a substituted or unsubstituted alkylamino group having 2 to 30 carbon atoms, a substituted or unsubstituted arylamino group having 6 to 60 ring carbon atoms, a thiol group, a substituted or unsubstituted alkylthio group having 1 to 30 carbon atoms, or a substituted or unsubstituted arylthio group having 6 to 30 ring carbon atoms.

**2.** The organic electroluminescence device according to claim 1, wherein

the emitting layer further comprises a fluorescent compound M1, and
the singlet energy $S_1(M2)$ of the compound M2 and a singlet energy $S_1(M1)$ of the compound M1 satisfy a

relationship of a numerical formula (Numerical Formula 2) below,

$$S_1(M2) > S_1(M1)\ldots\text{(Numerical Formula 2)}.$$

**3.** The organic electroluminescence device according to claim 1 or 2, wherein

in the compound M3, a pair of $R_{31}$ and $R_{32}$, a pair of $R_{32}$ and $R_{33}$, a pair of $R_{33}$ and $R_{34}$, a pair of $R_{35}$ and $R_{36}$, a pair of $R_{36}$ and $R_{37}$, and a pair of $R_{37}$ and $R_{38}$ are not mutually bonded, or
the compound M3 is represented by a formula (31), (32), (33), (34), (35) or (36) below,

[Formula 5]

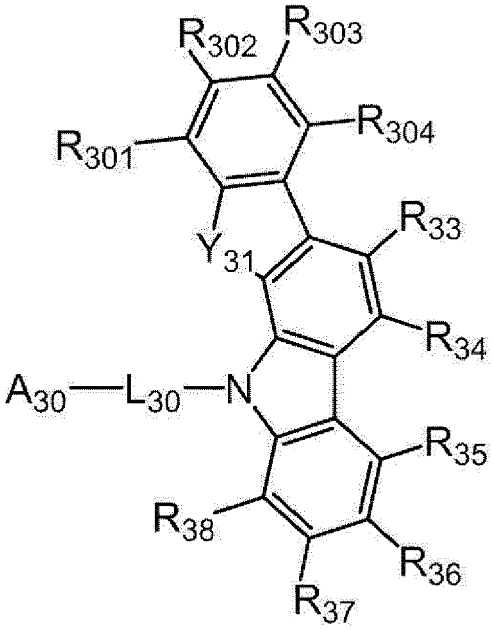

(31)

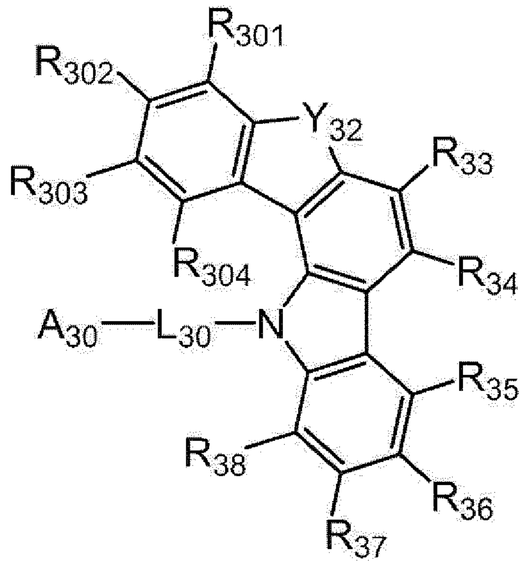

(32)

[Formula 6]

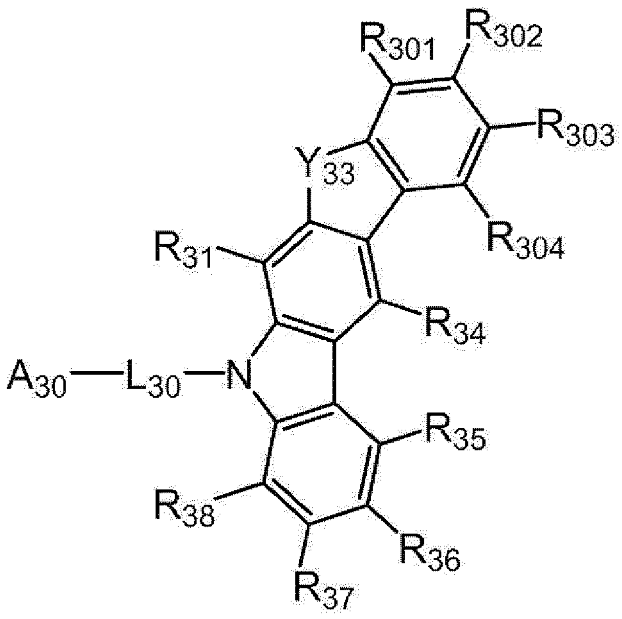

(33)

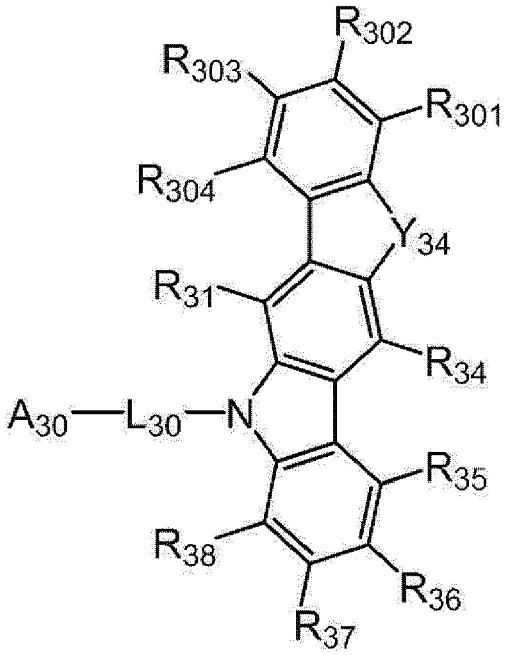

(34)

[Formula 7]

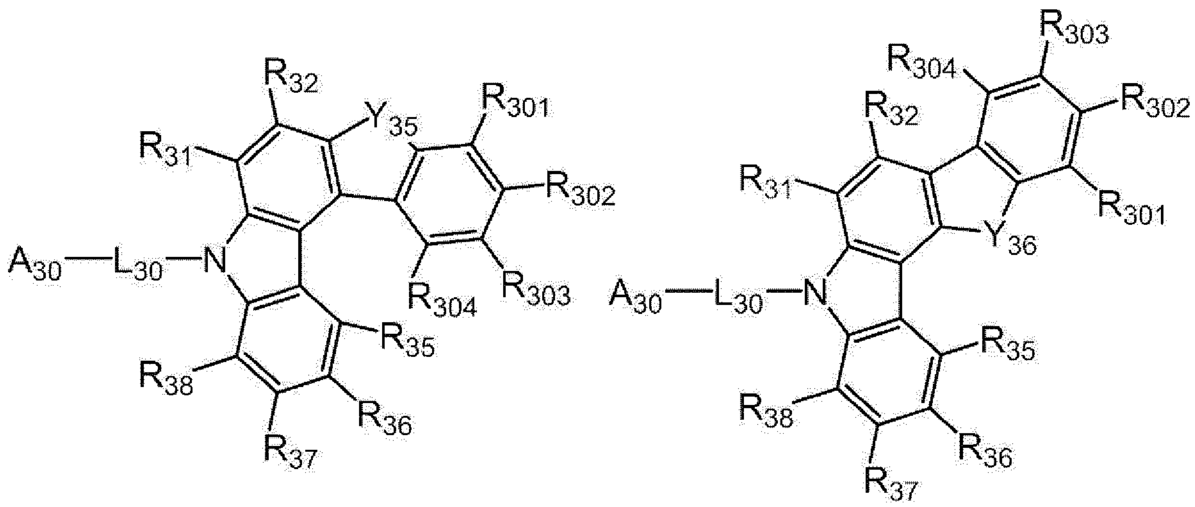

(35) (36)

where, in the formulae (31) to (36):

$Y_{31}$, $Y_{32}$, $Y_{33}$, $Y_{34}$, $Y_{35}$ and $Y_{36}$ are each independently an oxygen atom, a sulfur atom, $NR_{374}$ or $SiR_{375}R_{376}$; $R_{374}$, $R_{375}$ and $R_{376}$ are each independently a hydrogen atom or a substituent;
$A_{30}$, $L_{30}$ and $R_{31}$ to $R_{38}$ respectively represent the same as $A_{30}$, $L_{30}$ and $R_{31}$ to $R_{38}$ in the formula (3);
$R_{301}$ to $R_{304}$ are each independently a hydrogen atom or a substituent, or at least one pair of a pair of $R_{301}$ and $R_{302}$, a pair of $R_{302}$ and $R_{303}$, or a pair of $R_{303}$ and $R_{304}$ are mutually bonded to form a ring; and $R_{301}$ to $R_{304}$ as a substituent and $R_{374}$, $R_{375}$ and $R_{376}$ as a substituent are each independently a halogen atom, a cyano group, a substituted or unsubstituted aryl group having 6 to 30 ring carbon atoms, a substituted

or unsubstituted heterocyclic group having 5 to 30 ring atoms, a substituted or unsubstituted alkyl group having 1 to 30 carbon atoms, a substituted or unsubstituted alkyl halide group having 1 to 30 carbon atoms, a substituted or unsubstituted alkenyl group having 2 to 30 carbon atoms, a substituted or unsubstituted alkynyl group having 2 to 30 carbon atoms, a substituted or unsubstituted alkylsilyl group having 3 to 30 carbon atoms, a substituted or unsubstituted arylsilyl group having 6 to 60 ring carbon atoms, a substituted or unsubstituted arylphosphoryl group having 6 to 60 ring carbon atoms, a hydroxy group, a substituted or unsubstituted alkoxy group having 1 to 30 carbon atoms, a substituted or unsubstituted aryloxy group having 6 to 30 ring carbon atoms, an amino group, a substituted or unsubstituted alkylamino group having 2 to 30 carbon atoms, a substituted or unsubstituted arylamino group having 6 to 60 ring carbon atoms, a thiol group, a substituted or unsubstituted alkylthio group having 1 to 30 carbon atoms, or a substituted or unsubstituted arylthio group having 6 to 30 ring carbon atoms.

**4.** The organic electroluminescence device according to claim 3, wherein $Y_{31}$, $Y_{32}$, $Y_{33}$, $Y_{34}$, $Y_{35}$ and $Y_{36}$ are each independently an oxygen atom or a sulfur atom.

**5.** The organic electroluminescence device according to claim 3 or 4, wherein $R_{31}$ to $R_{38}$ are each independently a hydrogen atom, a substituted or unsubstituted aryl group having 6 to 30 ring carbon atoms, a substituted or unsubstituted heterocyclic group having 5 to 30 ring atoms, or a substituted or unsubstituted alkyl group having 1 to 30 carbon atoms.

**6.** The organic electroluminescence device according to claim 1 or 2, wherein at least one pair of a pair of $R_{31}$ and $R_{32}$, a pair of $R_{32}$ and $R_{33}$, a pair of $R_{33}$ and $R_{34}$, a pair of $R_{35}$ and $R_{36}$, a pair of $R_{36}$ and $R_{37}$, or a pair of $R_{37}$ and $R_{38}$ are mutually bonded to form a ring.

**7.** The organic electroluminescence device according to any one of claims 1 to 6, wherein $L_{30}$ is a substituted or unsubstituted phenylene group, a substituted or unsubstituted biphenylene group, or a substituted or unsubstituted terphenylene group.

**8.** The organic electroluminescence device according to any one of claims 1 to 7, wherein $X_{31}$, $X_{32}$, $X_{33}$, $X_{34}$, $X_{35}$ and $X_{36}$ are each independently an oxygen atom or a sulfur atom.

**9.** The organic electroluminescence device according to any one of claims 1 to 8, wherein

a pair of $R_{310}$ and $R_{311}$, a pair of $R_{311}$ and $R_{312}$, a pair of $R_{312}$ and $R_{313}$, a pair of $R_{314}$ and $R_{315}$, a pair of $R_{316}$ and $R_{317}$, a pair of $R_{317}$ and $R_{318}$, and a pair of $R_{318}$ and $R_{319}$ are not mutually bonded,
a pair of $R_{320}$ and $R_{321}$, a pair of $R_{321}$ and $R_{322}$, a pair of $R_{322}$ and $R_{323}$, a pair of $R_{324}$ and $R_{325}$, a pair of $R_{326}$ and $R_{327}$, a pair of $R_{327}$ and $R_{328}$, and a pair of $R_{328}$ and $R_{329}$ are not mutually bonded,
a pair of $R_{330}$ and $R_{331}$, a pair of $R_{331}$ and $R_{332}$, a pair of $R_{332}$ and $R_{333}$, a pair of $R_{335}$ and $R_{336}$, a pair of $R_{336}$ and $R_{337}$, and a pair of $R_{337}$ and $R_{338}$ are not mutually bonded,
a pair of $R_{340}$ and $R_{341}$, a pair of $R_{341}$ and $R_{342}$, a pair of $R_{342}$ and $R_{343}$, a pair of $R_{345}$ and $R_{346}$, a pair of $R_{346}$ and $R_{347}$, and a pair of $R_{347}$ and $R_{348}$ are not mutually bonded,
a pair of $R_{350}$ and $R_{351}$, a pair of $R_{351}$ and $R_{352}$, a pair of $R_{352}$ and $R_{353}$, a pair of $R_{354}$ and $R_{355}$, a pair of $R_{355}$ and $R_{356}$, a pair of $R_{356}$ and $R_{357}$, and a pair of $R_{358}$ and $R_{359}$ are not mutually bonded, and
a pair of $R_{360}$ and $R_{361}$, a pair of $R_{361}$ and $R_{362}$, a pair of $R_{362}$ and $R_{363}$, a pair of $R_{364}$ and $R_{365}$, a pair of $R_{365}$ and $R_{366}$, a pair of $R_{366}$ and $R_{367}$, and a pair of $R_{368}$ and $R_{369}$ are not mutually bonded.

**10.** The organic electroluminescence device according to any one of claims 1 to 9, wherein $A_{30}$ is the group represented by the formula (3a) or (3d).

**11.** A compound represented by a formula (301) below,

[Formula 8]

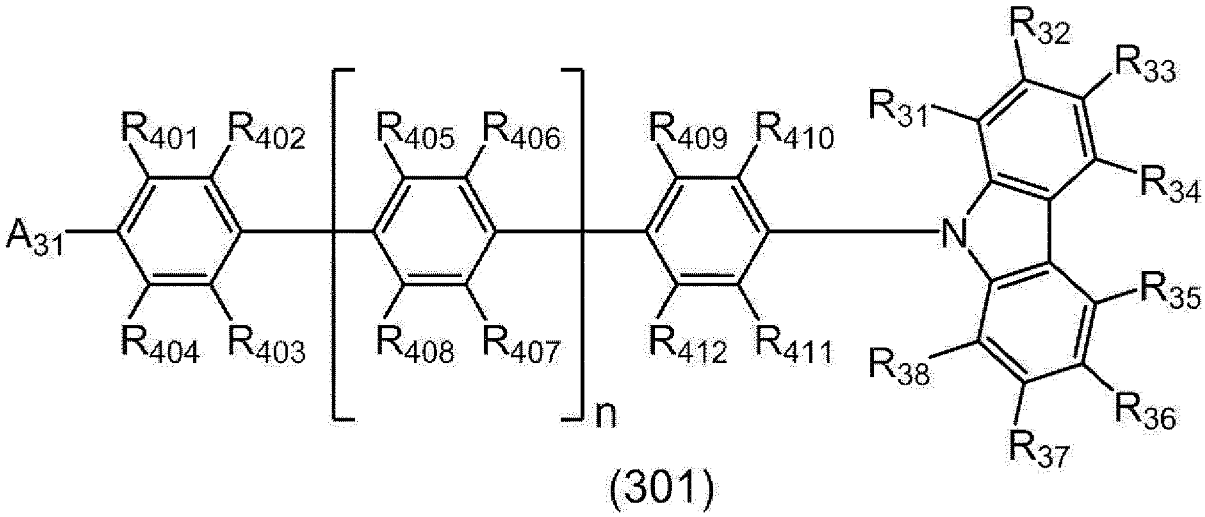

(301)

where, in the formula (301): $A_{31}$ is a group represented by a formula (31a), (31b), (31c), (31d), (31e) or (31f) below,

[Formula 9]

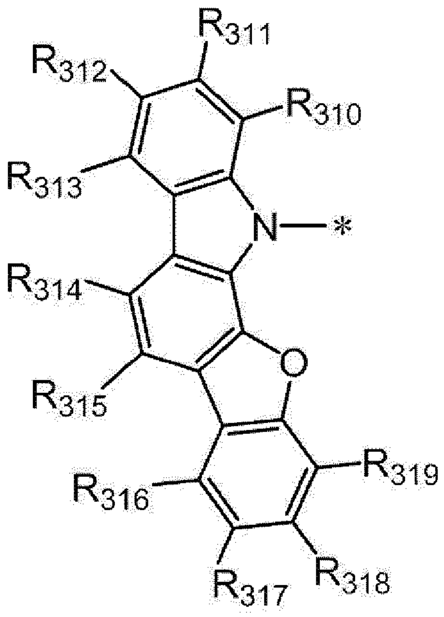

(31a)

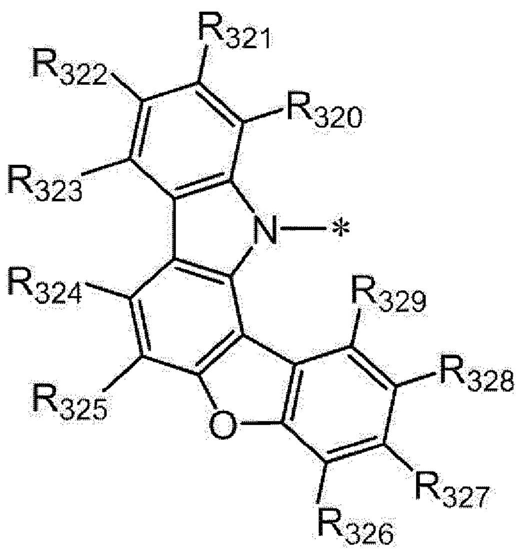

(31b)

[Formula 10]

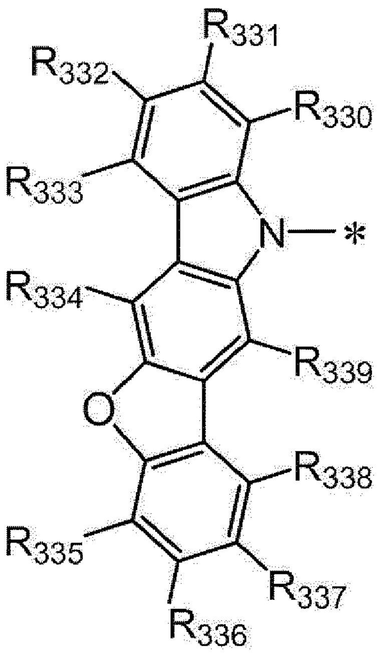

(31c)

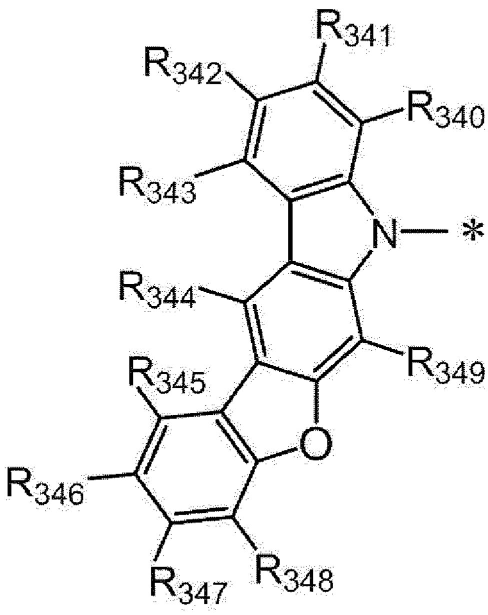

(31d)

[Formula 11]

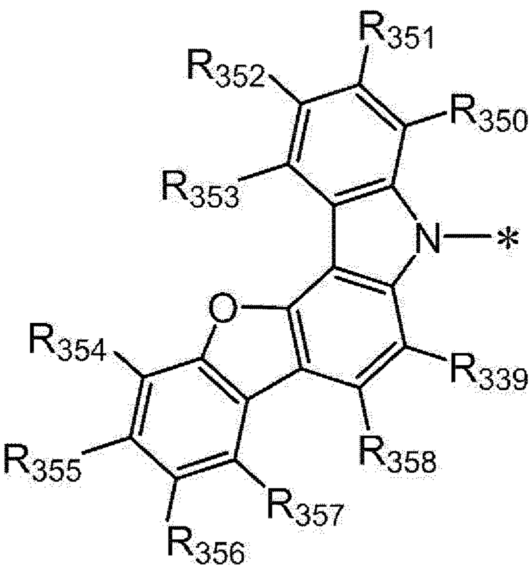

(31e)

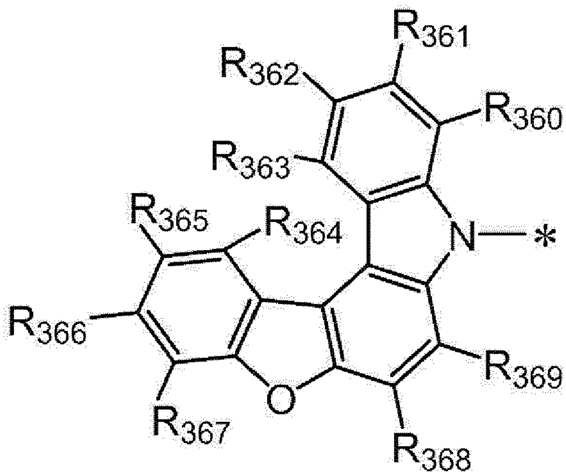

(31f)

where, in the formulae (31a), (31b), (31c), (31d), (31e) and (31f):

$R_{310}$ to $R_{319}$ are each independently a hydrogen atom or a substituent;
$R_{320}$ to $R_{329}$ are each independently a hydrogen atom or a substituent;
$R_{330}$ to $R_{339}$ are each independently a hydrogen atom or a substituent;
$R_{340}$ to $R_{349}$ are each independently a hydrogen atom or a substituent;
$R_{350}$ to $R_{359}$ are each independently a hydrogen atom or a substituent;

$R_{360}$ to $R_{369}$ are each independently a hydrogen atom or a substituent;
$R_{310}$ to $R_{319}$, $R_{320}$ to $R_{329}$, $R_{330}$ to $R_{339}$, $R_{340}$ to $R_{349}$, $R_{350}$ to $R_{359}$ and $R_{360}$ to $R_{369}$ as a substituent are each independently a halogen atom, a substituted or unsubstituted aryl group having 6 to 30 ring carbon atoms, a substituted or unsubstituted heterocyclic group having 5 to 30 ring atoms, a substituted or unsubstituted alkyl group having 1 to 30 carbon atoms, a substituted or unsubstituted alkyl halide group having 1 to 30 carbon atoms, a substituted or unsubstituted alkenyl group having 2 to 30 carbon atoms, a substituted or unsubstituted alkynyl group having 2 to 30 carbon atoms, a substituted or unsubstituted alkylsilyl group having 3 to 30 carbon atoms, a substituted or unsubstituted arylsilyl group having 6 to 60 ring carbon atoms, a substituted or unsubstituted arylphosphoryl group having 6 to 60 ring carbon atoms, a hydroxy group, a substituted or unsubstituted alkoxy group having 1 to 30 carbon atoms, a substituted or unsubstituted aryloxy group having 6 to 30 ring carbon atoms, an amino group, a substituted or unsubstituted alkylamino group having 2 to 30 carbon atoms, a substituted or unsubstituted arylamino group having 6 to 60 ring carbon atoms, a thiol group, a substituted or unsubstituted alkylthio group having 1 to 30 carbon atoms, or a substituted or unsubstituted arylthio group having 6 to 30 ring carbon atoms; and
* represents a bonding position to a benzene ring bonded with $R_{401}$ to $R_{404}$, and
where, in the formula (301):

$R_{31}$ to $R_{38}$ are each independently a hydrogen atom or a substituent;
$R_{401}$ to $R_{412}$ are each independently a hydrogen atom or a substituent;
$R_{31}$ to $R_{38}$ as a substituent are each independently a halogen atom, a substituted or unsubstituted aryl group having 6 to 30 ring carbon atoms, a substituted or unsubstituted heterocyclic group having 5 to 30 ring atoms, a substituted or unsubstituted alkyl group having 1 to 30 carbon atoms, a substituted or unsubstituted alkyl halide group having 1 to 30 carbon atoms, a substituted or unsubstituted alkenyl group having 2 to 30 carbon atoms, a substituted or unsubstituted alkynyl group having 2 to 30 carbon atoms, a substituted or unsubstituted alkylsilyl group having 3 to 30 carbon atoms, a substituted or unsubstituted arylsilyl group having 6 to 60 ring carbon atoms, a substituted or unsubstituted arylphosphoryl group having 6 to 60 ring carbon atoms, a hydroxy group, a substituted or unsubstituted alkoxy group having 1 to 30 carbon atoms, a substituted or unsubstituted aryloxy group having 6 to 30 ring carbon atoms, an amino group, a substituted or unsubstituted alkylamino group having 2 to 30 carbon atoms, a substituted or unsubstituted arylamino group having 6 to 60 ring carbon atoms, a thiol group, a substituted or unsubstituted alkylthio group having 1 to 30 carbon atoms, or a substituted or unsubstituted arylthio group having 6 to 30 ring carbon atoms; and
$R_{401}$ to $R_{412}$ as a substituent are each independently a halogen atom, a substituted or unsubstituted aryl group having 6 to 30 ring carbon atoms, a substituted or unsubstituted heterocyclic group having 5 to 30 ring atoms, a substituted or unsubstituted alkyl group having 1 to 30 carbon atoms, a substituted or unsubstituted alkyl halide group having 1 to 30 carbon atoms, a substituted or unsubstituted alkenyl group having 2 to 30 carbon atoms, a substituted or unsubstituted alkynyl group having 2 to 30 carbon atoms, a substituted or unsubstituted alkylsilyl group having 3 to 30 carbon atoms, a substituted or unsubstituted arylsilyl group having 6 to 60 ring carbon atoms, a substituted or unsubstituted aryl phosphoryl group having 6 to 60 ring carbon atoms, a hydroxy group, a substituted or unsubstituted alkoxy group having 1 to 30 carbon atoms, a substituted or unsubstituted aryloxy group having 6 to 30 ring carbon atoms, a thiol group, a substituted or unsubstituted alkylthio group having 1 to 30 carbon atoms, or a substituted or unsubstituted arylthio group having 6 to 30 ring carbon atoms;
n is 0, 1, 2 or 3;
when a plurality of $R_{405}$ are present, the plurality of $R_{405}$ are mutually the same or different;
when a plurality of $R_{406}$ are present, the plurality of $R_{406}$ are mutually the same or different;
when a plurality of $R_{407}$ are present, the plurality of $R_{407}$ are mutually the same or different; and
when a plurality of $R_{408}$ are present, the plurality of $R_{408}$ are mutually the same or different.

**12.** The compound according to claim 11, wherein the compound is represented by a formula (302) below,

[Formula 12]

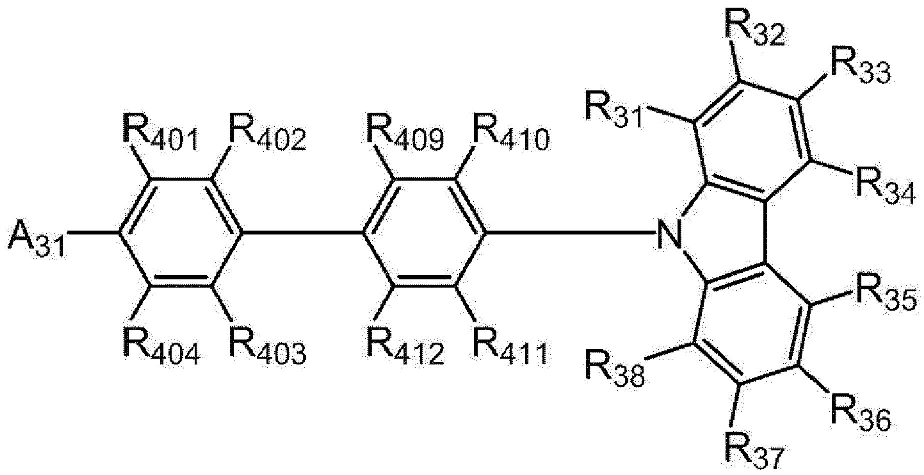

(302)

$A_{31}$, $R_{401}$ to $R_{404}$, $R_{409}$ to $R_{412}$ and $R_{31}$ to $R_{38}$ in the formula (302) respectively represent the same as $A_{31}$, $R_{401}$ to $R_{404}$, $R_{409}$ to $R_{412}$ and $R_{31}$ to $R_{38}$ in the formula (301).

**13.** A compound represented by a formula (310) below,

[Formula 13]

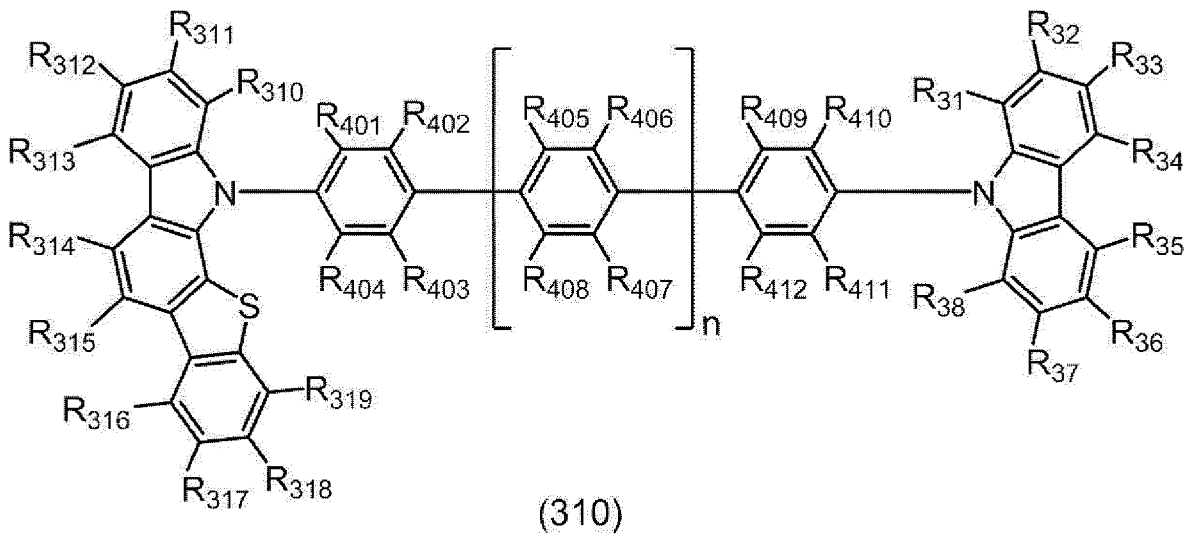

(310)

where, in the formula (310):

$R_{310}$ to $R_{319}$ are each independently a hydrogen atom or a substituent;
$R_{31}$ to $R_{38}$ are each independently a hydrogen atom or a substituent;
$R_{401}$ to $R_{412}$ are each independently a hydrogen atom or a substituent;
$R_{310}$ to $R_{319}$ as a substituent, $R_{31}$ to $R_{38}$ as a substituent and $R_{401}$ to $R_{412}$ as a substituent are each independently a halogen atom, a substituted or unsubstituted aryl group having 6 to 30 ring carbon atoms, a substituted or unsubstituted heterocyclic group having 5 to 30 ring atoms, a substituted or unsubstituted alkyl group having 1 to 30 carbon atoms, a substituted or unsubstituted alkyl halide group having 1 to 30 carbon atoms, a substituted or unsubstituted alkenyl group having 2 to 30 carbon atoms, a substituted or unsubstituted alkynyl group having 2 to 30 carbon atoms, a substituted or unsubstituted alkylsilyl group having 3 to 30 carbon atoms, a substituted or unsubstituted arylsilyl group having 6 to 60 ring carbon atoms, a substituted or unsubstituted arylphosphoryl group having 6 to 60 ring carbon atoms, a hydroxy group, a substituted or unsubstituted alkoxy group having 1 to 30 carbon atoms, a substituted or unsubstituted aryloxy group having 6 to 30 ring carbon atoms, an amino group, a substituted or unsubstituted alkylamino group having 2 to 30 carbon atoms, a substituted or unsubstituted or unsubsti-

tuted arylamino group having 6 to 60 ring carbon atoms, a thiol group, a substituted or unsubstituted alkylthio group having 1 to 30 carbon atoms, or a substituted or unsubstituted arylthio group having 6 to 30 ring carbon atoms;
n is 0, 1, 2 or 3;
when a plurality of $R_{405}$ are present, the plurality of $R_{405}$ are mutually the same or different;
when a plurality of $R_{406}$ are present, the plurality of $R_{406}$ are mutually the same or different;
when a plurality of $R_{407}$ are present, the plurality of $R_{407}$ are mutually the same or different; and
when a plurality of $R_{408}$ are present, the plurality of $R_{408}$ are mutually the same or different.

**14.** The compound according to claim 13, wherein the compound is represented by a formula (311) below,

[Formula 14]

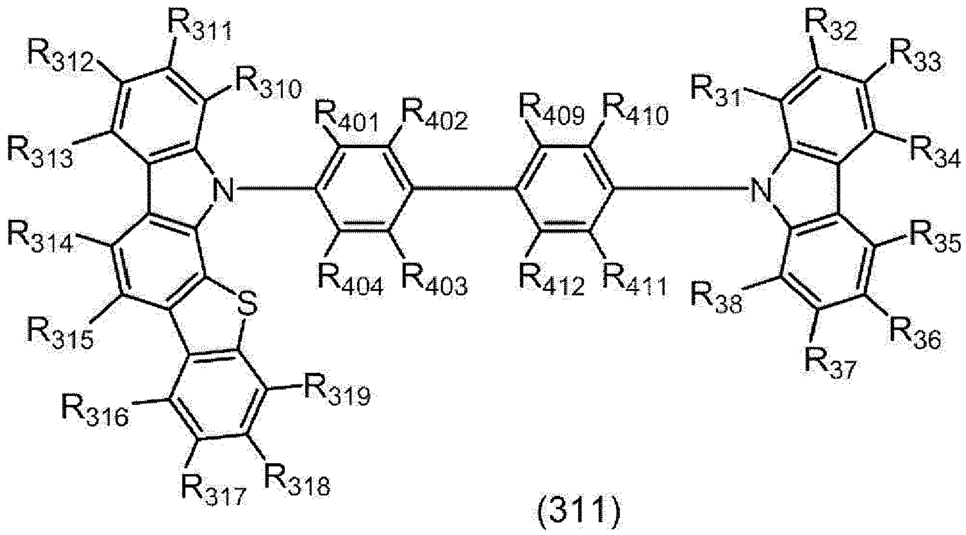

(311)

$R_{310}$ to $R_{319}$, $R_{401}$ to $R_{404}$, $R_{409}$ to $R_{412}$ and $R_{31}$ to $R_{38}$ in the formula (311) respectively represent the same as $R_{310}$ to $R_{319}$, $R_{401}$ to $R_{404}$, $R_{409}$ to $R_{412}$ and $R_{31}$ to $R_{38}$ in the formula (310).

**15.** An organic electroluminescence device comprising:

an anode;
a cathode;
an emitting layer provided between the anode and the cathode, wherein
the emitting layer comprises a delayed fluorescent compound M2 and the compound according to any one of claims 11 to 14.

## Patentansprüche

**1.** Eine organische Elektrolumineszenzvorrichtung, die folgendes umfasst:

eine Anode;
eine Kathode;
eine emittierende Schicht, die zwischen der Anode und der Kathode bereitgestellt wird, wobei die emittierende Schicht eine verzögert fluoreszierende Verbindung M2 und eine Verbindung M3, die durch die folgende Formel (3) dargestellt wird, umfasst, und
eine Singulett-Energie $S_1(M2)$ der Verbindung M2 und eine Singulett-Energie $S_1(M3)$ der Verbindung M3 die Beziehung der folgenden numerischen Formel (Numerische Formel 1) erfüllen,

$$S_1(M3) > S_1(M2) \ldots \text{(Numerische Formel 1)},$$

[Formel 1]

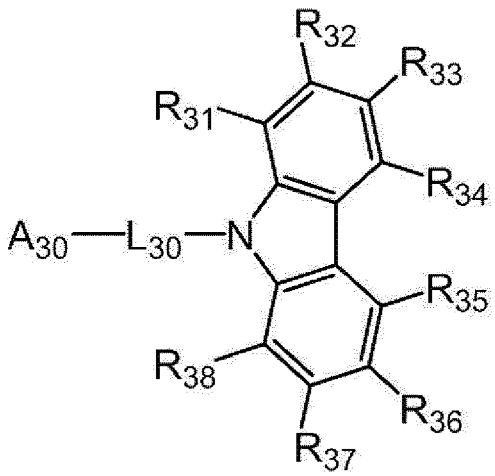

(3)

wobei in der Formel (3): $A_{30}$ eine Gruppe ist, die durch die folgenden Formeln (3a), (3b), (3c), (3d), (3e) oder (3f) dargestellt wird,

[Formel 2]

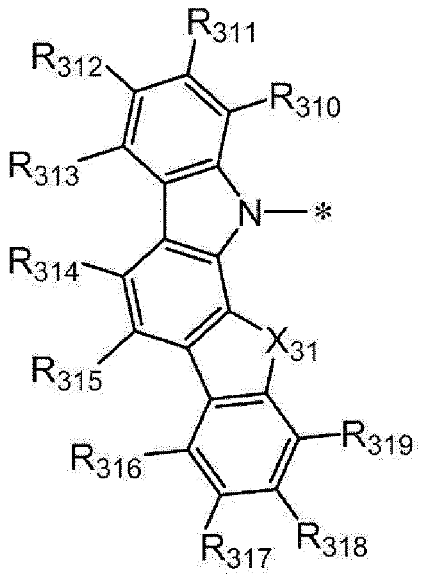

(3a)

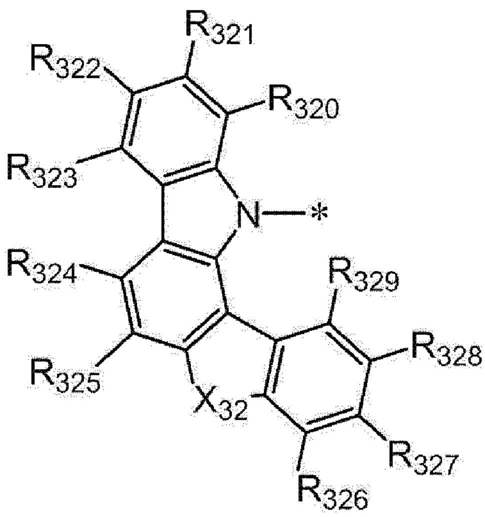

(3b)

[Formel 3]

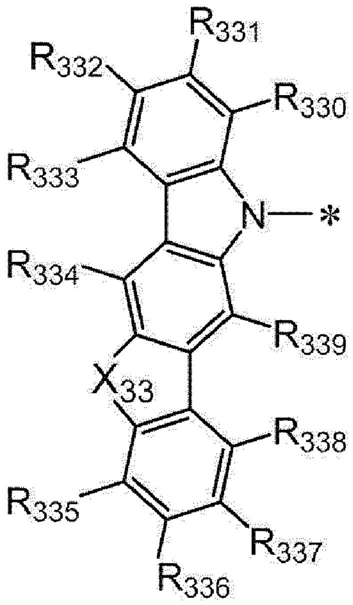

(3c)

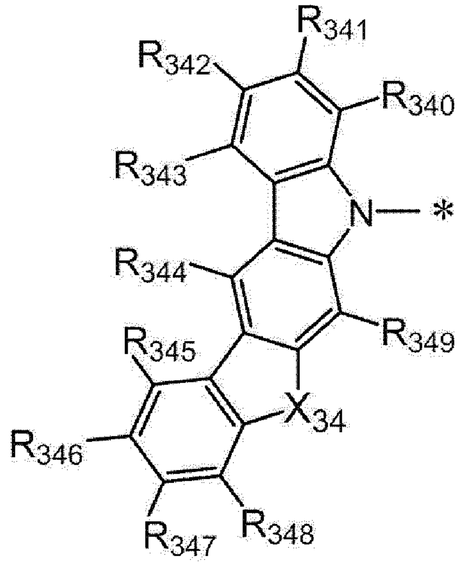

(3d)

[Formel 4]

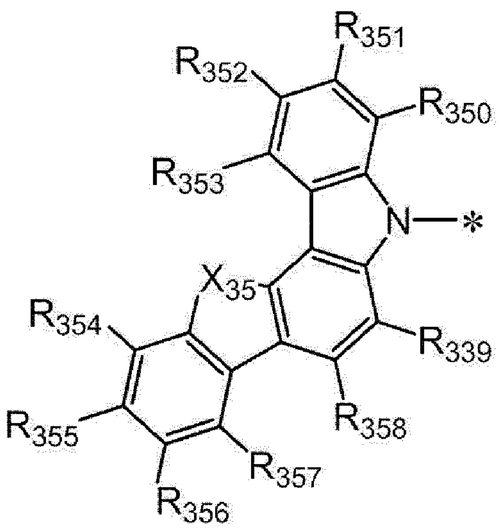

(3e)

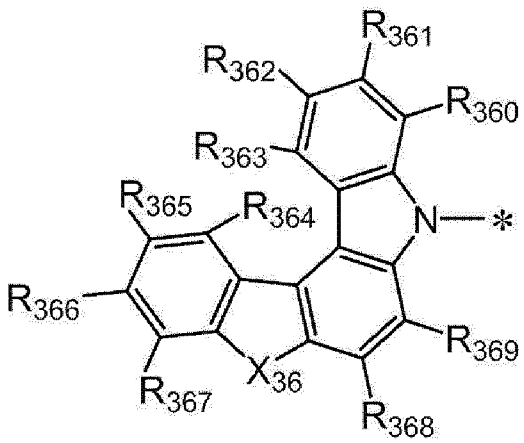

(3f)

wobei in den Formeln (3a), (3b), (3c), (3d), (3e) und (3f):

$X_{31}$, $X_{32}$, $X_{33}$, $X_{34}$, $X_{35}$ und $X_{36}$ jeweils unabhängig voneinander ein Sauerstoffatom, ein Schwefelatom oder $SiR_{372}R_{373}$ sind;

$R_{372}$ und $R_{373}$ jeweils unabhängig voneinander ein Wasserstoffatom oder ein Substituent sind;

$R_{310}$ bis $R_{319}$ jeweils unabhängig voneinander ein Wasserstoffatom oder ein Substituent sind, oder mindestens ein Paar aus einem Paar aus $R_{310}$ und $R_{311}$, einem Paar aus $R_{311}$ und $R_{312}$, einem Paar aus $R_{312}$ und $R_{313}$, einem Paar aus $R_{314}$ und $R_{315}$, einem Paar aus $R_{316}$ und $R_{317}$, einem Paar aus $R_{317}$ und $R_{318}$, oder einem Paar aus $R_{318}$ und $R_{319}$ miteinander verbunden ist, um einen Ring zu bilden;

$R_{320}$ bis $R_{329}$ jeweils unabhängig voneinander ein Wasserstoffatom oder ein Substituent sind, oder mindestens ein Paar aus einem Paar aus $R_{320}$ und $R_{321}$, einem Paar aus $R_{321}$ und $R_{322}$, einem Paar aus $R_{322}$ und $R_{323}$, einem Paar aus $R_{324}$ und $R_{325}$, einem Paar aus $R_{326}$ und $R_{327}$, einem Paar aus $R_{327}$ und $R_{328}$, oder einem Paar aus $R_{328}$ und $R_{329}$ miteinander verbunden ist, um einen Ring zu bilden;

$R_{330}$ bis $R_{339}$ jeweils unabhängig voneinander ein Wasserstoffatom oder ein Substituent sind, oder mindestens ein Paar aus einem Paar aus $R_{330}$ und $R_{331}$, einem Paar aus $R_{331}$ und $R_{332}$, einem Paar aus $R_{332}$ und $R_{333}$, einem Paar aus $R_{335}$ und $R_{336}$, einem Paar aus $R_{336}$ und $R_{337}$, oder einem Paar aus $R_{337}$ und $R_{338}$ miteinander verbunden ist, um einen Ring zu bilden;

$R_{340}$ bis $R_{349}$ jeweils unabhängig voneinander ein Wasserstoffatom oder ein Substituent sind, oder mindestens ein Paar aus einem Paar aus $R_{340}$ und $R_{341}$, einem Paar aus $R_{341}$ und $R_{342}$, einem Paar aus $R_{342}$ und $R_{343}$, einem Paar aus $R_{345}$ und $R_{346}$, einem Paar aus $R_{346}$ und $R_{347}$, oder einem Paar aus $R_{347}$ und $R_{348}$ miteinander verbunden ist, um einen Ring zu bilden;

$R_{350}$ bis $R_{359}$ jeweils unabhängig voneinander ein Wasserstoffatom oder ein Substituent sind, oder mindestens ein Paar aus einem Paar aus $R_{350}$ und $R_{351}$, einem Paar aus $R_{351}$ und $R_{352}$, einem Paar aus $R_{352}$ und $R_{353}$, einem Paar aus $R_{354}$ und $R_{355}$, einem Paar aus $R_{355}$ und $R_{356}$, einem Paar aus $R_{356}$ und $R_{357}$, oder einem Paar aus $R_{358}$ und $R_{359}$ miteinander verbunden ist, um einen Ring zu bilden;

$R_{360}$ bis $R_{369}$ jeweils unabhängig voneinander ein Wasserstoffatom oder ein Substituent sind, oder mindestens ein Paar aus einem Paar aus $R_{360}$ und $R_{361}$, einem Paar aus $R_{361}$ und $R_{362}$, einem Paar aus $R_{362}$ und $R_{363}$, einem Paar aus $R_{364}$ und $R_{365}$, einem Paar aus $R_{365}$ und $R_{366}$, einem Paar aus $R_{366}$ und $R_{367}$ oder einem Paar aus $R_{368}$ und $R_{369}$ miteinander verbunden ist, um einen Ring zu bilden;

$R_{372}$ und $R_{373}$ als Substituenten und $R_{310}$ bis $R_{319}$, $R_{320}$ bis $R_{329}$, $R_{330}$ bis $R_{339}$, $R_{340}$ bis $R_{349}$, $R_{350}$ bis $R_{359}$ und $R_{360}$ bis $R_{369}$ als Substituenten jeweils unabhängig voneinander ein Halogenatom, eine Cyanogruppe, eine substituierte oder unsubstituierte Arylgruppe mit 6 bis 30 Ringkohlenstoffatomen, eine substituierte oder unsubstituierte heterocyclische Gruppe mit 5 bis 30 Ringatomen, eine substituierte oder unsubstituierte Alkylgruppe mit 1 bis 30 Kohlenstoffatomen, eine substituierte oder unsubstituierte Alkylhalogenidgruppe mit 1 bis 30 Kohlenstoffatomen, eine substituierte oder unsubstituierte Alkenylgruppe mit 2 bis 30 Kohlenstoffatomen, eine substituierte oder unsubstituierte Alkinylgruppe mit 2 bis 30 Kohlenstoffatomen, eine substituierte oder unsubstituierte Alkylsilylgruppe mit 3 bis 30 Kohlenstoffatomen, eine substituierte oder unsubstituierte Arylsilylgruppe mit 6 bis 60 Ringkohlenstoffatomen, eine substituierte oder unsubstituierte Arylphosphorylgruppe mit 6 bis 60 Ringkohlenstoffatomen, eine Hydroxygruppe, eine substituierte oder unsubstituierte Alkoxygruppe mit 1 bis 30 Kohlenstoffatomen, eine substituierte oder unsubstituierte Aryloxygruppe mit 6 bis 30 Ringkohlenstoffatomen, eine Aminogruppe, eine substituierte oder unsubstituierte Alkylaminogruppe mit 2 bis 30 Kohlenstoffatomen, eine substituierte oder unsubstituierte Arylaminogruppe mit 6 bis 60 Ringkohlenstoffatomen, eine Thiolgruppe, eine substituierte oder unsubstituierte Alkylthiogruppe mit 1 bis 30 Kohlenstoffatomen oder eine substituierte oder unsubstituierte Arylthiogruppe mit 6 bis 30 Ringkohlenstoffatomen sind;

und

* eine Bindungsposition zu $L_{30}$ darstellt, und

wobei in der Formel (3):

$L_{30}$ eine substituierte oder unsubstituierte Arylengruppe mit 6 bis 22 Ringkohlenstoffatomen, eine durch die Bindung von zwei substituierten oder unsubstituierten Arylengruppen mit 6 bis 22 Ringkohlenstoffatomen gebildete Gruppe oder eine durch die Bindung von drei substituierten oder unsubstituierten Arylengruppen mit 6 bis 22 Ringkohlenstoffatomen gebildete Gruppe ist;

$R_{31}$ bis $R_{38}$ jeweils unabhängig voneinander ein Wasserstoffatom oder ein Substituent sind, oder mindestens ein Paar aus einem Paar aus $R_{31}$ und $R_{32}$, einem Paar aus $R_{32}$ und $R_{33}$, einem Paar aus $R_{33}$ und $R_{34}$, einem Paar aus $R_{35}$ und $R_{36}$, einem Paar aus $R_{36}$ und $R_{37}$, oder einem Paar aus $R_{37}$ und $R_{38}$ miteinander verbunden ist, um einen Ring zu bilden; und

ein Substituent zur Substitution von $L_{30}$, und $R_{31}$ bis $R_{38}$ als Substituenten jeweils unabhängig voneinander ein Halogenatom, eine Cyanogruppe, eine substituierte oder unsubstituierte Arylgruppe mit 6 bis 30 Ringkohlenstoffatomen, eine substituierte oder unsubstituierte heterocyclische Gruppe mit 5 bis 30 Ringatomen, eine substituierte oder unsubstituierte Alkylgruppe mit 1 bis 30 Kohlenstoffatomen, eine substituierte oder unsubstituierte Alkylhalogenidgruppe mit 1 bis 30 Kohlenstoffatomen, eine substituierte oder unsubstituierte Alkenylgruppe mit 2 bis 30 Kohlenstoffatomen, eine substituierte oder unsubstituierte Alkinylgruppe mit 2 bis 30 Kohlenstoffatomen, eine substituierte oder unsubstituierte Alkylsilylgruppe mit 3 bis 30 Kohlenstoffatomen, eine substituierte oder unsubstituierte Arylsilylgruppe mit 6 bis 60 Ringkohlenstoffatomen, eine substituierte oder unsubstituierte Arylphosphorylgruppe mit 6 bis 60 Ringkohlenstoffatomen, eine Hydroxygruppe, eine substituierte oder unsubstituierte Alkoxygruppe mit 1 bis 30 Kohlenstoffatomen, eine substituierte oder unsubstituierte Aryloxygruppe mit 6 bis

30 Ringkohlenstoffatomen, eine Aminogruppe, eine substituierte oder unsubstituierte Alkylaminogruppe mit 2 bis 30 Kohlenstoffatomen, eine substituierte oder unsubstituierte Arylaminogruppe mit 6 bis 60 Ringkohlenstoffatomen, eine Thiolgruppe, eine substituierte oder unsubstituierte Alkylthiogruppe mit 1 bis 30 Kohlenstoffatomen oder eine substituierte oder unsubstituierte Arylthiogruppe mit 6 bis 30 Ringkohlenstoffatomen sind.

**2.** Die organische Elektrolumineszenzvorrichtung gemäß Anspruch 1, wobei

die emittierende Schicht ferner eine fluoreszierende Verbindung M1 umfasst, und
die Singulett-Energie $S_1(M2)$ der Verbindung M2 und die Singulett-Energie $S_1(M1)$ der Verbindung M1 die Beziehung der folgenden numerischen Formel (Numerische Formel 2) erfüllen,

$$S_1(M2) > S_1(M1)\ldots\text{(Numerische Formel 2)}.$$

**3.** Die organische Elektrolumineszenzvorrichtung gemäß Anspruch 1 oder 2, wobei
in der Verbindung M3 ein Paar aus $R_{31}$ und $R_{32}$, ein Paar aus $R_{32}$ und $R_{33}$, ein Paar aus $R_{33}$ und $R_{34}$, ein Paar aus $R_{35}$ und $R_{36}$, ein Paar aus $R_{36}$ und $R_{37}$, und ein Paar aus $R_{37}$ und $R_{38}$ nicht aneinander gebunden ist, oder

die Verbindung M3 durch eine der nachstehenden Formeln (31), (32), (33), (34), (35) oder (36) dargestellt wird,

[Formel 5]

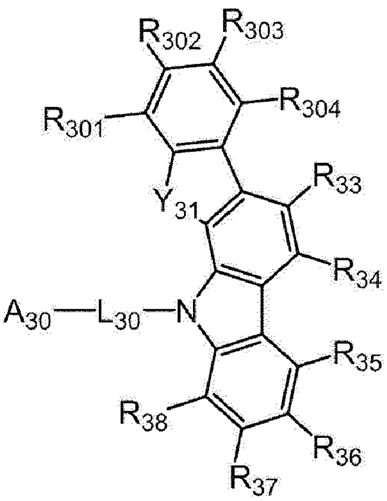

(31)

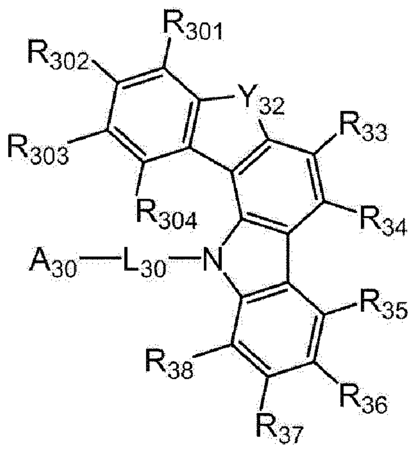

(32)

[Formel 6]

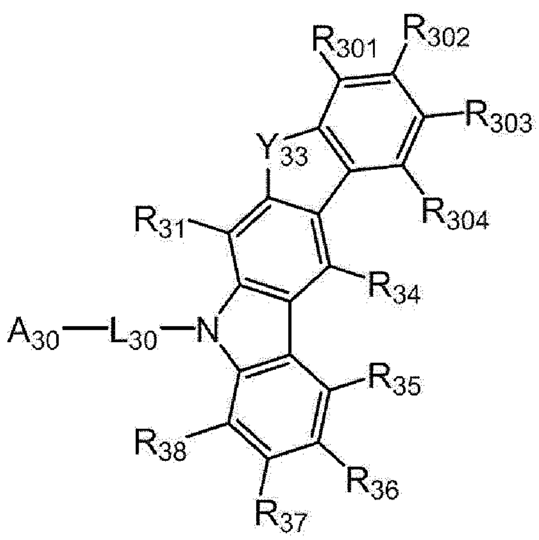

(33)

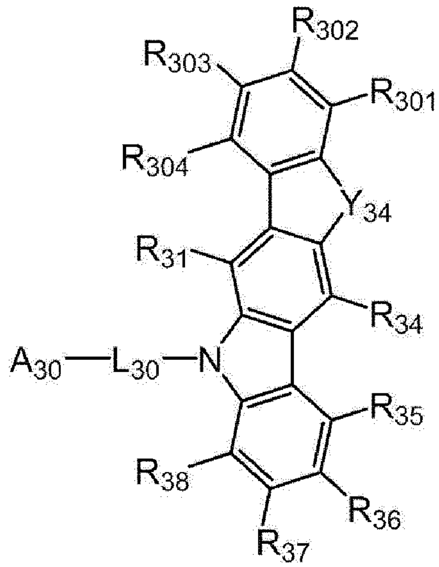

(34)

[Formel 7]

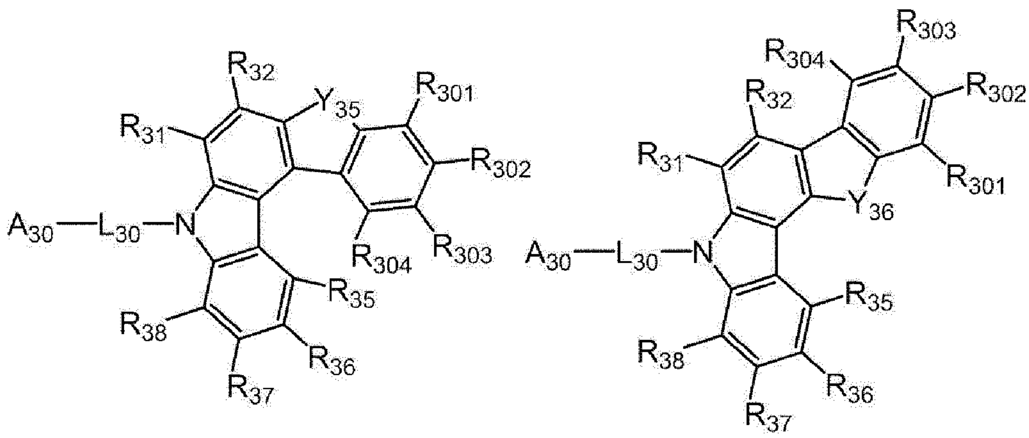

(35)

(36)

wobei in den Formeln (31) bis (36):

$Y_{31}$, $Y_{32}$, $Y_{33}$, $Y_{34}$, $Y_{35}$ und $Y_{36}$ jeweils unabhängig ein Sauerstoffatom, ein Schwefelatom, $NR_{374}$ oder $SiR_{375}R_{376}$ sind;

$R_{374}$, $R_{375}$ und $R_{376}$ jeweils unabhängig voneinander ein Wasserstoffatom oder ein Substituent sind;

$A_{30}$, $L_{30}$ und $R_{31}$ bis $R_{38}$ jeweils dasselbe wie $A_{30}$, $L_{30}$ und $R_{31}$ bis $R_{38}$ in der Formel (3) darstellen;

$R_{301}$ bis $R_{304}$ jeweils unabhängig voneinander ein Wasserstoffatom oder ein Substituent sind, oder mindestens ein Paar aus einem Paar aus $R_{301}$ und $R_{302}$, einem Paar aus $R_{302}$ und $R_{303}$, oder einem Paar aus $R_{303}$ und $R_{304}$ aneinander gebunden ist, um einen Ring zu bilden; und

$R_{301}$ bis $R_{304}$ als Substituent und $R_{374}$, $R_{375}$ und $R_{376}$ als Substituent jeweils unabhängig voneinander ein Halogenatom, eine Cyanogruppe, eine substituierte oder unsubstituierte Arylgruppe mit 6 bis 30 Ringkohlenstoffatomen, eine substituierte oder unsubstituierte heterocyclische Gruppe mit 5 bis 30 Ringatomen, eine substituierte oder unsubstituierte Alkylgruppe mit 1 bis 30 Kohlenstoffatomen, eine substituierte oder unsubstituierte Alkylhalogenidgruppe mit 1 bis 30 Kohlenstoffatomen, eine substituierte oder unsubstituierte

Alkenylgruppe mit 2 bis 30 Kohlenstoffatomen, eine substituierte oder unsubstituierte Alkinylgruppe mit 2 bis 30 Kohlenstoffatomen, eine substituierte oder unsubstituierte Alkylsilylgruppe mit 3 bis 30 Kohlenstoffatomen, eine substituierte oder unsubstituierte Arylsilylgruppe mit 6 bis 60 Ringkohlenstoffatomen, eine substituierte oder unsubstituierte Arylphosphorylgruppe mit 6 bis 60 Ringkohlenstoffatomen, eine Hydroxygruppe, eine substituierte oder unsubstituierte Alkoxygruppe mit 1 bis 30 Kohlenstoffatomen, eine substituierte oder unsubstituierte Aryloxygruppe mit 6 bis 30 Ringkohlenstoffatomen, eine Aminogruppe, eine substituierte oder unsubstituierte Alkylaminogruppe mit 2 bis 30 Kohlenstoffatomen, eine substituierte oder unsubstituierte Arylaminogruppe mit 6 bis 60 Ringkohlenstoffatomen, eine Thiolgruppe, eine substituierte oder unsubstituierte Alkylthiogruppe mit 1 bis 30 Kohlenstoffatomen oder eine substituierte oder unsubstituierte Arylthiogruppe mit 6 bis 30 Ringkohlenstoffatomen sind.

4. Die organische Elektrolumineszenzvorrichtung gemäß Anspruch 3, wobei $Y_{31}$, $Y_{32}$, $Y_{33}$, $Y_{34}$, $Y_{35}$ und $Y_{36}$ jeweils unabhängig voneinander ein Sauerstoffatom oder ein Schwefelatom sind.

5. Die organische Elektrolumineszenzvorrichtung gemäß Anspruch 3 oder 4, wobei $R_{31}$ bis $R_{38}$ jeweils unabhängig voneinander ein Wasserstoffatom, eine substituierte oder unsubstituierte Arylgruppe mit 6 bis 30 Ringkohlenstoffatomen, eine substituierte oder unsubstituierte heterocyclische Gruppe mit 5 bis 30 Ringatomen oder eine substituierte oder unsubstituierte Alkylgruppe mit 1 bis 30 Kohlenstoffatomen sind.

6. Die organische Elektrolumineszenzvorrichtung gemäß Anspruch 1 oder 2, wobei mindestens ein Paar aus einem Paar aus $R_{31}$ und $R_{32}$, einem Paar aus $R_{32}$ und $R_{33}$, einem Paar aus $R_{33}$ und $R_{34}$, einem Paar aus $R_{35}$ und $R_{36}$, einem Paar aus $R_{36}$ und $R_{37}$, oder einem Paar aus $R_{37}$ und $R_{38}$ aneinander gebunden ist, um einen Ring zu bilden.

7. Die organische Elektrolumineszenzvorrichtung gemäß einem der Ansprüche 1 bis 6, wobei $L_{30}$ eine substituierte oder unsubstituierte Phenylengruppe, eine substituierte oder unsubstituierte Biphenylengruppe, oder eine substituierte oder unsubstituierte Terphenylengruppe ist.

8. Die organische Elektrolumineszenzvorrichtung gemäß einem der Ansprüche 1 bis 7, wobei $X_{31}$, $X_{32}$, $X_{33}$, $X_{34}$, $X_{35}$ und $X_{36}$ jeweils unabhängig voneinander ein Sauerstoffatom oder ein Schwefelatom sind.

9. Die organische Elektrolumineszenzvorrichtung gemäß einem der Ansprüche 1 bis 8, wobei

ein Paar aus $R_{310}$ und $R_{311}$, ein Paar aus $R_{311}$ und $R_{312}$, ein Paar aus $R_{312}$ und $R_{313}$, ein Paar aus $R_{314}$ und $R_{315}$, ein Paar aus $R_{316}$ und $R_{317}$, ein Paar aus $R_{317}$ und $R_{318}$ und ein Paar aus $R_{318}$ und $R_{319}$ nicht aneinander gebunden ist,
ein Paar aus $R_{320}$ und $R_{321}$, ein Paar aus $R_{321}$ und $R_{322}$, ein Paar aus $R_{322}$ und $R_{323}$, ein Paar aus $R_{324}$ und $R_{325}$, ein Paar aus $R_{326}$ und $R_{327}$, ein Paar aus $R_{327}$ und $R_{328}$ und ein Paar aus $R_{328}$ und $R_{329}$ nicht aneinander gebunden ist,
ein Paar aus $R_{330}$ und $R_{331}$, ein Paar aus $R_{331}$ und $R_{332}$, ein Paar aus $R_{332}$ und $R_{333}$, ein Paar aus $R_{335}$ und $R_{336}$, ein Paar aus $R_{336}$ und $R_{337}$ und ein Paar aus $R_{337}$ und $R_{338}$ nicht aneinander gebunden ist,
ein Paar aus $R_{340}$ und $R_{341}$, ein Paar aus $R_{341}$ und $R_{342}$, ein Paar aus $R_{342}$ und $R_{343}$, ein Paar aus $R_{345}$ und $R_{346}$, ein Paar aus $R_{346}$ und $R_{347}$ und ein Paar aus $R_{347}$ und $R_{348}$ nicht aneinander gebunden ist,
ein Paar aus $R_{350}$ und $R_{351}$, ein Paar aus $R_{351}$ und $R_{352}$, ein Paar aus $R_{352}$ und $R_{353}$, ein Paar aus $R_{354}$ und $R_{355}$, ein Paar aus $R_{355}$ und $R_{356}$, ein Paar aus $R_{356}$ und $R_{357}$ und ein Paar aus $R_{358}$ und $R_{359}$ nicht aneinander gebunden ist, und
ein Paar aus $R_{360}$ und $R_{361}$, ein Paar aus $R_{361}$ und $R_{362}$, ein Paar aus $R_{362}$ und $R_{363}$, ein Paar aus $R_{364}$ und $R_{365}$, ein Paar aus $R_{365}$ und $R_{366}$, ein Paar aus $R_{366}$ und $R_{367}$ und ein Paar aus $R_{368}$ und $R_{369}$ nicht aneinander gebunden ist.

10. Die organische Elektrolumineszenzvorrichtung gemäß einem der Ansprüche 1 bis 9, wobei $A_{30}$ die durch die Formel (3a) oder (3d) dargestellte Gruppe ist.

11. Eine Verbindung, die durch die nachstehende Formel (301) dargestellt wird,

[Formel 8]

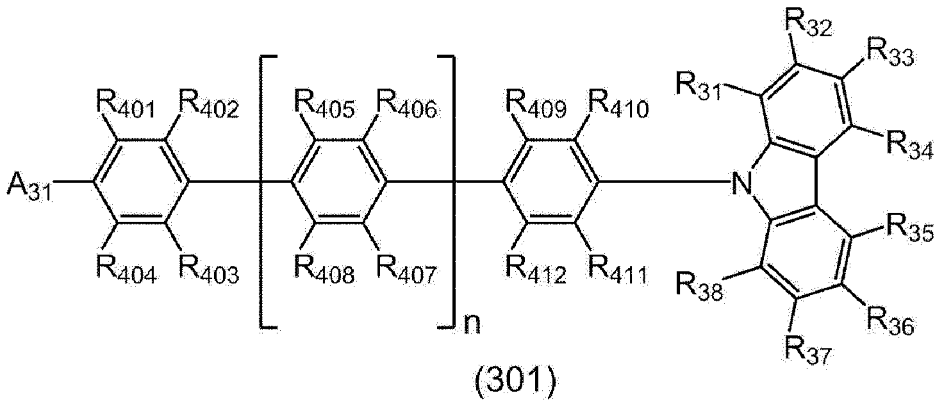

(301)

wobei in der Formel (301): $A_{31}$ eine Gruppe ist, die durch eine der folgenden Formeln (31a), (31b), (31c), (31d), (31e) oder (31f) dargestellt wird,

[Formel 9]

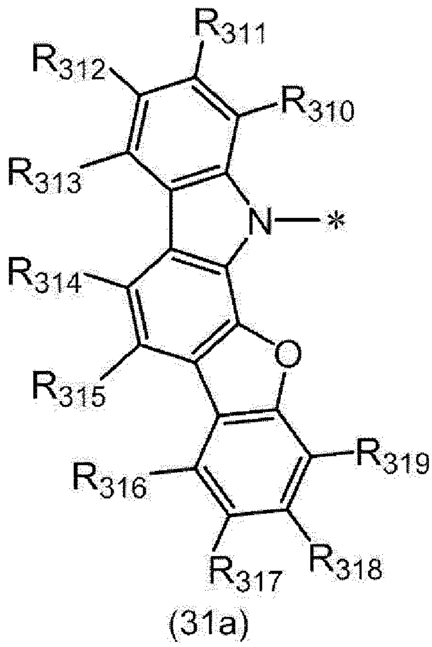

(31a)

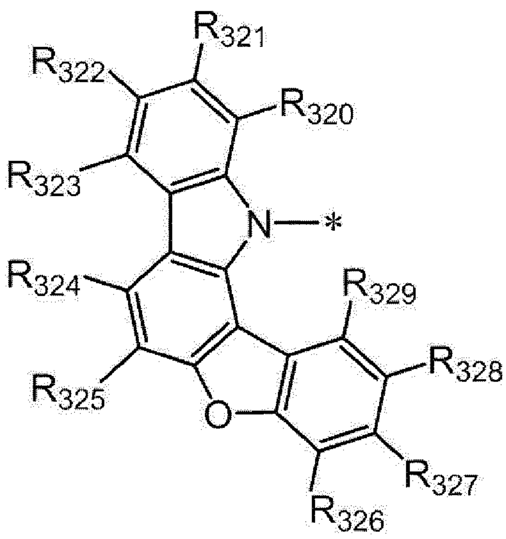

(31b)

[Formel 10]

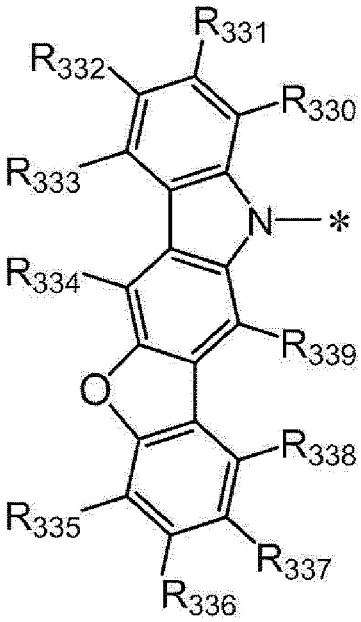

(31c)

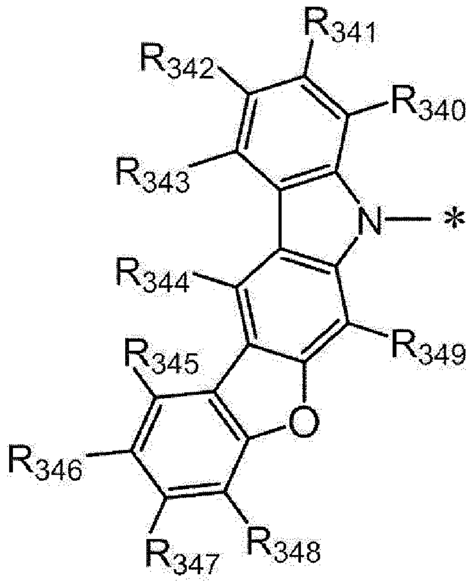

(31d)

[Formel 11]

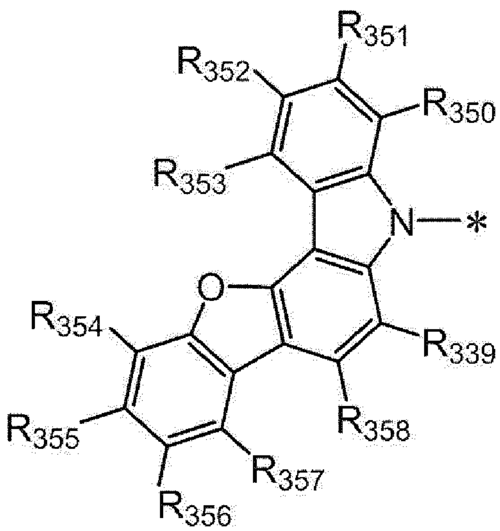

(31e)

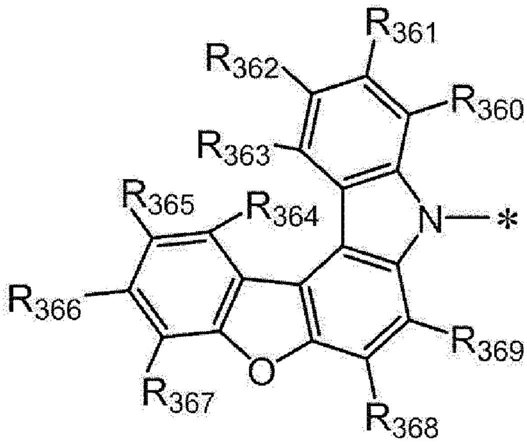

(31f)

wobei in den Formeln (31a), (31b), (31c), (31d), (31e) und (31f):

$R_{310}$ bis $R_{319}$ jeweils unabhängig voneinander ein Wasserstoffatom oder ein Substituent sind;
$R_{320}$ bis $R_{329}$ jeweils unabhängig voneinander ein Wasserstoffatom oder ein Substituent sind;
$R_{330}$ bis $R_{339}$ jeweils unabhängig voneinander ein Wasserstoffatom oder ein Substituent sind;
$R_{340}$ bis $R_{349}$ jeweils unabhängig voneinander ein Wasserstoffatom oder ein Substituent sind;
$R_{350}$ bis $R_{359}$ jeweils unabhängig voneinander ein Wasserstoffatom oder ein Substituent sind;
$R_{360}$ bis $R_{369}$ jeweils unabhängig voneinander ein Wasserstoffatom oder ein Substituent sind;
$R_{310}$ bis $R_{319}$, $R_{320}$ bis $R_{329}$, $R_{330}$ bis $R_{339}$, $R_{340}$ bis $R_{349}$, $R_{350}$ bis $R_{359}$ und $R_{360}$ bis $R_{369}$ als Substituenten

jeweils unabhängig voneinander ein Halogenatom, eine substituierte oder unsubstituierte Arylgruppe mit 6 bis 30 Ringkohlenstoffatomen, eine substituierte oder unsubstituierte heterocyclische Gruppe mit 5 bis 30 Ringatomen, eine substituierte oder unsubstituierte Alkylgruppe mit 1 bis 30 Kohlenstoffatomen, eine substituierte oder unsubstituierte Alkylhalogenidgruppe mit 1 bis 30 Kohlenstoffatomen, eine substituierte oder unsubstituierte Alkenylgruppe mit 2 bis 30 Kohlenstoffatomen, eine substituierte oder unsubstituierte Alkinylgruppe mit 2 bis 30 Kohlenstoffatomen, eine substituierte oder unsubstituierte Alkylsilylgruppe mit 3 bis 30 Kohlenstoffatomen, eine substituierte oder unsubstituierte Arylsilylgruppe mit 6 bis 60 Ringkohlenstoffatomen, eine substituierte oder unsubstituierte Arylphosphorylgruppe mit 6 bis 60 Ringkohlenstoffatomen, eine Hydroxygruppe, eine substituierte oder unsubstituierte Alkoxygruppe mit 1 bis 30 Kohlenstoffatomen, eine substituierte oder unsubstituierte Aryloxygruppe mit 6 bis 30 Ringkohlenstoffatomen, eine Aminogruppe, eine substituierte oder unsubstituierte Alkylaminogruppe mit 2 bis 30 Kohlenstoffatomen, eine substituierte oder unsubstituierte Arylaminogruppe mit 6 bis 60 Ringkohlenstoffatomen, eine Thiolgruppe, eine substituierte oder unsubstituierte Alkylthiogruppe mit 1 bis 30 Kohlenstoffatomen oder eine substituierte oder unsubstituierte Arylthiogruppe mit 6 bis 30 Ringkohlenstoffatomen sind; und
* eine Bindungsposition zu einem Benzolring darstellt, der an $R_{401}$ bis $R_{404}$ gebunden ist, und
wobei in der Formel (301):

$R_{31}$ bis $R_{38}$ jeweils unabhängig voneinander ein Wasserstoffatom oder ein Substituent sind;
$R_{401}$ bis $R_{412}$ jeweils unabhängig voneinander ein Wasserstoffatom oder ein Substituent sind;
$R_{31}$ bis $R_{38}$ als Substituenten jeweils unabhängig voneinander ein Halogenatom, eine substituierte oder unsubstituierte Arylgruppe mit 6 bis 30 Ringkohlenstoffatomen, eine substituierte oder unsubstituierte heterocyclische Gruppe mit 5 bis 30 Ringatomen, eine substituierte oder unsubstituierte Alkylgruppe mit 1 bis 30 Kohlenstoffatomen, eine substituierte oder unsubstituierte Alkylhalogenidgruppe mit 1 bis 30 Kohlenstoffatomen, eine substituierte oder unsubstituierte Alkenylgruppe mit 2 bis 30 Kohlenstoffatomen, eine substituierte oder unsubstituierte Alkinylgruppe mit 2 bis 30 Kohlenstoffatomen, eine substituierte oder unsubstituierte Alkylsilylgruppe mit 3 bis 30 Kohlenstoffatomen, eine substituierte oder unsubstituierte Arylsilylgruppe mit 6 bis 60 Ringkohlenstoffatomen, eine substituierte oder unsubstituierte Arylphosphorylgruppe mit 6 bis 60 Ringkohlenstoffatomen, eine Hydroxygruppe, eine substituierte oder unsubstituierte Alkoxygruppe mit 1 bis 30 Kohlenstoffatomen, eine substituierte oder unsubstituierte Aryloxygruppe mit 6 bis 30 Ringkohlenstoffatomen, eine Aminogruppe, eine substituierte oder unsubstituierte Alkylaminogruppe mit 2 bis 30 Kohlenstoffatomen, eine substituierte oder unsubstituierte Arylaminogruppe mit 6 bis 60 Ringkohlenstoffatomen, eine Thiolgruppe, eine substituierte oder unsubstituierte Alkylthiogruppe mit 1 bis 30 Kohlenstoffatomen oder eine substituierte oder unsubstituierte Arylthiogruppe mit 6 bis 30 Ringkohlenstoffatomen sind; und
$R_{401}$ bis $R_{412}$ als Substituenten jeweils unabhängig voneinander ein Halogenatom, eine substituierte oder unsubstituierte Arylgruppe mit 6 bis 30 Ringkohlenstoffatomen, eine substituierte oder unsubstituierte heterocyclische Gruppe mit 5 bis 30 Ringatomen, eine substituierte oder unsubstituierte Alkylgruppe mit 1 bis 30 Kohlenstoffatomen, eine substituierte oder unsubstituierte Alkylhalogenidgruppe mit 1 bis 30 Kohlenstoffatomen, eine substituierte oder unsubstituierte Alkenylgruppe mit 2 bis 30 Kohlenstoffatomen, eine substituierte oder unsubstituierte Alkinylgruppe mit 2 bis 30 Kohlenstoffatomen, eine substituierte oder unsubstituierte Alkylsilylgruppe mit 3 bis 30 Kohlenstoffatomen, eine substituierte oder unsubstituierte Arylsilylgruppe mit 6 bis 60 Ringkohlenstoffatomen, eine substituierte oder unsubstituierte Arylphosphorylgruppe mit 6 bis 60 Ringkohlenstoffatomen, eine Hydroxygruppe, eine substituierte oder unsubstituierte Alkoxygruppe mit 1 bis 30 Kohlenstoffatomen, eine substituierte oder unsubstituierte Aryloxygruppe mit 6 bis 30 Ringkohlenstoffatomen, eine Thiolgruppe, eine substituierte oder unsubstituierte Alkylthiogruppe mit 1 bis 30 Kohlenstoffatomen oder eine substituierte oder unsubstituierte Arylthiogruppe mit 6 bis 30 Ringkohlenstoffatomen sind;
n 0, 1, 2 oder 3 ist;
wenn mehrere $R_{405}$ vorhanden sind, die mehreren $R_{405}$ untereinander gleich oder verschieden sind;
wenn mehrere $R_{406}$ vorhanden sind, die mehreren $R_{406}$ untereinander gleich oder verschieden sind;
wenn mehrere $R_{407}$ vorhanden sind, die mehreren $R_{407}$ untereinander gleich oder verschieden sind; und
wenn mehrere $R_{408}$ vorhanden sind, die mehreren $R_{408}$ untereinander gleich oder verschieden sind.

**12.** Die Verbindung gemäß Anspruch 11, wobei die Verbindung durch die nachstehende Formel (302) dargestellt wird,

[Formel 12]

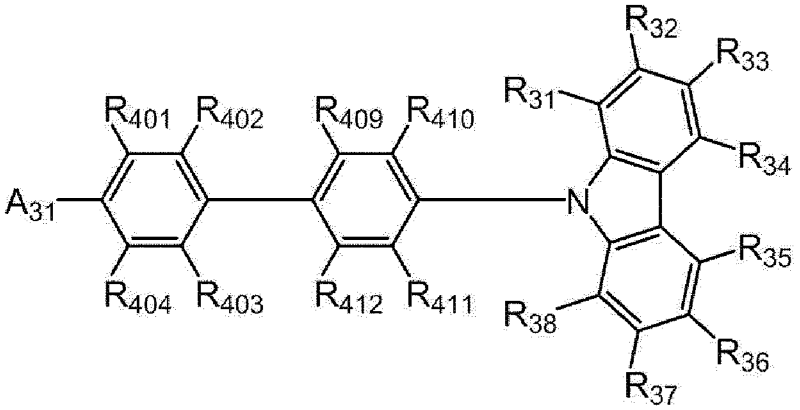

(302)

$A_{31}$, $R_{401}$ bis $R_{404}$, $R_{409}$ bis $R_{412}$ und $R_{31}$ bis $R_{38}$ in der Formel (302) jeweils dasselbe darstellen wie $A_{31}$, $R_{401}$ bis $R_{404}$, $R_{409}$ bis $R_{412}$ und $R_{31}$ bis $R_{38}$ in der Formel (301).

**13.** Eine Verbindung, die durch die nachstehende Formel (310) dargestellt wird,

[Formel 13]

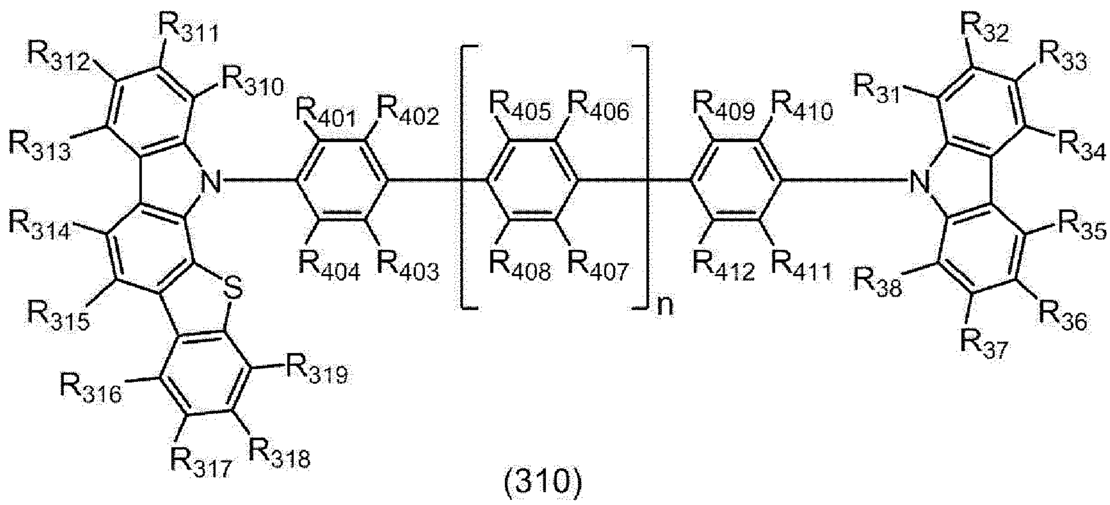

(310)

wobei in der Formel (310):

$R_{310}$ bis $R_{319}$ jeweils unabhängig voneinander ein Wasserstoffatom oder ein Substituent sind;
$R_{31}$ bis $R_{38}$ jeweils unabhängig voneinander ein Wasserstoffatom oder ein Substituent sind;
$R_{401}$ bis $R_{412}$ jeweils unabhängig voneinander ein Wasserstoffatom oder ein Substituent sind;
$R_{310}$ bis $R_{319}$ als Substituent, $R_{31}$ bis $R_{38}$ als Substituent und $R_{401}$ bis $R_{412}$ als Substituent jeweils unabhängig voneinander ein Halogenatom, eine substituierte oder unsubstituierte Arylgruppe mit 6 bis 30 Ringkohlenstoffatomen, eine substituierte oder unsubstituierte heterocyclische Gruppe mit 5 bis 30 Ringatomen, eine substituierte oder unsubstituierte Alkylgruppe mit 1 bis 30 Kohlenstoffatomen, eine substituierte oder unsubstituierte Alkylhalogenidgruppe mit 1 bis 30 Kohlenstoffatomen, eine substituierte oder unsubstituierte Alkenylgruppe mit 2 bis 30 Kohlenstoffatomen, eine substituierte oder unsubstituierte Alkinylgruppe mit 2 bis 30 Kohlenstoffatomen, eine substituierte oder unsubstituierte Alkylsilylgruppe mit 3 bis 30 Kohlenstoffatomen, eine substituierte oder unsubstituierte Arylsilylgruppe mit 6 bis 60 Ringkohlenstoffatomen, eine substituierte oder unsubstituierte Arylphosphorylgruppe mit 6 bis 60 Ringkohlenstoffatomen, eine Hydroxygruppe, eine substituierte oder unsubstituierte Alkoxygruppe mit 1 bis 30 Kohlenstoffatomen, eine substituierte oder unsubstituierte Aryloxygruppe mit 6 bis 30 Ringkohlenstoffatomen, eine Aminogruppe, eine substituierte oder unsubstituierte Alkylaminogruppe

mit 2 bis 30 Kohlenstoffatomen, eine substituierte oder unsubstituierte Arylaminogruppe mit 6 bis 60 Ringkohlenstoffatomen, eine Thiolgruppe, eine substituierte oder unsubstituierte Alkylthiogruppe mit 1 bis 30 Kohlenstoffatomen oder eine substituierte oder unsubstituierte Arylthiogruppe mit 6 bis 30 Ringkohlenstoffatomen sind;
n 0, 1, 2 oder 3 ist;
wenn mehrere $R_{405}$ vorhanden sind, die mehreren $R_{405}$ untereinander gleich oder verschieden sind;
wenn mehrere $R_{406}$ vorhanden sind, die mehreren $R_{406}$ untereinander gleich oder verschieden sind;
wenn mehrere $R_{407}$ vorhanden sind, die mehreren $R_{407}$ untereinander gleich oder verschieden sind; und
wenn mehrere $R_{408}$ vorhanden sind, die mehreren $R_{408}$ untereinander gleich oder verschieden sind.

**14.** Die Verbindung gemäß Anspruch 13, wobei die Verbindung durch die nachstehende Formel (311) dargestellt wird,

[Formel 14]

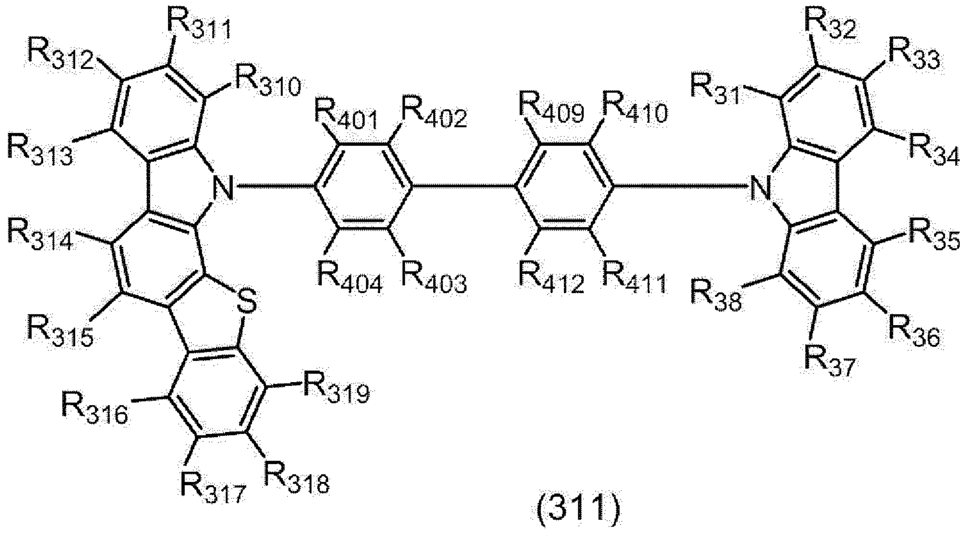

(311)

$R_{310}$ bis $R_{319}$, $R_{401}$ bis $R_{404}$, $R_{409}$ bis $R_{412}$ und $R_{31}$ bis $R_{38}$ in der Formel (311) jeweils dasselbe darstellen wie $R_{310}$ bis $R_{319}$, $R_{401}$ bis $R_{404}$, $R_{409}$ bis $R_{412}$ und $R_{31}$ bis $R_{38}$ in der Formel (310).

**15.** Eine organische Elektrolumineszenzvorrichtung, die Folgendes umfasst:

eine Anode;
eine Kathode;
eine emittierende Schicht, die zwischen der Anode und der Kathode vorgesehen ist,
wobei
die emittierende Schicht eine verzögert fluoreszierende Verbindung M2 und die Verbindung gemäß einem der Ansprüche 11 bis 14 umfasst.

## Revendications

**1.** Un dispositif électroluminescent organique comprenant:

une anode ;
une cathode ;
une couche émettrice disposée entre l'anode et la cathode, dans laquelle
la couche émettrice comprend un composé fluorescent retardé M2, et un composé M3 représenté par la formule (3) suivante, et
une énergie singulet $S_1(M2)$ du composé M2 et une énergie singulet $S_1(M3)$ du composé M3 satisfont une relation de la formule numérique (Formule numérique 1) suivante,

$$S_1(M3) > S_1(M2) \ldots \text{(Formule numérique 1)},$$

[Formule 1]

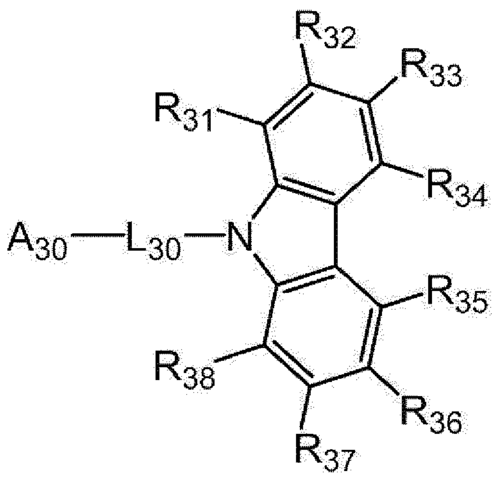

(3)

où, dans la formule (3) : $A_{30}$ est un groupe représenté par une formule (3a), (3b), (3c), (3d), (3e) ou (3f) suivante,

[Formule 2]

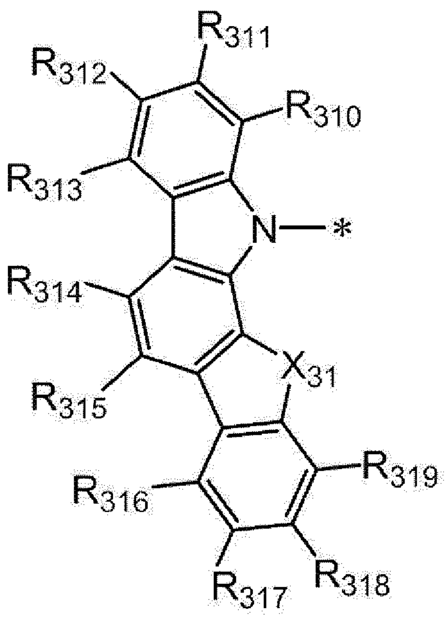

(3a)

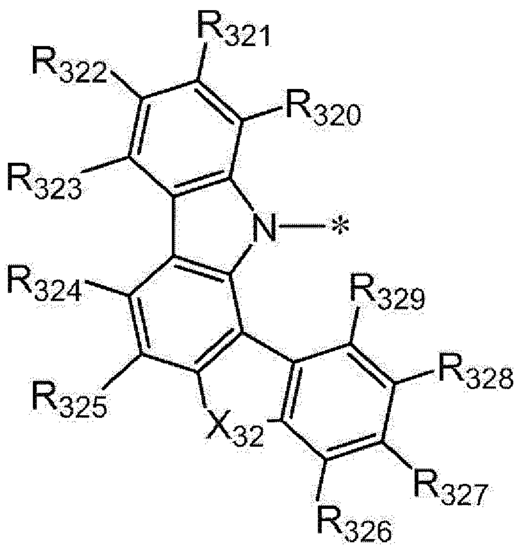

(3b)

[Formule 3]

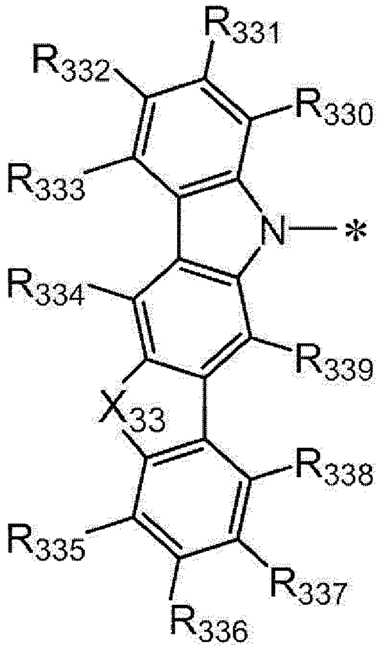

(3c)

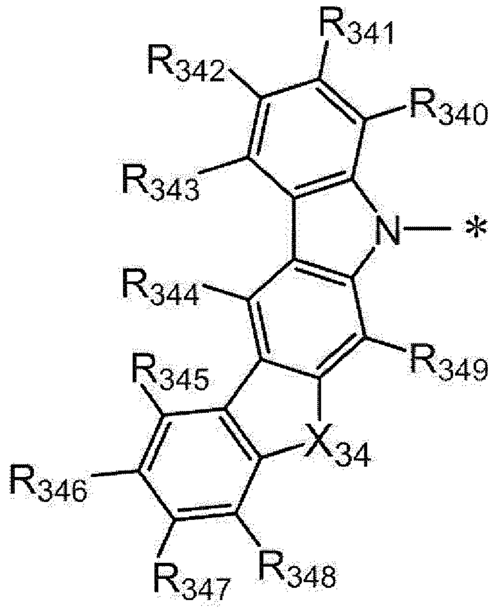

(3d)

[Formule 4]

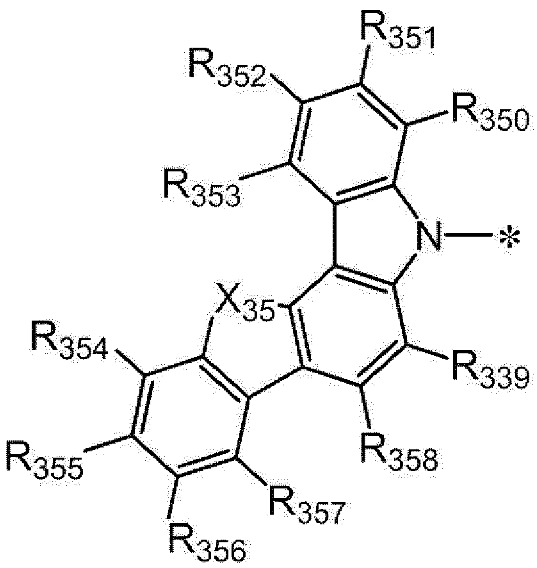

(3e)

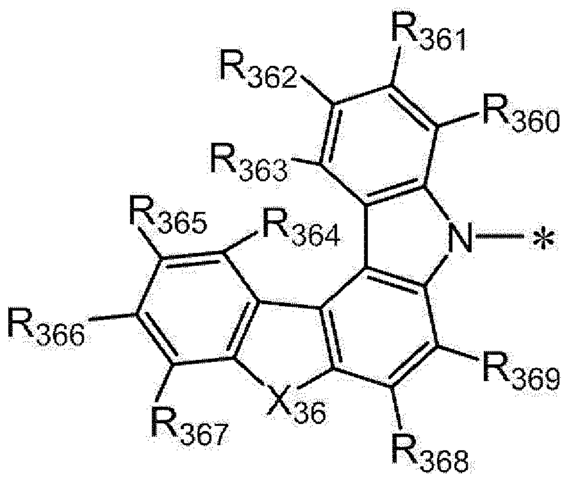

(3f)

où, dans les formules (3a), (3b), (3c), (3d), (3e) et (3f) :

$X_{31}$, $X_{32}$, $X_{33}$, $X_{34}$, $X_{35}$ et $X_{36}$ sont chacun indépendamment un atome d'oxygène, un atome de soufre ou $SiR_{372}R_{373}$;

$R_{372}$ et $R_{373}$ sont chacun indépendamment un atome d'hydrogène ou un substituant ;

$R_{310}$ à $R_{319}$ sont chacun indépendamment un atome d'hydrogène ou un substituant, ou au moins une paire

d'une paire de $R_{310}$ et $R_{311}$, une paire de $R_{311}$ et $R_{312}$, une paire de $R_{312}$ et $R_{313}$, une paire de $R_{314}$ et $R_{315}$, une paire de $R_{316}$ et $R_{317}$, une paire de $R_{317}$ et $R_{318}$, ou une paire de $R_{318}$ et $R_{319}$ est mutuellement liée pour former un anneau;

$R_{320}$ à $R_{329}$ sont chacun indépendamment un atome d'hydrogène ou un substituant, ou au moins une paire d'une paire de $R_{320}$ et $R_{321}$, une paire de $R_{321}$ et $R_{322}$, une paire de $R_{322}$ et $R_{323}$, une paire de $R_{324}$ et $R_{325}$, une paire de $R_{326}$ et $R_{327}$, une paire de $R_{327}$ et $R_{328}$, ou une paire de $R_{328}$ et $R_{329}$ est mutuellement liée pour former un anneau;

$R_{330}$ à $R_{339}$ sont chacun indépendamment un atome d'hydrogène ou un substituant, ou au moins une paire d'une paire de $R_{330}$ et $R_{331}$, une paire de $R_{331}$ et $R_{332}$, une paire de $R_{332}$ et $R_{333}$, une paire de $R_{335}$ et $R_{336}$, une paire de $R_{336}$ et $R_{337}$, ou une paire de $R_{337}$ et $R_{338}$ est mutuellement liée pour former un anneau;

$R_{340}$ à $R_{349}$ sont chacun indépendamment un atome d'hydrogène ou un substituant, ou au moins une paire d'une paire de $R_{340}$ et $R_{341}$, une paire de $R_{341}$ et $R_{342}$, une paire de $R_{342}$ et $R_{343}$, une paire de $R_{345}$ et $R_{346}$, une paire de $R_{346}$ et $R_{347}$, ou une paire de $R_{347}$ et $R_{348}$ est mutuellement liée pour former un anneau;

$R_{350}$ à $R_{359}$ sont chacun indépendamment un atome d'hydrogène ou un substituant, ou au moins une paire d'une paire de $R_{350}$ et $R_{351}$, une paire de $R_{351}$ et $R_{352}$, une paire de $R_{352}$ et $R_{353}$, une paire de $R_{354}$ et $R_{355}$, une paire de $R_{355}$ et $R_{356}$, une paire de $R_{356}$ et $R_{357}$, ou une paire de $R_{358}$ et $R_{359}$ est mutuellement liée pour former un anneau;

$R_{360}$ à $R_{369}$ sont chacun indépendamment un atome d'hydrogène ou un substituant, ou au moins une paire d'une paire de $R_{360}$ et $R_{361}$, une paire de $R_{361}$ et $R_{362}$, une paire de $R_{362}$ et $R_{363}$, une paire de $R_{364}$ et $R_{365}$, une paire de $R_{365}$ et $R_{366}$, une paire de $R_{366}$ et $R_{367}$, ou une paire de $R_{368}$ et $R_{369}$ est mutuellement liée pour former un anneau;

$R_{372}$ et $R_{373}$ comme substituant et $R_{310}$ à $R_{319}$, $R_{320}$ à $R_{329}$, $R_{330}$ à $R_{339}$, $R_{340}$ à $R_{349}$, $R_{350}$ à $R_{359}$ et $R_{360}$ à $R_{369}$ comme substituant sont chacun indépendamment un atome d'halogène, un groupe cyano, un groupe aryle substitué ou non substitué ayant de 6 à 30 atomes de carbone de cycle, un groupe hétérocyclique substitué ou non substitué ayant de 5 à 30 atomes de carbone de cycle, un groupe alkyle substitué ou non substitué ayant de 1 à 30 atomes de carbone, un groupe alkyle halogéné substitué ou non substitué ayant de 1 à 30 atomes de carbone, un groupe alcényle substitué ou non substitué ayant de 2 à 30 atomes de carbone, un groupe alcynyle substitué ou non substitué ayant de 2 à 30 atomes de carbone, un groupe alkylsilyle substitué ou non substitué ayant de 3 à 30 atomes de carbone, un groupe arylsilyle substitué ou non substitué ayant de 6 à 60 atomes de carbone de cycle, un groupe arylphosphoryle substitué ou non substitué ayant de 6 à 60 atomes de carbone de cycle, un groupe hydroxy, un groupe alcoxy substitué ou non substitué ayant de 1 à 30 atomes de carbone, un groupe aryloxy substitué ou non substitué ayant de 6 à 30 atomes de carbone de cycle, un groupe amino, un groupe alkylamino substitué ou non substitué ayant de 2 à 30 atomes de carbone, un groupe arylamino substitué ou non substitué ayant de 6 à 60 atomes de carbone de cycle, un groupe thiol, un groupe alkylthio substitué ou non substitué ayant de 1 à 30 atomes de carbone, ou un groupe arylthio substitué ou non substitué ayant de 6 à 30 atomes de carbone de cycle; et

* représente une position de liaison avec $L_{30}$, et
où, dans la formule (3):

$L_{30}$ est un groupe arylène substitué ou non substitué ayant de 6 à 22 atomes de carbone de cycle, un groupe formé par la liaison de deux groupes arylène substitués ou non substitués ayant de 6 à 22 atomes de carbone de cycle, ou un groupe formé par la liaison de trois groupes arylène substitués ou non substitués ayant de 6 à 22 atomes de carbone de cycle;

$R_{31}$ à $R_{38}$ sont chacun indépendamment un atome d'hydrogène ou un substituant, ou au moins une paire d'une paire de $R_{31}$ et $R_{32}$, une paire de $R_{32}$ et $R_{33}$, une paire de $R_{33}$ et $R_{34}$, une paire de $R_{35}$ et $R_{36}$, une paire de $R_{36}$ et $R_{37}$, ou une paire de $R_{37}$ et $R_{38}$ est mutuellement liée pour former un anneau; et

un substituant pour substituer $L_{30}$, et $R_{31}$ à $R_{38}$ comme substituant sont chacun indépendamment un atome d'halogène, un groupe cyano, un groupe aryle substitué ou non substitué ayant 6 à 30 atomes de carbone de cycle, un groupe hétérocyclique substitué ou non substitué ayant 5 à 30 atomes de cycle, un groupe alkyle substitué ou non substitué ayant 1 à 30 atomes de carbone, un groupe alkyle halogéné substitué ou non substitué ayant de 1 à 30 atomes de carbone, un groupe alcényle substitué ou non substitué ayant de 2 à 30 atomes de carbone, un groupe alcynyle substitué ou non substitué ayant de 2 à 30 atomes de carbone, un groupe alkylsilyle substitué ou non substitué ayant de 3 à 30 atomes de carbone, un groupe arylsilyle substitué ou non substitué ayant de 6 à 60 atomes de carbone de cycle, un groupe arylphosphoryle substitué ou non substitué ayant de 6 à 60 atomes de carbone de cycle, un groupe hydroxy, un groupe alcoxy substitué ou

non substitué ayant de 1 à 30 atomes de carbone, un groupe aryloxy substitué ou non substitué ayant de 6 à 30 atomes de carbone de cycle, un groupe amino, un groupe alkylamino substitué ou non substitué ayant 2 à 30 atomes de carbone, un groupe arylamino substitué ou non substitué ayant 6 à 60 atomes de carbone de cycle, un groupe thiol, un groupe alkylthio substitué ou non substitué ayant 1 à 30 atomes de carbone, ou un groupe arylthio substitué ou non substitué ayant 6 à 30 atomes de carbone de cycle.

**2.** Le dispositif électroluminescent organique selon la revendication 1, dans lequel

la couche émettrice comprend en outre un composé fluorescent M1, et
l'énergie singulet $S_1$(M2) du composé M2 et l'énergie singulet $S_1$(M1) du composé M1 satisfont une relation de la formule numérique (Formule numérique 2) suivante,

$$S_1(M2) > S_1(M1)...(\text{Formule numérique 2}).$$

**3.** Le dispositif électroluminescent organique selon la revendication 1 ou 2, dans lequel dans le composé M3, une paire de $R_{31}$ et $R_{32}$, une paire de $R_{32}$ et $R_{33}$, une paire de $R_{33}$ et $R_{34}$, une paire de $R_{35}$ et $R_{36}$, une paire de $R_{36}$ et $R_{37}$, et une paire de $R_{37}$ et $R_{38}$ ne sont pas mutuellement liées, ou

le composé M3 est représenté par une des formules (31), (32), (33), (34), (35) ou (36) suivantes,

[Formule 5]

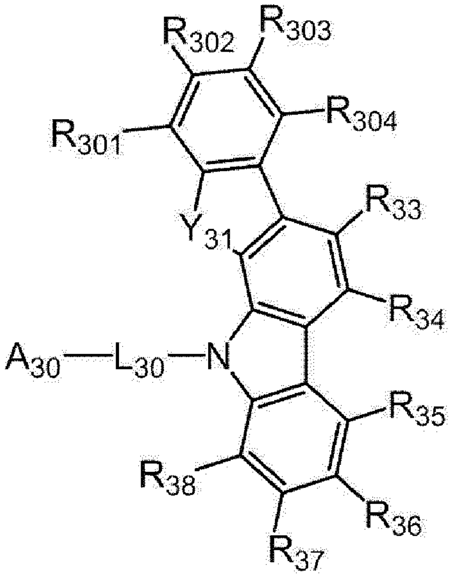

(31)

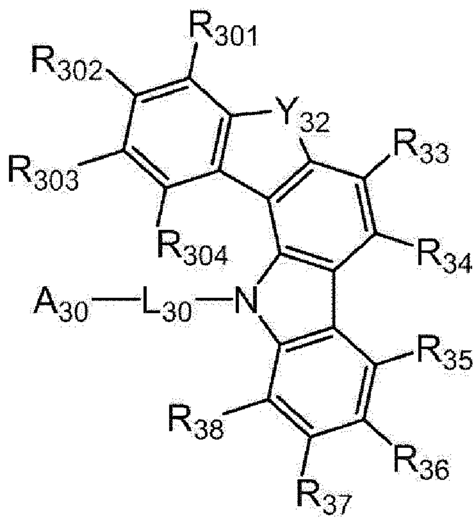

(32)

[Formule 6]

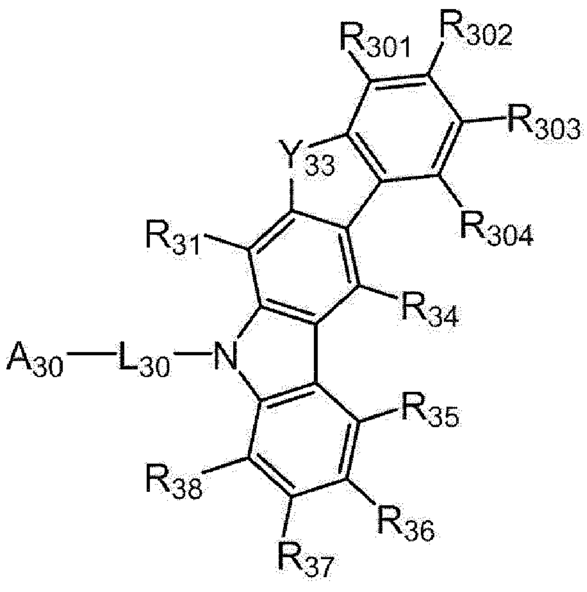

(33)

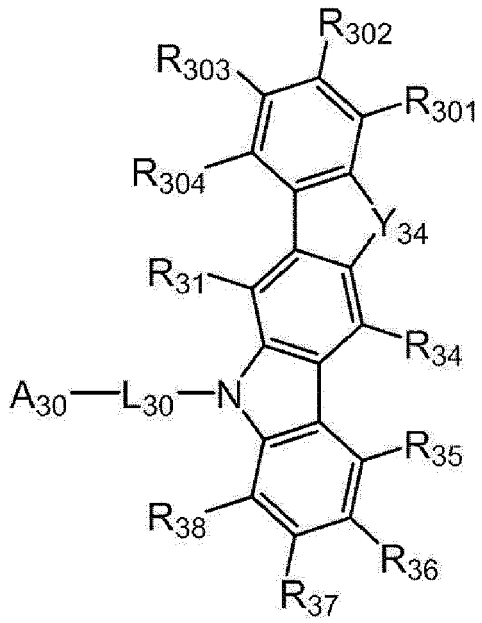

(34)

[Formule 7]

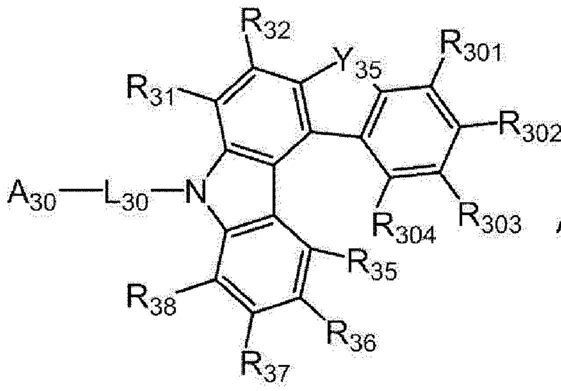

(35)

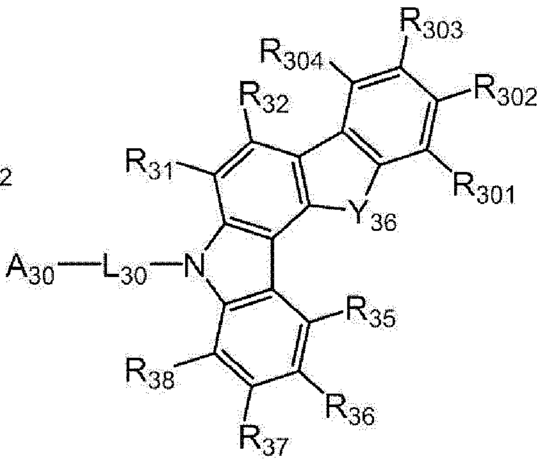

(36)

où, dans les formules (31) à (36):

$Y_{31}$, $Y_{32}$, $Y_{33}$, $Y_{34}$, $Y_{35}$ et $Y_{36}$ sont chacun indépendamment un atome d'oxygène, un atome de soufre, $NR_{374}$ ou $SiR_{375}R_{376}$;
$R_{374}$, $R_{375}$ et $R_{376}$ sont chacun indépendamment un atome d'hydrogène ou un substituant;
$A_{30}$, $L_{30}$ et $R_{31}$ à $R_{38}$ représentent respectivement les mêmes que $A_{30}$, $L_{30}$ et $R_{31}$ à $R_{38}$ dans la formule (3);
$R_{301}$ à $R_{304}$ sont chacun indépendamment un atome d'hydrogène ou un substituant, ou au moins une paire d'une paire de $R_{301}$ et $R_{302}$, une paire de $R_{302}$ et $R_{303}$, ou une paire de $R_{303}$ et $R_{304}$ est mutuellement liée pour former un anneau; et
$R_{301}$ à $R_{304}$ comme substituant et $R_{374}$, $R_{375}$ et $R_{376}$ comme substituant sont chacun indépendamment un atome d'halogène, un groupe cyano, un groupe aryle substitué ou non substitué ayant de 6 à 30 atomes de carbone de cycle, un groupe hétérocyclique substitué ou non substitué ayant de 5 à 30 atomes de carbone de cycle, un groupe alkyle substitué ou non substitué ayant de 1 à 30 atomes de carbone, un groupe alkyle halogéné substitué ou non substitué ayant de 1 à 30 atomes de carbone, un groupe alcényle substitué ou non substitué ayant de 2 à 30 atomes de carbone, un groupe alcynyle substitué ou non substitué

ayant de 2 à 30 atomes de carbone, un groupe alkylsilyle substitué ou non substitué ayant de 3 à 30 atomes de carbone, un groupe arylsilyle substitué ou non substitué ayant de 6 à 60 atomes de carbone de cycle, un groupe arylphosphoryle substitué ou non substitué ayant de 6 à 60 atomes de carbone de cycle, un groupe hydroxy, un groupe alcoxy substitué ou non substitué ayant de 1 à 30 atomes de carbone, un groupe aryloxy substitué ou non substitué ayant de 6 à 30 atomes de carbone de cycle, un groupe amino, un groupe alkylamino substitué ou non substitué ayant 2 à 30 atomes de carbone, un groupe arylamino substitué ou non substitué ayant 6 à 60 atomes de carbone, un groupe thiol, un groupe alkylthio substitué ou non substitué ayant 1 à 30 atomes de carbone, ou un groupe arylthio substitué ou non substitué ayant 6 à 30 atomes de carbone de cycle.

**4.** Le dispositif électroluminescent organique selon la revendication 3, dans lequel $Y_{31}$, $Y_{32}$, $Y_{33}$, $Y_{34}$, $Y_{35}$ et $Y_{36}$ sont chacun indépendamment un atome d'oxygène ou un atome de soufre.

**5.** Le dispositif électroluminescent organique selon la revendication 3 ou 4, dans lequel $R_{31}$ à $R_{38}$ sont chacun indépendamment un atome d'hydrogène, un groupe aryle substitué ou non substitué ayant 6 à 30 atomes de carbone de cycle, un groupe hétérocyclique substitué ou non substitué ayant 5 à 30 atomes de cycle, ou un groupe alkyle substitué ou non substitué ayant 1 à 30 atomes de carbone.

**6.** Le dispositif électroluminescent organique selon la revendication 1 ou 2, dans lequel au moins une paire d'une paire de $R_{31}$ et $R_{32}$, une paire de $R_{32}$ et $R_{33}$, une paire de $R_{33}$ et $R_{34}$, une paire de $R_{35}$ et $R_{36}$, une paire de $R_{36}$ et $R_{37}$, ou une paire de $R_{37}$ et $R_{38}$ est mutuellement liée pour former un anneau.

**7.** Le dispositif électroluminescent organique selon l'une des revendications 1 à 6, dans lequel $L_{30}$ est un groupe phénylène substitué ou non substitué, un groupe biphénylène substitué ou non substitué, ou un groupe terphénylène substitué ou non substitué.

**8.** Le dispositif électroluminescent organique selon l'une des revendications 1 à 7, dans lequel $X_{31}$, $X_{32}$, $X_{33}$, $X_{34}$, $X_{35}$ et $X_{36}$ sont chacun indépendamment un atome d'oxygène ou un atome de soufre.

**9.** Le dispositif électroluminescent organique selon l'une des revendications 1 à 8, dans lequel

une paire de $R_{310}$ et $R_{311}$, une paire de $R_{311}$ et $R_{312}$, une paire de $R_{312}$ et $R_{313}$, une paire de $R_{314}$ et $R_{315}$, une paire de $R_{316}$ et $R_{317}$, une paire de $R_{317}$ et $R_{318}$, et une paire de $R_{318}$ et $R_{319}$ ne sont pas mutuellement liées,
une paire de $R_{320}$ et $R_{321}$, une paire de $R_{321}$ et $R_{322}$, une paire de $R_{322}$ et $R_{323}$, une paire de $R_{324}$ et $R_{325}$, une paire de $R_{326}$ et $R_{327}$, une paire de $R_{327}$ et $R_{328}$, et une paire de $R_{328}$ et $R_{329}$ ne sont pas mutuellement liées,
une paire de $R_{330}$ et $R_{331}$, une paire de $R_{331}$ et $R_{332}$, une paire de $R_{332}$ et $R_{333}$, une paire de $R_{335}$ et $R_{336}$, une paire de $R_{336}$ et $R_{337}$, et une paire de $R_{337}$ et $R_{338}$ ne sont pas mutuellement liées,
une paire de $R_{340}$ et $R_{341}$, une paire de $R_{341}$ et $R_{342}$, une paire de $R_{342}$ et $R_{343}$, une paire de $R_{345}$ et $R_{346}$, une paire de $R_{346}$ et $R_{347}$, et une paire de $R_{347}$ et $R_{348}$ ne sont pas mutuellement liées,
une paire de $R_{350}$ et $R_{351}$, une paire de $R_{351}$ et $R_{352}$, une paire de $R_{352}$ et $R_{353}$, une paire de $R_{354}$ et $R_{355}$, une paire de $R_{355}$ et $R_{356}$, une paire de $R_{356}$ et $R_{357}$, et une paire de $R_{358}$ et $R_{359}$ ne sont pas mutuellement liées, et
une paire de $R_{360}$ et $R_{361}$, une paire de $R_{361}$ et $R_{362}$, une paire de $R_{362}$ et $R_{363}$, une paire de $R_{364}$ et $R_{365}$, une paire de $R_{365}$ et $R_{366}$, une paire de $R_{366}$ et $R_{367}$, et une paire de $R_{368}$ et $R_{369}$ ne sont pas mutuellement liées.

**10.** Le dispositif électroluminescent organique selon l'une des revendications 1 à 9, dans lequel $A_{30}$ est le groupe représenté par la formule (3a) ou (3d).

**11.** Un composé représenté par la formule (301) suivante,

[Formule 8]

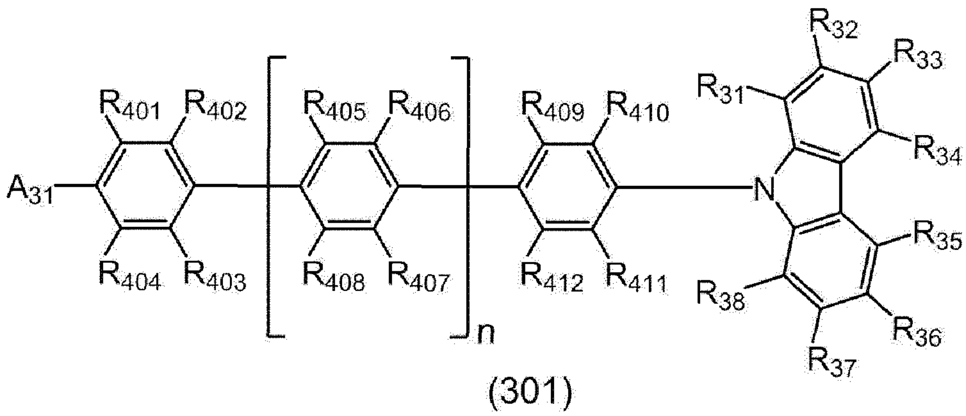

(301)

où, dans la formule (301): $A_{31}$ est un groupe représenté par une des formules (31a), (31b), (31c), (31d), (31e) ou (31f) suivantes,

[Formule 9]

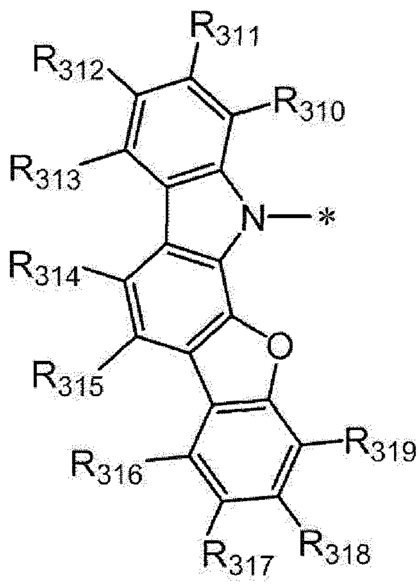

(31a)

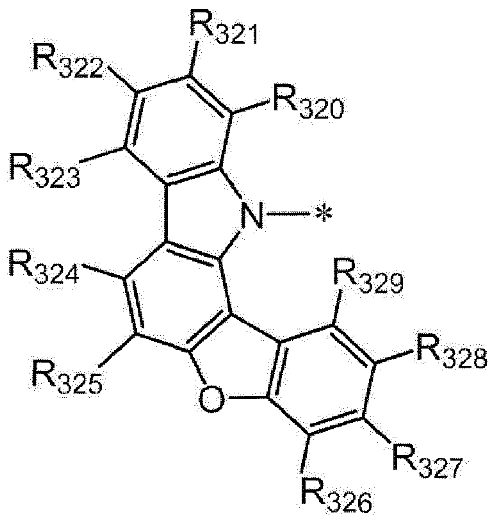

(31b)

[Formule 10]

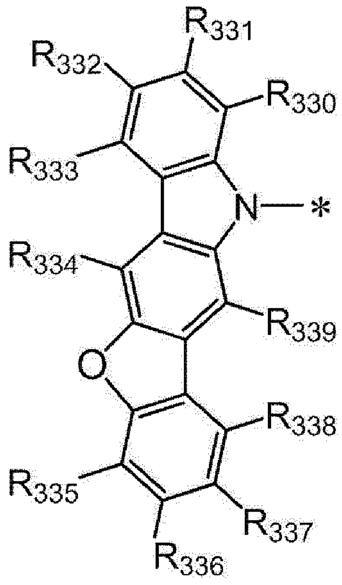

(31c)

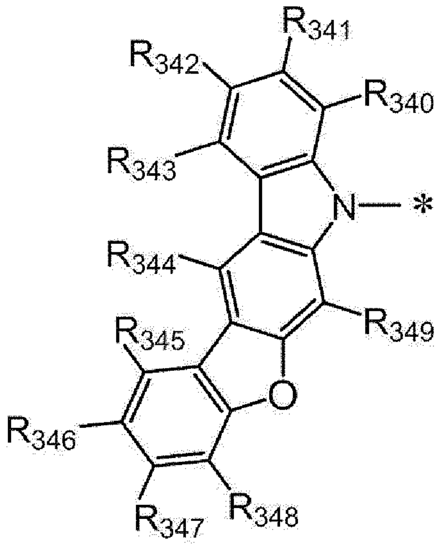

(31d)

[Formule 11]

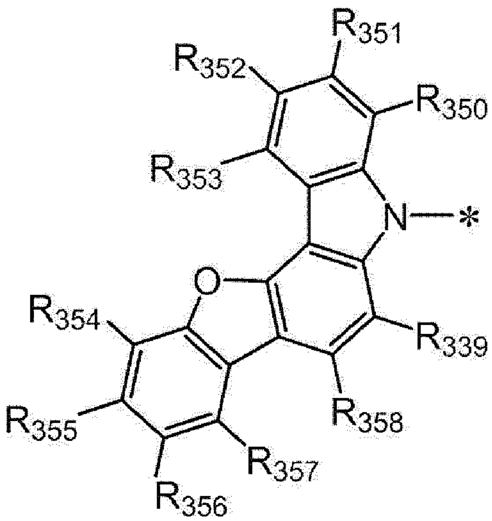

(31e)

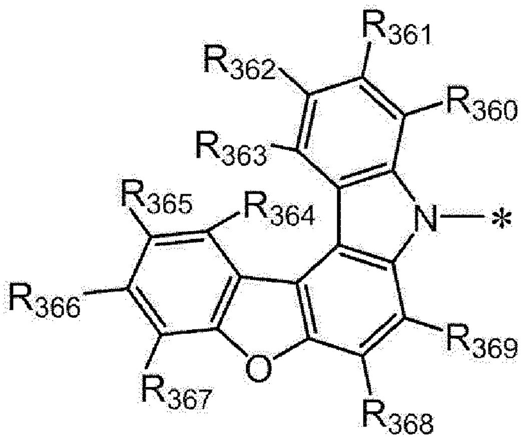

(31f)

où, dans les formules (31a), (31b), (31c), (31d), (31e) et (31f):

$R_{310}$ à $R_{319}$ sont chacun indépendamment un atome d'hydrogène ou un substituant;
$R_{320}$ à $R_{329}$ sont chacun indépendamment un atome d'hydrogène ou un substituant;
$R_{330}$ à $R_{339}$ sont chacun indépendamment un atome d'hydrogène ou un substituant;
$R_{340}$ à $R_{349}$ sont chacun indépendamment un atome d'hydrogène ou un substituant;
$R_{350}$ à $R_{359}$ sont chacun indépendamment un atome d'hydrogène ou un substituant;
$R_{360}$ à $R_{369}$ sont chacun indépendamment un atome d'hydrogène ou un substituant;
$R_{310}$ à $R_{319}$, $R_{320}$ à $R_{329}$, $R_{330}$ à $R_{339}$, $R_{340}$ à $R_{349}$, $R_{350}$ à $R_{359}$ et $R_{360}$ à $R_{369}$ comme substituant sont chacun indépendamment un atome d'halogène, un groupe aryle substitué ou non substitué ayant 6 à 30 atomes de carbone de cycle, un groupe hétérocyclique substitué ou non substitué ayant 5 à 30 atomes de cycle, un groupe alkyle substitué ou non substitué ayant 1 à 30 atomes de carbone, un groupe alkyle halogéné substitué ou non substitué ayant de 1 à 30 atomes de carbone, un groupe alcényle substitué ou non substitué ayant de 2 à 30 atomes de carbone, un groupe alcynyle substitué ou non substitué ayant de

2 à 30 atomes de carbone, un groupe alkylsilyle substitué ou non substitué ayant de 3 à 30 atomes de carbone, un groupe arylsilyle substitué ou non substitué ayant de 6 à 60 atomes de carbone de cycle, un groupe arylphosphoryle substitué ou non substitué ayant de 6 à 60 atomes de carbone de cycle, un groupe hydroxy, un groupe alcoxy substitué ou non substitué ayant de 1 à 30 atomes de carbone, un groupe aryloxy substitué ou non substitué ayant de 6 à 30 atomes de carbone de cycle, un groupe amino, un groupe alkylamino substitué ou non substitué ayant de 2 à 30 atomes de carbone, un groupe arylamino substitué ou non substitué ayant de 6 à 60 atomes de carbone de cycle, un groupe thiol, un groupe alkylthio substitué ou non substitué ayant de 1 à 30 atomes de carbone, ou un groupe arylthio substitué ou non substitué ayant de 6 à 30 atomes de carbone de cycle; et
* représente une position de liaison à un anneau benzénique lié à $R_{401}$ à $R_{404}$, et
où, dans la formule (301):

$R_{31}$ à $R_{38}$ sont chacun indépendamment un atome d'hydrogène ou un substituant;
$R_{401}$ à $R_{412}$ sont chacun indépendamment un atome d'hydrogène ou un substituant;
$R_{31}$ à $R_{38}$ comme substituant sont chacun indépendamment un atome d'halogène, un groupe aryle substitué ou non substitué ayant 6 à 30 atomes de carbone de cycle, un groupe hétérocyclique substitué ou non substitué ayant 5 à 30 atomes de cycle, un groupe alkyle substitué ou non substitué ayant 1 à 30 atomes de carbone, un groupe alkyle halogéné substitué ou non substitué ayant 1 à 30 atomes de carbone, un groupe alcényle substitué ou non substitué ayant de 2 à 30 atomes de carbone, un groupe alcynyle substitué ou non substitué ayant de 2 à 30 atomes de carbone, un groupe alkylsilyle substitué ou non substitué ayant de 3 à 30 atomes de carbone, un groupe arylsilyle substitué ou non substitué ayant de 6 à 60 atomes de carbone de cycle, un groupe arylphosphoryle substitué ou non substitué ayant de 6 à 60 atomes de carbone de cycle, un groupe hydroxy, un groupe alcoxy substitué ou non substitué ayant de 1 à 30 atomes de carbone, un groupe aryloxy substitué ou non substitué ayant de 6 à 30 atomes de carbone de cycle, un groupe amino, un groupe alkylamino substitué ou non substitué ayant de 2 à 30 atomes de carbone, un groupe arylamino substitué ou non substitué ayant de 6 à 60 atomes de carbone de cycle, un groupe thiol, un groupe alkylthio substitué ou non substitué ayant de 1 à 30 atomes de carbone, ou un groupe arylthio substitué ou non substitué ayant de 6 à 30 atomes de carbone de cycle; et
$R_{401}$ à $R_{412}$ comme substituant sont chacun indépendamment un atome d'halogène, un groupe aryle substitué ou non substitué ayant 6 à 30 atomes de carbone de cycle, un groupe hétérocyclique substitué ou non substitué ayant 5 à 30 atomes de carbone de cycle, un groupe alkyle substitué ou non substitué ayant 1 à 30 atomes de carbone, un groupe alkyle halogéné substitué ou non substitué ayant 1 à 30 atomes de carbone, un groupe alcényle substitué ou non substitué ayant 2 à 30 atomes de carbone, un groupe alcynyle substitué ou non substitué ayant 2 à 30 atomes de carbone, un groupe alkylsilyle substitué ou non substitué ayant de 3 à 30 atomes de carbone, un groupe arylsilyle substitué ou non substitué ayant de 6 à 60 atomes de carbone de cycle, un groupe arylphosphoryle substitué ou non substitué ayant de 6 à 60 atomes de carbone de cycle, un groupe hydroxy, un groupe alcoxy substitué ou non substitué ayant de 1 à 30 atomes de carbone, un groupe aryloxy substitué ou non substitué ayant de 6 à 30 atomes de carbone de cycle, un groupe thiol, un groupe alkylthio substitué ou non substitué ayant de 1 à 30 atomes de carbone, ou un groupe arylthio substitué ou non substitué ayant de 6 à 30 atomes de carbone de cycle;
n est 0, 1, 2 ou 3 ;

en présence d'une pluralité de $R_{405}$, la pluralité de $R_{405}$ est mutuellement identique ou différente ;
en présence d'une pluralité de $R_{406}$, la pluralité de $R_{406}$ est mutuellement identique ou différente ;
en présence d'une pluralité de $R_{407}$, la pluralité de $R_{407}$ est mutuellement identique ou différente ; et
en présence d'une pluralité de $R_{408}$, la pluralité de $R_{408}$ est mutuellement identique ou différente.

**12.** Le composé selon la revendication 11, dans lequel le composé est représenté par la formule (302) suivante,

[Formule 12]

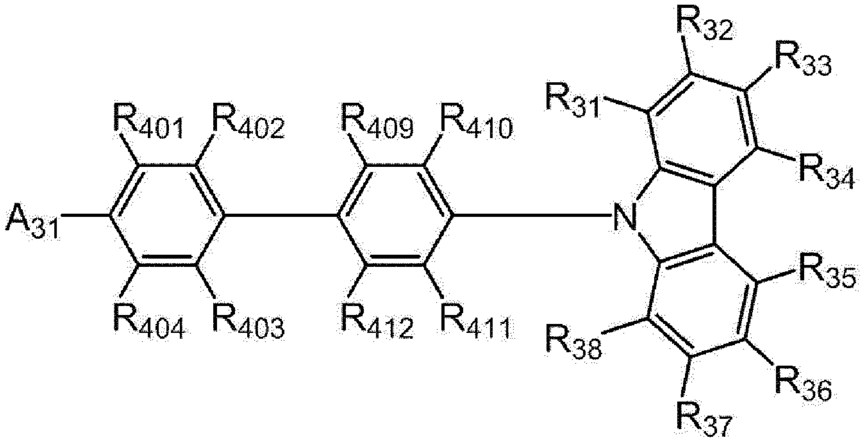

(302)

$A_{31}$, $R_{401}$ à $R_{404}$, $R_{409}$ à $R_{412}$ et $R_{31}$ à $R_{38}$ dans la formule (302) représentent respectivement les mêmes que $A_{31}$, $R_{401}$ à $R_{404}$, $R_{409}$ à $R_{412}$ et $R_{31}$ à $R_{38}$ dans la formule (301).

**13.** Un composé représenté par la formule (310) suivante,

[Formule 13]

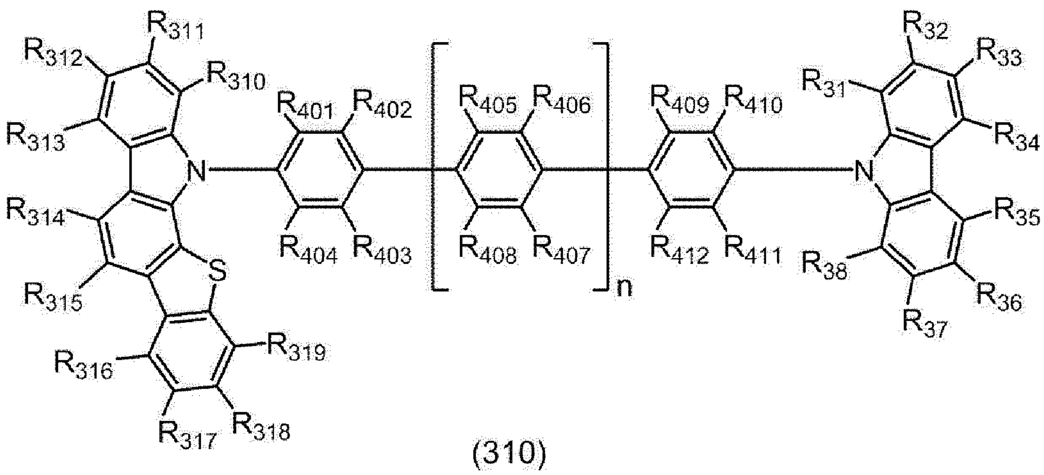

(310)

où, dans la formule (310):

$R_{310}$ à $R_{319}$ sont chacun indépendamment un atome d'hydrogène ou un substituant;
$R_{31}$ à $R_{38}$ sont chacun indépendamment un atome d'hydrogène ou un substituant;
$R_{401}$ à $R_{412}$ sont chacun indépendamment un atome d'hydrogène ou un substituant;
$R_{310}$ à $R_{319}$ comme substituant, $R_{31}$ à $R_{38}$ comme substituant et $R_{401}$ à $R_{412}$ comme substituant sont chacun indépendamment un atome d'halogène, un groupe aryle substitué ou non substitué ayant de 6 à 30 atomes de carbone de cycle, un groupe hétérocyclique substitué ou non substitué ayant de 5 à 30 atomes de cycle, un groupe alkyle substitué ou non substitué ayant de 1 à 30 atomes de carbone, un groupe alkyle halogéné substitué ou non substitué ayant de 1 à 30 atomes de carbone, un groupe alcényle substitué ou non substitué ayant de 2 à 30 atomes de carbone, un groupe alcynyle substitué ou non substitué ayant de 2 à 30 atomes de carbone, un groupe alkylsilyle substitué ou non substitué ayant de 3 à 30 atomes de carbone, un groupe arylsilyle substitué ou non substitué ayant de 6 à 60 atomes de carbone de cycle, un groupe arylphosphoryle substitué ou non substitué ayant de 6 à 60 atomes de carbone de cycle, un groupe hydroxy, un groupe alcoxy substitué ou non substitué ayant de 1 à 30 atomes de carbone, un groupe aryloxy substitué ou non substitué ayant de 6 à 30 atomes de carbone de cycle, un groupe amino, un groupe alkylamino substitué ou non substitué ayant 2 à 30 atomes de carbone, un groupe arylamino substitué ou non substitué ayant 6 à 60 atomes de carbone de cycle, un groupe thiol, un groupe alkylthio substitué ou non substitué ayant 1 à 30 atomes de carbone, ou un groupe arylthio substitué ou non substitué ayant 6 à 30 atomes de carbone de cycle;

n est 0, 1, 2 ou 3 ;
en présence d'une pluralité de $R_{405}$, la pluralité de $R_{405}$ est mutuellement identique ou différente;
en présence d'une pluralité de $R_{406}$, la pluralité de $R_{406}$ est mutuellement identique ou différente;
en présence d'une pluralité de $R_{407}$, la pluralité de $R_{407}$ est mutuellement identique ou différente; et
en présence d'une pluralité de $R_{408}$, la pluralité de $R_{408}$ est mutuellement identique ou différente.

**14.** Le composé selon la revendication 13, dans lequel le composé est représenté par la formule (311) suivante,

[Formule 14]

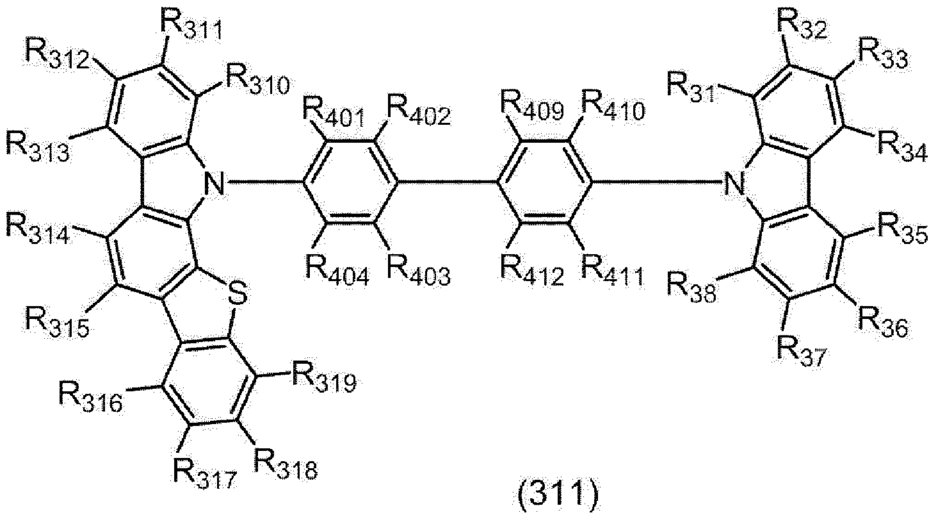

(311)

$R_{310}$ à $R_{319}$, $R_{401}$ à $R_{404}$, $R_{409}$ à $R_{412}$ et $R_{31}$ à $R_{38}$ dans la formule (311) représentent respectivement les mêmes que $R_{310}$ à $R_{319}$, $R_{401}$ à $R_{404}$, $R_{409}$ à $R_{412}$ et $R_{31}$ à $R_{38}$ dans la formule (310).

**15.** Un dispositif électroluminescent organique comprenant :

une anode ;
une cathode ;
une couche émettrice placée entre l'anode et la cathode, dans laquelle
la couche émettrice comprend un composé à fluorescence retardée M2 et le composé selon l'une quelconque des revendications 11 à 14.

FIG. 1

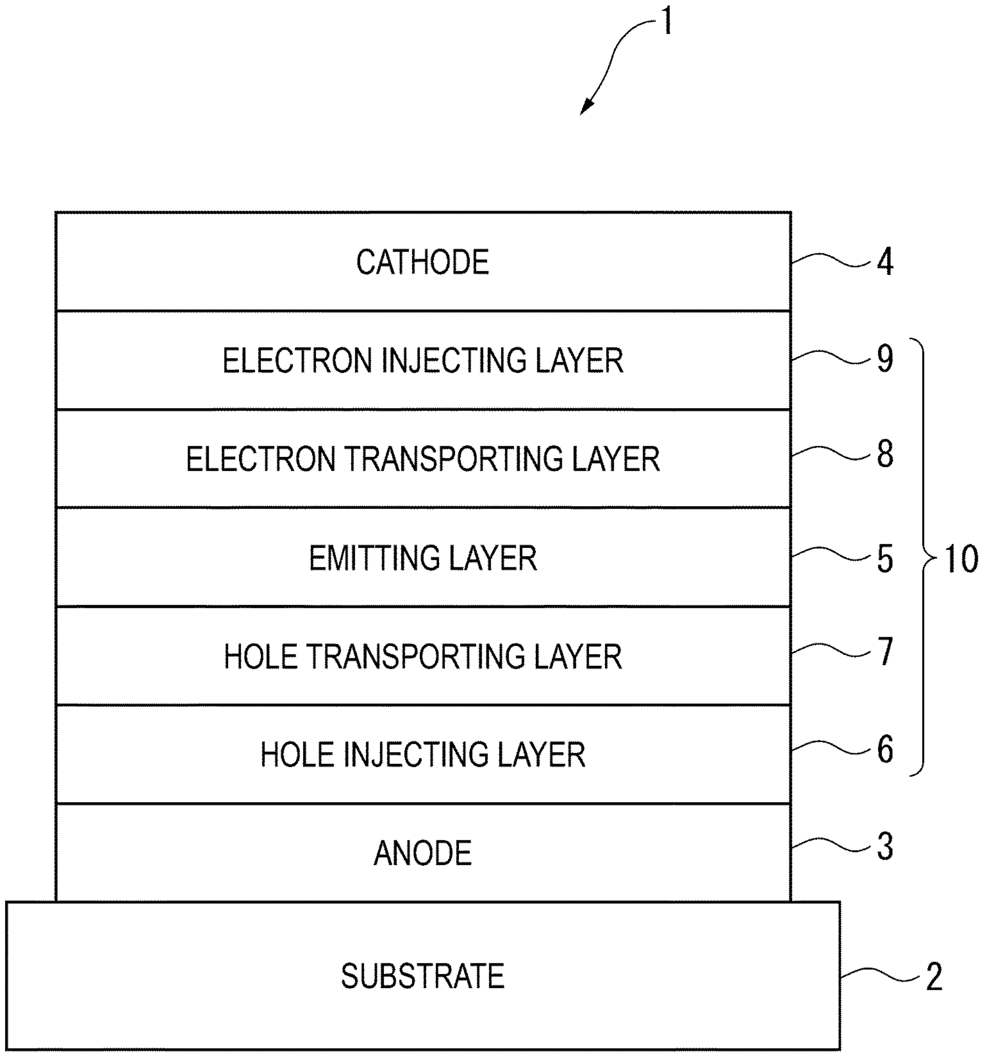
1
CATHODE
4
ELECTRON INJECTING LAYER
9
ELECTRON TRANSPORTING LAYER
8
EMITTING LAYER
5
10
HOLE TRANSPORTING LAYER
7
HOLE INJECTING LAYER
6
ANODE
3
SUBSTRATE
2

FIG.2

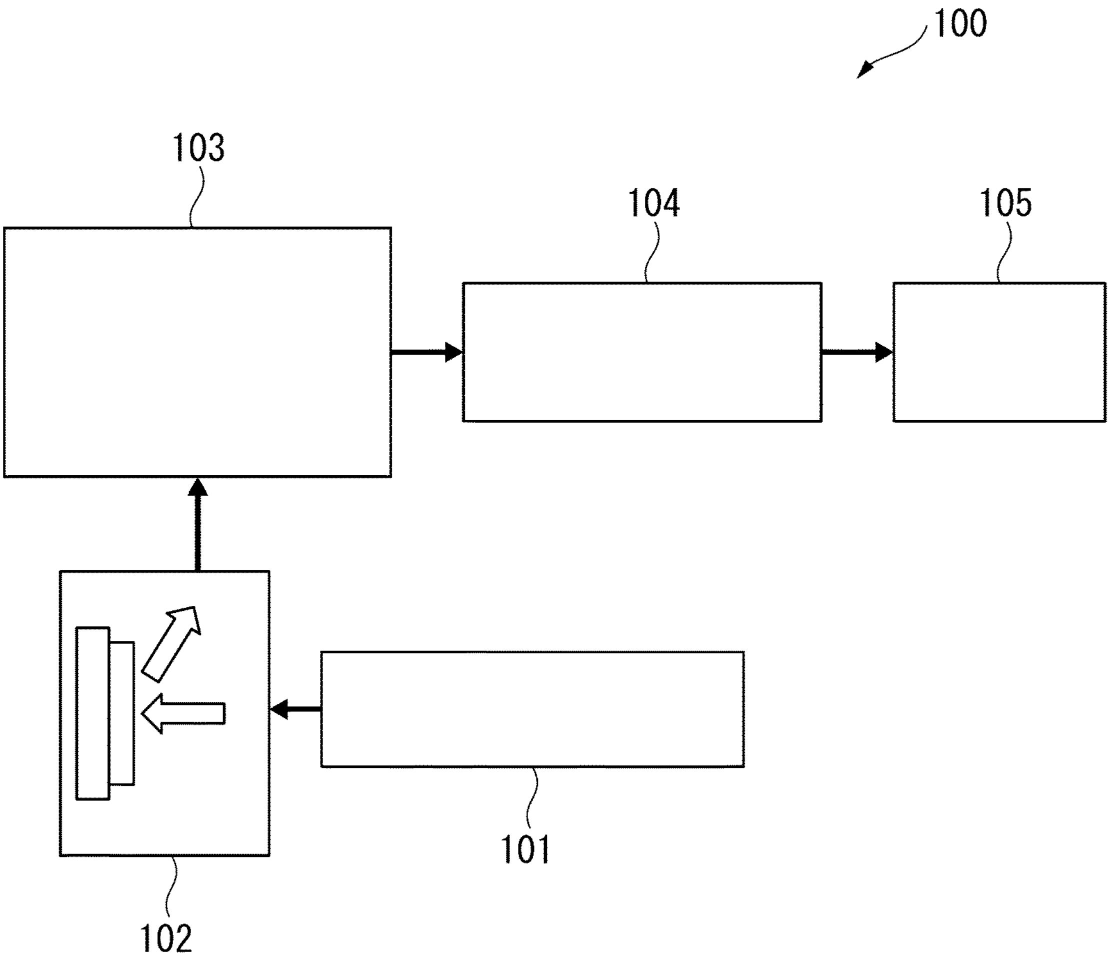

FIG.3

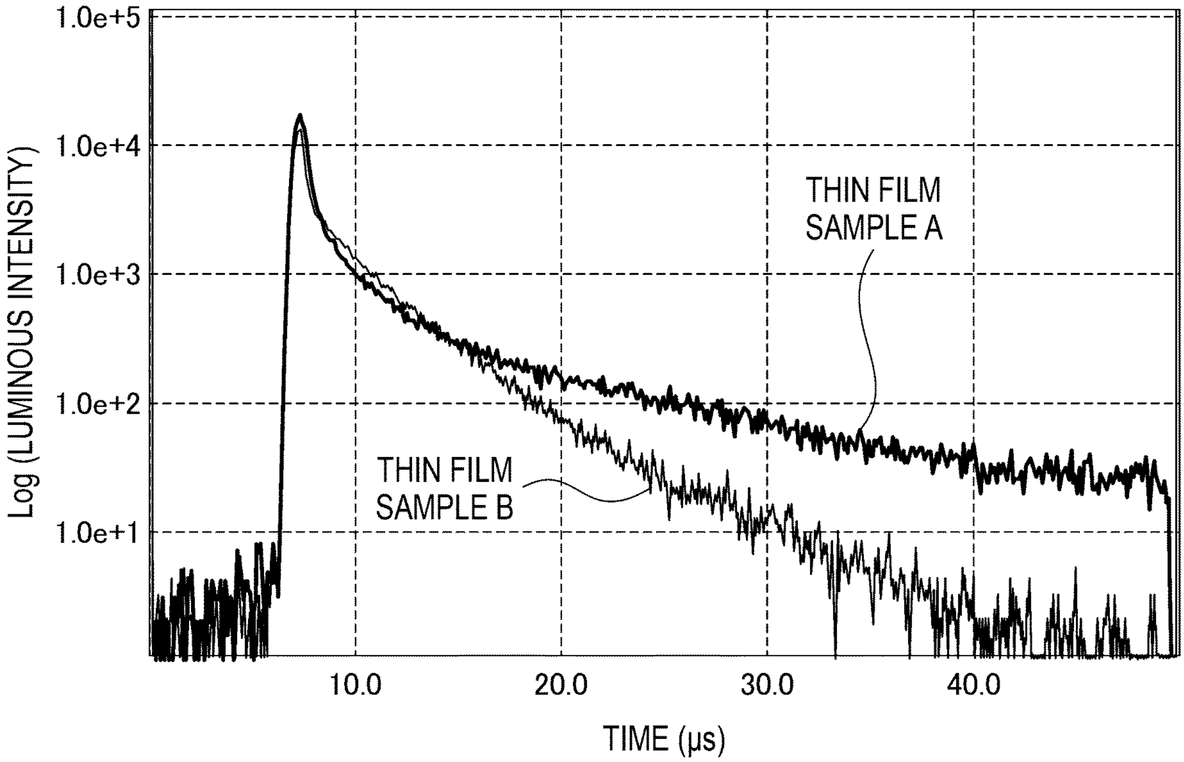
1.0e+5
1.0e+4
1.0e+3
1.0e+2
1.0e+1
Log (LUMINOUS INTENSITY)
THIN FILM
SAMPLE A
THIN FILM
SAMPLE B
10.0
20.0
30.0
40.0
TIME (μs)

FIG.4

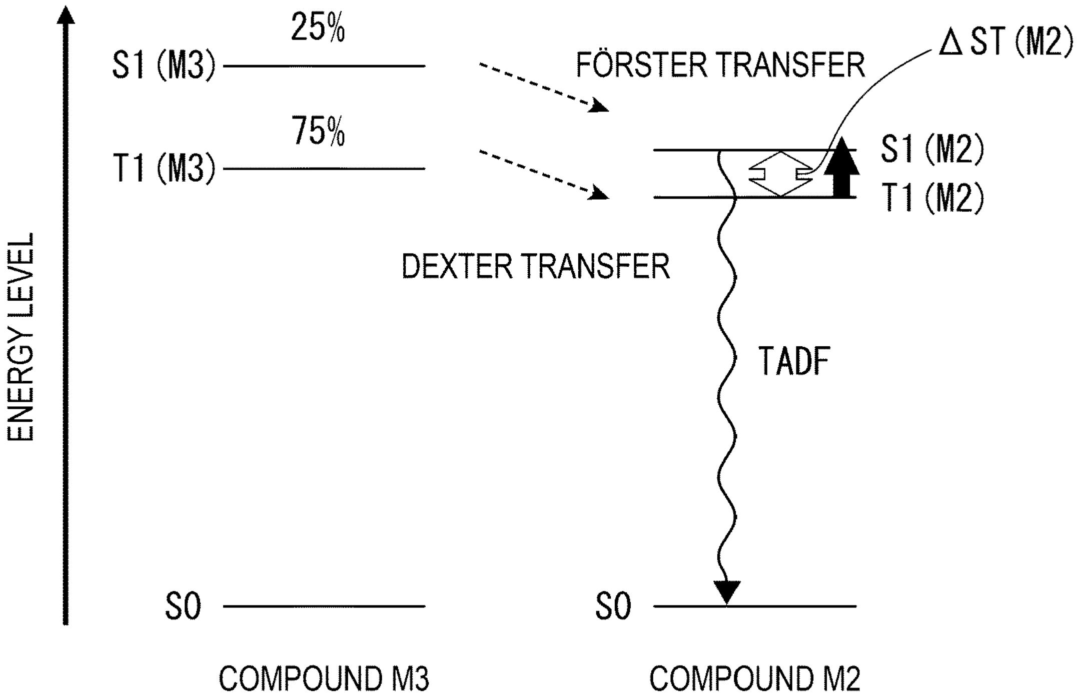
25%
S1 (M3)
75%
T1 (M3)
FÖRSTER TRANSFER
ΔST (M2)
S1 (M2)
T1 (M2)
DEXTER TRANSFER
TADF
ENERGY LEVEL
S0
S0
COMPOUND M3
COMPOUND M2

FIG.5

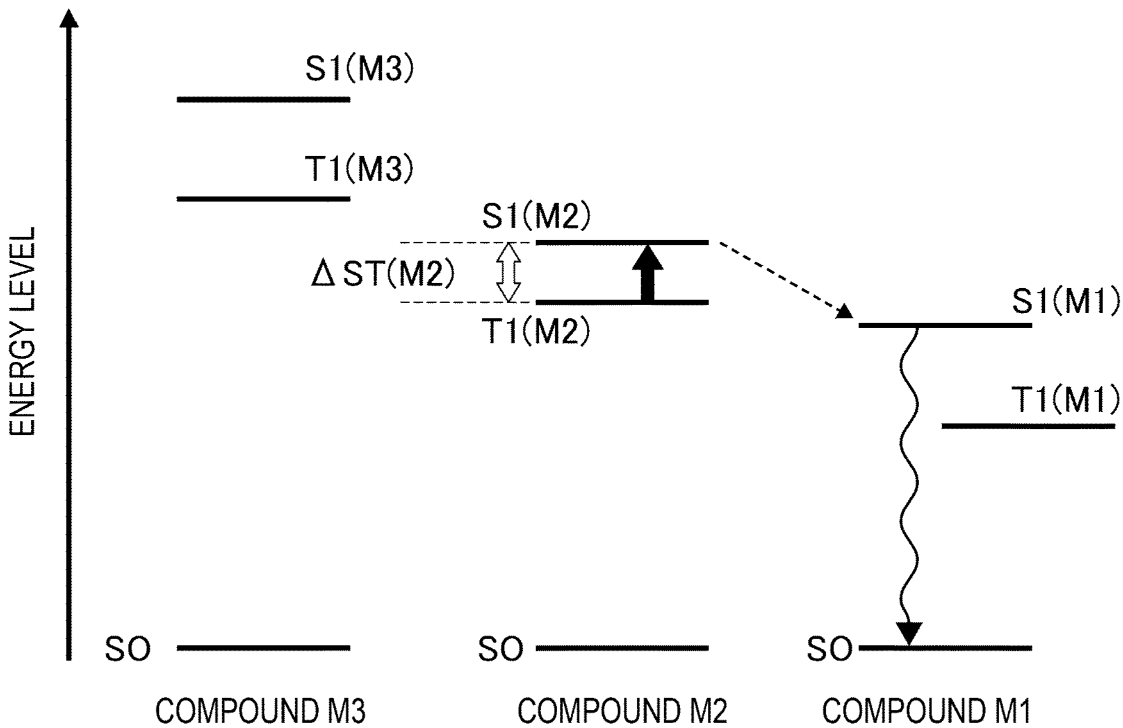
ENERGY LEVEL
S1(M3)
T1(M3)
S1(M2)
ΔST(M2)
T1(M2)
S1(M1)
T1(M1)
SO
SO
SO
COMPOUND M3
COMPOUND M2
COMPOUND M1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 107793398
- WO 2014166585 A
- KR 2014143618
- EP 3015457 A1
- WO 2017118155 A1
- CN 107325035 A
- WO 2015022987 A1


### Non-patent literature cited in the description


- *ADACHI,* 01 April 2012, 261-268
- *Nature Communications,* 2014, vol. 5, 4016
- *Yuki Hando-tai no Debaisu Bussei (Device Physics of Organic Semiconductors),* 261-268
- *Nature,* 2012, vol. 492, 234-238
- **MORRIS et al.** *J. Phys. Chem.,* 1976, vol. 80, 969